(19) Europäisches Patentamt
European Patent Office
Office européen des brevets

(11) EP 4 456 882 B1

(12) **EUROPEAN PATENT SPECIFICATION**

(45) Date of publication and mention of the grant of the patent:
**13.05.2026 Bulletin 2026/20**

(21) Application number: **22844203.4**

(22) Date of filing: **27.12.2022**

(51) International Patent Classification (IPC):
***A61K 9/51*** *(2006.01)* ***A61K 9/127*** *(2025.01)*
***A61K 9/00*** *(2006.01)* ***A61K 39/12*** *(2006.01)*
***A61K 47/10*** *(2017.01)* ***A61K 47/24*** *(2006.01)*
***A61K 47/34*** *(2017.01)* ***A61K 48/00*** *(2006.01)*
***A61P 37/04*** *(2006.01)* ***A61K 39/00*** *(2006.01)*
***A61P 31/14*** *(2006.01)*

(52) Cooperative Patent Classification (CPC):
**A61K 9/5123; A61K 9/0019; A61K 9/1272; A61K 9/5146; A61K 39/12; A61K 47/10; A61K 47/24; A61K 47/34; A61P 31/14; A61P 37/04; C12N 15/88;** A61K 2039/572; A61K 2039/575; A61K 2039/6018; C12N 2770/20034

(86) International application number:
**PCT/EP2022/087877**

(87) International publication number:
**WO 2023/126404 (06.07.2023 Gazette 2023/27)**

(54) **LIPID-BASED FORMULATIONS FOR ADMINISTRATION OF RNA**

LIPIDBASIERTE FORMULIERUNGEN ZUR VERABREICHUNG VON RNA

FORMULATIONS À BASE DE LIPIDES POUR L'ADMINISTRATION D'ARN

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**

(30) Priority: **28.12.2021 PCT/EP2021/087748**

(43) Date of publication of application:
**06.11.2024 Bulletin 2024/45**

(73) Proprietor: **BioNTech SE**
**55131 Mainz (DE)**

(72) Inventors:
- **MENINA, Sara**
  **55131 Mainz (DE)**
- **MEHRAVAR, Ehsan**
  **55131 Mainz (DE)**
- **HEFESHA, Hossam**
  **55131 Mainz (DE)**
- **THANKI, Kaushik**
  **55131 Mainz (DE)**
- **HAAS, Heinrich**
  **55131 Mainz (DE)**

(74) Representative: **Müller, Christian Stefan Gerd**
**ZSP Patentanwälte PartG mbB**
**Hansastraße 32**
**80686 München (DE)**

(56) References cited:
**WO-A1-2016/210190 WO-A1-2019/191780**
**WO-A1-2021/236855 WO-A2-2021/226597**

- **NOGUEIRA SARA S. ET AL: "Polysarcosine-Functionalized Lipid Nanoparticles for Therapeutic mRNA Delivery", vol. 3, no. 11, 25 November 2020 (2020-11-25), pages 10634 - 10645, XP055809176, ISSN: 2574-0970, Retrieved from the Internet <URL:https://pubs.acs.org/doi/pdf/10.1021/acsanm.0c01834> DOI: 10.1021/acsanm.0c01834**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SCHOENMAKER LINDE ET AL: "mRNA-lipid nanoparticle COVID-19 vaccines: Structure and stability", INTERNATIONAL JOURNAL OF PHARMACEUTICS, vol. 601, 1 May 2021 (2021-05-01), NL, pages 120586, XP055828800, ISSN: 0378-5173, DOI: 10.1016/ j.ijpharm.2021.120586**
- **PILKINGTON EMILY H ET AL: "From influenza to COVID-19: Lipid nanoparticle mRNA vaccines at the frontiers of infectious diseases", ACTA BIOMATERIALIA, ELSEVIER, AMSTERDAM, NL, vol. 131, 18 June 2021 (2021-06-18), pages 16 - 40, XP086746520, ISSN: 1742-7061, [retrieved on 20210618], DOI: 10.1016/J.ACTBIO.2021.06.023**


Remarks:

The complete document including Reference Table(s) and the Sequence Listing(s) can be downloaded from the EPO website

## Description

### Technical Field

The present disclosure relates to compositions comprising RNA and a cationically ionizable lipid for delivering RNA to target tissues after administration, in particular after parenteral administration. The present disclosure also relates to methods for delivering RNA to cells of a subject or for treating or preventing a disease or disorder in a subject, wherein the methods comprise administering to a subject a composition of the present disclosure. The RNA compositions in some embodiments comprise single-stranded RNA such as mRNA which encodes a peptide or polypeptide of interest, such as a pharmaceutically active peptide or polypeptide. The RNA is taken up by cells of a target tissue and the RNA is translated into the encoded peptide or polypeptide, which may exhibit its physiological activity. Thus, the present disclosure also relates to methods for delivering a pharmaceutically active peptide or protein to a subject or for treating or preventing a disease or disorder in a subject, wherein the methods comprise administering to a subject an RNA composition of the present disclosure, wherein the RNA encodes the pharmaceutically active peptide or protein.

### Background

The use of RNA to deliver foreign genetic information into target cells offers an attractive alternative to DNA. The advantages of RNA include transient expression and non-transforming character. RNA does not require nucleus infiltration for expression and moreover cannot integrate into the host genome, thereby eliminating the risk of oncogenesis.
RNA can be delivered to a target through different vehicles, based mostly upon cationic polymers or lipids, which combine with the RNA to form nanoparticles. The nanoparticles are intended to protect the RNA from degradation, enable delivery of the RNA to the target site and facilitate cellular uptake and processing by the target cells. The efficiency of RNA delivery depends, in part, on the molecular composition of the nanoparticle and can be influenced by numerous parameters, including particle size, formulation, and charge or grafting with molecular moieties, such as polyethylene glycol (PEG) or other ligands. Nogueira et al. (ACS Appl. Nano Mater. 2020, 3, 10634-10645) discloses polysarcosine-functionalized lipid nanoparticles for therapeutic mRNA delivery.
There is a need of providing formulations for the delivery of pharmaceutically active RNA to a target tissue where the delivered RNA is efficiently translated into the peptide or polypeptide it codes for. The inventors surprisingly found that the RNA formulations described herein fulfill the above-mentioned requirements.

### Summary

The present invention generally provides a composition comprising RNA, a cationically ionizable lipid comprising the structure of formula (1) shown below, and at least one polysarcosine-conjugated lipid. The inventors found that RNA formulations containing this cationically ionizable lipid of formula (I) can be manufactured in a robust and reproducible manner. They are suitable as RNA delivery systems and display comparable characteristics with respect to known systems.
The RNA formulations described herein are useful as RNA delivery vehicles for *in vivo* application, such as for pharmaceutical application.
In a first aspect, the invention provides a composition comprising: (i) RNA; and (ii) a cationically ionizable lipid comprising the structure of the following formula (I):

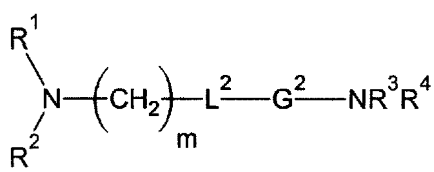

wherein

- each of $R^1$ and $R^2$ is independently $R^5$ or $-G^1-L^1-R^6$, wherein at least one of $R^1$ and $R^2$ is $-G^1-L^1-R^6$;
- each of $R^3$ and $R^4$ is independently selected from the group consisting of $C_{1-6}$ alkyl, $C_{2-6}$ alkenyl, aryl, and $C_{3-10}$ cycloalkyl;
- each of $R^5$ and $R^6$ is independently a non-cyclic hydrocarbyl group having at least 10 carbon atoms;
- each of $G^1$ and $G^2$ is independently unsubstituted $C_{1-12}$ alkylene or $C_{2-12}$ alkenylene;
- each of $L^1$ and $L^2$ is independently selected from the group consisting of -O(C=O)-, -(C=O)O-, -C(=O)-, -O-, $-S(O)_x-$, -S-S-, -C(=O)S-, -SC(=O)-, $-NR^aC(=O)-$, $-C(=O)NR^a-$, $-NR^aC(=O)NR^a-$, $-OC(=O)NR^a-$ and $-NR^aC(=O)O-$;

$R^a$ is H or $C_{1-12}$ alkyl;
m is 0, 1, 2, 3, or 4; and
x is 0, 1 or 2.

In some embodiments, each $L^1$ is independently selected from the group consisting of -O(C=O)-, -(C=O)O-, -C(=O)S-, -SC(=O)-, -$NR^a$C(=O)-, and -C(=O)$NR^a$-. In some embodiments, each $L^1$ is independently -O(C=O)- or -(C=O)O-.

In some embodiments, $L^2$ is selected from the group consisting of -O(C=O)-, -(C=O)O-, -C(=O)-, -C(=O)S-, -SC(=O)-, -$NR^a$C(=O)-, and -C(=O)$NR^a$-. In some embodiments, $L^2$ is -O(C=O)- or -(C=O)O-.

In some embodiments, $R^5$ is a straight alkyl or alkenyl group having at least 10 carbon atoms, preferably at least 14 carbon atoms, more preferably at least 16 carbon atoms. In some embodiments, $R^5$ is a straight alkyl group or a straight alkenyl group having at least 2 carbon-carbon double bonds. In some embodiments, $R^5$ has the following structure:

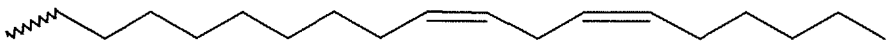,

wherein  represents the bond by which $R^5$ is bound to the remainder of the compound. In some embodiments, each $G^1$ is independently unsubstituted straight $C_{1-12}$ alkylene or $C_{2-12}$ alkenylene, preferably unsubstituted, straight $C_{6-12}$ alkylene or $C_{6-12}$ alkenylene. In some embodiments, each $G^1$ is independently unsubstituted, straight $C_{8-12}$ alkylene or $C_{8-12}$ alkenylene, such as unsubstituted straight $C_{6-10}$ alkylene or $C_{6-10}$ alkenylene. In some embodiments, each $G^1$ is unsubstituted straight $C_8$ alkylene.

In some embodiments, each $R^6$ is independently a straight hydrocarbyl group having at least 10 carbon atoms. In some embodiments, each $R^6$ is attached to $L^1$ via an internal carbon atom of $R^6$. In some embodiments, each $R^6$ is independently selected from the group consisting of:

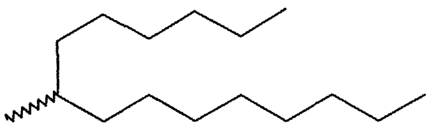, 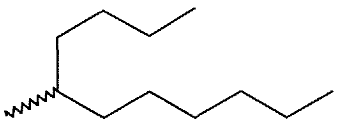, 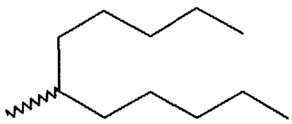,

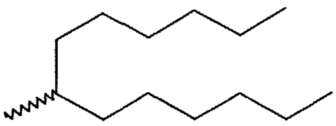, 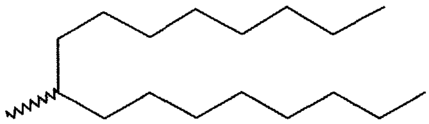,

and

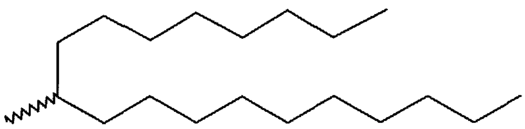,

wherein  represents the bond by which $R^6$ is bound to $L^1$.

In some embodiments, one of $R^1$ and $R^2$ is $R^5$ and the other is -$G^1$-$L^1$-$R^6$.

In some embodiments, each of $R^1$ and $R^2$ is independently -$G^1$-$L^1$-$R^6$.

In some embodiments, each of $R^3$ and $R^4$ is independently $C_{1-6}$ alkyl or $C_{2-6}$ alkenyl, preferably $C_{1-4}$ alkyl or $C_{2-4}$ alkenyl. In some embodiments, each of $R^3$ and $R^4$ is $C_{1-3}$ alkyl, such as methyl or ethyl. In some embodiments, each of $R^3$ and $R^4$ is methyl.

In some embodiments, $G^2$ is unsubstituted $C_{2-10}$ alkylene or $C_{2-10}$ alkenylene, preferably unsubstituted $C_{2-6}$ alkylene or $C_{2-6}$ alkenylene. In some embodiments, $G^2$ is unsubstituted $C_{2-4}$ alkylene or $C_{2-4}$ alkenylene, such as ethylene or trimethylene.

In some embodiments, m is 0, 1, 2 or 3. In some embodiments m is 0. In some embodiments, m is 2.

In some embodiments, the cationically ionizable lipid comprises the structure of one of the following formulas (IIIa) or (IIIb):

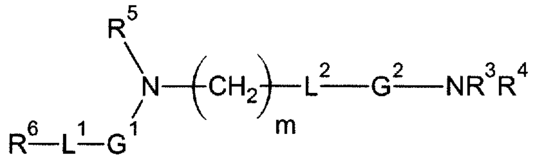

(IIIa)

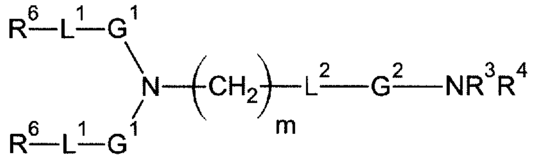

(IIIb),

wherein

each of $R^3$ and $R^4$ is independently $C_{1-4}$ alkyl or $C_{2-4}$ alkenyl;
$R^5$ is a straight alkyl or alkenyl group having at least 16 carbon atoms;
each $R^6$ is independently a straight hydrocarbyl group having at least 10 carbon atoms, wherein $R^6$ is attached to $L^1$ via an internal carbon atom of $R^6$;
each $G^1$ is independently unsubstituted, straight $C_{6-12}$ alkylene or $C_{6-12}$ alkenylene;
$G^2$ is unsubstituted $C_{2-6}$ alkylene or $C_{2-6}$ alkenylene;
each of $L^1$ and $L^2$ is independently -O(C=O)- or -(C=O)O-; and
m is 0, 1, 2 or 3.

In some embodiments, each of $R^3$ and $R^4$ is $C_{1-3}$ alkyl, such as methyl or ethyl.
In some embodiments, $R^5$ is a straight alkyl or alkenyl group having at least 16 carbon atoms, wherein the alkenyl group has at least 2 carbon-carbon double bonds.
In some embodiments, each $G^1$ is independently unsubstituted, straight $C_{8-10}$ alkylene or unsubstituted, straight $C_{8-10}$ alkenylene, such as unsubstituted, straight $C_8$ alkylene.
In some embodiments, $G^2$ is unsubstituted $C_{2-4}$ alkylene or $C_{2-4}$ alkenylene, such as ethylene or trimethylene.
In some embodiments, m is 0. In some embodiments, m is 2.
In some embodiments, the cationically ionizable lipid comprises one of the following structures (IV-1), (IV-2), and (IV-3):

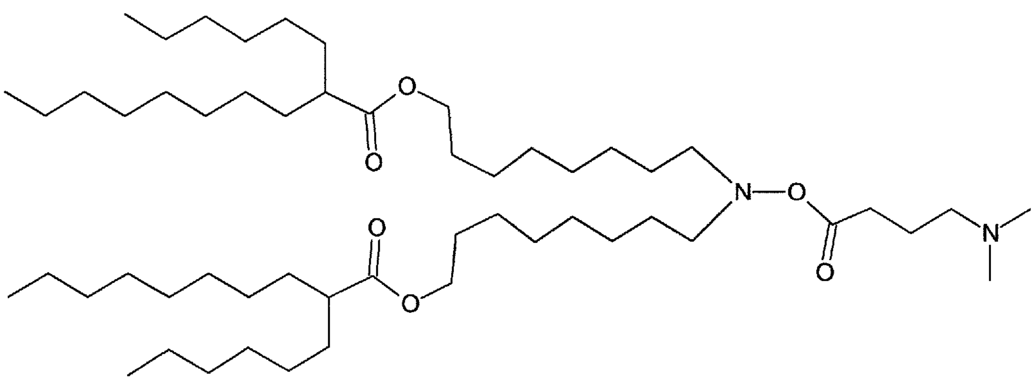

(IV-1);

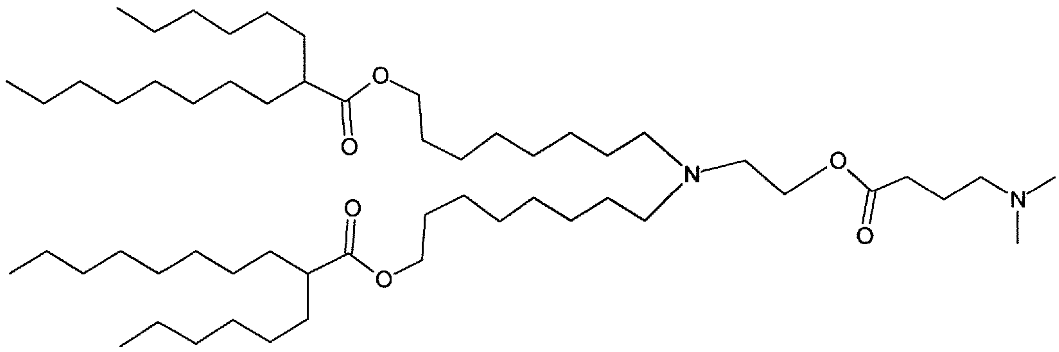

(IV-2);

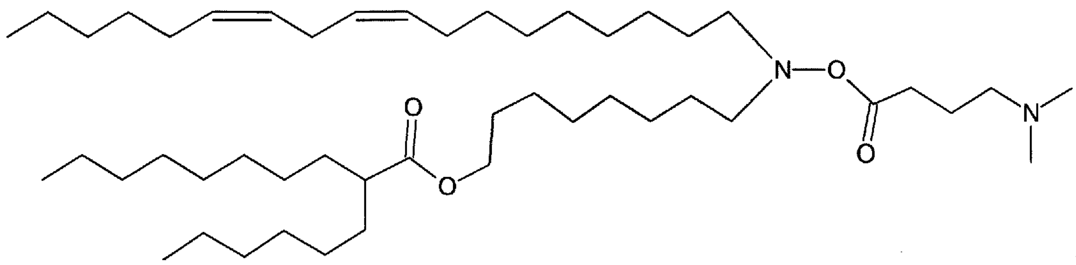

(IV-3).

In some embodiments, the cationically ionizable lipid comprises from about 20 mol % to about 80 mol %, preferably from about 25 mol % to about 65 mol %, more preferably from about 30 mol % to about 50 mol % of the total lipid present in the composition. In some embodiments, the cationically ionizable lipid comprises from about 40 mol % to about 50 mol % of the total lipid present in the composition. In some embodiments, the cationically ionizable lipid comprises from about 55 mol % to about 65 mol % of the total lipid present in the composition.

The composition further comprises a polymer-conjugated lipid.

The polymer-conjugated lipid is a polysarcosine-conjugated lipid. Thus, the composition further comprises at least one polysarcosine-conjugated lipid (such as two or more polysarcosine-conjugated lipids).

In some embodiments, the polysarcosine comprises between 2 and 200 sarcosine units, preferably between 5 and 100 sarcosine units, more preferably between 10 and 50 sarcosine units, more preferably between 15 and 40 sarcosine units, more preferably about 23 sarcosine units.

In some embodiments, the polysarcosine-conjugated lipid comprises the structure of the following general formula (V):

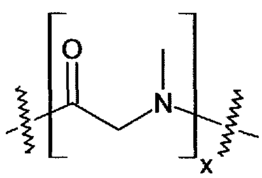

wherein x is the number of sarcosine units.

In some embodiments, the polysarcosine-conjugated lipid has the structure of the following general formula (VI):

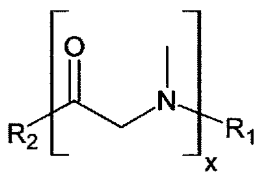

wherein one of $R^1$ and $R^2$ comprises a hydrophobic group and the other is H, a hydrophilic group or a functional group optionally comprising a targeting moiety; and x is the number of sarcosine units. In some embodiments, $R^1$ is H, a hydrophilic group or a functional group optionally comprising a targeting moiety; and $R^2$ comprises one or two straight alkyl or alkenyl groups each having at least 12 carbon atoms, preferably at least 14 carbon atoms.

In some embodiments, the polysarcosine-conjugated lipid has the structure of the following general formula (VII):

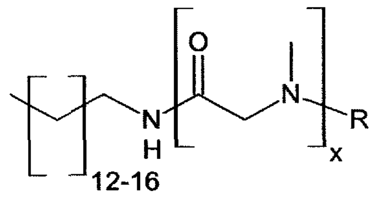

wherein R is H, a hydrophilic group or a functional group optionally comprising a targeting moiety; and x is the number of sarcosine units.

In some embodiments, the polysarcosine-conjugated lipid has the structure of the following formula (VII-1):

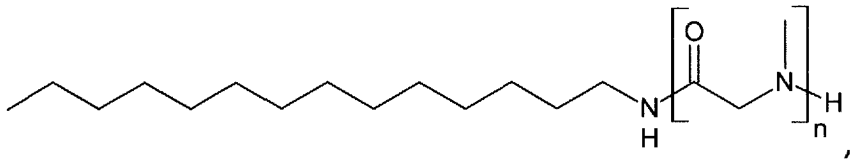

wherein n is 23.

In some embodiments, the polysarcosine-conjugated lipid comprises from about 0.1 mol % to about 5 mol % of the total lipid present in the composition. In some embodiments, the polysarcosine-conjugated lipid comprises from about 0.5 mol % to about 4.5 mol % of the total lipid present in the composition. In some embodiments, the polysarcosine-conjugated lipid comprises from about 1 mol % to about 4 mol % of the total lipid present in the composition. In some embodiments, the polysarcosine-conjugated lipid comprises from about 3 mol % to about 4 mol % of the total lipid present in the composition.

In some embodiments, the polymer-conjugated lipid is a pegylated lipid.

In some embodiments, the pegylated lipid has the structure of the following general formula (VIII):

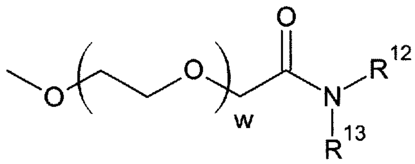

or a pharmaceutically acceptable salt, tautomer or stereoisomer thereof, wherein:
each of $R^{12}$ and $R^{13}$ is each independently a straight or branched alkyl, alkenyl, or alkynyl chain, wherein each of the alkyl, alkenyl, and alkynyl chains independently contains from 10 to 30 carbon atoms and is optionally interrupted by one or more ester bonds; and w has a mean value ranging from 30 to 60. In some embodiments, each of $R^{12}$ and $R^{13}$ is independently a straight alkyl chain containing from 10 to 18 carbon atoms, preferably from 12 to 16 carbon atoms. In some embodiments, $R^{12}$ and $R^{13}$ are identical. In some embodiments, each of $R^{12}$ and $R^{13}$ is a straight alkyl chain containing 12 carbon atoms. In some embodiments, each of $R^{12}$ and $R^{13}$ is a straight alkyl chain containing 14 carbon atoms. In some embodiments, each of $R^{12}$ and $R^{13}$ is a straight alkyl chain containing 16 carbon atoms. In some embodiments, $R^{12}$ and $R^{13}$ are different. In some embodiments, one of $R^{12}$ and $R^{13}$ is a straight alkyl chain containing 12 carbon atoms and the other of $R^{12}$ and $R^{13}$ is a straight alkyl chain containing 14 carbon atoms. In some embodiments, z has a mean value ranging from 40 to 50, such as a mean value of 45.

In some embodiments, the pegylated lipid comprises from about 1 mol % to about 10 mol % of the total lipid present in the composition. In some embodiments, the pegylated lipid comprises from about 1 mol % to about 5 mol % of the total lipid present in the composition. In some embodiments, the pegylated lipid comprises from about 1 mol % to about 2.5 mol % of the total lipid present in the composition.

In some embodiments, the composition further comprises one or more additional lipids. In some embodiments, the one or more additional lipids are selected from the group consisting of phospholipids, steroids, and combinations thereof.

In some embodiments, the composition comprises the cationically ionizable lipid; a polymer-conjugated lipid; a phospholipid; and a steroid.

According to the invention, the composition comprises at least one polysarcosine-conjugated lipid.

In some embodiments, the phospholipid is selected from the group consisting of phosphatidylcholines, phosphatidylethanolamines, phosphatidylglycerols, phosphatidic acids, phosphatidylserines and sphingomyelins, more preferably selected from the group consisting of distearoylphosphatidylcholine (DSPC), dioleoylphosphatidylcholine

(DOPC), dimyristoylphosphatidylcholine (DMPC), dipentadecanoylphosphatidylcholine, dilauroylphosphatidylcholine, dipalmitoylphosphatidylcholine (DPPC), diarachidoylphosphatidylcholine (DAPC), dibehenoylphosphatidylcholine (DBPC), ditricosanoylphosphatidylcholine (DTPC), dilignoceroylphatidylcholine (DLPC), palmitoyloleoyl-phosphatidylcholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), dioleoylphosphatidylethanolamine (DOPE), distearoyl-phosphatidylethanolamine (DSPE), dipalmitoyl-phosphatidylethanolamine (DPPE), dimyristoyl-phosphatidylethanolamine (DMPE), dilauroyl-phosphatidylethanolamine (DLPE), and diphytanoyl-phosphatidylethanolamine (DPyPE). In some embodiments, the phospholipid is DSPC. In some embodiments, the phospholipid is DOPE.

In some embodiments, the phospholipid comprises from about 5 mol % to about 40 mol % of the total lipid present in the composition. In some embodiments, the phospholipid comprises from about 5 mol % to about 20 mol % of the total lipid present in the composition. In some embodiments, the phospholipid comprises from about 5 mol % to about 15 mol % of the total lipid present in the composition.

In some embodiments, the steroid comprises a sterol such as cholesterol.

In some embodiments, the steroid comprises from about 10 mol % to about 65 mol % of the total lipid present in the composition. In some embodiments, the steroid comprises from about 20 mol % to about 60 mol % of the total lipid present in the composition. In some embodiments, the steroid comprises from about 30 mol % to about 50 mol % of the total lipid present in the composition. In some embodiments, the steroid comprises from about 25 mol % to about 35 mol % of the total lipid present in the composition.

In some embodiments, the composition comprises the cationically ionizable lipid, a polysarcosine-conjugated lipid, a phospholipid, and a steroid, wherein the cationically ionizable lipid is one of the structures (IV-1), (IV-2), and (IV-3), preferably (IV-1) or (IV-3), more preferably (IV-3); the polysarcosine-conjugated lipid has formula (VII-1); the phospholipid is DSPC; and the steroid is cholesterol.

In some embodiments, the composition comprises the cationically ionizable lipid, a polysarcosine-conjugated lipid, a phospholipid, and a steroid, wherein the cationically ionizable lipid is one of the structures (IV-1), (IV-2), and (IV-3), preferably (IV-2); the polysarcosine-conjugated lipid has formula (VII-1); the phospholipid is DOPE; and the steroid is cholesterol.

In some embodiments, the composition comprises the cationically ionizable lipid, a pegylated lipid, a phospholipid, and a steroid, wherein the cationically ionizable lipid is one of the structures (IV-1), (IV-2), and (IV-3), preferably (IV-1) or (IV-3), more preferably (IV-3); the pegylated lipid is DMG-PEG 2000; the phospholipid is DSPC; and the steroid is cholesterol.

In some embodiments, the composition comprises the cationically ionizable lipid, a pegylated lipid, a phospholipid, and a steroid, wherein the cationically ionizable lipid is one of the structures (IV-1), (IV-2), and (IV-3), preferably (IV-2); the pegylated lipid is DMG-PEG 2000; the phospholipid is DOPE; and the steroid is cholesterol.

In some embodiments, the composition comprises the cationically ionizable lipid, a polysarcosine-conjugated lipid, a phospholipid, and a steroid, wherein the cationically ionizable lipid comprises from about 30 mol % to about 50 mol of the total lipid present in the composition; the polysarcosine-conjugated lipid comprises from about 1 mol % to about 4.5 mol %, such as from about 3 mol % to about 4 mol %, of the total lipid present in the composition; the phospholipid comprises from about 5 mol % to about 15 mol % of the total lipid present in the composition; and the steroid comprises from about 30 mol % to about 50 mol % of the total lipid present in the composition. In some embodiments, the cationically ionizable lipid comprises from about 40 mol % to about 50 mol % of the total lipid present in the composition.

In some embodiments, the composition comprises the cationically ionizable lipid, a polysarcosine-conjugated lipid, a phospholipid, and a steroid, wherein the cationically ionizable lipid comprises from about 40 mol % to about 50 mol of the total lipid present in the composition; the polysarcosine-conjugated lipid comprises from about 3 mol % to about 4 mol % of the total lipid present in the composition; the phospholipid comprises from about 5 mol % to about 15 mol % of the total lipid present in the composition; and the steroid comprises from about 35 mol % to about 45 mol % of the total lipid present in the composition. In some of these embodiments, the cationically ionizable lipid is one of the structures (IV-1), (IV-2), and (IV-3), preferably (IV-3); the polysarcosine-conjugated lipid has formula (VII-1); the phospholipid is DSPC; and the steroid is cholesterol. In some of these embodiments, the N/P value in the composition is at least about 4. In some embodiments, the N/P value ranges from 4 to 20, 4 to 12, 4 to 10, 4 to 8, or 5 to 7. In some embodiments, the N/P value is about 6. In some embodiments, the N/P value is about 8. In some embodiments, the N/P value is about 12.

In some embodiments, the composition comprises the cationically ionizable lipid, a polysarcosine-conjugated lipid, a phospholipid, and a steroid, wherein the cationically ionizable lipid is one of the structures (IV-1), (IV-2), and (IV-3), preferably (IV-3), and comprises from about 40 mol % to about 50 mol of the total lipid present in the composition; the polysarcosine-conjugated lipid has formula (VII-1) and comprises from about 3 mol % to about 4 mol % of the total lipid present in the composition; the phospholipid is DSPC and comprises from about 5 mol % to about 15 mol % of the total lipid present in the composition; and the steroid is is cholesterol and comprises from about 35 mol % to about 45 mol % of the total lipid present in the composition. In some of these embodiments, the N/P value in the composition is at least about 4. In

some embodiments, the N/P value ranges from 4 to 20, 4 to 12, 4 to 10, 4 to 8, or 5 to 7. In some embodiments, the N/P value is about 6. In some embodiments, the N/P value is about 8. In some embodiments, the N/P value is about 12.

In some embodiments, the composition comprises the cationically ionizable lipid, a polysarcosine-conjugated lipid, a phospholipid, and a steroid, wherein the cationically ionizable lipid comprises from about 55 mol % to about 65 mol % of the total lipid present in the composition; the polysarcosine-conjugated lipid comprises from about 1 mol % to about 4.5 mol %, such as from about 3 mol % to about 4 mol %, of the total lipid present in the composition; the phospholipid comprises from about 5 mol % to about 15 mol % of the total lipid present in the composition; and the steroid comprises from about 25 mol % to about 35 mol % of the total lipid present in the composition. In some of these embodiments, the cationically ionizable lipid is one of the structures (IV-1), (IV-2), and (IV-3), preferably (IV-2); the polysarcosine-conjugated lipid has formula (VII-1); the phospholipid is DOPE; and the steroid is cholesterol. In some of these embodiments, the N/P value in the composition is at least about 4. In some embodiments, the N/P value ranges from 4 to 20, 4 to 12, 4 to 10, 4 to 8, or 5 to 7. In some embodiments, the N/P value is about 6. In some embodiments, the N/P value is about 8. In some embodiments, the N/P value is about 12.

In some embodiments, the composition comprises the cationically ionizable lipid, a polysarcosine-conjugated lipid, a phospholipid, and a steroid, wherein the cationically ionizable lipid is one of the structures (IV-1), (IV-2), and (IV-3), preferably (IV-2) and comprises from about 55 mol % to about 65 mol % of the total lipid present in the composition; the polysarcosine-conjugated lipid has formula (VII-1) and comprises from about 1 mol % to about 4.5 mol %, such as from about 3 mol % to about 4 mol %, of the total lipid present in the composition; the phospholipid is DOPE and comprises from about 5 mol % to about 15 mol % of the total lipid present in the composition; and the steroid is cholesterol and comprises from about 25 mol % to about 35 mol % of the total lipid present in the composition. In some of these embodiments, the N/P value in the composition is at least about 4. In some embodiments, the N/P value ranges from 4 to 20, 4 to 12, 4 to 10, 4 to 8, or 5 to 7. In some embodiments, the N/P value is about 6. In some embodiments, the N/P value is about 8. In some embodiments, the N/P value is about 12.

In some embodiments, the composition comprises the cationically ionizable lipid, a pegylated lipid, a phospholipid, and a steroid, wherein the cationically ionizable lipid comprises from about 30 mol % to about 50 mol % of the total lipid present in the composition; the pegylated lipid comprises from about 1 mol % to about 2.5 mol % of the total lipid present in the composition; the phospholipid comprises from about 5 mol % to about 15 mol % of the total lipid present in the composition; and the steroid comprises from about 30 mol % to about 50 mol % of the total lipid present in the composition. In some embodiments, the cationically ionizable lipid comprises from about 40 mol % to about 50 mol % of the total lipid present in the composition.

In some embodiments, the composition comprises the cationically ionizable lipid, a pegylated lipid, a phospholipid, and a steroid, wherein the cationically ionizable lipid comprises from about 40 mol % to about 50 mol of the total lipid present in the composition; the pegylated lipid comprises from about 1 mol % to about 2.5 mol % of the total lipid present in the composition; the phospholipid comprises from about 5 mol % to about 15 mol % of the total lipid present in the composition; and the steroid comprises from about 35 mol % to about 45 mol % of the total lipid present in the composition. In some of these embodiments, the cationically ionizable lipid is one of the structures (IV-1), (IV-2), and (IV-3), preferably (IV-3); the pegylated lipid is DMG-PEG 2000; the phospholipid is DSPC; and the steroid is cholesterol. In some of these embodiments, the N/P value in the composition is at least about 4. In some embodiments, the N/P value ranges from 4 to 20, 4 to 12, 4 to 10, 4 to 8, or 5 to 7. In some embodiments, the N/P value is about 6. In some embodiments, the N/P value is about 8. In some embodiments, the N/P value is about 12.

In some embodiments, the composition comprises the cationically ionizable lipid, a pegylated lipid, a phospholipid, and a steroid, wherein the cationically ionizable lipid is one of the structures (IV-1), (IV-2), and (IV-3), preferably (IV-3) and comprises from about 40 mol % to about 50 mol of the total lipid present in the composition; the pegylated lipid is DMG-PEG 2000 and comprises from about 1 mol % to about 2.5 mol % of the total lipid present in the composition; the phospholipid is DSPC and comprises from about 5 mol % to about 15 mol % of the total lipid present in the composition; and the steroid is cholesterol and comprises from about 35 mol % to about 45 mol % of the total lipid present in the composition. In some of these embodiments, the N/P value in the composition is at least about 4. In some embodiments, the N/P value ranges from 4 to 20, 4 to 12, 4 to 10, 4 to 8, or 5 to 7. In some embodiments, the N/P value is about 6. In some embodiments, the N/P value is about 8. In some embodiments, the N/P value is about 12.

In some embodiments, the composition comprises the cationically ionizable lipid, a pegylated lipid, a phospholipid, and a steroid, wherein the cationically ionizable lipid comprises from about 55 mol % to about 65 mol % of the total lipid present in the composition; the pegylated lipid comprises from about 1 mol % to about 2.5 mol % of the total lipid present in the composition; the phospholipid comprises from about 5 mol % to about 15 mol % of the total lipid present in the composition; and the steroid comprises from about 25 mol % to about 35 mol % of the total lipid present in the composition. In some of these embodiments, the cationically ionizable lipid is one of the structures (IV-1), (IV-2), and (IV-3), preferably (IV-2); the pegylated lipid is DMG-PEG 2000; the phospholipid is DOPE; and the steroid is cholesterol. In some of these embodiments, the N/P value in the composition is at least about 4. In some embodiments, the N/P value ranges from 4 to 20, 4 to 12, 4 to 10, 4 to 8, or 5 to 7. In some embodiments, the N/P value is about 6. In some embodiments, the N/P value is

about 8. In some embodiments, the N/P value is about 12.

In some embodiments, the composition comprises the cationically ionizable lipid, a pegylated lipid, a phospholipid, and a steroid, wherein the cationically ionizable lipid is one of the structures (IV-1), (IV-2), and (IV-3), preferably (IV-2) and comprises from about 55 mol % to about 65 mol of the total lipid present in the composition; the pegylated lipid is DMG-PEG 2000 and comprises from about 1 mol % to about 2.5 mol % of the total lipid present in the composition; the phospholipid is DOPE and comprises from about 5 mol % to about 15 mol % of the total lipid present in the composition; and the steroid is cholesterol and comprises from about 25 mol % to about 35 mol % of the total lipid present in the composition. In some of these embodiments, the N/P value in the composition is at least about 4. In some embodiments, the N/P value ranges from 4 to 20, 4 to 12, 4 to 10, 4 to 8, or 5 to 7. In some embodiments, the N/P value is about 6. In some embodiments, the N/P value is about 8. In some embodiments, the N/P value is about 12.

In some embodiments, at least a portion of (i) the RNA, (ii) the cationically ionizable lipid, and, if present, (iii) the one or more additional lipids is present in nanoparticles, such as lipid nanoparticles (LNPs).

In some embodiments, the nanoparticles (such as LNPs) have a size of from about 30 nm to about 500 nm. In some embodiments, the nanoparticles have a size of from about 40 nm to about 200 nm, such as from about 50 nm to about 180 nm, from about 60 nm to about 160 nm, from about 80 nm to about 150 nm or from about 80 nm to about 120 nm.

In some embodiments, the RNA is mRNA.

In some embodiments, the RNA comprises a modified nucleoside in place of uridine. In some embodiments, the modified nucleoside is selected from pseudouridine ($\psi$), N1-methyl-pseudouridine (m1$\psi$), and 5-methyl-uridine (m5U).

In some embodiments, the RNA has a coding sequence which is codon-optimized.

In some embodiments, has a coding sequence whose G/C content is increased compared to the wild-type coding sequence.

In some embodiments, the RNA (i) comprises a modified nucleoside in place of uridine; (ii) has a coding sequence which is codon-optimized; and (iii) has a coding sequence whose G/C content is increased compared to the wild-type coding sequence. In some embodiments, the modified nucleoside is selected from pseudouridine ($\psi$), N1-methyl-pseudouridine (m1$\psi$), and 5-methyl-uridine (m5U).

In some embodiments, the RNA comprises at least one of the following: a 5' cap; a 5' UTR; a 3' UTR; and a poly-A sequence.

In some embodiments, the RNA comprises all of the following: a 5' cap; a 5' UTR; a 3' UTR; and a poly-A sequence.

In some embodiments, the poly-A sequence comprises at least 100 A nucleotides. In some embodiments, the poly-A sequence is an interrupted sequence of A nucleotides.

In some embodiments, the 5' cap is a cap1 structure. In some embodiments, the 5' cap is cap2 structure.

In some embodiments, the RNA encodes one or more peptides or proteins. In some embodiments, the one or more peptides or proteins are pharmaceutically active peptides or proteins. In some embodiments, the one or more peptides or proteins comprise an epitope for inducing an immune response against an antigen in a subject. In some embodiments, the one or more peptides or proteins are pharmaceutically active peptides or proteins and comprise an epitope for inducing an immune response against an antigen in a subject.

In some embodiments, the pharmaceutically active peptide or protein and/or the antigen or epitope is derived from or is a SARS-CoV-2 spike (S) protein, an immunogenic variant thereof, or an immunogenic fragment of the SARS-CoV-2 S protein or the immunogenic variant thereof. In some embodiments, the RNA comprises an open reading frame (ORF) encoding an amino acid sequence comprising a SARS-CoV-2 S protein, an immunogenic variant thereof, or an immunogenic fragment of the SARS-CoV-2 S protein or the immunogenic variant thereof.

In some embodiments, the SARS-CoV-2 S protein variant has proline residue substitutions at positions 986 and 987 of SEQ ID NO: 11.

In some embodiments, the SARS-CoV-2 S protein variant has at least 80% identity to the amino acid sequence of amino acids 17 to 1273 of SEQ ID NO: 11 or the amino acid sequence of amino acids 17 to 1273 of SEQ ID NO: 12.

In some embodiments, the fragment comprises the receptor binding domain (RBD) of the SARS-CoV-2 S protein.

In some embodiments, the fragment of (i) the SARS-CoV-2 S protein or (ii) the immunogenic variant of the SARS-CoV-2 S protein has at least 80% identity to the amino acid sequence of amino acids 327 to 528 of SEQ ID NO: 11.

In a second aspect, the invention provides a method for delivering RNA to cells of a subject, the method comprising administering to a subject a composition of the first aspect. In some embodiments of the second aspect, the composition is administered parenterally, such as intramuscularly.

In a third aspect, the invention provides a method for delivering a pharmaceutically active peptide or protein to a subject, the method comprising administering to a subject a composition of the first aspect, wherein the RNA encodes the pharmaceutically active peptide or protein. In some embodiments of the third aspect, the composition is administered parenterally, such as intramuscularly.

In a fourth aspect, the invention provides a method for treating or preventing a disease or disorder in a subject, the

method comprising administering to a subject a composition of the first aspect, wherein delivering the RNA to cells of the subject is beneficial in treating or preventing the disease or disorder. In some embodiments of the fourth aspect, the composition is administered parenterally, such as intramuscularly.

In a fifth aspect, the invention provides a method for treating or preventing a disease or disorder in a subject, the method comprising administering to a subject a composition of the first aspect, wherein the RNA encodes a pharmaceutically active peptide or protein and wherein delivering the pharmaceutically active peptide or protein to the subject is beneficial in treating or preventing the disease or disorder. In some embodiments of the fifth aspect, the composition is administered parenterally, such as intramuscularly.

Further embodiments of the above aspects are described herein.

In some embodiments, the RNA described herein (e.g., contained in the compositions/formulations of the present disclosure and/or used in the methods of the present disclosure) is single-stranded RNA (in particular, mRNA) that may be translated into the respective protein upon entering cells, e.g., cells of a recipient. In addition to wild-type or codon-optimized sequences encoding an amino acid sequence comprising the amino acid sequence of a peptide or polypeptide having biological activity, e.g., a pharmaceutically active peptide or polypeptide such as antigen sequence (peptide or polypeptide comprising an epitope), the RNA may contain one or more structural elements optimized for maximal efficacy of the RNA with respect to stability and translational efficiency (5' cap, 5' UTR, 3' UTR, poly(A)-tail). In some embodiments, the RNA contains all of these elements. In some embodiments, beta-S-ARCA(D1) ($m_2^{7,2'-O}$GppSpG) or $m_2^{7,3'-O}$Gppp($m_1^{2'O}$)ApG may be utilized as specific capping structure at the 5'-end of the RNA drug substances. As 5'-UTR sequence, the 5'-UTR sequence of the human alpha-globin mRNA, optionally with an optimized 'Kozak sequence' to increase translational efficiency may be used. As 3'-UTR sequence, a combination of two sequence elements (FI element) derived from the "amino terminal enhancer of split" (AES) mRNA (called F) and the mitochondrial encoded 12S ribosomal RNA (called I) placed between the coding sequence and the poly(A)-tail to assure higher maximum protein levels and prolonged persistence of the mRNA may be used. These were identified by an *ex vivo* selection process for sequences that confer RNA stability and augment total protein expression (see WO 2017/060314).

Alternatively, the 3'-UTR may be two re-iterated 3'-UTRs of the human beta-globin mRNA. Furthermore, a poly(A)-tail measuring 110 nucleotides in length, consisting of a stretch of 30 adenosine residues, followed by a 10 nucleotide linker sequence (of random nucleotides) and another 70 adenosine residues may be used. This poly(A)-tail sequence was designed to enhance RNA stability and translational efficiency.

The amino acid sequence comprising the amino acid sequence of a peptide or polypeptide having biological activity, e.g., a pharmaceutically active peptide or polypeptide such as antigen sequence, may comprise amino acid sequences other than the amino acid sequence of a peptide or polypeptide having biological activity. Such other amino acid sequences may support the function or activity of the peptide or polypeptide having biological activity. In some embodiments, such other amino acid sequences comprise an amino acid sequence enhancing antigen processing and/or presentation. Alternatively, or additionally, such other amino acid sequences comprise an amino acid sequence which breaks immunological tolerance.

## Brief description of the Figures

**Figure 1.** Characteristics of RNA LNP formulations containing different cationically ionizable lipids of formula (I) disclosed herein (i.e., lipid EA-405, HY-405, or HY-501) and the polysarcosine-conjugated lipid C14pSar23 (abbreviated as "pSar" in the figure): (A) particle size and PDI; (B) zeta-potential; (C) accessible RNA; and (D) free RNA (lane 1: EA-405_pSar, lane 2: HY-405_pSar, lane 3: HY-501_pSar, lane 4: EA-405-NP6_47.5%, lane 5: HY-405-NP6_47.5%, lane 6: HY-501-NP6_47.5%).

**Figure 2.** (A) *In vitro* expression and (B) viability of RNA LNP formulations containing different cationically ionizable lipids (EA-405, HY-405, and HY-501) in skeletal muscle cell line (C2C12), a hepatocarcinoma cell line (HepG2) and a murine macrophage cell line (Raw cell). Firefly luciferase expression 24h post-incubation with 12.5, 25 and 50 ng per well of mRNA-loaded LNPs.

**Figure 3.** (A) *In vivo* whole body bioluminescence imaging (BLI) of animals (n=3) at 6h (upper row), 24h (middle row) and *ex-vivo* BLI (bottom row). (B) *In vivo* luciferase expression in muscle region 6 and 24 h post-transfection for the LNP formulations containing different cationically ionizable lipids and C14pSar23.

**Figure 4.** (A) Terminal complement complex (SC5b-9) formation after incubation of human serum with LNPs containing different cationically ionizable lipids and control items. The horizontal dashed line shows the level of SC5b-9 formation for PBS. (B) Hemolysis analysis after incubation (in neutral pH condition) of human red blood cells with LNP formulations containing different cationically ionizable lipids and controls.

**Figure 5.** Characteristics of RNA LNP formulations containing HY-501 and C14pSar23: (A) particle size and PDI; (B) zeta-potential; (C) accessible RNA; and (D) free RNA.

**Figure 6.** (A) Terminal complement complex (SC5b-9) formation after incubation of human serum with HY-501 containing LNP formulations and control items. The horizontal dashed line shows the level of SC5b-9 formation for PBS. (B) Hemolysis analysis after incubation (in neutral pH condition) of whole human blood with HY-501 containing LNP formulations.

**Figure 7.** Quantification of S1 protein expression after transfection with C14pSar23 containing LNPs using HY-501 as cationic lipid. (A) Variation in pSar mol% vs. MFI (Mean Fluorescence Intensity) of the overall cell population. Data was fitted using a quadratic polynomial function. (B) Viability of all tested formulations. Plotted are mean values (n=3) ± StDev.

**Figure 8.** Characteristics of RNA LNP formulations containing HY-405 and C14pSar23: (A) particle size and PDI; (B) zeta-potential; (C) accessible RNA; and (D) free RNA.

**Figure 9.** (A) Terminal complement complex (SCSb-9) formation after incubation of human serum with LNP formulations and control items. The horizontal dashed line shows the level of SCSb-9 formation for PBS. The horizontal dashed line shows the level of SC5b-9 formation for PBS. (B) Hemolysis analysis after incubation (in neutral pH condition) of whole human blood with HY405 containing LNP formulations.

**Figure 10.** (A) Accessible RNA measured via Ribogreen Assay. (B) Agarose gel electrophoresis image of LNP formulations containing HY501 or HY405 (first injection).

**Figure 11.** (A) Accessible RNA measured via Ribogreen Assay. (B) Agarose gel electrophoresis image of LNP formulations containing HY501 or HY405 (second injection).

**Figure 12.** Quantification of S1 protein expression after transfection with LNP formulations containing HY405 or HY501. (A) MFI (Mean Fluorescence Intensity) of the overall cell population. (B) Viability of all tested formulations. Plotted are mean values (n=3) +/- standard deviation (SD).

**Figure 13.** Quantification of S1 protein expression after transfection with LNP formulations containing BM, HY405 or HY501. MFI: Mean Fluorescence Intensity of the overall cell population. Viability is viability of all tested formulations. Plotted are mean values (n=3) +/-SD.

**Figure 14.** Serological analysis using anti-S1-ELISA to detect SARS-CoV-2 S protein specific antibodies; after first immunization, animals were bled weekly and serological analysis was performed.

**Figure 15.** Graphs showing the $pVNT_{50}$ values for group 2 (HY-501), group 3 (HY-405) and control (PBS) at different time points (D29, D28, D35, D42, D49, D56).

**Figure 16.** Graphs showing IFNy-secretion from splenocytes after restimulation with (A) an overlapping peptide mix specific for the SARS-CoV-2 S protein, (B) SARS-CoV-2 RBD peptide pool, or (C) unrelated peptide.

**Figure 17.** Physicochemical characteristics of EA-405, HY-501, HY-405 LNP formulations including (A) size and polydispersity index (PDI), (B) zeta potential and (C) encapsulation efficiency, RNA accessibility and free RNA.

**Figure 18.** Representative (A) *in vivo* dorsal, (B) *in vivo* ventral, and (C) *ex vivo* bioluminescence images (BLI) of BALB/c mice injected with 1 µg/leg modified mRNA coding for luciferase via intramuscular route over time. Relative luminescence scale is indicated. For (C), organs are set from top to bottom as following: heart, lung, liver, spleen, kidneys.

**Figure 19.** Quantification of the bioluminescent signal measured in BALB/c mice injected with 1 µg/leg LNPs containing modified mRNA coding for luciferase 6 h post-injection in (A) muscle, (B) liver regions *in vivo,* (C) spleen and (D) organs *ex vivo.* Data represent the mean of 3 mice/group (n=3) ± SD for *in vivo* BLI and 1 mouse/group (n=1) for *ex vivo* BLI, expressed as total flux (photons/sec).

**Figure 20.** Physicochemical characteristics of LNP optimization in terms of N/P ratio (3-12). Bar graphs include size and charge (a, b, and c), encapsulation efficiency and RNA accessibility (d, e, and f) and free RNA (g, h, and i) of formulations containing EA-405, HY-501 and HY-405, respectively.

**Figure *21. In* ***vitro* luciferase expression of EA-405 (a, b, and c), HY-501 (d, e, and f) and HY-405 (g, h, and i) in C2C12, HepG2 and RAW cell lines using 12.5 ng, 25 ng and 50 ng mRNA dose.

**Figure 22.** Representative (A) *in vivo* dorsal, (B) *in vivo* ventral, and (C) *ex vivo* bioluminescence images (BLI) of BALB/c mice injected with 1 µg/leg mod. mRNA-luciferase containing LNPs with different N/P ratios, via intramuscular route over 48 h. Relative luminescence scale is indicated accordingly. For (C), organs are set from top to bottom as following: heart, lung, liver, spleen, kidneys.

**Figure 23.** Quantification of the bioluminescent signal measured in BALB/c mice injected with 1 µg/leg LNPs containing mod. mRNA coding for luciferase (with different N/P ratios; 4, 6 and 12) 6 h post-injection in (a) muscle, (b) liver regions *in vivo,* (c) spleen and (d) organs *ex vivo.* Data represent the mean of 3 mice/group (n=3) ± SD for *in vivo* BLI and 1 mouse/group (n=1) for *ex vivo* BLI, expressed as total flux (photons/sec).

**Figure 24.** Antibody titers (measured by ELISA) of EA-405, HY-501 and HY-405 formulations vs. BM and buffer control (PBS) at (a) day 14 after prime, (b) day 28 after boost and (c) day 42 as endpoint. IFN-y ELISpot analysis (d) performed with total splenocytes collected at day 42.

**Figure 25.** Physicochemical characteristics of EA-405 LNPs in terms of (a) size and polydispersity index (PDI), (b) zeta potential, (c) encapsulated RNA/accessible RNA and (d) free RNA.

**Figure 26.** Representative (A) *in vivo* dorsal and (B) *in vivo* ventral bioluminescence images (BLI) of BALB/c mice injected with 1 μg/leg mod. mRNA coding for luciferase via intramuscular route at 6 h (A and B) and 24 h (only A). Relative luminescence scale is indicated.

**Figure 27.** Quantification of bioluminescent signal measured in BALB/c mice injected with 1 μg/leg LNPs containing luciferase encoding mod. mRNA in (a) muscle (6 h and 24 h post-injection); (b) muscle (over 9 days "kinetics"); (c) liver (*in vivo* expressed as total flux (photons/sec)); (d) IFN-y ELISpot analysis. Data represent the mean of 3 mice/group and 2 sites (n=6) ± SD for *in vivo* BLI and 3 mouse/group (n=3) for liver and ELISpot.

**Description of the Sequences**

The following table provides a listing of certain sequences referenced herein.

TABLE 1: DESCRIPTION OF THE SEQUENCES

| SEQ ID NO: | Description | SEQUENCE |
|---|---|---|
| **Sec/MITD** | | |
| 1 | Sec (aa) | MRVMAPRTLILLLSGALALTETWAGS |
| 2 | MITD (aa) | IVGIVAGLAVLAVVVIGAVVATVMCRRKSSGGKGGSYSQAASSDSAQGSDVSLTA |
| **P2P16** | | |
| 3 | P2P16 (aa) | KKQYIKANSKFIGITELKKLGGGKRGGGKKMTNSVDDALINSTKIYSYFPSVISKVNQGAQGKKL |
| **GS Linker** | | |
| 4 | GS Linker 1 | GGSGGGGSGG |
| 5 | GS Linker 2 | GSSGGGGSPGGGSS |
| **5'-UTR (hAg-Kozak)** | | |
| 6 | 5'-UTR | AACUAGUAUUCUUCUGGUCCCCACAGACUCAGAGAGAACCCGCCACC |
| **3'-UTR (FI element)** | | |
| 7 | 3'-UTR | CUGGUACUGCAUGCACGCAAUGCUAGCUGCCCCUUUCCCGUCCUGGGUACCCCGAGUCUCCCCCGACCUCGGGU<br>CCCAGGUAUGCUCCCACCUCCACCUGCCCCACUCACCACCUCUGCUAGUUCCAGACACCUCCCAAGCACGCAGCAA<br>UGCAGCUCAAAACGCUUAGCCUAGCCACACCCCCACGGGAAACAGCAGUGAUUAACCUUUAGCAAUAAACGAA<br>AGUUUAACUAAGCUAUACUAACCCCAGGGUUGGUCAAUUUCGUGCCAGCCACACC |
| **A30L70** | | |
| 8 | A30L70 | AAAAAAAAAAAAAAAAAAAAAAAAAAAAAAGCAUAUGACUAAAAAAAAAAAAAAAAAAAAAAAAAAAAAAAA<br>AAAAAAAAAAAAAAAAAAAAAAAAAAAAAAAAAAAAAA |

| | | |
|---|---|---|
| **Helper epitopes** | | |
| 9 | P2 | QYIKANSKFIGITEL |
| 10 | P16 | MTNSVDDALINSTKIYSYFPSVISKVNQGAQG |
| **SARS-CoV-2 S protein sequences** | | |
| 11 | S protein | MFVFLVLLPLVSSQCVNLTTRTQLPPAYTNSFTRGVYYPDKVFRSSVLHSTQDLFLPFFSNVTWFHAIHVSGTNGTKRFDN<br>PVLPFNDGVYFASTEKSNIIRGWIFGTTLDSKTQSLLIVNNATNVVIKVCEFQFCNDPFLGVYYHKNNKSWMESEFRVYSS<br>ANNCTFEYVSQPFLMDLEGKQGNFKNLREFVFKNIDGYFKIYSKHTPINLVRDLPQGFSALEPLVDLPIGINITRFQTLLALH<br>RSYLTPGDSSSGWTAGAAAYYVGYLQPRTFLLKYNENGTITDAVDCALDPLSETKCTLKSFTVEKGIYQTSNFRVQPTESIV<br>RFPNITNLCPFGEVFNATRFASVYAWNRKRISNCVADYSVLYNSASFSTFKCYGVSPTKLNDLCFTNVYADSFVIRGDEVR<br>QIAPGQTGKIADYNYKLPDDFTGCVIAWNSNNLDSKVGGNYNYLYRLFRKSNLKPFERDISTEIYQAGSTPCNGVEGFNC<br>YFPLQSYGFQPTNGVGYQPYRVVVLSFELLHAPATVCGPKKSTNLVKNKCVNFNFNGLTGTGVLTESNKKFLPFQQFGRD<br>IADTTDAVRDPQTLEILDITPCSFGGVSVITPGTNTSNQVAVLYQDVNCTEVPVAIHADQLTPTWRVYSTGSNVFQTRAG<br>CLIGAEHVNNSYECDIPIGAGICASYQTQTNSPRRARSVASQSIIAYTMSLGAENSVAYSNNSIAIPTNFTISVTTEILPVSMT<br>KTSVDCTMYICGDSTECSNLLLQYGSFCTQLNRALTGIAVEQDKNTQEVFAQVKQIYKTPPIKDFGGFNFSQILPDPSKPSK<br>RSFIEDLLFNKVTLADAGFIKQYGDCLGDIAARDLICAQKFNGLTVLPPLLTDEMIAQYTSALLAGTITSGWTFGAGAALQI<br>PFAMQMAYRFNGIGVTQNVLYENQKLIANQFNSAIGKIQDSLSSTASALGKLQDVVNQNAQALNTLVKQLSSNFGAISS<br>VLNDILSRLDKVEAEVQIDRLITGRLQSLQTYVTQQLIRAAEIRASANLAATKMSECVLGQSKRVDFCGKGYHLMSFPQSA<br>PHGVVFLHVTYVPAQEKNFTTAPAICHDGKAHFPREGVFVSNGTHWFVTQRNFYEPQIITTDNTFVSGNCDVVIGIVNN<br>TVYDPLQPELDSFKEELDKYFKNHTSPDVDLGDISGINASVVNIQKEIDRLNEVAKNLNESLIDLQELGKYEQYIKWPWYI<br>WLGFIAGLIAIVMVTIMLCCMTSCCSCLKGCCSCGSCCKFDEDDSEPVLKGVKLHYT |

| | | |
|---|---|---|
| 12 | S protein PP | MFVFLVLLPLVSSQCVNLTTRTQLPPAYTNSFTRGVYYPDKVFRSSVLHSTQDLFLPFFSNVTWFHAIHVSGTNGTKRFDN PVLPFNDGVYFASTEKSNIIRGWIFGTTLDSKTQSLLIVNNATNVVIKVCEFQFCNDPFLGVYYHKNNKSWMESEFRVYSS ANNCTFEYVSQPFLMDLEGKQGNFKNLREFVFKNIDGYFKIYSKHTPINLVRDLPQGFSALEPLVDLPIGINITRFQTLLALH RSYLTPGDSSSGWTAGAAAYYVGYLQPRTFLLKYNENGTITDAVDCALDPLSETKCTLKSFTVEKGIYQTSNFRVQPTESIV RFPNITNLCPFGEVFNATRFASVYAWNRKRISNCVADYSVLYNSASFSTFKCYGVSPTKLNDLCFTNVYADSFVIRGDEVR QIAPGQTGKIADYNYKLPDDFTGCVIAWNSNNLDSKVGGNYNYLYRLFRKSNLKPFERDISTEIYQAGSTPCNGVEGFNC YFPLQSYGFQPTNGVGYQPYRVVVLSFELLHAPATVCGPKKSTNLVKNKCVNFNFNGLTGTGVLTESNKKFLPFQQFGRD IADTTDAVRDPQTLEILDITPCSFGGVSVITPGTNTSNQVAVLYQDVNCTEVPVAIHADQLTPTWRVYSTGSNVFQTRAG CLIGAEHVNNSYECDIPIGAGICASYQTQTNSPRRARSVASQSIIAYTMSLGAENSVAYSNNSIAIPTNFTISVTTEILPVSMT KTSVDCTMYICGDSTECSNLLLQYGSFCTQLNRALTGIAVEQDKNTQEVFAQVKQIYKTPPIKDFGGFNFSQILPDPSKPSK RSFIEDLLFNKVTLADAGFIKQYGDCLGDIAARDLICAQKFNGLTVLPPLLTDEMIAQYTSALLAGTITSGWTFGAGAALQI PFAMQMAYRFNGIGVTQNVLYENQKLIANQFNSAIGKIQDSLSSTASALGKLQDVVNQNAQALNTLVKQLSSNFGAISS VLNDILSRLDPPEAEVQIDRLITGRLQSLQTYVTQQLIRAAEIRASANLAATKMSECVLGQSKRVDFCGKGYHLMSFPQSA PHGVVFLHVTYVPAQEKNFTTAPAICHDGKAHFPREGVFVSNGTHWFVTQRNFYEPQIITTDNTFVSGNCDVVIGIVNN TVYDPLQPELDSFKEELDKYFKNHTSPDVDLGDISGINASVVNIQKEIDRLNEVAKNLNESLIDLQELGKYEQYIKWPWYI WLGFIAGLIAIVMVTIMLCCMTSCCSCLKGCCSCGSCCKFDEDDSEPVLKGVKLHYT |
| Trimerization domain | | |
| 13 | Foldon | GSGYIPEAPRDGQAYVRKDGEWVLLSTFLGRSLEVLFQGPG |
| SARS-CoV-2 Vaccine constructs | | |

| | | |
|---|---|---|
| 14 | Construct 1 | MFVFLVLLPLVSSQCVVRFPNITNLCPFGEVFNATRFASVYAWNRKRISNCVADYSVLYNSASFSTFKCYGVSPTKLNDLCF<br>TNVYADSFVIRGDEVRQIAPGQTGKIADYNYKLPDDFTGCVIAWNSNNLDSKVGGNYNYLYRLFRKSNLKPFERDISTEIY<br>QAGSTPCNGVEGFNCYFPLQSYGFQPTNGVGYQPYRVVVLSFELLHAPATVCGPKGSPGSGSGSGYIPEAPRDGQAYVR<br>KDGEWVLLSTFLGRSLEVLFQGPG |
| 15 | Construct 3c | MFVFLVLLPLVSSQCVNLTVRFPNITNLCPFGEVFNATRFASVYAWNRKRISNCVADYSVLYNSASFSTFKCYGVSPTKLN<br>DLCFTNVYADSFVIRGDEVRQIAPGQTGKIADYNYKLPDDFTGCVIAWNSNNLDSKVGGNYNYLYRLFRKSNLKPFERDIS<br>TEIYQAGSTPCNGVEGFNCYFPLQSYGFQPTNGVGYQPYRVVVLSFELLHAPATVCGPKGSPGSGSGSGYIPEAPRDGQA<br>YVRKDGEWVLLSTFLGSGSGSEQYIKWPWYIWLGFIAGLIAIVMVTIMLCCMTSCCSCLKGCCSCGSCC |
| 16 | Construct 3d | MDWIWRILFLVGAATGAHSQMQVRFPNITNLCPFGEVFNATRFASVYAWNRKRISNCVADYSVLYNSASFSTFKCYGVS<br>PTKLNDLCFTNVYADSFVIRGDEVRQIAPGQTGKIADYNYKLPDDFTGCVIAWNSNNLDSKVGGNYNYLYRLFRKSNLKP<br>FERDISTEIYQAGSTPCNGVEGFNCYFPLQSYGFQPTNGVGYQPYRVVVLSFELLHAPATVCGPKGSPGSGSGSGYIPEAP<br>RDGQAYVRKDGEWVLLSTFLGSGSGSEQYIKWPWYIWLGFIAGLIAIVMVTIMLCCMTSCCSCLKGCCSCGSCC |
| SARS-CoV-2 Vaccine RNA constructs | | |
| 17 | Construct 2 | agaauaaacu aguauucuuc ugguccccac agacucagag agaacccgcc accauguucg 60<br>uguuccuggu gcugcugccu cuggugucca gccagugugu gaaccugacc accagaacac 120<br>agcugccucc agccuacacc aacagcuuua ccagaggcgu guacuacccc gacaaggugu 180<br>ucagauccag cgugcugcac ucuacccagg accuguuccu gccuuucuuc agcaacguga 240<br>ccugguucca cgccauccac guguccggca ccaauggcac caagagauuc gacaaccccg 300<br>ugcugcccuu caacgacggg guguacuuug ccagcaccga gaaguccaac aucaucagag 360<br>gcuggaucuu cggcaccaca cuggacagca agacccagag ccugcugauc gugaacaacg 420<br>ccaccaacgu ggucaucaaa gugugcgagu uccaguucug caacgacccc uuccugggcg 480 |

| | |
|---|---|
| ucuacuacca caagaacaac aagagcugga uggaaagcga guuccgggug uacagcagcg | 540 |
| ccaacaacug caccuucgag uacguguccc agccuuuccu gauggaccug gaaggcaagc | 600 |
| agggcaacuu caagaaccug cgcgaguucg uguuuaagaa caucgacggc uacuucaaga | 660 |
| ucuacagcaa gcacaccccu aucaaccucg ugcgggaucu gccucagggc uucucugcuc | 720 |
| uggaaccccu gguggaucug cccaucggca ucaacaucac ccgguuucag acacugcugg | 780 |
| cccugcacag aagcuaccug acaccuggcg auagcagcag cggauggaca gcuggugccg | 840 |
| ccgcuuacua ugugggcuac cugcagccua gaaccuuccu gcugaaguac aacgagaacg | 900 |
| gcaccaucac cgacgccgug gauugugcuc uggauccucu gagcgagaca aagugcaccc | 960 |
| ugaaguccuu caccguggaa aagggcaucu accagaccag caacuuccgg gugcagccca | 1020 |
| ccgaauccau cgugcgguuc cccaauauca ccaaucugug ccccuucggc gagguguuca | 1080 |
| augccaccag auucgccucu guguacgccu ggaaccggaa gcggaucagc aauugcgugg | 1140 |
| ccgacuacuc cgugcuguac aacuccgcca gcuucagcac cuucaagugc uacggcgugu | 1200 |
| ccccuaccaa gcugaacgac cugugcuuca caaacgugua cgccgacagc uucgugaucc | 1260 |
| ggggagauga agugcggcag auugccccug gacagacagg caagaucgcc gacuacaacu | 1320 |
| acaagcugcc cgacgacuuc accggcugug ugauugccug gaacagcaac aaccuggacu | 1380 |
| ccaaagucgg cggcaacuac aauuaccugu accggcuguu ccggaagucc aaucugaagc | 1440 |
| ccuucgagcg ggacaucucc accgagaucu aucaggccgg cagcaccccu uguaacggcg | 1500 |
| uggaaggcuu caacugcuac uucccacugc aguccuacgg cuuucagccc acaaauggcg | 1560 |
| ugggcuauca gcccuacaga guggguggugc ugagcuucga acugcugcau gccccugcca | 1620 |
| cagugugcgg cccuaagaaa agcaccaauc ucgugaagaa caaaugcgug aacuucaacu | 1680 |
| ucaacggccu gaccggcacc ggcgugcuga cagagagcaa caagaaguuc cugccauucc | 1740 |
| agcaguuugg ccgggauauc gccgauacca cagacgccgu uagagauccc cagacacugg | 1800 |

| | | | |
|---|---|---|---|
| | | aaauccugga caucaccccu ugcagcuucg gcggagaguc ugugaucacc ccuggcacca | 1860 |
| | | acaccagcaa ucagguggca gugcuguacc aggacgugaa cuguaccgaa gugcccgugg | 1920 |
| | | ccauucacgc cgaucagcug acaccuacau ggcgggugua cuccaccggc agcaaugugu | 1980 |
| | | uucagaccag agccggcugu cugaucggag ccgagcacgu gaacaauagc uacgagugcg | 2040 |
| | | acaucccccau cggcgcugga aucugcgcca gcuaccagac acagacaaac agcccucgga | 2100 |
| | | gagccagaag cguggccagc cagagcauca uugccuacac aaugucucug ggcgccgaga | 2160 |
| | | acagcguggc cuacuccaac aacucuaucg cuaucccccac caacuucacc aucagcguga | 2220 |
| | | ccacagagau ccugccugug uccaugacca agaccagcgu ggacugcacc auguacaucu | 2280 |
| | | gcggcgauuc caccgagugc uccaaccugc ugcugcagua cggcagcuuc ugcacccagc | 2340 |
| | | ugaauagagc ccugacaggg aucgccgugg aacaggacaa gaacacccaa gagguguucg | 2400 |
| | | cccaagugaa gcagaucuac aagaccccuc cuaucaagga cuucggcggc uucaauuuca | 2460 |
| | | gccagauucu gcccgauccu agcaagccca gcaagcggag cuucaucgag gaccugcugu | 2520 |
| | | ucaacaaagu gacacuggcc gacgccggcu ucaucaagca guauggcgau ugucugggcg | 2580 |
| | | acauugccgc cagggaucug auuugcgccc agaaguuuaa cggacugaca gugcugccuc | 2640 |
| | | cucugcugac cgaugagaug aucgcccagu acacaucugc ccugcuggcc ggcacaauca | 2700 |
| | | caagcggcug gacauuugga gcaggcgccg cucugcagau ccccuuugcu augcagaugg | 2760 |
| | | ccuaccgguu caacggcauc ggagugaccc agaaugugcu guacgagaac cagaagcuga | 2820 |
| | | ucgccaacca guucaacagc gccaucggca agauccagga cagccugagc agcacagcaa | 2880 |
| | | gcgcccuggg aaagcugcag gacgugguca accagaaugc ccaggcacug aacacccugg | 2940 |
| | | ucaagcagcu guccuccaac uucggcgcca ucagcucugu gcugaacgau auccugagca | 3000 |
| | | gacuggaccc uccugaggcc gaggugcaga ucgacagacu gaucacaggc agacugcaga | 3060 |
| | | gccuccagac auacgugacc cagcagcuga ucagagccgc cgagauuaga gccucugcca | 3120 |

| | | | |
|---|---|---|---|
| | | aucuggccgc caccaagaug ucugagugug ugcugggcca gagcaagaga guggacuuuu | 3180 |
| | | gcggcaaggg cuaccaccug augagcuucc cucagucugc cccucacggc gugguguuuc | 3240 |
| | | ugcacgugac auaugugccc gcucaagaga agaauuucac caccgcucca gccaucugcc | 3300 |
| | | acgacggcaa agcccacuuu ccuagagaag gcguguucgu guccaacggc acccauuggu | 3360 |
| | | ucgugacaca gcggaacuuc uacgagcccc agaucaucac caccgacaac accuucgugu | 3420 |
| | | cuggcaacug cgacgucgug aucggcauug ugaacaauac cguguacgac ccucugcagc | 3480 |
| | | ccgagcugga cagcuucaaa gaggaacugg acaaguacuu uaagaaccac acaagccccg | 3540 |
| | | acguggaccu gggcgauauc agcggaauca augccagcgu cgugaacauc cagaaagaga | 3600 |
| | | ucgaccggcu gaacgaggug gccaagaauc ugaacgagag ccugaucgac cugcaagaac | 3660 |
| | | uggggaagua cgagcaguac aucaaguggc ccugguacau cuggcugggc uuuaucgccg | 3720 |
| | | gacugauugc caucgugaug gucacaauca ugcuguguug caugaccagc ugcuguagcu | 3780 |
| | | gccugaaggg cuguuguagc uguggcagcu gcugcaaguu cgacgaggac gauucugagc | 3840 |
| | | ccgugcugaa gggcgugaaa cugcacuaca caugaugacu cgagcuggua cugcaugcac | 3900 |
| | | gcaaugcuag cugccccuuu cccguccugg guaccccgag ucuccccccga ccucgggucc | 3960 |
| | | cagguaugcu cccaccucca ccugccccac ucaccaccuc ugcuaguucc agacaccucc | 4020 |
| | | caagcacgca gcaaugcagc ucaaaacgcu uagccuagcc acacccccac gggaaacagc | 4080 |
| | | agugauuaac cuuuagcaau aaacgaaagu uuaacuaagc uauacuaacc ccaggguugg | 4140 |
| | | ucaauuucgu gccagccaca cccuggagcu agcaaaaaaa aaaaaaaaaa aaaaaaaaaa | 4200 |
| | | aaagcauaug acuaaaaaaa aaaaaaaaaa aaaaaaaaaa aaaaaaaaaa aaaaaaaaaa | 4260 |
| | | aaaaaaaaaa aaaaaaaaaa aaa | 4283 |
| **18** | **Construct 1** | agaauaaacu aguauucuuc ugguccccac agacucagag agaacccgcc accauguuug | 60 |
| | | uguuucuugu gcugcugccu cuugugucuu cucagugugu ggugagauuu ccaaauauua | 120 |

| | | | |
|---|---|---|---|
| | | caaaucugug uccauuugga gaaguguuua augcaacaag auuugcaucu guguaugcau | 180 |
| | | ggaauagaaa aagaauuucu aauugugugg cugauuauuc ugugcuguau aauagugcuu | 240 |
| | | cuuuuuccac auuuaaaugu uauggagugu cuccaacaaa auuaaaugau uuauguuuua | 300 |
| | | caaaugugua ugcugauucu uuugugauca gaggugauga agugagacag auugcccccg | 360 |
| | | gacagacagg aaaaauugcu gauuacaauu acaaacugcc ugaugauuuu acaggaugug | 420 |
| | | ugauugcuug gaauucuaau aauuuagauu cuaaaguggg aggaaauuac aauuaucugu | 480 |
| | | acagacuguu uagaaaauca aaucugaaac cuuuugaaag agauauuuca acagaaauuu | 540 |
| | | aucaggcugg aucaacaccu uguaauggag uggaaggauu uaauuguuau uuuccauuac | 600 |
| | | agagcuaugg auuucagcca accaauggug ugggauauca gccauauaga guggguggugc | 660 |
| | | ugucuuuuga acugcugcau gcaccugcaa cagugugugg accuaaaggc ucccccggcu | 720 |
| | | ccggcuccgg aucugguuau auuccugaag cuccaagaga ugggcaagcu uacguucgua | 780 |
| | | aagauggcga augguauua cuuucuaccu uuuuaggccg gucccuggag gugcuguucc | 840 |
| | | agggccccgg cugaugacuc gagcugguac ugcaugcacg caaugcuagc ugccccuuuc | 900 |
| | | ccguccuggg uaccccgagu cucccccgac cucggguccc agguaugcuc ccaccuccac | 960 |
| | | cugccccacu caccaccucu gcuaguucca gacaccuccc aagcacgcag caaugcagcu | 1020 |
| | | caaaacgcuu agccuagcca caccccccacg ggaaacagca gugauuaacc uuuagcaaua | 1080 |
| | | aacgaaaguu uaacuaagcu auacuaaccc caggguuggu caauuucgug ccagccacac | 1140 |
| | | ccuggagcua gcaaaaaaaa aaaaaaaaaa aaaaaaaaaa aagcauauga cuaaaaaaaa | 1200 |
| | | aaaaaaaaaa aaaaaaaaaa aaaaaaaaaa aaaaaaaaaa aaaaaaaaaa aaaaaaaaaa | 1260 |
| | | aa | 1262 |
| 19 | Construct 3c | agaauaaacu aguauucuuc ugguccccac agacucagag agaacccgcc accauguuug | 60 |
| | | uguuucuugu gcugcugccu cuugugucuu cucagugugu gaauuugaca gugagauuuc | 120 |

| | | | |
|---|---|---|---|
| | | caaauauuac aaaucugugu ccauuuggag aaguguuuaa ugcaacaaga uuugcaucug | 180 |
| | | uguaugcaug gaauagaaaa agaauuucua auuguguggc ugauuauucu gugcuguaua | 240 |
| | | auagugcuuc uuuuuccaca uuuaaauguu auggaguguc uccaacaaaa uuaaaugauu | 300 |
| | | uauguuuuac aaauguguau gcugauucuu uugugaucag aggugaugaa gugagacaga | 360 |
| | | uugcccccgg acagacagga aaaauugcug auuacaauua caaacugccu gaugauuuua | 420 |
| | | caggaugugu gauugcuugg aauucuaaua auuuagauuc uaaagugggga ggaaauuaca | 480 |
| | | auuaucugua cagacuguuu agaaaaucaa aucugaaacc uuuugaaaga gauauuucaa | 540 |
| | | cagaaauuua ucaggcugga ucaacaccuu guaauggagu ggaaggauuu aauuguuauu | 600 |
| | | uuccauuaca gagcuaugga uuucagccaa ccaauggugu gggauaucag ccauauagag | 660 |
| | | ugguggugcu gucuuuugaa cugcugcaug caccugcaac agugugugga ccuaaaggcu | 720 |
| | | cccccggcuc cggcuccgga ucugguuaua uuccugaagc uccaagagau gggcaagcuu | 780 |
| | | acguucguaa agauggcgaa uggguauuac uuucuaccuu uuuaggaagc ggcagcggau | 840 |
| | | cugaacagua cauuaaaugg ccuugguaca uuuggcuugg auuuauugca ggauuaauug | 900 |
| | | caauugugau ggugacaauu auguuauguu guaugacauc auguuguucu uguuuaaaag | 960 |
| | | gauguuguuc uuguggaagc uguuguugau gacucgagcu gguacugcau gcacgcaaug | 1020 |
| | | cuagcugccc cuuucccguc cuggguaccc cgagucuccc ccgaccucgg gucccaggua | 1080 |
| | | ugcucccacc uccaccugcc ccacucacca ccucugcuag uuccagacac cucccaagca | 1140 |
| | | cgcagcaaug cagcucaaaa cgcuuagccu agccacaccc ccacgggaaa cagcagugau | 1200 |
| | | uaaccuuuag caauaaacga aaguuuaacu aagcuauacu aaccccaggg uuggucaauu | 1260 |
| | | ucgugccagc cacacccugg agcuagcaaa aaaaaaaaaa aaaaaaaaaa aaaaaaagca | 1320 |
| | | uaugacuaaa aaaaaaaaaa aaaaaaaaaa aaaaaaaaaa aaaaaaaaaa aaaaaaaaaa | 1380 |
| | | aaaaaaaaaa aaaaaaa | 1397 |

| | | | |
|---|---|---|---|
| 20 | Construct 3d | agaauaaacu aguauucuuc uggucccac agacucagag agaacccgcc accauggauu | 60 |
| | | ggauuuggag aauccuguuc cucgugggag ccgcuacagg agcccacucc cagaugcagg | 120 |
| | | ugagauuucc aaauauuaca aaucugugc cauuuggaga aguguuuaau gcaacaagau | 180 |
| | | uugcaucugu guaugcaugg aauagaaaaa gaauuucuaa uuguguggcu gauuauucug | 240 |
| | | ugcuguauaa uagugcuucu uuuuccacau uuaaauguua uggagugucu ccaacaaaau | 300 |
| | | uaaaugauuu auguuuuaca aauguguaug cugauucuuu ugugaucaga ggugaugaag | 360 |
| | | ugagacagau ugcccccgga cagacaggaa aaauugcuga uuacaauuac aaacugccug | 420 |
| | | augauuuuac aggaugugug auugcuugga auucuaauaa uuuagauucu aaagugggag | 480 |
| | | gaaauuacaa uuaucuguac agacuguuua gaaaaucaaa ucugaaaccu uuugaaagag | 540 |
| | | auauuucaac agaaauuuau caggcuggau caacaccuug uaauggagug gaaggauuua | 600 |
| | | auuguuauuu uccauuacag agcuauggau uucagccaac caauggugug ggauaucagc | 660 |
| | | cauauagagu gguggugcug ucuuuugaac ugcugcaugc accugcaaca gugugggac | 720 |
| | | cuaaaggcuc ccccggcucc ggcuccggau cugguuauau uccugaagcu ccaagagaug | 780 |
| | | ggcaagcuua cguucguaaa gauggcgaau ggguauuacu uucuaccuuu uuaggaagcg | 840 |
| | | gcagcggauc ugaacaguac auuaaauggc cuugguacau uuggcuugga uuuauugcag | 900 |
| | | gauuaauugc aauugugaug gugacaauua uguuauguug uaugacauca uguuguucuu | 960 |
| | | guuuaaaagg auguuguucu uguggaagcu guuguugaug acucgagcug guacugcaug | 1020 |
| | | cacgcaaugc uagcugcccc uuucccgucc uggguacccc gagucucccc cgaccucggg | 1080 |
| | | ucccagguau gcucccaccu ccaccugccc cacucaccac cucugcuagu uccagacacc | 1140 |
| | | ucccaagcac gcagcaaugc agcucaaaac gcuuagccua gccacacccc cacgggaaac | 1200 |
| | | agcagugauu aaccuuuagc aauaaacgaa aguuuaacua agcuauacua accccagggu | 1260 |
| | | uggucaauuu cgugccagcc acacccugga gcuagcaaaa aaaaaaaaaa aaaaaaaaaa | 1320 |

| | | |
|---|---|---|
| | | aaaaaagcau augacuaaaa aaaaaaaaaa aaaaaaaaaa aaaaaaaaaa aaaaaaaaaa 1380<br>aaaaaaaaaa aaaaaaaaaa aaaaaa 1406 |

## Detailed Description of the Invention

Although the present disclosure is further described in more detail below, it is to be understood that this disclosure is not limited to the particular methodologies, protocols and reagents described herein as these may vary. It is also to be understood that the terminology used herein is for the purpose of describing particular embodiments only, and is not intended to limit the scope of the present disclosure which will be limited only by the appended claims. Unless defined otherwise, all technical and scientific terms used herein have the same meanings as commonly understood by one of ordinary skill in the art.

In the following, the elements of the present disclosure will be described in more detail. These elements are listed with specific embodiments, however, it should be understood that they may be combined in any manner and in any number to create additional embodiments. The variously described examples and preferred embodiments should not be construed to limit the present disclosure to only the explicitly described embodiments. This description should be understood to support and encompass embodiments which combine the explicitly described embodiments with any number of the disclosed and/or preferred elements. Furthermore, any permutations and combinations of all described elements in this application should be considered disclosed by the description of the present application unless the context indicates otherwise.

The practice of the present disclosure will employ, unless otherwise indicated, conventional chemistry, biochemistry, cell biology, immunology, and recombinant DNA techniques which are explained in the literature in the field.

Throughout this specification and the claims which follow, unless the context requires otherwise, the word "comprise", and variations such as "comprises" and "comprising", will be understood to imply the inclusion of a stated feature, element, member, integer or step or group of features, elements, members, integers or steps but not the exclusion of any other feature, element, member, integer or step or group of features, elements, members, integers or steps. The term "consisting essentially of" limits the scope of a claim or disclosure to the specified features, elements, members, integers, or steps and those that do not materially affect the basic and novel characteristic(s) of the claim or disclosure. The term "consisting of" limits the scope of a claim or disclosure to the specified features, elements, members, integers, or steps. The term "comprising" encompasses the term "consisting essentially of" which, in turn, encompasses the term "consisting of". Thus, at each occurrence in the present application, the term "comprising" may be replaced with the term "consisting essentially of" or "consisting of". Likewise, at each occurrence in the present application, the term "consisting essentially of" may be replaced with the term "consisting of".

The terms "a", "an" and "the" and similar references used in the context of describing the present disclosure (especially in the context of the claims) are to be construed to cover both the singular and the plural, unless otherwise indicated herein or clearly contradicted by the context.

All methods described herein can be performed in any suitable order unless otherwise indicated herein or otherwise clearly contradicted by the context.

The use of any and all examples, or exemplary language (e.g., "such as"), provided herein is intended merely to better illustrate the present disclosure and does not pose a limitation on the scope of the present disclosure otherwise claimed. No language in the specification should be construed as indicating any non-claimed element essential to the practice of the present disclosure.

The term "optional" or "optionally" as used herein means that the subsequently described event, circumstance or condition may or may not occur, and that the description includes instances where said event, circumstance, or condition occurs and instances in which it does not occur.

Where used herein, "and/or" is to be taken as specific disclosure of each of the two specified features or components with or without the other. For example, "X and/or Y" is to be taken as specific disclosure of each of (i) X, (ii) Y, and (iii) X and Y, just as if each is set out individually herein.

In the context of the present disclosure, the term "about" denotes an interval of accuracy that the person of ordinary skill will understand to still ensure the technical effect of the feature in question. The term typically indicates deviation from the indicated numerical value by ±10%, ±5%, ±4%, ±3%, ±2%, ±1%, ±0.9%, ±0.8%, ±0.7%, ±0.6%, ±0.5%, ±0.4%, ±0.3%, ±0.2%, ±0.1%, ±0.05%, and for example ±0.01%. In some embodiments, "about" indicates deviation from the indicated numerical value by ±10%. In some embodiments, "about" indicates deviation from the indicated numerical value by ±5%. In some embodiments, "about" indicates deviation from the indicated numerical value by ±4%. In some embodiments, "about" indicates deviation from the indicated numerical value by ±3%. In some embodiments, "about" indicates deviation from the indicated numerical value by ±2%. In some embodiments, "about" indicates deviation from the indicated numerical value by ±1%. In some embodiments, "about" indicates deviation from the indicated numerical value by ±0.9%. In some embodiments, "about" indicates deviation from the indicated numerical value by ±0.8%. In some embodiments, "about" indicates deviation from the indicated numerical value by ±0.7%. In some embodiments, "about" indicates deviation from the indicated numerical value by ±0.6%. In some embodiments, "about" indicates deviation from the indicated numerical value by ±0.5%. In some embodiments, "about" indicates deviation from the indicated numerical value by ±0.4%. In some embodiments, "about" indicates deviation from the indicated numerical

value by ±0.3%. In some embodiments, "about" indicates deviation from the indicated numerical value by ±0.2%. In some embodiments, "about" indicates deviation from the indicated numerical value by ±0.1%. In some embodiments, "about" indicates deviation from the indicated numerical value by ±0.05%. In some embodiments, "about" indicates deviation from the indicated numerical value by ±0.01%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect.

Recitation of ranges of values herein is merely intended to serve as a shorthand method of referring individually to each separate value falling within the range. Unless otherwise indicated herein, each individual value is incorporated into the specification as if it were individually recited herein.

Several documents are cited throughout the text of this specification. Nothing herein is to be construed as an admission that the invention is not entitled to antedate such disclosure by virtue of prior invention.

**Definitions**

In the following, definitions will be provided which apply to all aspects of the present disclosure. The following terms have the following meanings unless otherwise indicated. Any undefined terms have their art recognized meanings.

Terms such as "reduce" or "inhibit" as used herein means the ability to cause an overall decrease, for example, of about 5% or greater, about 10% or greater, about 15% or greater, about 20% or greater, about 25% or greater, about 30% or greater, about 40% or greater, about 50% or greater, or about 75% or greater, in the level. The term "inhibit" or similar phrases includes a complete or essentially complete inhibition, *i.e.* a reduction to zero or essentially to zero.

Terms such as "enhance" as used herein means the ability to cause an overall increase, or enhancement, for example, by at least about 5% or greater, about 10% or greater, about 15% or greater, about 20% or greater, about 25% or greater, about 30% or greater, about 40% or greater, about 50% or greater, about 75% or greater, or about 100% or greater in the level.

"Physiological pH" as used herein refers to a pH of about 7.4. In some embodiments, physiological pH is from 7.3 to 7.5. In some embodiments, physiological pH is from 7.35 to 7.45. In some embodiments, physiological pH is 7.3, 7.35, 7.4, 7.45, or 7.5.

As used in the present disclosure, "% w/v" refers to weight by volume percent, which is a unit of concentration measuring the amount of solute in grams (g) expressed as a percent of the total volume of solution in milliliters (mL).

As used in the present disclosure, "% by weight" refers to weight percent, which is a unit of concentration measuring the amount of a substance in grams (g) expressed as a percent of the total weight of the total composition in grams (g).

As used in the present disclosure, "mol %" is defined as the ratio of the number of moles of one component to the total number of moles of all components, multiplied by 100.

As used in the present disclosure, "mol % of the total lipid" is defined as the ratio of the number of moles of one lipid component to the total number of moles of all lipids, multiplied by 100. In this context, in some embodiments, the term "total lipid" includes lipids and lipid-like material.

The term "ionic strength" refers to the mathematical relationship between the number of different kinds of ionic species in a particular solution and their respective charges. Thus, ionic strength I is represented mathematically by the formula:

$$I = \frac{1}{2} \cdot \sum_i z_i^2 \cdot c_i$$

in which c is the molar concentration of a particular ionic species and z the absolute value of its charge. The sum Σ is taken over all the different kinds of ions (i) in solution.

According to the disclosure, the term "ionic strength" in some embodiments relates to the presence of monovalent ions. Regarding the presence of divalent ions, in particular divalent cations, their concentration or effective concentration (presence of free ions) due to the presence of chelating agents is, in some embodiments, sufficiently low so as to prevent degradation of the nucleic acid. In some embodiments, the concentration or effective concentration of divalent ions is below the catalytic level for hydrolysis of the phosphodiester bonds between nucleotides such as RNA nucleotides. In some embodiments, the concentration of free divalent ions is 20 μM or less. In some embodiments, there are no or essentially no free divalent ions.

"Osmolality" refers to the concentration of a particular solute expressed as the number of osmoles of solute per kilogram of solvent.

The term "lyophilizing" or "lyophilization" refers to the freeze-drying of a substance by freezing it and then

reducing the surrounding pressure (e.g., below 15 Pa, such as below 10 Pa, below 5 Pa, or 1 Pa or less) to allow the frozen medium in the substance to sublimate directly from the solid phase to the gas phase. Thus, the terms "lyophilizing" and "freeze-drying" are used herein interchangeably.

The term "spray-drying" refers to spray-drying a substance by mixing (heated) gas with a fluid that is atomized (sprayed) within a vessel (spray dryer), where the solvent from the formed droplets evaporates, leading to a dry powder.

The term "reconstitute" relates to adding a solvent such as water to a dried product to return it to a liquid state such as its original liquid state.

The term "recombinant" in the context of the present disclosure means "made through genetic engineering". In some embodiments, a "recombinant object" in the context of the present disclosure is not occurring naturally.

The term "naturally occurring" as used herein refers to the fact that an object can be found in nature. For example, a peptide or nucleic acid that is present in an organism (including viruses) and can be isolated from a source in nature and which has not been intentionally modified by man in the laboratory is naturally occurring. The term "found in nature" means "present in nature" and includes known objects as well as objects that have not yet been discovered and/or isolated from nature, but that may be discovered and/or isolated in the future from a natural source.

As used herein, the terms "room temperature" and "ambient temperature" are used interchangeably herein and refer to temperatures from at least about 15°C, e.g., from about 15°C to about 35°C, from about 15°C to about 30°C, from about 15°C to about 25°C, or from about 17°C to about 22°C. Such temperatures will include 15°C, 16°C, 17°C, 18°C, 19°C, 20°C, 21°C and 22°C.

The term "EDTA" refers to ethylenediaminetetraacetic acid disodium salt. All concentrations are given with respect to the EDTA disodium salt.

The term "cryoprotectant" relates to a substance that is added to a formulation in order to protect the active ingredients during the freezing stages.

The term "lyoprotectant" relates to a substance that is added to a formulation in order to protect the active ingredients during the drying stages.

According to the present disclosure, the term "peptide" refers to substances which comprise about two or more, about 3 or more, about 4 or more, about 6 or more, about 8 or more, about 10 or more, about 13 or more, about 16 or more, about 20 or more, and up to about 50, about 100 or about 150, consecutive amino acids linked to one another via peptide bonds. The term "polypeptide" refers to large peptides, in particular peptides having at least about 151 amino acids. "Peptides" and "polypeptides" are both protein molecules, although the terms "protein" and "polypeptide" are used herein usually as synonyms.

The term "biological activity" means the response of a biological system to a molecule. Such biological systems may be, for example, a cell or an organism. In some embodiments, such response is therapeutically or pharmaceutically useful.

The term "portion" refers to a fraction. With respect to a particular structure such as an amino acid sequence or protein the term "portion" thereof may designate a continuous or a discontinuous fraction of said structure.

The terms "part" and "fragment" are used interchangeably herein and refer to a continuous element. For example, a part of a structure such as an amino acid sequence or protein refers to a continuous element of said structure. When used in context of a composition, the term "part" means a portion of the composition. For example, a part of a composition may be any portion from 0.1% to 99.9% (such as 0.1%, 0.5%, 1%, 5%, 10%, 50%, 90%, or 99%) of said composition.

"Fragment", with reference to an amino acid sequence (peptide or polypeptide), relates to a part of an amino acid sequence, i.e. a sequence which represents the amino acid sequence shortened at the N-terminus and/or C-terminus. A fragment shortened at the C-terminus (N-terminal fragment) is obtainable, *e.g.,* by translation of a truncated open reading frame that lacks the 3'-end of the open reading frame. A fragment shortened at the N-terminus (C-terminal fragment) is obtainable, e.g., by translation of a truncated open reading frame that lacks the 5'-end of the open reading frame, as long as the truncated open reading frame comprises a start codon that serves to initiate translation. A fragment of an amino acid sequence comprises, *e.g.,* at least 50 %, at least 60 %, at least 70 %, at least 80%, at least 90% of the amino acid residues from an amino acid sequence. A fragment of an amino acid sequence comprises, e.g., at least 6, in particular at least 8, , at least 10, at least 12, at least 15, at least 20, at least 30, at least 50, or at least 100 consecutive amino acids from an amino acid sequence. A fragment of an amino acid sequence comprises, e.g., a sequence of up to 8, in particular up to 10, up to 12, up to 15, up to 20, up to 30 or up to 55, consecutive amino acids of the amino acid sequence.

"Variant," as used herein and with reference to an amino acid sequence (peptide or polypeptide), is meant an amino acid sequence that differs from a parent amino acid sequence by virtue of at least one amino acid (e.g., a different amino acid, or a modification of the same amino acid). The parent amino acid sequence may be a naturally occurring or wild type (WT) amino acid sequence, or may be a modified version of a wild type amino acid sequence. In some embodiments, the variant amino acid sequence has at least one amino acid difference as compared to the parent amino acid sequence, e.g., from 1 to about 20 amino acid differences, such as from 1 to about 10 or from 1 to about 5 amino acid differences compared to the parent.

By "wild type" or "WT" or "native" herein is meant an amino acid sequence that is found in nature, including allelic variations. A wild type amino acid sequence, peptide or polypeptide has an amino acid sequence that has not been intentionally modified.

For the purposes of the present disclosure, "variants" of an amino acid sequence (peptide or polypeptide) may comprise amino acid insertion variants, amino acid addition variants, amino acid deletion variants and/or amino acid substitution variants. The term "variant" includes all mutants, splice variants, post-translationally modified variants, conformations, isoforms, allelic variants, species variants, and species homologs, in particular those which are naturally occurring. The term "variant" includes, in particular, fragments of an amino acid sequence. Amino acid insertion variants comprise insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible. Amino acid addition variants comprise amino- and/or carboxy-terminal fusions of one or more amino acids, such as 1, 2, 3, 5, 10, 20, 30, 50, or more amino acids. Amino acid deletion variants are characterized by the removal of one or more amino acids from the sequence, such as by removal of 1, 2, 3, 5, 10, 20, 30, 50, or more amino acids. The deletions may be in any position of the protein. Amino acid deletion variants that comprise the deletion at the N-terminal and/or C-terminal end of the protein are also called N-terminal and/or C-terminal truncation variants. Amino acid substitution variants are characterized by at least one residue in the sequence being removed and another residue being inserted in its place. Preference is given to the modifications being in positions in the amino acid sequence which are not conserved between homologous peptides or polypeptides and/or to replacing amino acids with other ones having similar properties. In some embodiments, amino acid changes in peptide and polypeptide variants are conservative amino acid changes, i.e., substitutions of similarly charged or uncharged amino acids. A conservative amino acid change involves substitution of one of a family of amino acids which are related in their side chains. Naturally occurring amino acids are generally divided into four families: acidic (aspartate, glutamate), basic (lysine, arginine, histidine), non-polar (alanine, valine, leucine, isoleucine, proline, phenylalanine, methionine, tryptophan), and uncharged polar (glycine, asparagine, glutamine, cysteine, serine, threonine, tyrosine) amino acids. Phenylalanine, tryptophan, and tyrosine are sometimes classified jointly as aromatic amino acids. In some embodiments, conservative amino acid substitutions include substitutions within the following groups:

- glycine, alanine;
- valine, isoleucine, leucine;
- aspartic acid, glutamic acid;
- asparagine, glutamine;
- serine, threonine;
- lysine, arginine; and
- phenylalanine, tyrosine.

In some embodiments the degree of similarity, such as identity between a given amino acid sequence and an amino acid sequence which is a variant of said given amino acid sequence, will be at least about 60%, 70%, 80%, 81%, 82%, 83%, 84%, 85%, 86%, 87%, 88%, 89%, 90%, 91%, 92%, 93%, 94%, 95%, 96%, 97%, 98%, or 99%. In some embodiments, the degree of similarity or identity is given for an amino acid region which is at least about 10%, at least about 20%, at least about 30%, at least about 40%, at least about 50%, at least about 60%, at least about 70%, at least about 80%, at least about 90% or about 100% of the entire length of the reference amino acid sequence. For example, if the reference amino acid sequence consists of 200 amino acids, the degree of similarity or identity is given, e.g., for at least about 20, at least about 40, at least about 60, at least about 80, at least about 100, at least about 120, at least about 140, at least about 160, at least about 180, or about 200 amino acids, in some embodiments continuous amino acids. In some embodiments, the degree of similarity or identity is given for the entire length of the reference amino acid sequence. The alignment for determining sequence similarity, such as sequence identity, can be done with art known tools, such as using the best sequence alignment, for example, using Align, using standard settings, preferably EMBOSS::needle, Matrix: Blosum62, Gap Open 10.0, Gap Extend 0.5.

"Sequence similarity" indicates the percentage of amino acids that either are identical or that represent conservative amino acid substitutions. "Sequence identity" between two amino acid sequences indicates the percentage of amino acids that are identical between the sequences. "Sequence identity" between two nucleic acid sequences indicates the percentage of nucleotides that are identical between the sequences.

The terms "% identical" and "% identity" or similar terms are intended to refer, in particular, to the percentage of nucleotides or amino acids which are identical in an optimal alignment between the sequences to be compared. Said percentage is purely statistical, and the differences between the two sequences may be but are not necessarily randomly distributed over the entire length of the sequences to be compared. Comparisons of two sequences are usually carried out by comparing the sequences, after optimal alignment, with respect to a segment or "window of comparison", in order to

identify local regions of corresponding sequences. The optimal alignment for a comparison may be carried out manually or with the aid of the local homology algorithm by Smith and Waterman, 1981, Ads App. Math. 2, 482, with the aid of the local homology algorithm by Neddleman and Wunsch, 1970, J. Mol. Biol. 48, 443, with the aid of the similarity search algorithm by Pearson and Lipman, 1988, Proc. Natl Acad. Sci. USA 88, 2444, or with the aid of computer programs using said algorithms (GAP, BESTFIT, FASTA, BLAST P, BLAST N and TFASTA in Wisconsin Genetics Software Package, Genetics Computer Group, 575 Science Drive, Madison, Wis.). In some embodiments, percent identity of two sequences is determined using the BLASTN or BLASTP algorithm, as available on the United States National Center for Biotechnology Information (NCBI) website (*e.g.,* at blast.ncbi.nim.nih.gov/Blast.cgi?PAGE_TYPE=BlastSearch&BLAST_SPEC=-blast2seq&LINK_LOC =align2seq). In some embodiments, the algorithm parameters used for BLASTN algorithm on the NCBI website include: (i) Expect Threshold set to 10; (ii) Word Size set to 28; (iii) Max matches in a query range set to 0; (iv) Match/Mismatch Scores set to 1, -2; (v) Gap Costs set to Linear; and (vi) the filter for low complexity regions being used. In some embodiments, the algorithm parameters used for BLASTP algorithm on the NCBI website include: (i) Expect Threshold set to 10; (ii) Word Size set to 3; (iii) Max matches in a query range set to 0; (iv) Matrix set to BLOSUM62; (v) Gap Costs set to Existence: 11 Extension: 1; and (vi) conditional compositional score matrix adjustment.

Percentage identity is obtained by determining the number of identical positions at which the sequences to be compared correspond, dividing this number by the number of positions compared *(e.g.,* the number of positions in the reference sequence) and multiplying this result by 100.

In some embodiments, the degree of similarity or identity is given for a region which is at least about 50%, at least about 60%, at least about 70%, at least about 80%, at least about 90% or about 100% of the entire length of the reference sequence. For example, if the reference nucleic acid sequence consists of 200 nucleotides, the degree of identity is given for at least about 100, at least about 120, at least about 140, at least about 160, at least about 180, or about 200 nucleotides, in some embodiments continuous nucleotides. In some embodiments, the degree of similarity or identity is given for the entire length of the reference sequence. Homologous amino acid sequences exhibit according to the disclosure at least 40%, in particular at least 50%, at least 60%, at least 70%, at least 80%, at least 90% and, e.g., at least 95%, at least 98 or at least 99% identity of the amino acid residues.

The amino acid sequence variants described herein may readily be prepared by the skilled person, for example, by recombinant DNA manipulation. The manipulation of DNA sequences for preparing peptides or polypeptides having substitutions, additions, insertions or deletions, is described in detail in Molecular Cloning: A Laboratory Manual, 4th Edition, M.R. Green and J. Sambrook eds., Cold Spring Harbor Laboratory Press, Cold Spring Harbor 2012, for example. Furthermore, the peptides, polypeptides and amino acid variants described herein may be readily prepared with the aid of known peptide synthesis techniques such as, for example, by solid phase synthesis and similar methods.

In some embodiments, a fragment or variant of an amino acid sequence (peptide or polypeptide) is a "functional fragment" or "functional variant". The term "functional fragment" or "functional variant" of an amino acid sequence relates to any fragment or variant exhibiting one or more functional properties identical or similar to those of the amino acid sequence from which it is derived, *i.e.*, it is functionally equivalent. With respect to antigens or antigenic sequences, one particular function is one or more immunogenic activities displayed by the amino acid sequence from which the fragment or variant is derived. The term "functional fragment" or "functional variant", as used herein, in particular refers to a variant molecule or sequence that comprises an amino acid sequence that is altered by one or more amino acids compared to the amino acid sequence of the parent molecule or sequence and that is still capable of fulfilling one or more of the functions of the parent molecule or sequence, e.g., inducing an immune response. In some embodiments, the modifications in the amino acid sequence of the parent molecule or sequence do not significantly affect or alter the characteristics of the molecule or sequence. In different embodiments, the function of the functional fragment or functional variant may be reduced but still significantly present, e.g., function of the functional fragment or functional variant may be at least 50%, at least 60%, at least 70%, at least 80%, or at least 90% of the parent molecule or sequence. However, in other embodiments, function of the functional fragment or functional variant may be enhanced compared to the parent molecule or sequence.

An amino acid sequence (peptide or polypeptide) "derived from" a designated amino acid sequence (peptide or polypeptide) refers to the origin of the first amino acid sequence. In some embodiments, the amino acid sequence which is derived from a particular amino acid sequence has an amino acid sequence that is identical, essentially identical or homologous to that particular sequence or a fragment thereof. Amino acid sequences derived from a particular amino acid sequence may be variants of that particular sequence or a fragment thereof. For example, it will be understood by one of ordinary skill in the art that the antigens suitable for use herein may be altered such that they vary in sequence from the naturally occurring or native sequences from which they were derived, while retaining the desirable activity of the native sequences.

In some embodiments, "isolated" means removed (e.g., purified) from the natural state or from an artificial composition, such as a composition from a production process. For example, a nucleic acid, peptide or polypeptide naturally present in a living animal is not "isolated", but the same nucleic acid, peptide or polypeptide partially or completely separated from the coexisting materials of its natural state is "isolated". An isolated nucleic acid, peptide or polypeptide can exist in substantially purified form, or can exist in a non-native environment such as, for example, a host cell.

The term "transfection" relates to the introduction of nucleic acids, in particular RNA, into a cell. For purposes of the present disclosure, the term "transfection" also includes the introduction of a nucleic acid into a cell or the uptake of a nucleic acid by such cell, wherein the cell may be present in a subject, *e.g.,* a patient, or the cell may be *in vitro, e.g.,* outside of a patient. Thus, according to the present disclosure, a cell for transfection of a nucleic acid described herein can be present *in vitro* or *in vivo, e.g.* the cell can form part of an organ, a tissue and/or the body of a patient. According to the disclosure, transfection can be transient or stable. For some applications of transfection, it is sufficient if the transfected genetic material is only transiently expressed. RNA can be transfected into cells to transiently express its coded protein. Since the nucleic acid introduced in the transfection process is usually not integrated into the nuclear genome, the foreign nucleic acid will be diluted through mitosis or degraded. Cells allowing episomal amplification of nucleic acids greatly reduce the rate of dilution. If it is desired that the transfected nucleic acid actually remains in the genome of the cell and its daughter cells, a stable transfection must occur. Such stable transfection can be achieved by using virus-based systems or transposon-based systems for transfection, for example. Generally, nucleic acid encoding antigen is transiently transfected into cells. RNA can be transfected into cells to transiently express its coded protein.

The disclosure includes analogs of a peptide or polypeptide. According to the present disclosure, an analog of a peptide or polypeptide is a modified form of said peptide or polypeptide from which it has been derived and has at least one functional property of said peptide or polypeptide. *E.g.,* a pharmacological active analog of a peptide or polypeptide has at least one of the pharmacological activities of the peptide or polypeptide from which the analog has been derived. Such modifications include any chemical modification and comprise single or multiple substitutions, deletions and/or additions of any molecules associated with the peptide or polypeptide, such as carbohydrates, lipids and/or peptides or polypeptides. In some embodiments, "analogs" of peptides or polypeptides include those modified forms resulting from glycosylation, acetylation, phosphorylation, amidation, palmitoylation, myristoylation, isoprenylation, lipidation, alkylation, derivatization, introduction of protective/blocking groups, proteolytic cleavage or binding to an antibody or to another cellular ligand. The term "analog" also extends to all functional chemical equivalents of said peptides and polypeptides.

As used herein, the terms "linked", "fused", or "fusion" are used interchangeably. These terms refer to the joining together of two or more elements or components or domains.

As used herein "endogenous" refers to any material from or produced inside an organism, cell, tissue or system.

As used herein, the term "exogenous" refers to any material introduced from or produced outside an organism, cell, tissue or system.

According to various embodiments of the present disclosure, a nucleic acid such as RNA encoding a peptide or polypeptide is taken up by or introduced, *i.e.* transfected or transduced, into a cell which cell may be present *in vitro* or in a subject, resulting in expression of said peptide or polypeptide. The cell may, e.g., express the encoded peptide or polypeptide intracellularly (*e.g.* in the cytoplasm and/or in the nucleus), may secrete the encoded peptide or polypeptide, and/or may express it on the surface.

According to the present disclosure, terms such as "nucleic acid expressing" and "nucleic acid encoding" or similar terms are used interchangeably herein and with respect to a particular peptide or polypeptide mean that the nucleic acid, if present in the appropriate environment, e.g. within a cell, can be expressed to produce said peptide or polypeptide.

The term "expression" as used herein includes the transcription and/or translation of a particular nucleotide sequence.

In the context of the present disclosure, the term "transcription" relates to a process, wherein the genetic code in a DNA sequence is transcribed into RNA (especially mRNA). Subsequently, the RNA may be translated into peptide or polypeptide.

With respect to RNA, the term "expression" or "translation" relates to the process in the ribosomes of a cell by which a strand of mRNA directs the assembly of a sequence of amino acids to make a peptide or polypeptide.

A medical preparation, in particular kit, described herein may comprise instructional material or instructions. As used herein, "instructional material" or "instructions" includes a publication, a recording, a diagram, or any other medium of expression which can be used to communicate the usefulness of the compositions and methods of the present disclosure. The instructional material of the kit of the present disclosure may, for example, be affixed to a container which contains the compositions/formulations of the present disclosure or be shipped together with a container which contains the compositions/formulations. Alternatively, the instructional material may be shipped separately from the container with the intention that the instructional material and the compositions be used cooperatively by the recipient.

Prodrugs of a particular compound described herein are those compounds that upon administration to an individual undergo chemical conversion under physiological conditions to provide the particular compound. Additionally, prodrugs can be converted to the particular compound by chemical or biochemical methods in an *ex vivo* environment. For example, prodrugs can be slowly converted to the particular compound when, for example, placed in a transdermal patch reservoir with a suitable enzyme or chemical reagent. Exemplary prodrugs are esters (using an alcohol or a carboxy group contained in the particular compound) or amides (using an amino or a carboxy group contained in the particular compound) which are hydrolyzable *in vivo.* Specifically, any amino group which is contained in the particular compound and which bears at least one hydrogen atom can be converted into a prodrug form. Typical N-prodrug forms include carbamates,

Mannich bases, enamines, and enaminones.

In the present specification, a structural formula of a compound may represent a certain isomer of said compound. It is to be understood, however, that the present disclosure includes all isomers such as geometrical isomers, optical isomers based on an asymmetrical carbon, stereoisomers, tautomers and the like which occur structurally and isomer mixtures and is not limited to the description of the formula. Furthermore, in the present specification, a structural formula of a compound may represent a specific salt and/or solvate of said compound. It is to be understood, however, that the present disclosure includes all salts (e.g., pharmaceutically acceptable salts) and solvates (e.g., hydrates) and is not limited to the description of the specific salt and/or solvate.

"Isomers" are compounds having the same molecular formula but differ in structure ("structural isomers") or in the geometrical (spatial) positioning of the functional groups and/or atoms ("stereoisomers"). "Enantiomers" are a pair of stereoisomers which are non-superimposable mirror-images of each other. A "racemic mixture" or "racemate" contains a pair of enantiomers in equal amounts and is denoted by the prefix (±). "Diastereomers" are stereoisomers which are non-superimposable and which are not mirror-images of each other. "Tautomers" are structural isomers of the same chemical substance that spontaneously and reversibly interconvert into each other, even when pure, due to the migration of individual atoms or groups of atoms; *i.e.,* the tautomers are in a dynamic chemical equilibrium with each other. An example of tautomers are the isomers of the keto-enol-tautomerism. "Conformers" are stereoisomers that can be interconverted just by rotations about formally single bonds, and include - in particular - those leading to different 3-dimentional forms of (hetero)cyclic rings, such as chair, half-chair, boat, and twist-boat forms of cyclohexane.

The term "solvate" as used herein refers to an addition complex of a dissolved material in a solvent (such as an organic solvent (e.g., an aliphatic alcohol (such as methanol, ethanol, n-propanol, isopropanol), acetone, acetonitrile, ether, and the like), water or a mixture of two or more of these liquids), wherein the addition complex exists in the form of a crystal or mixed crystal. The amount of solvent contained in the addition complex may be stoichiometric or non-stoichiometric. A "hydrate" is a solvate wherein the solvent is water.

In isotopically labeled compounds one or more atoms are replaced by a corresponding atom having the same number of protons but differing in the number of neutrons. For example, a hydrogen atom may be replaced by a deuterium or tritium atom. Exemplary isotopes which can be used in the present disclosure include deuterium, tritium, $^{11}C$, $^{13}C$, $^{14}C$, $^{15}N$, $^{18}F$, $^{32}P$, $^{32}S$, $^{35}S$, $^{36}Cl$, and $^{125}I$.

The term "average diameter" refers to the mean hydrodynamic diameter of particles as measured by dynamic light scattering (DLS) with data analysis using the so-called cumulant algorithm, which provides as results the so-called $Z_{average}$ with the dimension of a length, and the polydispersity index (PDI), which is dimensionless (Koppel, D., J. Chem. Phys. 57, 1972, pp 4814-4820, ISO 13321). Here "average diameter", "diameter" or "size" for particles is used synonymously with this value of the $Z_{average}$.

In some embodiments, the "polydispersity index" is calculated based on dynamic light scattering measurements by the so-called cumulant analysis as mentioned in the definition of the "average diameter". Under certain prerequisites, it can be taken as a measure of the size distribution of an ensemble of nanoparticles.

The "radius of gyration" (abbreviated herein as $R_g$) of a particle about an axis of rotation is the radial distance of a point from the axis of rotation at which, if the whole mass of the particle is assumed to be concentrated, its moment of inertia about the given axis would be the same as with its actual distribution of mass. Mathematically, $R_g$ is the root mean square distance of the particle's components from either its center of mass or a given axis. For example, for a macromolecule composed of *n* mass elements, of masses $m_i$ ($i$ = 1, 2, 3, ..., $n$), located at fixed distances $s_i$ from the center of mass, $R_g$ is the square-root of the mass average of $s_i^2$ over all mass elements and can be calculated as follows:

$$R_g = \left( \sum_{i=1}^{n} m_i \cdot s_i^2 \Big/ \sum_{i=1}^{n} m_i \right)^{1/2}$$

The radius of gyration can be determined or calculated experimentally, e.g., by using light scattering. In particular, for small scattering vectors $\vec{q}$ the structure function S is defined as follows:

$$S(\vec{q}) \approx N \cdot \left( 1 - \frac{q^2 \cdot R_g^2}{3} \right)$$

wherein N is the number of components (Guinier's law).

The "hydrodynamic radius" (which is sometimes called "Stokes radius" or "Stokes-Einstein radius") of a particle is the radius of a hypothetical hard sphere that diffuses at the same rate as said particle. The hydrodynamic radius is related to the mobility of the particle, taking into account not only size but also solvent effects. For example, a smaller charged

particle with stronger hydration may have a greater hydrodynamic radius than a larger charged particle with weaker hydration. This is because the smaller particle drags a greater number of water molecules with it as it moves through the solution. Since the actual dimensions of the particle in a solvent are not directly measurable, the hydrodynamic radius may be defined by the Stokes-Einstein equation:

$$R_h = \frac{k_B \cdot T}{6 \cdot \pi \cdot \eta \cdot D}$$

wherein $k_B$ is the Boltzmann constant; T is the temperature; $\eta$ is the viscosity of the solvent; and D is the diffusion coefficient. The diffusion coefficient can be determined experimentally, *e.g.*, by using dynamic light scattering (DLS). Thus, one procedure to determine the hydrodynamic radius of a particle or a population of particles (such as the hydrodynamic radius of particles contained in a sample or control composition as disclosed herein or the hydrodynamic radius of a particle peak obtained from subjecting such a sample or control composition to field-flow fractionation) is to measure the DLS signal of said particle or population of particles (such as DLS signal of particles contained in a sample or control composition as disclosed herein or the DLS signal of a particle peak obtained from subjecting such a sample or control composition to field-flow fractionation).

The expression "light scattering" as used herein refers to the physical process where light is forced to deviate from a straight trajectory by one or more paths due to localized nonuniformities in the medium through which the light passes.

The term "UV" means ultraviolet and designates a band of the electromagnetic spectrum with a wavelength from 10 nm to 400 nm, *i.e.,* shorter than that of visible light but longer than X-rays.

The expression "multi-angle light scattering" or "MALS" as used herein relates to a technique for measuring the light scattered by a sample into a plurality of angles. "Multi-angle" means in this respect that scattered light can be detected at different discrete angles as measured, for example, by a single detector moved over a range including the specific angles selected or an array of detectors fixed at specific angular locations. In certain embodiments, the light source used in MALS is a laser source (MALLS: multi-angle laser light scattering). Based on the MALS signal of a composition comprising particles and by using an appropriate formalism (*e.g.,* Zimm plot, Berry plot, or Debye plot), it is possible to determine the radius of gyration ($R_g$) and, thus, the size of said particles. Preferably, the Zimm plot is a graphical presentation using the following equation:

$$\frac{R_\theta}{K^*c} = M_w P(\theta) - 2A_2 c M_w^2 P^2(\theta)$$

wherein *c* is the mass concentration of the particles in the solvent (g/mL); $A_2$ is the second virial coefficient (mol·mL/g$^2$); $P(\vartheta)$ is a form factor relating to the dependence of scattered light intensity on angle; $R_\vartheta$ is the excess Rayleigh ratio (cm$^{-1}$); and $K^*$ is an optical constant that is equal to $4\pi^2\eta_o$ (d*n*/d*c*)$^2\lambda_0^{-4}N_A^{-1}$, where $\eta_o$ is the refractive index of the solvent at the incident radiation (vacuum) wavelength, $\lambda_0$ is the incident radiation (vacuum) wavelength (nm), $N_A$ is Avogadro's number (mol$^{-1}$), and dn/dc is the differential refractive index increment (mL/g) (cf., *e.g.,* Buchholz et al. (Electrophoresis 22 (2001), 4118-4128); B.H. Zimm (J. Chem. Phys. 13 (1945), 141; P. Debye (J. Appl. Phys. 15 (1944): 338; and W. Burchard (Anal. Chem. 75 (2003), 4279-4291). Preferably, the Berry plot is calculated using the following term or the reciprocal thereof:

$$\sqrt{\frac{R_\theta}{K^*c}}$$

wherein *c, $R_\vartheta$* and *K** are as defined above. Preferably, the Debye plot is calculated using the following term or the reciprocal thereof:

$$\frac{K^*c}{R_\theta}$$

wherein *c, $R_\vartheta$* and K* are as defined above.

The expression "dynamic light scattering" or "DLS" as used herein refers to a technique to determine the size and size distribution profile of particles, in particular with respect to the hydrodynamic radius of the particles. A monochromatic light source, usually a laser, is shot through a polarizer and into a sample. The scattered light then goes through a second

polarizer where it is detected and the resulting image is projected onto a screen. The particles in the solution are being hit with the light and diffract the light in all directions. The diffracted light from the particles can either interfere constructively (light regions) or destructively (dark regions). This process is repeated at short time intervals and the resulting set of speckle patterns are analyzed by an autocorrelator that compares the intensity of light at each spot over time.

The expression "static light scattering" or "SLS" as used herein refers to a technique to determine the size and size distribution profile of particles, in particular with respect to the radius of gyration of the particles, and/or the molar mass of particles. A high-intensity monochromatic light, usually a laser, is launched in a solution containing the particles. One or many detectors are used to measure the scattering intensity at one or many angles. The angular dependence is needed to obtain accurate measurements of both molar mass and size for all macromolecules of radius. Hence simultaneous measurements at several angles relative to the direction of incident light, known as multi-angle light scattering (MALS) or multi-angle laser light scattering (MALLS), is generally regarded as the standard implementation of static light scattering.

**Nucleic Acids**

The term "nucleic acid" comprises deoxyribonucleic acid (DNA), ribonucleic acid (RNA), combinations thereof, and modified forms thereof. The term comprises genomic DNA, cDNA, mRNA, recombinantly produced and chemically synthesized molecules. In some embodiments, a nucleic acid is DNA. In some embodiments, a nucleic acid is RNA. In some embodiments, a nucleic acid is a mixture of DNA and RNA. A nucleic acid may be present as a single-stranded or double-stranded and linear or covalently circularly closed molecule. A nucleic acid can be isolated. The term "isolated nucleic acid" means, according to the present disclosure, that the nucleic acid (i) was amplified *in vitro,* for example via polymerase chain reaction (PCR) for DNA or *in vitro* transcription (using, *e.g.,* an RNA polymerase) for RNA, (ii) was produced recombinantly by cloning, (iii) was purified, for example, by cleavage and separation by gel electrophoresis, or (iv) was synthesized, for example, by chemical synthesis. The term "nucleoside" (abbreviated herein as "N") relates to compounds which can be thought of as nucleotides without a phosphate group. While a nucleoside is a nucleobase linked to a sugar (*e.g.,* ribose or deoxyribose), a nucleotide is composed of a nucleoside and one or more phosphate groups. Examples of nucleosides include cytidine, uridine, pseudouridine, adenosine, and guanosine.

The five standard nucleosides which usually make up naturally occurring nucleic acids are uridine, adenosine, thymidine, cytidine and guanosine. The five nucleosides are commonly abbreviated to their one letter codes U, A, T, C and G, respectively. However, thymidine is more commonly written as "dT" ("d" represents "deoxy") as it contains a 2'-deoxyribofuranose moiety rather than the ribofuranose ring found in uridine. This is because thymidine is found in deoxyribonucleic acid (DNA) and not ribonucleic acid (RNA). Conversely, uridine is found in RNA and not DNA. The remaining three nucleosides may be found in both RNA and DNA. In RNA, they would be represented as A, C and G, whereas in DNA they would be represented as dA, dC and dG.

A modified purine (A or G) or pyrimidine (C, T, or U) base moiety is, in some embodiments, modified by one or more alkyl groups, e.g., one or more $C_{1-4}$ alkyl groups, e.g., one or more methyl groups. Particular examples of modified purine or pyrimidine base moieties include $N^7$-alkyl-guanine, $N^6$-alkyl-adenine, 5-alkyl-cytosine, 5-alkyl-uracil, and N(1)-alkyl-uracil, such as $N^7$-$C_{1-4}$ alkyl-guanine, $N^6$-$C_{1-4}$ alkyl-adenine, 5-$C_{1-4}$ alkyl-cytosine, 5-$C_{1-4}$ alkyl-uracil, and N(1)-$C_{14}$ alkyl-uracil, preferably $N^7$-methyl-guanine, $N^6$-methyl-adenine, 5-methyl-cytosine, 5-methyl-uracil, and N(1)-methyl-uracil.

Herein, the term "DNA" relates to a nucleic acid molecule which includes deoxyribonucleotide residues. In preferred embodiments, the DNA contains all or a majority of deoxyribonucleotide residues. As used herein, "deoxyribonucleotide" refers to a nucleotide which lacks a hydroxyl group at the 2'-position of a β-D-ribofuranosyl group. DNA encompasses without limitation, double stranded DNA, single stranded DNA, isolated DNA such as partially purified DNA, essentially pure DNA, synthetic DNA, recombinantly produced DNA, as well as modified DNA that differs from naturally occurring DNA by the addition, deletion, substitution and/or alteration of one or more nucleotides. Such alterations may refer to addition of non-nucleotide material to internal DNA nucleotides or to the end(s) of DNA. It is also contemplated herein that nucleotides in DNA may be non-standard nucleotides, such as chemically synthesized nucleotides or ribonucleotides. For the present disclosure, these altered DNAs are considered analogs of naturally-occurring DNA. A molecule contains "a majority of deoxyribonucleotide residues" if the content of deoxyribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard *(i.e.,* naturally occurring) nucleotide residues or analogs thereof).

DNA may be recombinant DNA and may be obtained by cloning of a nucleic acid, in particular cDNA. The cDNA may be obtained by reverse transcription of RNA.

The term "RNA" relates to a nucleic acid molecule which includes ribonucleotide residues. In preferred embodiments, the RNA contains all or a majority of ribonucleotide residues. As used herein, "ribonucleotide" refers to

a nucleotide with a hydroxyl group at the 2'-position of a β-D-ribofuranosyl group. RNA encompasses without limitation, double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered/modified nucleotides can be referred to as analogs of naturally occurring nucleotides, and the corresponding RNAs containing such altered/modified nucleotides (*i.e.,* altered/modified RNAs) can be referred to as analogs of naturally occurring RNAs. A molecule contains "a majority of ribonucleotide residues" if the content of ribonucleotide residues in the molecule is more than 50% (such as at least 55%, at least 60%, at least 65%, at least 70%, at least 75%, at least 80%, at least 85%, at least 90%, at least 95%, at least 96%, at least 97%, at least 98%, at least 99%), based on the total number of nucleotide residues in the molecule. The total number of nucleotide residues in a molecule is the sum of all nucleotide residues (irrespective of whether the nucleotide residues are standard (*i.e.,* naturally occurring) nucleotide residues or analogs thereof). "RNA" includes mRNA, tRNA, ribosomal RNA (rRNA), small nuclear RNA (snRNA), self-amplifying RNA (saRNA), single-stranded RNA (ssRNA), dsRNA, inhibitory RNA (such as antisense ssRNA, small interfering RNA (siRNA), or microRNA (miRNA)), activating RNA (such as small activating RNA) and immunostimulatory RNA (isRNA). In some embodiments, "RNA" refers to mRNA.

The term "*in vitro* transcription" or "IVT" as used herein means that the transcription (*i.e.,* the generation of RNA) is conducted in a cell-free manner. *I.e.*, IVT does not use living/cultured cells but rather the transcription machinery extracted from cells (*e.g.,* cell lysates or the isolated components thereof, including an RNA polymerase (preferably T7, T3 or SP6 polymerase)).

**mRNA**

According to the present disclosure, the term "mRNA" means "messenger-RNA" and includes a "transcript" which may be generated by using a DNA template. Generally, mRNA encodes a peptide or polypeptide.

mRNA is single-stranded but may contain self-complementary sequences that allow parts of the mRNA to fold and pair with itself to form double helices.

According to the present disclosure, "dsRNA" means double-stranded RNA and is RNA with two partially or completely complementary strands.

In preferred embodiments of the present disclosure, the mRNA relates to an RNA transcript which encodes a peptide or polypeptide.

In some embodiments, the mRNA which preferably encodes a peptide or polypeptide has a length of at least 45 nucleotides (such as at least 60, at least 90, at least 100, at least 200, at least 300, at least 400, at least 500, at least 600, at least 700, at least 800, at least 900, at least 1,000, at least 1,500, at least 2,000, at least 2,500, at least 3,000, at least 3,500, at least 4,000, at least 4,500, at least 5,000, at least 6,000, at least 7,000, at least 8,000, at least 9,000 nucleotides), preferably up to 15,000, such as up to 14,000, up to 13,000, up to 12,000 nucleotides, up to 11,000 nucleotides or up to 10,000 nucleotides.

As established in the art, mRNA generally contains a 5' untranslated region (5'-UTR), a peptide/polypeptide coding region and a 3' untranslated region (3'-UTR). In some embodiments, the mRNA is produced by *in vitro* transcription or chemical synthesis. In some embodiments, the mRNA is produced by *in vitro* transcription using a DNA template. The *in vitro* transcription methodology is known to the skilled person; cf., *e.g.,* Molecular Cloning: A Laboratory Manual, 4th Edition, M.R. Green and J. Sambrook eds., Cold Spring Harbor Laboratory Press, Cold Spring Harbor 2012. Furthermore, a variety of *in vitro* transcription kits is commercially available, *e.g.,* from Thermo Fisher Scientific (such as TranscriptAid™ T7 kit, MEGAscript® T7 kit, MAXIscript®), New England BioLabs Inc. (such as HiScribe™ T7 kit, HiScribe™ T7 ARCA mRNA kit), Promega (such as RiboMAX™, HeLaScribe®, Riboprobe® systems), Jena Bioscience (such as SP6 or T7 transcription kits), and Epicentre (such as AmpliScribe™). For providing modified mRNA, correspondingly modified nucleotides, such as modified naturally occurring nucleotides, non-naturally occurring nucleotides and/or modified non-naturally occurring nucleotides, can be incorporated during synthesis (preferably *in vitro* transcription), or modifications can be effected in and/or added to the mRNA after transcription.

In some embodiments, mRNA is *in vitro* transcribed mRNA (IVT-RNA) and may be obtained by *in vitro* transcription of an appropriate DNA template. The promoter for controlling transcription can be any promoter for any RNA polymerase. Particular examples of RNA polymerases are the T7, T3, and SP6 RNA polymerases. Preferably, the *in vitro* transcription is controlled by a T7 or SP6 promoter. A DNA template for *in vitro* transcription may be obtained by cloning of a nucleic acid, in particular cDNA, and introducing it into an appropriate vector for *in vitro* transcription. The cDNA may be obtained by reverse transcription of RNA.

In some embodiments of the present disclosure, the mRNA is "replicon mRNA" or simply a "replicon", in particular "self-replicating mRNA" or "self-amplifying mRNA". In certain embodiments, the replicon or self-replicating mRNA is

derived from or comprises elements derived from an ssRNA virus, in particular a positive-stranded ssRNA virus such as an alphavirus. Alphaviruses are typical representatives of positive-stranded RNA viruses. Alphaviruses replicate in the cytoplasm of infected cells (for review of the alphaviral life cycle see José et al., Future Microbiol., 2009, vol. 4, pp. 837-856). The total genome length of many alphaviruses typically ranges between 11,000 and 12,000 nucleotides, and the genomic RNA typically has a 5'-cap, and a 3' poly(A) tail. The genome of alphaviruses encodes non-structural proteins (involved in transcription, modification and replication of viral RNA and in protein modification) and structural proteins (forming the virus particle). There are typically two open reading frames (ORFs) in the genome. The four non-structural proteins (nsP1-nsP4) are typically encoded together by a first ORF beginning near the 5' terminus of the genome, while alphavirus structural proteins are encoded together by a second ORF which is found downstream of the first ORF and extends near the 3' terminus of the genome. Typically, the first ORF is larger than the second ORF, the ratio being roughly 2:1. In cells infected by an alphavirus, only the nucleic acid sequence encoding non-structural proteins is translated from the genomic RNA, while the genetic information encoding structural proteins is translatable from a subgenomic transcript, which is an RNA molecule that resembles eukaryotic messenger RNA (mRNA; Gould et al., 2010, Antiviral Res., vol. 87 pp. 111-124). Following infection, i.e. at early stages of the viral life cycle, the (+) stranded genomic RNA directly acts like a messenger RNA for the translation of the open reading frame encoding the non-structural poly-protein (nsP1234). Alphavirus-derived vectors have been proposed for delivery of foreign genetic information into target cells or target organisms. In simple approaches, the open reading frame encoding alphaviral structural proteins is replaced by an open reading frame encoding a protein of interest. Alphavirus-based trans-replication systems rely on alphavirus nucleotide sequence elements on two separate nucleic acid molecules: one nucleic acid molecule encodes a viral replicase, and the other nucleic acid molecule is capable of being replicated by said replicase in trans (hence the designation trans-replication system). Trans-replication requires the presence of both these nucleic acid molecules in a given host cell. The nucleic acid molecule capable of being replicated by the replicase in trans must comprise certain alphaviral sequence elements to allow recognition and RNA synthesis by the alphaviral replicase.

In some embodiments of the present disclosure, the RNA (in particular, mRNA) described herein (e.g., contained in the compositions/formulations of the present disclosure and/or used in the methods of the present disclosure) contains one or more modifications, e.g., in order to increase its stability and/or increase translation efficiency and/or decrease immunogenicity and/or decrease cytotoxicity. For example, in order to increase expression of the RNA (in particular, mRNA), it may be modified within the coding region, *i.e.,* the sequence encoding the expressed peptide or polypeptide, preferably without altering the sequence of the expressed peptide or polypeptide. Such modifications are described, for example, in WO 2007/036366 and PCT/EP2019/056502, and include the following: a 5'-cap structure; an extension or truncation of the naturally occurring poly(A) tail; an alteration of the 5'- and/or 3'-untranslated regions (UTR) such as introduction of a UTR which is not related to the coding region of said RNA; the replacement of one or more naturally occurring nucleotides with synthetic nucleotides; and codon optimization (e.g., to alter, preferably increase, the GC content of the RNA).

In some embodiments, the RNA (in particular, mRNA) described herein comprises a 5'-cap structure. In some embodiments, the mRNA does not have uncapped 5'-triphosphates. In some embodiments, the RNA (in particular, mRNA)may comprise a conventional 5'-cap and/or a 5'-cap analog. The term "conventional 5'-cap" refers to a cap structure found on the 5'-end of an mRNA molecule and generally consists of a guanosine 5'-triphosphate (Gppp) which is connected via its triphosphate moiety to the 5'-end of the next nucleotide of the mRNA (i.e., the guanosine is connected via a 5' to 5' triphosphate linkage to the rest of the mRNA). The guanosine may be methylated at position $N^7$ (resulting in the cap structure $m^7$Gppp). The term "5'-cap analog" includes a 5'-cap which is based on a conventional 5'-cap but which has been modified at either the 2'- or 3'-position of the $m^7$guanosine structure in order to avoid an integration of the 5'-cap analog in the reverse orientation (such 5'-cap analogs are also called anti-reverse cap analogs (ARCAs)). Particularly preferred 5'-cap analogs are those having one or more substitutions at the bridging and non-bridging oxygen in the phosphate bridge, such as phosphorothioate modified 5'-cap analogs at the β-phosphate (such as $m_2^{7,2'O}$G(5')ppSp(5')G (referred to as beta-S-ARCA or β-S-ARCA)), as described in PCT/EP2019/056502. Providing an RNA (in particular, mRNA)with a 5'-cap structure as described herein may be achieved by *in vitro* transcription of a DNA template in presence of a corresponding 5'-cap compound, wherein said 5'-cap structure is co-transcriptionally incorporated into the generated RNA (in particular, mRNA) strand, or the RNA (in particular, mRNA) may be generated, for example, by *in vitro* transcription, and the 5'-cap structure may be attached to the RNA post-transcriptionally using capping enzymes, for example, capping enzymes of vaccinia virus.

In some embodiments, the RNA (in particular, mRNA) comprises a 5'-cap structure selected from the group consisting of $m_2^{7,2'O}$G(5')ppSp(5')G (in particular its D1 diastereomer), $m_2^{7,3'O}$G(5')ppp(5')G, and $m_2^{7,3'O}$Gppp($m_1^{2'-O}$)ApG. In some embodiments, RNA encoding a peptide or polypeptide comprising an antigen or epitope comprises $m_2^{7,2'O}$G(5')ppSp(5')G (in particular its D1 diastereomer) as 5'-cap structure.

In some embodiments, the RNA (in particular, mRNA) comprises a cap0, cap1, or cap2, preferably cap1 or cap2. According to the present disclosure, the term "cap0" means the structure "$m^7$GpppN", wherein N is any nucleoside bearing an OH moiety at position 2'. According to the present disclosure, the term "cap1" means the structure "$m^7$GpppNm",

wherein Nm is any nucleoside bearing an $OCH_3$ moiety at position 2'. According to the present disclosure, the term "cap2" means the structure "$m^7$GpppNmNm", wherein each Nm is independently any nucleoside bearing an $OCH_3$ moiety at position 2'.

The 5'-cap analog beta-S-ARCA (β-S-ARCA) has the following structure:

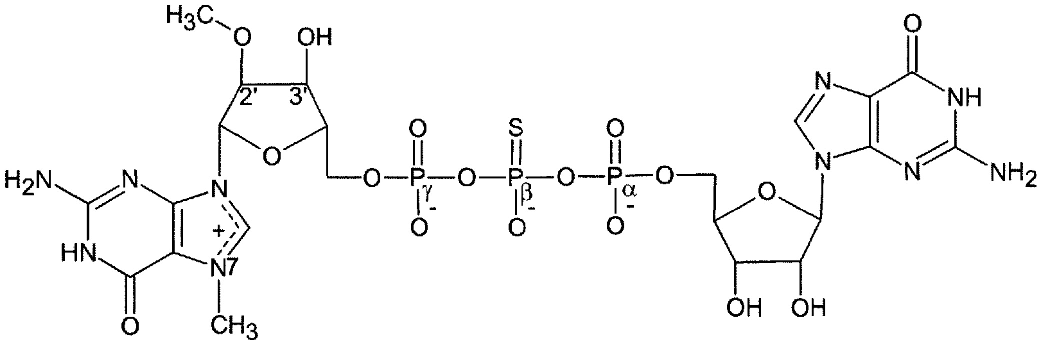

The "D1 diastereomer of beta-S-ARCA" or "beta-S-ARCA(D1)" is the diastereomer of beta-S-ARCA which elutes first on an HPLC column compared to the D2 diastereomer of beta-S-ARCA (beta-S-ARCA(D2)) and thus exhibits a shorter retention time. The HPLC preferably is an analytical HPLC. In some embodiments, a Supelcosil LC-18-T RP column, preferably of the format: 5 µm, 4.6 x 250 mm is used for separation, whereby a flow rate of 1.3 ml/min can be applied. In some embodiments, a gradient of methanol in ammonium acetate, for example, a 0-25% linear gradient of methanol in 0.05 M ammonium acetate, pH = 5.9, within 15 min is used. UV-detection (VWD) can be performed at 260 nm and fluorescence detection (FLD) can be performed with excitation at 280 nm and detection at 337 nm.

The 5'-cap analog $m_2^{7,3'\text{-}O}$Gppp($m_1^{2'\text{-}O}$)ApG (also referred to as $m_2^{7,3'O}$G(5')ppp(5')$m^{2'\text{-}O}$ApG) which is a building block of a cap1 has the following structure:

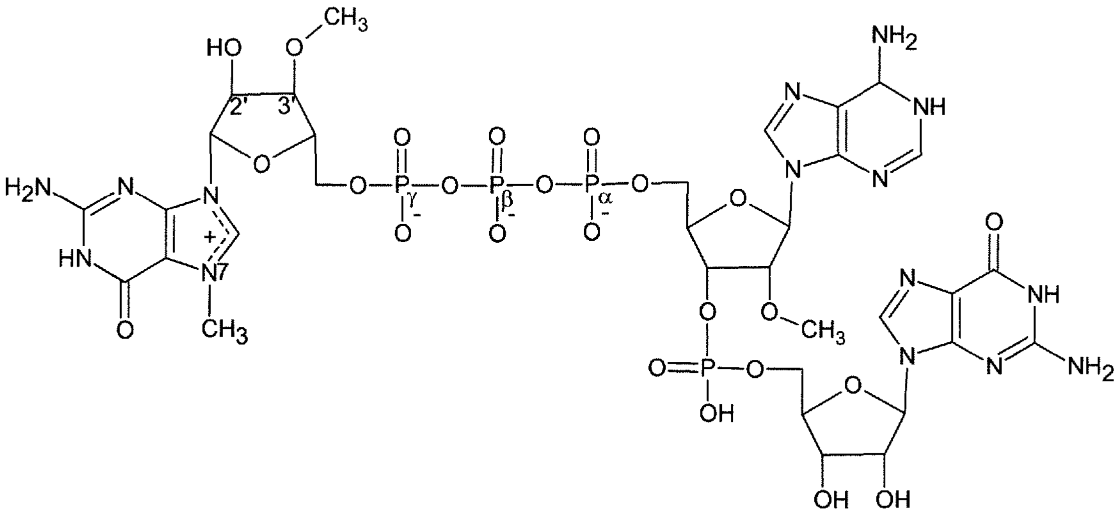

An exemplary cap0 mRNA comprising β-S-ARCA and mRNA has the following structure:

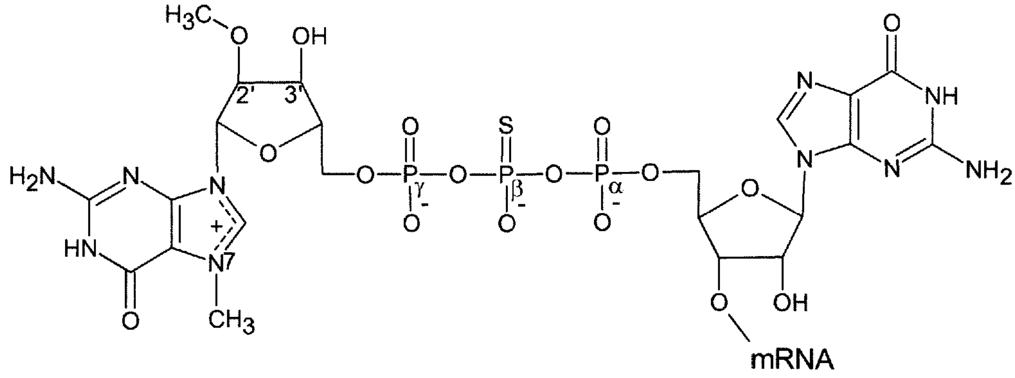

An exemplary cap0 mRNA comprising $m_2^{7,3'O}G(5')ppp(5')G$ and mRNA has the following structure:

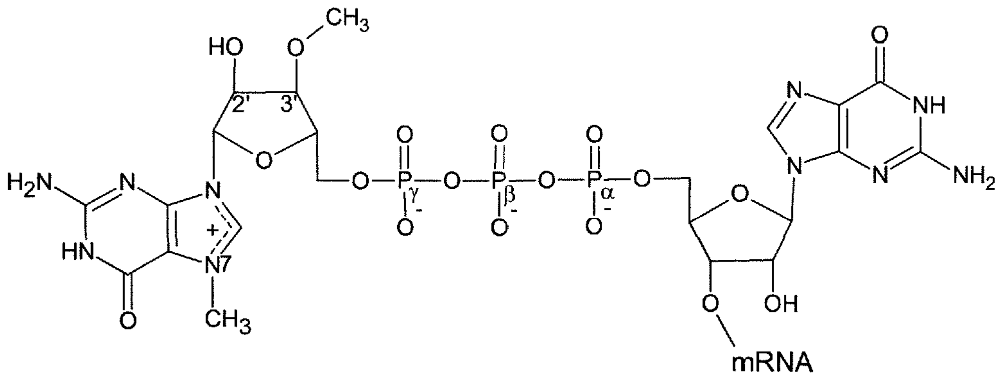

An exemplary cap1 mRNA comprising $m_2^{7,3'-O}Gppp(m_1^{2'-O})ApG$ and mRNA has the following structure:

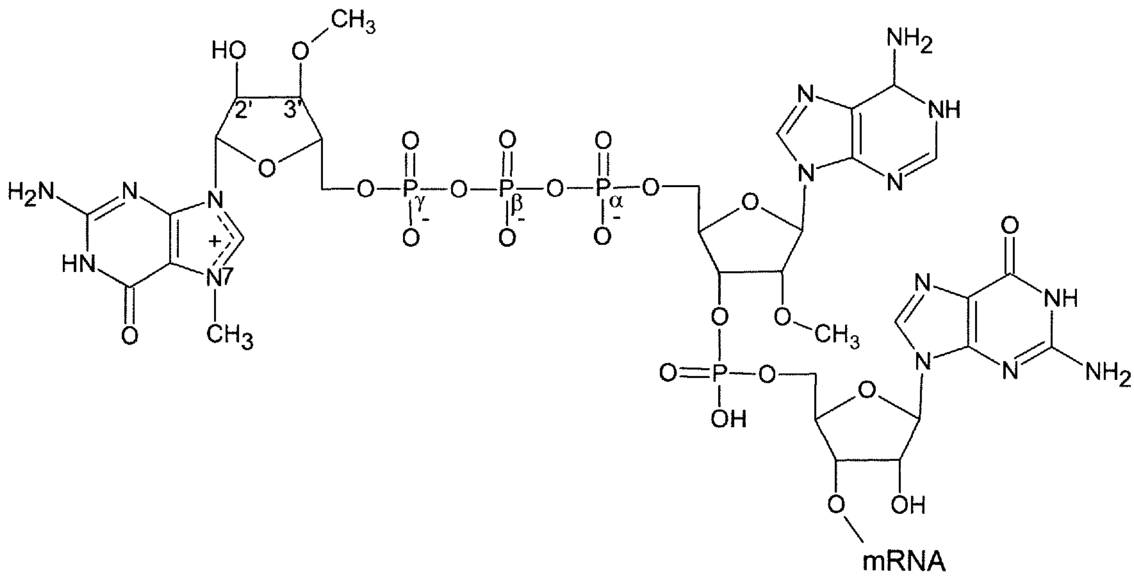

As used herein, the term "poly-A tail" or "poly-A sequence" refers to an uninterrupted or interrupted sequence of adenylate residues which is typically located at the 3'-end of an mRNA molecule. Poly-A tails or poly-A sequences are known to those of skill in the art and may follow the 3'-UTR in the RNAs (in particular, mRNAs) described herein. An uninterrupted poly-A tail is characterized by consecutive adenylate residues. In nature, an uninterrupted poly-A tail is typical. RNAs (in particular, mRNAs) disclosed herein can have a poly-A tail attached to the free 3'-end of the RNA by a template-independent RNA polymerase after transcription or a poly-A tail encoded by DNA and transcribed by a template-dependent RNA polymerase.

It has been demonstrated that a poly-A tail of about 120 A nucleotides has a beneficial influence on the levels of mRNA in transfected eukaryotic cells, as well as on the levels of protein that is translated from an open reading frame that is

present upstream (5') of the poly-A tail (Holtkamp et al., 2006, Blood, vol. 108, pp. 4009-4017).

The poly-A tail may be of any length. In some embodiments, a poly-A tail comprises, essentially consists of, or consists of at least 20, at least 30, at least 40, at least 80, or at least 100 and up to 500, up to 400, up to 300, up to 200, or up to 150 A nucleotides, and, in particular, about 120 A nucleotides. In this context, "essentially consists of" means that most nucleotides in the poly-A tail, typically at least 75%, at least 80%, at least 85%, at least 90%, at least 95%, at least 96%, at least 97%, at least 98%, or at least 99% by number of nucleotides in the poly-A tail are A nucleotides, but permits that remaining nucleotides are nucleotides other than A nucleotides, such as U nucleotides (uridylate), G nucleotides (guanylate), or C nucleotides (cytidylate). In this context, "consists of" means that all nucleotides in the poly-A tail, *i.e., 100%* by number of nucleotides in the poly-A tail, are A nucleotides. The term "A nucleotide" or "A" refers to adenylate.

In some embodiments, a poly-A tail is attached during RNA transcription, *e.g.,* during preparation of *in vitro* transcribed RNA, based on a DNA template comprising repeated dT nucleotides (deoxythymidylate) in the strand complementary to the coding strand. The DNA sequence encoding a poly-A tail (coding strand) is referred to as poly(A) cassette.

In some embodiments, the poly(A) cassette present in the coding strand of DNA essentially consists of dA nucleotides, but is interrupted by a random sequence of the four nucleotides (dA, dC, dG, and dT). Such random sequence may be 5 to 50, 10 to 30, or 10 to 20 nucleotides in length. Such a cassette is disclosed in WO 2016/005324.

Any poly(A) cassette disclosed in WO 2016/005324 A1 may be used in the present disclosure. A poly(A) cassette that essentially consists of dA nucleotides, but is interrupted by a random sequence having an equal distribution of the four nucleotides (dA, dC, dG, dT) and having a length of *e.g.,* 5 to 50 nucleotides shows, on DNA level, constant propagation of plasmid DNA in *E. coli* and is still associated, on RNA level, with the beneficial properties with respect to supporting RNA stability and translational efficiency is encompassed. Consequently, in some embodiments, the poly-A tail contained in an RNA (in particular, mRNA) molecule described herein essentially consists of A nucleotides, but is interrupted by a random sequence of the four nucleotides (A, C, G, U). Such random sequence may be 5 to 50, 10 to 30, or 10 to 20 nucleotides in length.

In some embodiments, no nucleotides other than A nucleotides flank a poly-A tail at its 3'- end, *i.e.,* the poly-A tail is not masked or followed at its 3'-end by a nucleotide other than A. In some embodiments, a poly-A tail may comprise at least 20, at least 30, at least 40, at least 80, or at least 100 and up to 500, up to 400, up to 300, up to 200, or up to 150 nucleotides. In some embodiments, the poly-A tail may essentially consist of at least 20, at least 30, at least 40, at least 80, or at least 100 and up to 500, up to 400, up to 300, up to 200, or up to 150 nucleotides. In some embodiments, the poly-A tail may consist of at least 20, at least 30, at least 40, at least 80, or at least 100 and up to 500, up to 400, up to 300, up to 200, or up to 150 nucleotides. In some embodiments, the poly-A tail comprises the poly-A tail shown in SEQ ID NO: 8. In some embodiments, the poly-A tail comprises at least 100 nucleotides. In some embodiments, the poly-A tail comprises about 150 nucleotides. In some embodiments, the poly-A tail comprises about 120 nucleotides.

In some embodiments, RNA (in particular, mRNA) described in present disclosure comprises a 5'-UTR and/or a 3'-UTR. The term "untranslated region" or "UTR" relates to a region in a DNA molecule which is transcribed but is not translated into an amino acid sequence, or to the corresponding region in an RNA molecule, such as an mRNA molecule. An untranslated region (UTR) can be present 5' (upstream) of an open reading frame (5'-UTR) and/or 3' (downstream) of an open reading frame (3'-UTR). A 5'-UTR, if present, is located at the 5'-end, upstream of the start codon of a protein-encoding region. A 5'-UTR is downstream of the 5'-cap (if present), e.g., directly adjacent to the 5'-cap. A 3'-UTR, if present, is located at the 3'-end, downstream of the termination codon of a protein-encoding region, but the term "3'-UTR" does generally not include the poly-A sequence. Thus, the 3'-UTR is upstream of the poly-A sequence (if present), *e.g.,* directly adjacent to the poly-A sequence. Incorporation of a 3'-UTR into the 3'-non translated region of an RNA (preferably mRNA) molecule can result in an enhancement in translation efficiency. A synergistic effect may be achieved by incorporating two or more of such 3'-UTRs (which are preferably arranged in a head-to-tail orientation; cf., e.g., Holtkamp et al., Blood 108, 4009-4017 (2006)). The 3'-UTRs may be autologous or heterologous to the RNA (e.g., mRNA) into which they are introduced. In certain embodiments, the 3'-UTR is derived from a globin gene or mRNA, such as a gene or mRNA of alpha2-globin, alpha1-globin, or beta-globin, e.g., beta-globin, e.g., human beta-globin. For example, the RNA (e.g., mRNA) may be modified by the replacement of the existing 3'-UTR with or the insertion of one or more, e.g., two copies of a 3'-UTR derived from a globin gene, such as alpha2-globin, alpha1-globin, beta-globin, e.g., beta-globin, e.g., human beta-globin.

A particularly preferred 5'-UTR comprises the nucleotide sequence of SEQ ID NO: 6. A particularly preferred 3'-UTR comprises the nucleotide sequence of SEQ ID NO: 7.

In some embodiments, RNA comprises a 5'-UTR comprising the nucleotide sequence of SEQ ID NO: 6, or a nucleotide sequence having at least 99%, 98%, 97%, 96%, 95%, 90%, 85%, or 80% identity to the nucleotide sequence of SEQ ID NO: 6.

In some embodiments, RNA comprises a 3'-UTR comprising the nucleotide sequence of SEQ ID NO: 7, or a nucleotide sequence having at least 99%, 98%, 97%, 96%, 95%, 90%, 85%, or 80% identity to the nucleotide sequence of SEQ ID NO: 7.

The RNA (in particular, mRNA) described herein may have modified ribonucleotides in order to increase its stability and/or decrease immunogenicity and/or decrease cytotoxicity. For example, in some embodiments, uridine in the RNA (in particular, mRNA) described herein is replaced (partially or completely, preferably completely) by a modified nucleoside. In some embodiments, the modified nucleoside is a modified uridine.

In some embodiments, the modified uridine replacing uridine is selected from the group consisting of pseudouridine (ψ), N1-methyl-pseudouridine (m1ψ), 5-methyl-uridine (m5U), and combinations thereof.

In some embodiments, the modified nucleoside replacing (partially or completely, preferably completely) uridine in the mRNA may be any one or more of 3-methyl-uridine (m3U), 5-methoxy-uridine (mo5U), 5-aza-uridine, 6-aza-uridine, 2-thio-5-aza-uridine, 2-thio-uridine (s2U), 4-thio-uridine (s4U), 4-thio-pseudouridine, 2-thio-pseudouridine, 5-hydroxy-uridine (ho5U), 5-aminoallyl-uridine, 5-halo-uridine *(e.g.,* 5-iodo-uridineor 5-bromo-uridine), uridine 5-oxyacetic acid (cmo5U), uridine 5-oxyacetic acid methyl ester (mcmo5U), 5-carboxymethyl-uridine (cm5U), 1-carboxymethyl-pseudouridine, 5-carboxyhydroxymethyl-uridine (chm5U), 5-carboxyhydroxymethyl-uridine methyl ester (mchm5U), 5-methoxycarbonylmethyl-uridine (mcm5U), 5-methoxycarbonylmethyl-2-thio-uridine (mcm5s2U), 5-aminomethyl-2-thio-uridine (nm5s2U), 5-methylaminomethyl-uridine (mnm5U), 1-ethyl-pseudouridine, 5-methylaminomethyl-2-thio-uridine (mnm5s2U), 5-methylaminomethyl-2-seleno-uridine (mnm5se2U), 5-carbamoylmethyl-uridine (ncm5U), 5-carboxymethylaminomethyl-uridine (cmnm5U), 5-carboxymethylaminomethyl-2-thio-uridine (cmnm5s2U), 5-propynyl-uridine, 1-propynyl-pseudouridine, 5-taurinomethyl-uridine (τm5U), 1-taurinomethyl-pseudouridine, 5-taurinomethyl-2-thio-uridine(τm5s2U), 1-taurinomethyl-4-thio-pseudouridine), 5-methyl-2-thio-uridine (m5s2U), 1-methyl-4-thio-pseudouridine (m1s4ψ), 4-thio-1-methyl-pseudouridine, 3-methyl-pseudouridine (m3ψ), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine (m5D), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine (acp3U), 1-methyl-3-(3-amino-3-carboxypropyl)pseudouridine (acp3 ψ), 5-(isopentenylaminomethyl)uridine (inm5U), 5-(isopentenylaminomethyl)-2-thio-uridine (inm5s2U), α-thio-uridine, 2'-O-methyl-uridine (Um), 5,2'-O-dimethyl-uridine (m5Um), 2'-O-methyl-pseudouridine (ψm), 2-thio-2'-O-methyl-uridine (s2Um), 5-methoxycarbonylmethyl-2'-O-methyl-uridine (mcm5Um), 5-carbamoylmethyl-2'-O-methyl-uridine (ncm5Um), 5-carboxymethylaminomethyl-2'-O-methyl-uridine (cmnm5Um), 3,2'-O-dimethyl-uridine (m3Um), 5-(isopentenylaminomethyl)-2'-O-methyl-uridine (inm5Um), 1-thio-uridine, deoxythymidine, 2'-F-ara-uridine, 2'-F-uridine, 2'-OH-ara-uridine, 5-(2-carbomethoxyvinyl) uridine, 5-[3-(1-E-propenylamino)uridine, or any other modified uridine known in the art.

An RNA (preferably mRNA) which is modified by pseudouridine (replacing partially or completely, preferably completely, uridine) is referred to herein as "Ψ-modified", whereas the term "m1Ψ-modified" means that the RNA (preferably mRNA) contains N(1)-methylpseudouridine (replacing partially or completely, preferably completely, uridine). Furthermore, the term "m5U-modified" means that the RNA (preferably mRNA) contains 5-methyluridine (replacing partially or completely, preferably completely, uridine). Such Ψ- or m1Ψ- or m5U-modified RNAs usually exhibit decreased immunogenicity compared to their unmodified forms and, thus, are preferred in applications where the induction of an immune response is to be avoided or minimized. In some embodiments, the RNA (preferably mRNA) contains N(1)-methylpseudouridine replacing completely uridine.

The codons of the RNA (in particular, mRNA) described in the present disclosure may further be optimized, e.g., to increase the GC content of the RNA and/or to replace codons which are rare in the cell (or subject) in which the peptide or polypeptide of interest is to be expressed by codons which are synonymous frequent codons in said cell (or subject). In some embodiments, the amino acid sequence encoded by the RNA (in particular, mRNA) described in the present disclosure is encoded by a coding sequence which is codon-optimized and/or the G/C content of which is increased compared to wild type coding sequence. This also includes embodiments, wherein one or more sequence regions of the coding sequence are codon-optimized and/or increased in the G/C content compared to the corresponding sequence regions of the wild type coding sequence. In some embodiments, the codon-optimization and/or the increase in the G/C content preferably does not change the sequence of the encoded amino acid sequence.

The term "codon-optimized" refers to the alteration of codons in the coding region of a nucleic acid molecule to reflect the typical codon usage of a host organism without preferably altering the amino acid sequence encoded by the nucleic acid molecule. Within the context of the present disclosure, coding regions may be codon-optimized for optimal expression in a subject to be treated using the RNA (in particular, mRNA) described herein. Codon-optimization is based on the finding that the translation efficiency is also determined by a different frequency in the occurrence of tRNAs in cells. Thus, the sequence of RNA (in particular, mRNA) may be modified such that codons for which frequently occurring tRNAs are available are inserted in place of "rare codons".

In some embodiments, the guanosine/cytosine (G/C) content of the coding region of the RNA (in particular, mRNA) described herein is increased compared to the G/C content of the corresponding coding sequence of the wild type RNA, wherein the amino acid sequence encoded by the RNA is preferably not modified compared to the amino acid sequence encoded by the wild type RNA. This modification of the RNA sequence is based on the fact that the sequence of any RNA region to be translated is important for efficient translation of that RNA. Sequences having an increased G

(guanosine)/C (cytosine) content are more stable than sequences having an increased A (adenosine)/U (uracil) content. In respect to the fact that several codons code for one and the same amino acid (so-called degeneration of the genetic code), the most favorable codons for the stability can be determined (so-called alternative codon usage). Depending on the amino acid to be encoded by the RNA, there are various possibilities for modification of the RNA sequence, compared to its wild type sequence. In particular, codons which contain A and/or U nucleotides can be modified by substituting these codons by other codons, which code for the same amino acids but contain no A and/or U or contain a lower content of A and/or U nucleotides.

In various embodiments, the G/C content of the coding region of the RNA (in particular, mRNA) described herein is increased by at least 10%, at least 20%, at least 30%, at least 40%, at least 50%, at least 55%, or even more compared to the G/C content of the coding region of the wild type RNA.

A combination of the above described modifications, *i.e.,* incorporation of a 5'-cap structure, incorporation of a poly-A sequence, unmasking of a poly-A sequence, alteration of the 5'-and/or 3'-UTR (such as incorporation of one or more 3'-UTRs), replacing one or more naturally occurring nucleotides with synthetic nucleotides *(e.g.,* 5-methylcytidine for cytidine and/or pseudouridine (Ψ) or N(1)-methylpseudouridine (m1Ψ) or 5-methyluridine (m5U) for uridine), and codon optimization, has a synergistic influence on the stability of RNA (preferably mRNA) and increase in translation efficiency. Thus, in some embodiments, the RNA (in particular, mRNA) described in the present disclosure contains a combination of at least two, at least three, at least four or all five of the above-mentioned modifications, *i.e.,* (i) incorporation of a 5'-cap structure, (ii) incorporation of a poly-A sequence, unmasking of a poly-A sequence; (iii) alteration of the 5'- and/or 3'-UTR (such as incorporation of one or more 3'-UTRs); (iv) replacing one or more naturally occurring nucleotides with synthetic nucleotides *(e.g., 5*-methylcytidine for cytidine and/or pseudouridine (Ψ) or N(1)-methylpseudouridine (m1Ψ) or 5-methyluridine (m5U) for uridine), and (v) codon optimization.

Some aspects of the disclosure involve the targeted delivery of the mRNA disclosed herein to certain cells or tissues. In some embodiments, the disclosure involves targeting the lymphatic system, in particular secondary lymphoid organs, more specifically spleen. Targeting the lymphatic system, in particular secondary lymphoid organs, more specifically spleen is in particular preferred if the RNA (in particular, mRNA) administered is RNA (in particular, mRNA) encoding an antigen or epitope for inducing an immune response. In some embodiments, the target cell is a spleen cell. In some embodiments, the target cell is an antigen presenting cell such as a professional antigen presenting cell in the spleen. In some embodiments, the target cell is a dendritic cell in the spleen. The "lymphatic system" is part of the circulatory system and an important part of the immune system, comprising a network of lymphatic vessels that carry lymph. The lymphatic system consists of lymphatic organs, a conducting network of lymphatic vessels, and the circulating lymph. The primary or central lymphoid organs generate lymphocytes from immature progenitor cells. The thymus and the bone marrow constitute the primary lymphoid organs. Secondary or peripheral lymphoid organs, which include lymph nodes and the spleen, maintain mature naive lymphocytes and initiate an adaptive immune response.

Lipid-based mRNA delivery systems have an inherent preference to the liver, where, depending on the composition of the mRNA delivery sytems used, mRNA expression in the liver can be obtained. Liver accumulation is caused by the discontinuous nature of the hepatic vasculature or the lipid metabolism (liposomes and lipid or cholesterol conjugates). In some embodiments, the target organ for mRNA expression is liver and the target tissue is liver tissue. The delivery to such target tissue is preferred, in particular, if presence of mRNA or of the encoded peptide or polypeptide in this organ or tissue is desired and/or if it is desired to express large amounts of the encoded peptide or polypeptide and/or if systemic presence of the encoded peptide or polypeptide, in particular in significant amounts, is desired or required.

In some embodiments, after administration of the RNA (in particular, mRNA) compositions/formulations described herein, at least a portion of the RNA is delivered to a target cell or target organ. In some embodiments, at least a portion of the RNA is delivered to the cytosol of the target cell. In some embodiments, the RNA is RNA (in particular, mRNA) encoding a peptide or polypeptide and the RNA is translated by the target cell to produce the peptide or polypeptide. In some embodiments, the target cell is a cell in the liver. In some embodiments, the target cell is a muscle cell. In some embodiments, the target cell is an endothelial cell. In some embodiments the target cell is a tumor cell or a cell in the tumor microenvironment. In some embodiments, the target cell is a blood cell. In some embodiments, the target cell is a cell in the lymph nodes. In some embodiments, the target cell is a cell in the lung. In some embodiments, the target cell is a cell in the skin. In some embodiments, the target cell is a spleen cell. In some embodiments, the target cell is an antigen presenting cell such as a professional antigen presenting cell in the spleen. In some embodiments, the target cell is a dendritic cell in the spleen. In some embodiments, the target cell is a T cell. In some embodiments, the target cell is a B cell. In some embodiments, the target cell is a NK cell. In some embodiments, the target cell is a monocyte. Thus, RNA (in particular, mRNA) compositions/formulations described herein may be used for delivering RNA to such target cell.

Pharmaceutically active peptides or polypeptides

"Encoding" refers to the inherent property of specific sequences of nucleotides in a polynucleotide, such as a gene, a cDNA, or an RNA (in particular, mRNA), to serve as templates for synthesis of other polymers and macromolecules

in biological processes having either a defined sequence of nucleotides *(i.e.,* rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the noncoding strand, used as the template for transcription of a gene or cDNA, can be referred to as encoding the protein or other product of that gene or cDNA.

In some embodiments, RNA (in particular, mRNA) described in the present disclosure comprises a nucleic acid sequence encoding a peptide or polypeptide, e.g., a pharmaceutically active peptide or polypeptide.

In some embodiments, RNA (in particular, mRNA) described in the present disclosure comprises a nucleic acid sequence encoding a peptide or polypeptide, preferably a pharmaceutically active peptide or polypeptide, and is capable of expressing said peptide or polypeptide, in particular if transferred into a cell or subject. Thus, in some embodiments, the RNA (in particular, mRNA) described in the present disclosure contains a coding region (open reading frame (ORF)) encoding a peptide or polypeptide, e.g., encoding a pharmaceutically active peptide or polypeptide. In this respect, an "open reading frame" or "ORF" is a continuous stretch of codons beginning with a start codon and ending with a stop codon. Such nucleic acid encoding a pharmaceutically active peptide or polypeptide is also referred to herein as "pharmaceutically active nucleic acid". In particular, such RNA encoding a pharmaceutically active peptide or polypeptide is also referred to herein as "pharmaceutically active RNA" and such mRNA encoding a pharmaceutically active peptide or polypeptide is also referred to herein as "pharmaceutically active mRNA". In some embodiments, RNA used in the present disclosure comprises a nucleic acid sequence encoding more than one peptide or polypeptide, e.g., two, three, four or more peptides or polypeptides.

According to the present disclosure, the term "pharmaceutically active peptide or polypeptide" means a peptide or polypeptide that can be used in the treatment of an individual where the expression of the peptide or polypeptide would be of benefit, e.g., in ameliorating the symptoms of a disease. Preferably, a pharmaceutically active peptide or polypeptide has curative or palliative properties and may be administered to ameliorate, relieve, alleviate, reverse, delay onset of or lessen the severity of one or more symptoms of a disease. In some embodiments, a pharmaceutically active peptide or polypeptide has a positive or advantageous effect on the condition or disease state of an individual when administered to the individual in a therapeutically effective amount. A pharmaceutically active peptide or polypeptide may have prophylactic properties and may be used to delay the onset of a disease or to lessen the severity of such disease. The term "pharmaceutically active peptide or polypeptide" includes entire peptides or polypeptides, and can also refer to pharmaceutically active fragments thereof. It can also include pharmaceutically active variants and/or analogs of a peptide or polypeptide.

Specific examples of pharmaceutically active peptides and polypeptides include, but are not limited to, immunostimulants, e.g., cytokines, hormones, adhesion molecules, immunoglobulins, immunologically active compounds, growth factors, protease inhibitors, enzymes, receptors, apoptosis regulators, transcription factors, tumor suppressor proteins, structural proteins, reprogramming factors, genomic engineering proteins, and blood proteins. In some embodiments, the pharmaceutically active peptide and polypeptide includes a replacement protein.

An "immunostimulant" is any substance that stimulates the immune system by inducing activation or increasing activity of any of the immune system's components, in particular immune effector cells. The immunostimulant may be pro-inflammatory (e.g., when treating infections or cancer), or anti-inflammatory (e.g., when treating autoimmune diseases). According to one aspect, the immunostimulant is a cytokine or a variant thereof. Examples of cytokines include interferons, such as interferon-alpha (IFN-$\alpha$) or interferon-gamma (IFN-y), interleukins, such as IL2, IL7, IL12, IL15 and IL23, colony stimulating factors, such as M-CSF and GM-CSF, and tumor necrosis factor. According to another aspect, the immunostimulant includes an adjuvant-type immunostimulatory agent such as APC Toll-like Receptor agonists or costimulatory/cell adhesion membrane proteins. Examples of Toll-like Receptor agonists include costimulatory/adhesion proteins such as CD80, CD86, and ICAM-1.

The term "cytokines" relates to proteins which have a molecular weight of about 5 to 60 kDa and which participate in cell signaling (e.g., paracrine, endocrine, and/or autocrine signaling). In particular, when released, cytokines exert an effect on the behavior of cells around the place of their release. Examples of cytokines include lymphokines, interleukins, chemokines, interferons, and tumor necrosis factors (TNFs). According to the present disclosure, cytokines do not include hormones or growth factors. Cytokines differ from hormones in that (i) they usually act at much more variable concentrations than hormones and (ii) generally are made by a broad range of cells (nearly all nucleated cells can produce cytokines). Interferons are usually characterized by antiviral, antiproliferative and immunomodulatory activities. Interferons are proteins that alter and regulate the transcription of genes within a cell by binding to interferon receptors on the regulated cell's surface, thereby preventing viral replication within the cells. The interferons can be grouped into two types. Particular examples of cytokines include erythropoietin (EPO), colony stimulating factor (CSF), granulocyte colony stimulating factor (G-CSF), granulocyte-macrophage colony stimulating factor (GM-CSF), tumor necrosis factor (TNF), bone morphogenetic protein (BMP), interferon alfa (IFN$\alpha$), interferon beta (IFN$\beta$), interferon gamma (INFy), interleukin 2 (IL-2), interleukin 4 (IL-4), interleukin 10 (IL-10), interleukin 11 (IL-11), interleukin 12 (IL-12), interleukin 15 (IL-15), and

interleukin 21 (IL-21), as well as variants and derivatives thereof.

According to the disclosure, a cytokine may be a naturally occurring cytokine or a functional fragment or variant thereof. A cytokine may be human cytokine and may be derived from any vertebrate, especially any mammal. One particularly preferred cytokine is interferon-a. Immunostimulants may be provided to a subject by administering to the subject RNA encoding an immunostimulant in a formulation for preferential delivery of RNA to liver or liver tissue. The delivery of RNA to such target organ or tissue is preferred, in particular, if it is desired to express large amounts of the immunostimulant and/or if systemic presence of the immunostimulant, in particular in significant amounts, is desired or required.

RNA delivery systems have an inherent preference to the liver. This pertains to lipid-based particles, cationic and neutral nanoparticles, in particular lipid nanoparticles.

Examples of suitable immunostimulants for targeting liver are cytokines involved in T cell proliferation and/or maintenance. Examples of suitable cytokines include IL2 or IL7, fragments and variants thereof, and fusion proteins of these cytokines, fragments and variants, such as extended-PK cytokines.

In another embodiment, RNA encoding an immunostimulant may be administered in a formulation for preferential delivery of RNA to the lymphatic system, in particular secondary lymphoid organs, more specifically spleen. The delivery of an immunostimulant to such target tissue is preferred, in particular, if presence of the immunostimulant in this organ or tissue is desired (e.g., for inducing an immune response, in particular in case immunostimulants such as cytokines are required during T-cell priming or for activation of resident immune cells), while it is not desired that the immunostimulant is present systemically, in particular in significant amounts (e.g., because the immunostimulant has systemic toxicity).

Examples of suitable immunostimulants are cytokines involved in T cell priming. Examples of suitable cytokines include IL12, IL15, IFN-$\alpha$, or IFN-$\beta$, fragments and variants thereof, and fusion proteins of these cytokines, fragments and variants, such as extended-PK cytokines. Interferons (IFNs) are a group of signaling proteins made and released by host cells in response to the presence of several pathogens, such as viruses, bacteria, parasites, and also tumor cells.

In a typical scenario, a virus-infected cell will release interferons causing nearby cells to heighten their anti-viral defenses.

Based on the type of receptor through which they signal, interferons are typically divided among three classes: type I interferon, type II interferon, and type III interferon.

All type I interferons bind to a specific cell surface receptor complex known as the IFN-$\alpha/\beta$ receptor (IFNAR) that consists of IFNAR1 and IFNAR2 chains.

The type I interferons present in humans are IFN$\alpha$, IFN$\beta$, IFN$\varepsilon$, IFN$\kappa$ and IFN$\omega$. In general, type I interferons are produced when the body recognizes a virus that has invaded it. They are produced by fibroblasts and monocytes. Once released, type I interferons bind to specific receptors on target cells, which leads to expression of proteins that will prevent the virus from producing and replicating its RNA and DNA.

The IFN$\alpha$ proteins are produced mainly by plasmacytoid dendritic cells (pDCs). They are mainly involved in innate immunity against viral infection. The genes responsible for their synthesis come in 13 subtypes that are called IFNA1, IFNA2, IFNA4, IFNA5, IFNA6, IFNA7, IFNA8, IFNA10, IFNA13, IFNA14, IFNA16, IFNA17, IFNA21. These genes are found together in a cluster on chromosome 9.

The IFN$\beta$ proteins are produced in large quantities by fibroblasts. They have antiviral activity that is involved mainly in innate immune response. Two types of IFN$\beta$ have been described, IFN$\beta$1 and IFN$\beta$3. The natural and recombinant forms of IFN$\beta$1 have antiviral, antibacterial, and anticancer properties.

Type II interferon (IFNy in humans) is also known as immune interferon and is activated by IL12. Furthermore, type II interferons are released by cytotoxic T cells and T helper cells.

Type III interferons signal through a receptor complex consisting of IL10R2 (also called CRF2-4) and IFNLR1 (also called CRF2-12). Although discovered more recently than type I and type II IFNs, recent information demonstrates the importance of type III IFNs in some types of virus or fungal infections.

In general, type I and II interferons are responsible for regulating and activating the immune response.

According to the disclosure, a type I interferon is preferably IFN$\alpha$ or IFN$\beta$, more preferably IFN$\alpha$.

According to the disclosure, an interferon may be a naturally occurring interferon or a functional fragment or variant thereof. An interferon may be human interferon and may be derived from any vertebrate, especially any mammal.

Interleukins (ILs) are a group of cytokines (secreted proteins and signal molecules) that can be divided into four major groups based on distinguishing structural features. However, their amino acid sequence similarity is rather weak (typically 15-25% identity). The human genome encodes more than 50 interleukins and related proteins.

According to the disclosure, an interleukin may be a naturally occurring interleukin or a functional fragment or variant thereof. An interleukin may be human interleukin and may be derived from any vertebrate, especially any mammal.

Immunostimulant polypeptides described herein can be prepared as fusion or chimeric polypeptides that include an immunostimulant portion and a heterologous polypeptide (i.e., a polypeptide that is not an immunostimulant). The immunostimulant may be fused to an extended-PK group, which increases circulation half-life. Non-limiting examples of

extended-PK groups are described infra. It should be understood that other PK groups that increase the circulation half-life of immunostimulants such as cytokines, or variants thereof, are also applicable to the present disclosure. In certain embodiments, the extended-PK group is a serum albumin domain (e.g., mouse serum albumin, human serum albumin).

As used herein, the term "PK" is an acronym for "pharmacokinetic" and encompasses properties of a compound including, by way of example, absorption, distribution, metabolism, and elimination by a subject. As used herein, an "extended-PK group" refers to a protein, peptide, or moiety that increases the circulation half-life of a biologically active molecule when fused to or administered together with the biologically active molecule. Examples of an extended-PK group include serum albumin (e.g., HSA), Immunoglobulin Fc or Fc fragments and variants thereof, transferrin and variants thereof, and human serum albumin (HSA) binders (as disclosed in U.S. Publication Nos. 2005/0287153 and 2007/0003549). Other exemplary extended-PK groups are disclosed in Kontermann, Expert Opin Biol Ther, 2016 Jul;16(7):903-15.

As used herein, an "extended-PK" immunostimulant refers to an immunostimulant moiety in combination with an extended-PK group. In some embodiments, the extended-PK immunostimulant is a fusion protein in which an immunostimulant moiety is linked or fused to an extended-PK group.

In certain embodiments, the serum half-life of an extended-PK immunostimulant is increased relative to the immunostimulant alone (i.e., the immunostimulant not fused to an extended-PK group). In certain embodiments, the serum half-life of the extended-PK immunostimulant is at least 20%, at least 40%, at least 60%, at least 80%, at least 100%, at least 120%, at least 150%, at least 180%, at least 200%, at least 400%, at least 600%, at least 800%, or at least 1000% longer relative to the serum half-life of the immunostimulant alone. In certain embodiments, the serum half-life of the extended-PK immunostimulant is at least 1.5-fold, 2-fold, 2.5-fold, 3-fold, 3.5-fold, 4-fold, 4.5-fold, 5-fold, 6-fold, 7-fold, 8-fold, 10-fold, 12-fold, 13-fold, 15-fold, 17-fold, 20-fold, 22-fold, 25-fold, 27-fold, 30-fold, 35-fold, 40-fold, or 50-fold greater than the serum half-life of the immunostimulant alone. In certain embodiments, the serum half-life of the extended-PK immunostimulant is at least 10 hours, 15 hours, 20 hours, 25 hours, 30 hours, 35 hours, 40 hours, 50 hours, 60 hours, 70 hours, 80 hours, 90 hours, 100 hours, 110 hours, 120 hours, 130 hours, 135 hours, 140 hours, 150 hours, 160 hours, or 200 hours.

As used herein, "half-life" refers to the time taken for the serum or plasma concentration of a compound such as a peptide or polypeptide to reduce by 50%, *in vivo,* for example due to degradation and/or clearance or sequestration by natural mechanisms. An extended-PK immunostimulant suitable for use herein is stabilized *in vivo* and its half-life increased by, e.g., fusion to serum albumin (e.g., HSA or MSA), which resist degradation and/or clearance or sequestration. The half-life can be determined in any manner known per se, such as by pharmacokinetic analysis. Suitable techniques will be clear to the person skilled in the art, and may for example generally involve the steps of suitably administering a suitable dose of the amino acid sequence or compound to a subject; collecting blood samples or other samples from said subject at regular intervals; determining the level or concentration of the amino acid sequence or compound in said blood sample; and calculating, from (a plot of) the data thus obtained, the time until the level or concentration of the amino acid sequence or compound has been reduced by 50% compared to the initial level upon dosing. Further details are provided in, e.g., standard handbooks, such as Kenneth, A. et al., Chemical Stability of Pharmaceuticals: A Handbook for Pharmacists and in Peters et al., Pharmacokinetic Analysis: A Practical Approach (1996). Reference is also made to Gibaldi, M. et al., Pharmacokinetics, 2nd Rev. Edition, Marcel Dekker (1982).

In certain embodiments, the extended-PK group includes serum albumin, or fragments thereof or variants of the serum albumin or fragments thereof (all of which for the purpose of the present disclosure are comprised by the term "albumin"). Polypeptides described herein may be fused to albumin (or a fragment or variant thereof) to form albumin fusion proteins. Such albumin fusion proteins are described in U.S. Publication No. 20070048282.

As used herein, "albumin fusion protein" refers to a protein formed by the fusion of at least one molecule of albumin (or a fragment or variant thereof) to at least one molecule of a protein such as a therapeutic protein, in particular an immunostimulant. The albumin fusion protein may be generated by translation of a nucleic acid in which a polynucleotide encoding a therapeutic protein is joined in-frame with a polynucleotide encoding an albumin. The therapeutic protein and albumin, once part of the albumin fusion protein, may each be referred to as a "portion", "region" or "moiety" of the albumin fusion protein (e.g., a "therapeutic protein portion" or an "albumin protein portion"). In a highly preferred embodiment, an albumin fusion protein comprises at least one molecule of a therapeutic protein (including, but not limited to a mature form of the therapeutic protein) and at least one molecule of albumin (including but not limited to a mature form of albumin). In some embodiments, an albumin fusion protein is processed by a host cell such as a cell of the target organ for administered RNA, e.g. a liver cell, and secreted into the circulation. Processing of the nascent albumin fusion protein that occurs in the secretory pathways of the host cell used for expression of the RNA may include, but is not limited to signal peptide cleavage; formation of disulfide bonds; proper folding; addition and processing of carbohydrates (such as for example, N- and O-linked glycosylation); specific proteolytic cleavages; and/or assembly into multimeric proteins. An albumin fusion protein is preferably encoded by RNA in a non-processed form which in particular has a signal peptide at its N-terminus and following secretion by a cell is preferably present in the processed form wherein in particular the signal peptide has been cleaved off. In a most preferred embodiment, the "processed form of an albumin fusion protein" refers to an albumin fusion

protein product which has undergone N-terminal signal peptide cleavage, herein also referred to as a "mature albumin fusion protein". In preferred embodiments, albumin fusion proteins comprising a therapeutic protein have a higher plasma stability compared to the plasma stability of the same therapeutic protein when not fused to albumin. Plasma stability typically refers to the time period between when the therapeutic protein is administered *in vivo* and carried into the bloodstream and when the therapeutic protein is degraded and cleared from the bloodstream, into an organ, such as the kidney or liver, that ultimately clears the therapeutic protein from the body. Plasma stability is calculated in terms of the half-life of the therapeutic protein in the bloodstream. The half-life of the therapeutic protein in the bloodstream can be readily determined by common assays known in the art.

As used herein, "albumin" refers collectively to albumin protein or amino acid sequence, or an albumin fragment or variant, having one or more functional activities (e.g., biological activities) of albumin. In particular, "albumin" refers to human albumin or fragments or variants thereof especially the mature form of human albumin, or albumin from other vertebrates or fragments thereof, or variants of these molecules. The albumin may be derived from any vertebrate, especially any mammal, for example human, cow, sheep, or pig. Non-mammalian albumins include, but are not limited to, hen and salmon. The albumin portion of the albumin fusion protein may be from a different animal than the therapeutic protein portion.

In certain embodiments, the albumin is human serum albumin (HSA), or fragments or variants thereof, such as those disclosed in US 5,876,969, WO 2011/124718, WO 2013/075066, and WO 2011/0514789.

The terms, human serum albumin (HSA) and human albumin (HA) are used interchangeably herein. The terms, "albumin and "serum albumin" are broader, and encompass human serum albumin (and fragments and variants thereof) as well as albumin from other species (and fragments and variants thereof).

As used herein, a fragment of albumin sufficient to prolong the therapeutic activity or plasma stability of the therapeutic protein refers to a fragment of albumin sufficient in length or structure to stabilize or prolong the therapeutic activity or plasma stability of the protein so that the plasma stability of the therapeutic protein portion of the albumin fusion protein is prolonged or extended compared to the plasma stability in the non-fusion state.

The albumin portion of the albumin fusion proteins may comprise the full length of the albumin sequence, or may include one or more fragments thereof that are capable of stabilizing or prolonging the therapeutic activity or plasma stability. Such fragments may be of 10 or more amino acids in length or may include about 15, 20, 25, 30, 50, or more contiguous amino acids from the albumin sequence or may include part or all of specific domains of albumin. For instance, one or more fragments of HSA spanning the first two immunoglobulin-like domains may be used. In a preferred embodiment, the HSA fragment is the mature form of HSA.

Generally speaking, an albumin fragment or variant will be at least 100 amino acids long, preferably at least 150 amino acids long.

According to the disclosure, albumin may be naturally occurring albumin or a fragment or variant thereof. Albumin may be human albumin and may be derived from any vertebrate, especially any mammal.

Preferably, the albumin fusion protein comprises albumin as the N-terminal portion, and a therapeutic protein as the C-terminal portion. Alternatively, an albumin fusion protein comprising albumin as the C-terminal portion, and a therapeutic protein as the N-terminal portion may also be used. In other embodiments, the albumin fusion protein has a therapeutic protein fused to both the N-terminus and the C-terminus of albumin. In a preferred embodiment, the therapeutic proteins fused at the N- and C-termini are the same therapeutic proteins. In another preferred embodiment, the therapeutic proteins fused at the N- and C-termini are different therapeutic proteins. In some embodiments, the different therapeutic proteins are both cytokines.

In some embodiments, the therapeutic protein(s) is (are) joined to the albumin through (a) peptide linker(s). A peptide linker between the fused portions may provide greater physical separation between the moieties and thus maximize the accessibility of the therapeutic protein portion, for instance, for binding to its cognate receptor. The peptide linker may consist of amino acids such that it is flexible or more rigid. The linker sequence may be cleavable by a protease or chemically.

As used herein, the term "Fc region" refers to the portion of a native immunoglobulin formed by the respective Fc domains (or Fc moieties) of its two heavy chains. As used herein, the term "Fc domain" refers to a portion or fragment of a single immunoglobulin (Ig) heavy chain wherein the Fc domain does not comprise an Fv domain. In certain embodiments, an Fc domain begins in the hinge region just upstream of the papain cleavage site and ends at the C-terminus of the antibody. Accordingly, a complete Fc domain comprises at least a hinge domain, a CH2 domain, and a CH3 domain. In certain embodiments, an Fc domain comprises at least one of: a hinge (e.g., upper, middle, and/or lower hinge region) domain, a CH2 domain, a CH3 domain, a CH4 domain, or a variant, portion, or fragment thereof. In certain embodiments, an Fc domain comprises a complete Fc domain (i.e., a hinge domain, a CH2 domain, and a CH3 domain). In certain embodiments, an Fc domain comprises a hinge domain (or portion thereof) fused to a CH3 domain (or portion thereof). In certain embodiments, an Fc domain comprises a CH2 domain (or portion thereof) fused to a CH3 domain (or portion thereof). In certain embodiments, an Fc domain consists of a CH3 domain or portion thereof. In certain embodiments, an Fc domain consists of a hinge domain (or portion thereof) and a CH3 domain (or portion thereof). In certain embodiments, an

Fc domain consists of a CH2 domain (or portion thereof) and a CH3 domain. In certain embodiments, an Fc domain consists of a hinge domain (or portion thereof) and a CH2 domain (or portion thereof). In certain embodiments, an Fc domain lacks at least a portion of a CH2 domain (e.g., all or part of a CH2 domain). An Fc domain herein generally refers to a polypeptide comprising all or part of the Fc domain of an immunoglobulin heavy-chain. This includes, but is not limited to, polypeptides comprising the entire CH1, hinge, CH2, and/or CH3 domains as well as fragments of such peptides comprising only, e.g., the hinge, CH2, and CH3 domain. The Fc domain may be derived from an immunoglobulin of any species and/or any subtype, including, but not limited to, a human IgG1, IgG2, IgG3, IgG4, IgD, IgA, IgE, or IgM antibody. The Fc domain encompasses native Fc and Fc variant molecules. As set forth herein, it will be understood by one of ordinary skill in the art that any Fc domain may be modified such that it varies in amino acid sequence from the native Fc domain of a naturally occurring immunoglobulin molecule. In certain embodiments, the Fc domain has reduced effector function (e.g., FcγR binding).

The Fc domains of a polypeptide described herein may be derived from different immunoglobulin molecules. For example, an Fc domain of a polypeptide may comprise a CH2 and/or CH3 domain derived from an IgG1 molecule and a hinge region derived from an IgG3 molecule. In another example, an Fc domain can comprise a chimeric hinge region derived, in part, from an IgG1 molecule and, in part, from an IgG3 molecule. In another example, an Fc domain can comprise a chimeric hinge derived, in part, from an IgG1 molecule and, in part, from an IgG4 molecule.

In certain embodiments, an extended-PK group includes an Fc domain or fragments thereof or variants of the Fc domain or fragments thereof (all of which for the purpose of the present disclosure are comprised by the term "Fc domain"). The Fc domain does not contain a variable region that binds to antigen. Fc domains suitable for use in the present disclosure may be obtained from a number of different sources. In certain embodiments, an Fc domain is derived from a human immunoglobulin. In certain embodiments, the Fc domain is from a human IgG1 constant region. It is understood, however, that the Fc domain may be derived from an immunoglobulin of another mammalian species, including for example, a rodent (e.g. a mouse, rat, rabbit, guinea pig) or non-human primate (e.g. chimpanzee, macaque) species. Moreover, the Fc domain (or a fragment or variant thereof) may be derived from any immunoglobulin class, including IgM, IgG, IgD, IgA, and IgE, and any immunoglobulin isotype, including IgG1, IgG2, IgG3, and IgG4.

A variety of Fc domain gene sequences (e.g., mouse and human constant region gene sequences) are available in the form of publicly accessible deposits. Constant region domains comprising an Fc domain sequence can be selected lacking a particular effector function and/or with a particular modification to reduce immunogenicity. Many sequences of antibodies and antibody-encoding genes have been published and suitable Fc domain sequences (e.g. hinge, CH2, and/or CH3 sequences, or fragments or variants thereof) can be derived from these sequences using art recognized techniques.

In certain embodiments, the extended-PK group is a serum albumin binding protein such as those described in US2005/0287153, US2007/0003549, US2007/0178082, US2007/0269422, US2010/0113339, WO2009/083804, and WO2009/133208.

In certain embodiments, the extended-PK group is transferrin, as disclosed in US 7,176,278 and US 8,158,579.

In certain embodiments, the extended-PK group is a serum immunoglobulin binding protein such as those disclosed in US2007/0178082, US2014/0220017, and US2017/0145062.

In certain embodiments, the extended-PK group is a fibronectin (Fn)-based scaffold domain protein that binds to serum albumin, such as those disclosed in US2012/0094909.

Methods of making fibronectin-based scaffold domain proteins are also disclosed in US2012/0094909. A non-limiting example of a Fn3-based extended-PK group is Fn3(HSA), i.e., a Fn3 protein that binds to human serum albumin.

In certain aspects, the extended-PK immunostimulant, suitable for use according to the disclosure, can employ one or more peptide linkers. As used herein, the term "peptide linker" refers to a peptide or polypeptide sequence which connects two or more domains (e.g., the extended-PK moiety and an immunostimulant moiety) in a linear amino acid sequence of a polypeptide chain. For example, peptide linkers may be used to connect an immunostimulant moiety to a HSA domain.

Linkers suitable for fusing the extended-PK group to, e.g., an immunostimulant are well known in the art. Exemplary linkers include glycine-serine-polypeptide linkers, glycine-proline-polypeptide linkers, and proline-alanine polypeptide linkers. In certain embodiments, the linker is a glycine-serine-polypeptide linker, i.e., a peptide that consists of glycine and serine residues.

In some embodiments, a pharmaceutically active peptide or polypeptide comprises a replacement protein. In these embodiments, the present disclosure provides a method for treatment of a subject having a disorder requiring protein replacement *(e.g.,* protein deficiency disorders) comprising administering to the subject RNA (in particular, mRNA) as described herein encoding a replacement protein. The term "protein replacement" refers to the introduction of a protein (including functional variants thereof) into a subject having a deficiency in such protein. The term also refers to the introduction of a protein into a subject otherwise requiring or benefiting from providing a protein, e.g., suffering from protein insufficiency. The term "disorder characterized by a protein deficiency" refers to any disorder that presents with a pathology caused by absent or insufficient amounts of a protein. This term encompasses protein folding disorders, *i.e.,* conformational disorders, that result in a biologically inactive protein product. Protein insufficiency can be involved in infectious diseases, immunosuppression, organ failure, glandular problems, radiation illness, nutritional deficiency, poisoning, or other environmental or external insults.

The term "hormones" relates to a class of signaling molecules produced by glands, wherein signaling usually includes the following steps: (i) synthesis of a hormone in a particular tissue; (ii) storage and secretion; (iii) transport of the hormone to its target; (iv) binding of the hormone by a receptor; (v) relay and amplification of the signal; and (vi) breakdown of the hormone. Hormones differ from cytokines in that (1) hormones usually act in less variable concentrations and (2) generally are made by specific kinds of cells. In some embodiments, a "hormone" is a peptide or polypeptide hormone, such as insulin, vasopressin, prolactin, adrenocorticotropic hormone (ACTH), thyroid hormone, growth hormones (such as human grown hormone or bovine somatotropin), oxytocin, atrial-natriuretic peptide (ANP), glucagon, somatostatin, cholecystokinin, gastrin, and leptins.

The term "adhesion molecules" relates to proteins which are located on the surface of a cell and which are involved in binding of the cell with other cells or with the extracellular matrix (ECM). Adhesion molecules are typically transmembrane receptors and can be classified as calcium-independent *(e.g.,* integrins, immunoglobulin superfamily, lymphocyte homing receptors) and calcium-dependent (cadherins and selectins). Particular examples of adhesion molecules are integrins, lymphocyte homing receptors, selectins *(e.g.,* P-selectin), and addressins.

Integrins are also involved in signal transduction. In particular, upon ligand binding, integrins modulate cell signaling pathways, *e.g.,* pathways of transmembrane protein kinases such as receptor tyrosine kinases (RTK). Such regulation can lead to cellular growth, division, survival, or differentiation or to apoptosis. Particular examples of integrins include: $\alpha_1\beta_1$, $\alpha_2\beta_1$, $\alpha_3\beta_1$, $\alpha_4\beta_1$, $\alpha_5\beta_1$, $\alpha_6\beta_1$, $\alpha_7\beta_1$, $\alpha_L\beta_2$, $\alpha_M\beta_2$, $\alpha_{IIb}\beta_3$, $\alpha_V\beta_1$, $\alpha_V\beta_3$, $\alpha_V\beta_5$, $\alpha_V\beta_6$, $\alpha_V\beta_8$, and $\alpha_6\beta_4$.

The term "immunoglobulins" or "immunoglobulin superfamily" refers to molecules which are involved in the recognition, binding, and/or adhesion processes of cells. Molecules belonging to this superfamily share the feature that they contain a region known as immunoglobulin domain or fold. Members of the immunoglobulin superfamily include antibodies (e.g., IgG), T cell receptors (TCRs), major histocompatibility complex (MHC) molecules, co-receptors *(e.g.,* CD4, CD8, CD19), antigen receptor accessory molecules (e.g., CD-3y, CD3-6, CD-3$\varepsilon$, CD79a, CD79b), co-stimulatory or inhibitory molecules (e.g., CD28, CD80, CD86), and other.

The term "immunologically active compound" relates to any compound altering an immune response, e.g., by inducing and/or suppressing maturation of immune cells, inducing and/or suppressing cytokine biosynthesis, and/or altering humoral immunity by stimulating antibody production by B cells. Immunologically active compounds possess potent immunostimulating activity including, but not limited to, antiviral and antitumor activity, and can also down-regulate other aspects of the immune response, for example shifting the immune response away from a TH2 immune response, which is useful for treating a wide range of TH2 mediated diseases. Immunologically active compounds can be useful as vaccine adjuvants. Particular examples of immunologically active compounds include interleukins, colony stimulating factor (CSF), granulocyte colony stimulating factor (G-CSF), granulocyte-macrophage colony stimulating factor (GM-CSF), erythropoietin, tumor necrosis factor (TNF), interferons, integrins, addressins, selectins, homing receptors, and antigens, in particular tumor-associated antigens, pathogen-associated antigens (such as bacterial, parasitic, or viral antigens), allergens, and autoantigens. An immunologically active compound may be a vaccine antigen, *i.e.,* an antigen whose inoculation into a subject induces an immune response.

In some embodiments, RNA (in particular, mRNA) described in the present disclosure comprises a nucleic acid sequence encoding a peptide or polypeptide comprising an epitope for inducing an immune response against an antigen in a subject. The "peptide or polypeptide comprising an epitope for inducing an immune response against an antigen in a subject" is also designated herein as "vaccine antigen", "peptide and protein antigen" or simply "antigen".

In some embodiments, the RNA (in particular, mRNA) encoding vaccine antigen is a single-stranded, 5' capped mRNA that is translated into the respective protein upon entering cells of a subject being administered the RNA, e.g., antigen-presenting cells (APCs). Preferably, the RNA (i) contains structural elements optimized for maximal efficacy of the RNA with respect to stability and translational efficiency (5' cap, 5' UTR, 3' UTR, poly(A) sequence); (ii) is modified for optimized efficacy of the RNA (e.g., increased translation efficacy, decreased immunogenicity, and/or decreased cytotoxicity) (e.g., by replacing (partially or completely, preferably completely) naturally occurring nucleosides (in particular cytidine) with synthetic nucleosides (e.g., modified nucleosides selected from the group consisting of pseudouridine ($\psi$), N1-methyl-pseudouridine (m1$\psi$), and 5-methyl-uridine); and/or codon-optimization), or (iii) both (i) and (ii).

In some embodiments, beta-S-ARCA(D1) is utilized as specific capping structure at the 5'-end of the RNA. In some embodiments, the 5'-UTR comprises the nucleotide sequence of SEQ ID NO: 6, or a nucleotide sequence having at least 99%, 98%, 97%, 96%, 95%, 90%, 85%, or 80% identity to the nucleotide sequence of SEQ ID NO: 6. In some embodiments, the 3'-UTR comprises the nucleotide sequence of SEQ ID NO: 7, or a nucleotide sequence having at least 99%, 98%, 97%, 96%, 95%, 90%, 85%, or 80% identity to the nucleotide sequence of SEQ ID NO: 7. In some embodiments, the poly(A) sequence is 110 nucleotides in length and consists of a stretch of 30 adenosine residues, followed by a 10 nucleotide linker sequence and another 70 adenosine residues. This poly(A) sequence was designed to enhance RNA stability and translational efficiency in dendritic cells. In some embodiments, the poly(A) sequence

comprises the nucleotide sequence of SEQ ID NO: 8, or a nucleotide sequence having at least 99%, 98%, 97%, 96%, 95%, 90%, 85%, or 80% identity to the nucleotide sequence of SEQ ID NO: 8. In some embodiments, the RNA comprises a modified nucleoside in place of uridine. In some embodiments, the modified nucleoside replacing (partially or completely, preferably completely) uridine is selected from the group consisting of pseudouridine (ψ), N1-methyl-pseudouridine (m1ψ), and 5-methyl-uridine. In some embodiments, the RNA encoding the vaccine antigen has a coding sequence (a) which is codon-optimized, (b) the G/C content of which is increased compared to the wild type coding sequence, or (c) both (a) and (b).

In some embodiments, the RNA encoding the vaccine antigen is expressed in cells of the subject to provide the vaccine antigen. In some embodiments, expression of the vaccine antigen is at the cell surface. In some embodiments, the vaccine antigen is presented in the context of MHC. In some embodiments, the RNA encoding the vaccine antigen is transiently expressed in cells of the subject. In some embodiments, the RNA encoding the vaccine antigen is administered systemically. In some embodiments, after systemic administration of the RNA encoding the vaccine antigen, expression of the RNA encoding the vaccine antigen in spleen occurs. In some embodiments, after systemic administration of the RNA encoding the vaccine antigen, expression of the RNA encoding the vaccine antigen in antigen presenting cells, preferably professional antigen presenting cells occurs. In some embodiments, the antigen presenting cells are selected from the group consisting of dendritic cells, macrophages and B cells. In some embodiments, after systemic administration of the RNA encoding the vaccine antigen, no or essentially no expression of the RNA encoding the vaccine antigen in lung and/or liver occurs. In some embodiments, after systemic administration of the RNA encoding the vaccine antigen, expression of the RNA encoding the vaccine antigen in spleen is at least 5-fold the amount of expression in lung.

The vaccine antigen comprises an epitope for inducing an immune response against an antigen in a subject. Accordingly, the vaccine antigen comprises an antigenic sequence for inducing an immune response against an antigen in a subject. Such antigenic sequence may correspond to a target antigen or disease-associated antigen, e.g., a protein of an infectious agent (e.g., viral or bacterial antigen) or tumor antigen, or may correspond to an immunogenic variant thereof, or an immunogenic fragment of the target antigen or disease-associated antigen or the immunogenic variant thereof. Thus, the antigenic sequence may comprise at least an epitope of a target antigen or disease-associated antigen or an immunogenic variant thereof. The antigenic sequences, e.g., epitopes, suitable for use according to the disclosure typically may be derived from a target antigen, i.e. the antigen against which an immune response is to be elicited. For example, the antigenic sequences contained within the vaccine antigen may be a target antigen or a fragment or variant of a target antigen.

The antigenic sequence or a procession product thereof, e.g., a fragment thereof, may bind to the antigen receptor such as TCR or CAR carried by immune effector cells. In some embodiments, the antigenic sequence is selected from the group consisting of the antigen expressed by a target cell to which the immune effector cells are targeted or a fragment thereof, or a variant of the antigenic sequence or the fragment.

A vaccine antigen which may be provided to a subject according to the present disclosure by administering RNA encoding the vaccine antigen, preferably results in the induction of an immune response, e.g., in the stimulation, priming and/or expansion of immune effector cells, in the subject being provided the vaccine antigen. Said immune response, e.g., stimulated, primed and/or expanded immune effector cells, is preferably directed against a target antigen, in particular a target antigen expressed by diseased cells, tissues and/or organs, i.e., a disease-associated antigen. Thus, a vaccine antigen may comprise the disease-associated antigen, or a fragment or variant thereof. In some embodiments, such fragment or variant is immunologically equivalent to the disease-associated antigen.

In the context of the present disclosure, the term "fragment of an antigen" or "variant of an antigen" means an agent which results in the induction of an immune response, e.g., in the stimulation, priming and/or expansion of immune effector cells, which immune response, e.g., stimulated, primed and/or expanded immune effector cells, targets the antigen, i.e. a disease-associated antigen, in particular when presented by diseased cells, tissues and/or organs. Thus, the vaccine antigen may correspond to or may comprise the disease-associated antigen, may correspond to or may comprise a fragment of the disease-associated antigen or may correspond to or may comprise an antigen which is homologous to the disease-associated antigen or a fragment thereof. If the vaccine antigen comprises a fragment of the disease-associated antigen or an amino acid sequence which is homologous to a fragment of the disease-associated antigen said fragment or amino acid sequence may comprise an epitope of the disease-associated antigen to which the antigen receptor of the immune effector cells is targeted or a sequence which is homologous to an epitope of the disease-associated antigen. Thus, according to the disclosure, a vaccine antigen may comprise an immunogenic fragment of a disease-associated antigen or an amino acid sequence being homologous to an immunogenic fragment of a disease-associated antigen. An "immunogenic fragment of an antigen" according to the disclosure preferably relates to a fragment of an antigen which is capable of inducing an immune response against, e.g., stimulating, priming and/or expanding immune effector cells carrying an antigen receptor binding to, the antigen or cells expressing the antigen. It is preferred that the vaccine antigen (similar to the disease-associated antigen) provides the relevant epitope for binding by the antigen receptor present on the immune effector cells. In some embodiments, the vaccine antigen or a fragment thereof (similar to the disease-associated antigen) is expressed on the surface of a cell such as an antigen-presenting cell (optionally in the

context of MHC) so as to provide the relevant epitope for binding by immune effector cells. The vaccine antigen may be a recombinant antigen.

In some embodiments of all aspects of the invention, the RNA encoding the vaccine antigen is expressed in cells of a subject to provide the antigen or a procession product thereof for binding by the antigen receptor expressed by immune effector cells, said binding resulting in stimulation, priming and/or expansion of the immune effector cells. An "antigen" according to the present disclosure covers any substance that will elicit an immune response and/or any substance against which an immune response or an immune mechanism such as a cellular response and/or humoral response is directed. This also includes situations wherein the antigen is processed into antigen peptides and an immune response or an immune mechanism is directed against one or more antigen peptides, in particular if presented in the context of MHC molecules. In particular, an "antigen" relates to any substance, such as a peptide or polypeptide, that reacts specifically with antibodies or T-lymphocytes (T-cells). The term "antigen" may comprise a molecule that comprises at least one epitope, such as a T cell epitope. In some embodiments, an antigen is a molecule which, optionally after processing, induces an immune reaction, which may be specific for the antigen (including cells expressing the antigen). In some embodiments, an antigen is a disease-associated antigen, such as a tumor antigen, a viral antigen, or a bacterial antigen, or an epitope derived from such antigen. In some embodiments, an antigen is presented or present on the surface of cells of the immune system such as antigen presenting cells like dendritic cells or macrophages. An antigen or a procession product thereof such as a T cell epitope is in some embodiments bound by an antigen receptor. Accordingly, an antigen or a procession product thereof may react specifically with immune effector cells such as T-lymphocytes (T cells).

The term "autoantigen" or "self-antigen" refers to an antigen which originates from within the body of a subject *(i.e.,* the autoantigen can also be called "autologous antigen") and which produces an abnormally vigorous immune response against this normal part of the body. Such vigorous immune reactions against autoantigens may be the cause of "autoimmune diseases".

According to the present disclosure, any suitable antigen may be used, which is a candidate for an immune response, wherein the immune response may comprise a humoral or cellular immune response, or both. In the context of some embodiments of the present disclosure, the antigen is presented by a cell, such as by an antigen presenting cell, in the context of MHC molecules, which results in an immune response against the antigen. An antigen may be a product which corresponds to or is derived from a naturally occurring antigen. Such naturally occurring antigens may include or may be derived from allergens, viruses, bacteria, fungi, parasites and other infectious agents and pathogens or an antigen may also be a tumor antigen. According to the present disclosure, an antigen may correspond to a naturally occurring product, for example, a viral protein, or a part thereof.

The term "disease-associated antigen" is used in its broadest sense to refer to any antigen associated with a disease. A disease-associated antigen is a molecule which contains epitopes that will stimulate a host's immune system to make a cellular antigen-specific immune response and/or a humoral antibody response against the disease. Disease-associated antigens include pathogen-associated antigens, *i.e.,* antigens which are associated with infection by microbes, typically microbial antigens (such as bacterial or viral antigens), or antigens associated with cancer, typically tumors, such as tumor antigens.

In some embodiments, the antigen is a tumor antigen, *i.e.,* a part of a tumor cell, in particular those which primarily occur intracellularly or as surface antigens of tumor cells. In another embodiment, the antigen is a pathogen-associated antigen, *i.e.,* an antigen derived from a pathogen, e.g., from a virus, bacterium, unicellular organism, or parasite, for example a viral antigen such as viral ribonucleoprotein or coat protein. In some embodiments, the antigen should be presented by MHC molecules which results in modulation, in particular activation of cells of the immune system, such as CD4+ and CD8+ lymphocytes, in particular via the modulation of the activity of a T-cell receptor.

The term "tumor antigen" or "tumor-associated antigen" refers to a constituent of cancer cells which may be derived from the cytoplasm, the cell surface or the cell nucleus. In particular, it refers to those antigens which are produced intracellularly or as surface antigens on tumor cells. For example, tumor antigens include the carcinoembryonal antigen, $\alpha$1-fetoprotein, isoferritin, and fetal sulphoglycoprotein, $\alpha$2-H-ferroprotein and $\gamma$-fetoprotein, as well as various virus tumor antigens. According to some embodiments of the present disclosure, a tumor antigen comprises any antigen which is characteristic for tumors or cancers as well as for tumor or cancer cells with respect to type and/or expression level.

The term "viral antigen" refers to any viral component having antigenic properties, i.e., being able to provoke an immune response in an individual. The viral antigen may be a viral ribonucleoprotein or an envelope protein.

The term "bacterial antigen" refers to any bacterial component having antigenic properties, *i.e.* being able to provoke an immune response in an individual. The bacterial antigen may be derived from the cell wall or cytoplasm membrane of the bacterium.

The term "epitope" refers to an antigenic determinant in a molecule such as an antigen, *i.e.,* to a part in or fragment of the molecule that is recognized by the immune system, for example, that is recognized by antibodies, T cells or B cells, in particular when presented in the context of MHC molecules. An epitope of a protein may comprises a continuous or discontinuous portion of said protein and, e.g., may be between about 5 and about 100, between about 5 and about 50, between about 8 and about 30, or about 10 and about 25 amino acids in length, for example, the epitope may be preferably

9, 10, 11, 12, 13, 14, 15, 16, 17, 18, 19, 20, 21, 22, 23, 24, or 25 amino acids in length. In some embodiments, the epitope in the context of the present disclosure is a T cell epitope.

Terms such as "epitope", "fragment of an antigen", "immunogenic peptide" and "antigen peptide" are used interchangeably herein and, e.g., may relate to an incomplete representation of an antigen which is, e.g., capable of eliciting an immune response against the antigen or a cell expressing or comprising and presenting the antigen. In some embodiments, the terms relate to an immunogenic portion of an antigen. In some embodiments, it is a portion of an antigen that is recognized *(i.e.,* specifically bound) by a T cell receptor, in particular if presented in the context of MHC molecules. Certain preferred immunogenic portions bind to an MHC class I or class II molecule. The term "epitope" refers to a part or fragment of a molecule such as an antigen that is recognized by the immune system. For example, the epitope may be recognized by T cells, B cells or antibodies. An epitope of an antigen may include a continuous or discontinuous portion of the antigen and may be between about 5 and about 100, such as between about 5 and about 50, between about 8 and about 30, or between about 8 and about 25 amino acids in length, for example, the epitope may be 9, 10, 11, 12, 13, 14, 15, 16, 17, 18, 19, 20, 21, 22, 23, 24, or 25 amino acids in length. In some embodiments, an epitope is between about 10 and about 25 amino acids in length. The term "epitope" includes T cell epitopes.

The term "T cell epitope" refers to a part or fragment of a protein that is recognized by a T cell when presented in the context of MHC molecules. The term "major histocompatibility complex" and the abbreviation "MHC" includes MHC class I and MHC class II molecules and relates to a complex of genes which is present in all vertebrates. MHC proteins or molecules are important for signaling between lymphocytes and antigen presenting cells or diseased cells in immune reactions, wherein the MHC proteins or molecules bind peptide epitopes and present them for recognition by T cell receptors on T cells. The proteins encoded by the MHC are expressed on the surface of cells, and display both self-antigens (peptide fragments from the cell itself) and non-self-antigens (e.g., fragments of invading microorganisms) to a T cell. In the case of class I MHC/peptide complexes, the binding peptides are typically about 8 to about 10 amino acids long although longer or shorter peptides may be effective. In the case of class II MHC/peptide complexes, the binding peptides are typically about 10 to about 25 amino acids long and are in particular about 13 to about 18 amino acids long, whereas longer and shorter peptides may be effective.

The peptide and polypeptide antigen can be 2 to 100 amino acids, including for example, 5 amino acids, 10 amino acids, 15 amino acids, 20 amino acids, 25 amino acids, 30 amino acids, 35 amino acids, 40 amino acids, 45 amino acids, or 50 amino acids in length. In some embodiments, a peptide can be greater than 50 amino acids. In some embodiments, the peptide can be greater than 100 amino acids.

The peptide or polypeptide antigen can be any peptide or polypeptide that can induce or increase the ability of the immune system to develop antibodies and T cell responses to the peptide or polypeptide.

In some embodiments, vaccine antigen, *i.e.,* an antigen whose inoculation into a subject induces an immune response, is recognized by an immune effector cell. In some embodiments, the vaccine antigen if recognized by an immune effector cell is able to induce in the presence of appropriate co-stimulatory signals, stimulation, priming and/or expansion of the immune effector cell carrying an antigen receptor recognizing the vaccine antigen. In the context of the embodiments of the present disclosure, the vaccine antigen may be, e.g., presented or present on the surface of a cell, such as an antigen presenting cell.

In some embodiments, an antigen is expressed in a diseased cell (such as tumor cell or an infected cell).

In some embodiments, an antigen is presented by a diseased cell (such as tumor cell or an infected cell). In some embodiments, an antigen receptor is a TCR which binds to an epitope of an antigen presented in the context of MHC. In some embodiments, binding of a TCR when expressed by T cells and/or present on T cells to an antigen presented by cells such as antigen presenting cells results in stimulation, priming and/or expansion of said T cells. In some embodiments, binding of a TCR when expressed by T cells and/or present on T cells to an antigen presented on diseased cells results in cytolysis and/or apoptosis of the diseased cells, wherein said T cells release cytotoxic factors, e.g., perforins and granzymes.

In some embodiments, an antigen is expressed on the surface of a diseased cell (such as tumor cell or an infected cell). In some embodiments, an antigen receptor is a CAR which binds to an extracellular domain or to an epitope in an extracellular domain of an antigen. In some embodiments, a CAR binds to native epitopes of an antigen present on the surface of living cells. In some embodiments, binding of a CAR when expressed by T cells and/or present on T cells to an antigen present on cells such as antigen presenting cells results in stimulation, priming and/or expansion of said T cells. In some embodiments, binding of a CAR when expressed by T cells and/or present on T cells to an antigen present on diseased cells results in cytolysis and/or apoptosis of the diseased cells, wherein said T cells preferably release cytotoxic factors, e.g., perforins and granzymes.

According to some embodiments, an amino acid sequence enhancing antigen processing and/or presentation is fused, either directly or through a linker, to an antigenic peptide or polypeptide (antigenic sequence). Accordingly, in some embodiments, the RNA described herein comprises at least one coding region encoding an antigenic peptide or polypeptide and an amino acid sequence enhancing antigen processing and/or presentation.

In some embodiments, antigen for vaccination which may be administered in the form of RNA coding therefor

comprises a naturally occurring antigen or a fragment such as an epitope thereof.

Such amino acid sequences enhancing antigen processing and/or presentation are preferably located at the C-terminus of the antigenic peptide or polypeptide (and optionally at the C-terminus of an amino acid sequence which breaks immunological tolerance), without being limited thereto. Amino acid sequences enhancing antigen processing and/or presentation as defined herein preferably improve antigen processing and presentation. In some embodiments, the amino acid sequence enhancing antigen processing and/or presentation as defined herein includes, without being limited thereto, sequences derived from the human MHC class I complex (HLA-B51, haplotype A2, B27/B51, Cw2/Cw3), in particular a sequence comprising the amino acid sequence of SEQ ID NO: 2 or a functional variant thereof.

In some embodiments, an amino acid sequence enhancing antigen processing and/or presentation comprises the amino acid sequence of SEQ ID NO: 2, an amino acid sequence having at least 99%, 98%, 97%, 96%, 95%, 90%, 85%, or 80% identity to the amino acid sequence of SEQ ID NO: 2, or a functional fragment of the amino acid sequence of SEQ ID NO: 2, or the amino acid sequence having at least 99%, 98%, 97%, 96%, 95%, 90%, 85%, or 80% identity to the amino acid sequence of SEQ ID NO: 2. In some embodiments, an amino acid sequence enhancing antigen processing and/or presentation comprises the amino acid sequence of SEQ ID NO: 2.

Accordingly, in some embodiments, the RNA described herein comprises at least one coding region encoding an antigenic peptide or polypeptide and an amino acid sequence enhancing antigen processing and/or presentation, said amino acid sequence enhancing antigen processing and/or presentation preferably being fused to the antigenic peptide or polypeptide, more preferably to the C-terminus of the antigenic peptide or polypeptide as described herein.

Furthermore, a secretory sequence, e.g., a sequence comprising the amino acid sequence of SEQ ID NO: 1, may be fused to the N-terminus of the antigenic peptide or polypeptide. Amino acid sequences derived from tetanus toxoid of *Clostridium tetani* may be employed to overcome self-tolerance mechanisms in order to efficiently mount an immune response to self-antigens by providing T-cell help during priming.

It is known that tetanus toxoid heavy chain includes epitopes that can bind promiscuously to MHC class II alleles and induce $CD4^+$ memory T cells in almost all tetanus vaccinated individuals. In addition, the combination of tetanus toxoid (TT) helper epitopes with tumor-associated antigens is known to improve the immune stimulation compared to application of tumor-associated antigen alone by providing $CD4^+$-mediated T-cell help during priming. To reduce the risk of stimulating $CD8^+$ T cells with the tetanus sequences which might compete with the intended induction of tumor antigen-specific T-cell response, not the whole fragment C of tetanus toxoid is used as it is known to contain $CD8^+$ T-cell epitopes. Two peptide sequences containing promiscuously binding helper epitopes were selected alternatively to ensure binding to as many MHC class II alleles as possible. Based on the data of the *ex vivo* studies the well-known epitopes p2 (QYIKANSKFIGITEL; $TT_{830-844}$; SEQ ID NO: 9) and p16 (MTNSVDDALINSTKIYSYFPSVISKVNQGAQG; $TT_{578-609}$; SEQ ID NO: 10) were selected. The p2 epitope was already used for peptide vaccination in clinical trials to boost anti-melanoma activity.

Non-clinical data showed that RNA vaccines encoding both a tumor antigen plus promiscuously binding tetanus toxoid sequences lead to enhanced $CD8^+$ T-cell responses directed against the tumor antigen and improved break of tolerance. Immunomonitoring data from patients vaccinated with vaccines including those sequences fused in frame with the tumor antigen-specific sequences reveal that the tetanus sequences chosen are able to induce tetanus-specific T-cell responses in almost all patients.

According to some embodiments, an amino acid sequence which breaks immunological tolerance is fused, either directly or through a linker, e.g., a linker having the amino acid sequence according to SEQ ID NO: 4, to the antigenic peptide or polypeptide.

Such amino acid sequences which break immunological tolerance are preferably located at the C-terminus of the antigenic peptide or polypeptide (and optionally at the N-terminus of the amino acid sequence enhancing antigen processing and/or presentation, wherein the amino acid sequence which breaks immunological tolerance and the amino acid sequence enhancing antigen processing and/or presentation may be fused either directly or through a linker, e.g., a linker having the amino acid sequence according to SEQ ID NO: 5), without being limited thereto. Amino acid sequences which break immunological tolerance as defined herein preferably improve T cell responses. In some embodiments, the amino acid sequence which breaks immunological tolerance as defined herein includes, without being limited thereto, sequences derived from tetanus toxoid-derived helper sequences p2 and p16 (P2P16), in particular a sequence comprising the amino acid sequence of SEQ ID NO: 3 or a functional variant thereof.

In some embodiments, an amino acid sequence which breaks immunological tolerance comprises the amino acid sequence of SEQ ID NO: 3, an amino acid sequence having at least 99%, 98%, 97%, 96%, 95%, 90%, 85%, or 80% identity to the amino acid sequence of SEQ ID NO: 3, or a functional fragment of the amino acid sequence of SEQ ID NO: 3, or the amino acid sequence having at least 99%, 98%, 97%, 96%, 95%, 90%, 85%, or 80% identity to the amino acid sequence of SEQ ID NO: 3. In some embodiments, an amino acid sequence which breaks immunological tolerance comprises the amino acid sequence of SEQ ID NO: 3.

In the following, embodiments of vaccine RNAs are described, wherein certain terms used when describing elements thereof have the following meanings:

cap: 5'-cap structure selected from the group consisting of $m_2^{7,2'O}G(5')ppSp(5')G$ (in particular its D1 diastereomer), $m_2^{7,3'O}G(5')ppp(5')G$, and $m_2^{7,3'-O}Gppp(m_1^{2'-O})ApG$.

**hAg-Kozak:** 5'-UTR sequence of the human alpha-globin mRNA with an optimized 'Kozak sequence' to increase translational efficiency.

**sec/MITD:** Fusion-protein tags derived from the sequence encoding the human MHC class I complex (HLA-B51, haplotype A2, B27/B51, Cw2/Cw3), which have been shown to improve antigen processing and presentation. Sec corresponds to the 78 bp fragment coding for the secretory signal peptide, which guides translocation of the nascent polypeptide chain into the endoplasmatic reticulum. MITD corresponds to the transmembrane and cytoplasmic domain of the MHC class I molecule, also called MHC class I trafficking domain.

**Antigen:** Sequences encoding the respective vaccine antigen/epitope.

**Glycine-serine linker (GS):** Sequences coding for short peptide linkers predominantly consisting of the amino acids glycine (G) and serine (S), as commonly used for fusion proteins.

**P2P16:** Sequence coding for tetanus toxoid-derived helper epitopes to break immunological tolerance.

**FI element:** The 3'-UTR is a combination of two sequence elements derived from the "amino terminal enhancer of split" (AES) mRNA (called F) and the mitochondrial encoded 12S ribosomal RNA (called I). These were identified by an *ex vivo* selection process for sequences that confer RNA stability and augment total protein expression.

**A30L70:** A poly(A)-tail measuring 110 nucleotides in length, consisting of a stretch of 30 adenosine residues, followed by a 10 nucleotide linker sequence and another 70 adenosine residues designed to enhance RNA stability and translational efficiency in dendritic cells.

In some embodiments, vaccine RNA described herein has one of the following structures: cap-hAg-Kozak-sec-GS(1)-Antigen-GS(2)-P2P16-GS(3)-MITD-FI-A30L70 beta-S-ARCA(D1)-hAg-Kozak-sec-GS(1)-Antigen-GS(2)-P2P16-GS(3)-MITD-FI-A30L70

In some embodiments, vaccine antigen described herein has the structure: sec-GS(1)-Antigen-GS(2)-P2P16-GS(3)-MITD

In some embodiments, hAg-Kozak comprises the nucleotide sequence of SEQ ID NO: 6. In some embodiments, sec comprises the amino acid sequence of SEQ ID NO: 1. In some embodiments, P2P16 comprises the amino acid sequence of SEQ ID NO: 3. In some embodiments, MITD comprises the amino acid sequence of SEQ ID NO: 2. In some embodiments, GS(1) comprises the amino acid sequence of SEQ ID NO: 4. In some embodiments, GS(2) comprises the amino acid sequence of SEQ ID NO: 4. In some embodiments, GS(3) comprises the amino acid sequence of SEQ ID NO: 5. In some embodiments, FI comprises the nucleotide sequence of SEQ ID NO: 7. In some embodiments, A30L70 comprises the nucleotide sequence of SEQ ID NO: 8.

In some embodiments, the sequence encoding the vaccine antigen/epitope comprises a modified nucleoside replacing (partially or completely, preferably completely) uridine, wherein the modified nucleoside is selected from the group consisting of pseudouridine ($\psi$), N1-methyl-pseudouridine ($m1\psi$), and 5-methyl-uridine.

In some embodiments, the sequence encoding the vaccine antigen/epitope is codon-optimized.

In some embodiments, the G/C content of the sequence encoding the vaccine antigen/epitope is increased compared to the wild type coding sequence.

In some embodiments, an antigen receptor is an antibody or B cell receptor which binds to an epitope in an antigen. In some embodiments, an antibody or B cell receptor binds to native epitopes of an antigen.

The term "expressed on the cell surface" or "associated with the cell surface" means that a molecule such as an antigen is associated with and located at the plasma membrane of a cell, wherein at least a part of the molecule faces the extracellular space of said cell and is accessible from the outside of said cell, e.g., by antibodies located outside the cell. In this context, a part may be, e.g., at least 4, at least 8, at least 12, or at least 20 amino acids. The association may be direct or indirect. For example, the association may be by one or more transmembrane domains, one or more lipid anchors, or by the interaction with any other protein, lipid, saccharide, or other structure that can be found on the outer leaflet of the plasma membrane of a cell. For example, a molecule associated with the surface of a cell may be a transmembrane protein having an extracellular portion or may be a protein associated with the surface of a cell by interacting with another protein that is a transmembrane protein.

"Cell surface" or "surface of a cell" is used in accordance with its normal meaning in the art, and thus includes the outside of the cell which is accessible to binding by proteins and other molecules. An antigen is expressed on the surface of cells if it is located at the surface of said cells and is accessible to binding by, *e.g.,* antigen-specific antibodies added to the cells. In some embodiments, an antigen expressed on the surface of cells is an integral membrane protein having an extracellular portion which may be recognized by a CAR.

The term "extracellular portion" or "exodomain" in the context of the present disclosure refers to a part of a molecule such as a protein that is facing the extracellular space of a cell and preferably is accessible from the outside of said cell, e.g., by binding molecules such as antibodies located outside the cell. In some embodiments, the term refers to one or more extracellular loops or domains or a fragment thereof.

The terms "T cell" and "T lymphocyte" are used interchangeably herein and include T helper cells (CD4+ T cells) and cytotoxic T cells (CTLs, $CD8^+$ T cells) which comprise cytolytic T cells. The term "antigen-specific T cell" or similar terms relate to a T cell which recognizes the antigen to which the T cell is targeted, in particular when presented on the surface of antigen presenting cells or diseased cells such as cancer cells in the context of MHC molecules and preferably exerts effector functions of T cells. T cells are considered to be specific for antigen if the cells kill target cells expressing an antigen. T cell specificity may be evaluated using any of a variety of standard techniques, for example, within a chromium release assay or proliferation assay. Alternatively, synthesis of lymphokines (such as interferon-$\gamma$) can be measured.

The term "target" shall mean an agent such as a cell or tissue which is a target for an immune response such as a cellular immune response. Targets include cells that present an antigen or an antigen epitope, *i.e.,* a peptide fragment derived from an antigen. In some embodiments, the target cell is a cell expressing an antigen and presenting said antigen with class I MHC.

"Antigen processing" refers to the degradation of an antigen into processing products which are fragments of said antigen (e.g., the degradation of a polypeptide into peptides) and the association of one or more of these fragments (e.g., via binding) with MHC molecules for presentation by cells, such as antigen-presenting cells to specific T-cells. Antigen-presenting cells can be distinguished in professional antigen presenting cells and non-professional antigen presenting cells.

The term "professional antigen presenting cells" relates to antigen presenting cells which constitutively express the Major Histocompatibility Complex class II (MHC class II) molecules required for interaction with naive T cells. If a T cell interacts with the MHC class II molecule complex on the membrane of the antigen presenting cell, the antigen presenting cell produces a co-stimulatory molecule inducing activation of the T cell. Professional antigen presenting cells comprise dendritic cells and macrophages.

The term "non-professional antigen presenting cells" relates to antigen presenting cells which do not constitutively express MHC class II molecules, but upon stimulation by certain cytokines such as interferon-gamma. Exemplary, non-professional antigen presenting cells include fibroblasts, thymic epithelial cells, thyroid epithelial cells, glial cells, pancreatic beta cells or vascular endothelial cells.

The term "dendritic cell" (DC) refers to a subtype of phagocytic cells belonging to the class of antigen presenting cells. In some embodiments, dendritic cells are derived from hematopoietic bone marrow progenitor cells. These progenitor cells initially transform into immature dendritic cells. These immature cells are characterized by high phagocytic activity and low T cell activation potential. Immature dendritic cells constantly sample the surrounding environment for pathogens such as viruses and bacteria. Once they have come into contact with a presentable antigen, they become activated into mature dendritic cells and begin to migrate to the spleen or to the lymph node. Immature dendritic cells phagocytose pathogens and degrade their proteins into small pieces and upon maturation present those fragments at their cell surface using MHC molecules. Simultaneously, they upregulate cell-surface receptors that act as co-receptors in T cell activation such as CD80, CD86, and CD40 greatly enhancing their ability to activate T cells. They also upregulate CCR7, a chemotactic receptor that induces the dendritic cell to travel through the blood stream to the spleen or through the lymphatic system to a lymph node. Here they act as antigen-presenting cells and activate helper T cells and killer T cells as well as B cells by presenting them antigens, alongside non-antigen specific co-stimulatory signals. Thus, dendritic cells can actively induce a T cell- or B cell-related immune response. In some embodiments, the dendritic cells are splenic dendritic cells.

The term "macrophage" refers to a subgroup of phagocytic cells produced by the differentiation of monocytes. Macrophages which are activated by inflammation, immune cytokines or microbial products nonspecifically engulf and kill foreign pathogens within the macrophage by hydrolytic and oxidative attack resulting in degradation of the pathogen.

Peptides from degraded proteins are displayed on the macrophage cell surface where they can be recognized by T cells, and they can directly interact with antibodies on the B cell surface, resulting in T and B cell activation and further stimulation of the immune response. Macrophages belong to the class of antigen presenting cells. In some embodiments, the macrophages are splenic macrophages.

By "antigen-responsive CTL" is meant a $CD8^+$ T-cell that is responsive to an antigen or a peptide derived from said antigen, which is presented with class I MHC on the surface of antigen presenting cells.

According to the disclosure, CTL responsiveness may include sustained calcium flux, cell division, production of cytokines such as IFN-$\gamma$ and TNF-$\alpha$, up-regulation of activation markers such as CD44 and CD69, and specific cytolytic killing of tumor antigen expressing target cells. CTL responsiveness may also be determined using an artificial reporter that accurately indicates CTL responsiveness.

"Activation" or "stimulation", as used herein, refers to the state of a cell that has been sufficiently stimulated to induce detectable cellular proliferation, such as an immune effector cell such as T cell. Activation can also be associated with initiation of signaling pathways, induced cytokine production, and detectable effector functions. The term "activated immune effector cells" refers to, among other things, immune effector cells that are undergoing cell division.

The term "priming" refers to a process wherein an immune effector cell such as a T cell has its first contact with its specific antigen and causes differentiation into effector cells such as effector T cells.

The term "expansion" refers to a process wherein a specific entity is multiplied. In some embodiments, the term is used in the context of an immunological response in which immune effector cells are stimulated by an antigen, proliferate, and the specific immune effector cell recognizing said antigen is amplified. In some embodiments, expansion leads to differentiation of the immune effector cells.

The terms "immune response" and "immune reaction" are used herein interchangeably in their conventional meaning and refer to an integrated bodily response to an antigen and may refer to a cellular immune response, a humoral immune response, or both. According to the disclosure, the term "immune response to" or "immune response against" with respect to an agent such as an antigen, cell or tissue, relates to an immune response such as a cellular response directed against the agent. An immune response may comprise one or more reactions selected from the group consisting of developing antibodies against one or more antigens and expansion of antigen-specific T-lymphocytes, such as $CD4^+$ and $CD8^+$ T-lymphocytes, e.g. $CD8^+$ T-lymphocytes, which may be detected in various proliferation or cytokine production tests *in vitro.*

The terms "inducing an immune response" and "eliciting an immune response" and similar terms in the context of the present disclosure refer to the induction of an immune response, such as the induction of a cellular immune response, a humoral immune response, or both. The immune response may be protective/preventive/prophylactic and/or therapeutic. The immune response may be directed against any immunogen or antigen or antigen peptide, such as against a tumor-associated antigen or a pathogen-associated antigen (e.g., an antigen of a virus (such as influenza virus (A, B, or C), CMV or RSV)). "Inducing" in this context may mean that there was no immune response against a particular antigen or pathogen before induction, but it may also mean that there was a certain level of immune response against a particular antigen or pathogen before induction and after induction said immune response is enhanced. Thus, "inducing the immune response" in this context also includes "enhancing the immune response". In some embodiments, after inducing an immune response in an individual, said individual is protected from developing a disease such as an infectious disease or a cancerous disease or the disease condition is ameliorated by inducing an immune response.

The terms "cellular immune response", "cellular response", "cell-mediated immunity" or similar terms are meant to include a cellular response directed to cells characterized by expression of an antigen and/or presentation of an antigen with class I or class II MHC. The cellular response relates to cells called T cells or T lymphocytes which act as either "helpers" or "killers". The helper T cells (also termed $CD4^+$ T cells) play a central role by regulating the immune response and the killer cells (also termed cytotoxic T cells, cytolytic T cells, $CD8^+$ T cells or CTLs) kill cells such as diseased cells.

The term "humoral immune response" refers to a process in living organisms wherein antibodies are produced in response to agents and organisms, which they ultimately neutralize and/or eliminate. The specificity of the antibody response is mediated by T and/or B cells through membrane-associated receptors that bind antigen of a single specificity. Following binding of an appropriate antigen and receipt of various other activating signals, B lymphocytes divide, which produces memory B cells as well as antibody secreting plasma cell clones, each producing antibodies that recognize the identical antigenic epitope as was recognized by its antigen receptor. Memory B lymphocytes remain dormant until they are subsequently activated by their specific antigen. These lymphocytes provide the cellular basis of memory and the resulting escalation in antibody response when re-exposed to a specific antigen.

The term "antibody" as used herein, refers to an immunoglobulin molecule, which is able to specifically bind to an epitope on an antigen. In particular, the term "antibody" refers to a glycoprotein comprising at least two heavy (H) chains and two light (L) chains inter-connected by disulfide bonds. The term "antibody" includes monoclonal antibodies, recombinant antibodies, human antibodies, humanized antibodies, chimeric antibodies and combinations of any of the foregoing. Each heavy chain is comprised of a heavy chain variable region (VH) and a heavy chain constant region (CH). Each light chain is comprised of a light chain variable region (VL) and a light chain constant region (CL). The variable regions and constant regions are also referred to herein as variable domains and constant domains, respectively. The VH and VL regions can be further subdivided into regions of hypervariability, termed complementarity determining regions (CDRs), interspersed with regions that are more conserved, termed framework regions (FRs). Each VH and VL is composed of three CDRs and four FRs, arranged from amino-terminus to carboxy-terminus in the following order: FR1, CDR1, FR2, CDR2, FR3, CDR3, FR4. The CDRs of a VH are termed HCDR1, HCDR2 and HCDR3, the CDRs of a VL are termed LCDR1, LCDR2 and LCDR3. The variable regions of the heavy and light chains contain a binding domain that interacts with an antigen. The constant regions of an antibody comprise the heavy chain constant region (CH) and the light chain constant region (CL), wherein CH can be further subdivided into constant domain CH1, a hinge region, and constant domains CH2 and CH3 (arranged from amino-terminus to carboxy-terminus in the following order: CH1, CH2, CH3). The constant regions of the antibodies may mediate the binding of the immunoglobulin to host tissues or factors, including various cells of the immune system (e.g., effector cells) and the first component (C1q) of the classical complement system. Antibodies can be intact immunoglobulins derived from natural sources or from recombinant sources and can be immunoactive portions of intact immunoglobulins. Antibodies are typically tetramers of immunoglobulin molecules. Antibodies may exist in a variety of forms including, for example, polyclonal antibodies, monoclonal antibodies, Fv, Fab and $F(ab)_2$, as well as single chain antibodies and humanized antibodies.

The term "immunoglobulin" relates to proteins of the immunoglobulin superfamily, such as to antigen receptors

such as antibodies or the B cell receptor (BCR). The immunoglobulins are characterized by a structural domain, i.e., the immunoglobulin domain, having a characteristic immunoglobulin (Ig) fold. The term encompasses membrane bound immunoglobulins as well as soluble immunoglobulins. Membrane bound immunoglobulins are also termed surface immunoglobulins or membrane immunoglobulins, which are generally part of the BCR. Soluble immunoglobulins are generally termed antibodies. Immunoglobulins generally comprise several chains, typically two identical heavy chains and two identical light chains which are linked via disulfide bonds. These chains are primarily composed of immunoglobulin domains, such as the $V_L$ (variable light chain) domain, $C_L$ (constant light chain) domain, $V_H$ (variable heavy chain) domain, and the $C_H$ (constant heavy chain) domains $C_H1$, $C_H2$, $C_H3$, and $C_H4$. There are five types of mammalian immunoglobulin heavy chains, *i.e.,* $\alpha$, $\delta$, $\varepsilon$, $\gamma$, and $\mu$ which account for the different classes of antibodies, *i.e.,* IgA, IgD, IgE, IgG, and IgM. As opposed to the heavy chains of soluble immunoglobulins, the heavy chains of membrane or surface immunoglobulins comprise a transmembrane domain and a short cytoplasmic domain at their carboxy-terminus. In mammals there are two types of light chains, i.e., lambda and kappa. The immunoglobulin chains comprise a variable region and a constant region. The constant region is essentially conserved within the different isotypes of the immunoglobulins, wherein the variable part is highly divers and accounts for antigen recognition.

The terms "vaccination" and "immunization" describe the process of treating an individual for therapeutic or prophylactic reasons and relate to the procedure of administering one or more immunogen(s) or antigen(s) or derivatives thereof, in particular in the form of RNA (especially mRNA) coding therefor, as described herein to an individual and stimulating an immune response against said one or more immunogen(s) or antigen(s) or cells characterized by presentation of said one or more immunogen(s) or antigen(s).

By "cell characterized by presentation of an antigen" or "cell presenting an antigen" or "MHC molecules which present an antigen on the surface of an antigen presenting cell" or similar expressions is meant a cell such as a diseased cell, in particular a tumor cell or an infected cell, or an antigen presenting cell presenting the antigen or an antigen peptide, either directly or following processing, in the context of MHC molecules, such as MHC class I and/or MHC class II molecules. In some embodiments, the MHC molecules are MHC class I molecules.

The term "allergen" refers to a kind of antigen which originates from outside the body of a subject *(i.e.,* the allergen can also be called "heterologous antigen") and which produces an abnormally vigorous immune response in which the immune system of the subject fights off a perceived threat that would otherwise be harmless to the subject. "Allergies" are the diseases caused by such vigorous immune reactions against allergens. An allergen usually is an antigen which is able to stimulate a type-I hypersensitivity reaction in atopic individuals through immunoglobulin E (IgE) responses. Particular examples of allergens include allergens derived from peanut proteins *(e.g.,* Ara h 2.02), ovalbumin, grass pollen proteins *(e.g.,* Phl p 5), and proteins of dust mites (e.g., Der p 2).

The term "growth factors" refers to molecules which are able to stimulate cellular growth, proliferation, healing, and/or cellular differentiation. Typically, growth factors act as signaling molecules between cells. The term "growth factors" include particular cytokines and hormones which bind to specific receptors on the surface of their target cells. Examples of growth factors include bone morphogenetic proteins (BMPs), fibroblast growth factors (FGFs), vascular endothelial growth factors (VEGFs), such as VEGFA, epidermal growth factor (EGF), insulin-like growth factor, ephrins, macrophage colony-stimulating factor, granulocyte colony-stimulating factor, granulocyte macrophage colony-stimulating factor, neuregulins, neurotrophins *(e.g.,* brain-derived neurotrophic factor (BDNF), nerve growth factor (NGF)), placental growth factor (PGF), platelet-derived growth factor (PDGF), renalase (RNLS) (anti-apoptotic survival factor), T-cell growth factor (TCGF), thrombopoietin (TPO), transforming growth factors (transforming growth factor alpha (TGF-$\alpha$), transforming growth factor beta (TGF-$\beta$)), and tumor necrosis factor-alpha (TNF-$\alpha$). In some embodiments, a "growth factor" is a peptide or polypeptide growth factor.

The term "protease inhibitors" refers to molecules, in particular peptides or polypeptides, which inhibit the function of proteases. Protease inhibitors can be classified by the protease which is inhibited *(e.g.,* aspartic protease inhibitors) or by their mechanism of action (e.g., suicide inhibitors, such as serpins). Particular examples of protease inhibitors include serpins, such as alpha 1-antitrypsin, aprotinin, and bestatin.

The term "enzymes" refers to macromolecular biological catalysts which accelerate chemical reactions. Like any catalyst, enzymes are not consumed in the reaction they catalyze and do not alter the equilibrium of said reaction. Unlike many other catalysts, enzymes are much more specific. In some embodiments, an enzyme is essential for homeostasis of a subject, e.g., any malfunction (in particular, decreased activity which may be caused by any of mutation, deletion or decreased production) of the enzyme results in a disease. Examples of enzymes include herpes simplex virus type 1 thymidine kinase (HSV1-TK), hexosaminidase, phenylalanine hydroxylase, pseudocholinesterase, and lactase.

The term "receptors" refers to protein molecules which receive signals (in particular chemical signals called ligands) from outside a cell. The binding of a signal *(e.g.,* ligand) to a receptor causes some kind of response of the cell, *e.g.,* the intracellular activation of a kinase. Receptors include transmembrane receptors (such as ion channel-linked (ionotropic) receptors, G protein-linked (metabotropic) receptors, and enzyme-linked receptors) and intracellular receptors (such as cytoplasmic receptors and nuclear receptors). Particular examples of receptors include steroid hormone receptors, growth factor receptors, and peptide receptors *(i.e.,* receptors whose ligands are peptides), such as P-selectin

glycoprotein ligand-1 (PSGL-1). The term "growth factor receptors" refers to receptors which bind to growth factors.

The term "apoptosis regulators" refers to molecules, in particular peptides or polypeptides, which modulate apoptosis, *i.e.,* which either activate or inhibit apoptosis. Apoptosis regulators can be grouped into two broad classes: those which modulate mitochondrial function and those which regulate caspases. The first class includes proteins (e.g., BCL-2, BCL-xL) which act to preserve mitochondrial integrity by preventing loss of mitochondrial membrane potential and/or release of pro-apoptotic proteins such as cytochrome C into the cytosol. Also to this first class belong proapoptotic proteins (e.g., BAX, BAK, BIM) which promote release of cytochrome C. The second class includes proteins such as the inhibitors of apoptosis proteins *(e.g.,* XIAP) or FLIP which block the activation of caspases.

The term "transcription factors" relates to proteins which regulate the rate of transcription of genetic information from DNA to messenger RNA, in particular by binding to a specific DNA sequence. Transcription factors may regulate cell division, cell growth, and cell death throughout life; cell migration and organization during embryonic development; and/or in response to signals from outside the cell, such as a hormone. Transcription factors contain at least one DNA-binding domain which binds to a specific DNA sequence, usually adjacent to the genes which are regulated by the transcription factors. Particular examples of transcription factors include MECP2, FOXP2, FOXP3, the STAT protein family, and the HOX protein family.

The term "tumor suppressor proteins" relates to molecules, in particular peptides or polypeptides, which protect a cell from one step on the path to cancer. Tumor-suppressor proteins (usually encoded by corresponding tumor-suppressor genes) exhibit a weakening or repressive effect on the regulation of the cell cycle and/or promote apoptosis. Their functions may be one or more of the following: repression of genes essential for the continuing of the cell cycle; coupling the cell cycle to DNA damage (as long as damaged DNA is present in a cell, no cell division should take place); initiation of apoptosis, if the damaged DNA cannot be repaired; metastasis suppression (e.g., preventing tumor cells from dispersing, blocking loss of contact inhibition, and inhibiting metastasis); and DNA repair. Particular examples of tumor-suppressor proteins include p53, phosphatase and tensin homolog (PTEN), SWI/SNF (SWitch/Sucrose Non-Fermentable), von Hippel-Lindau tumor suppressor (pVHL), adenomatous polyposis coli (APC), CD95, suppression of tumorigenicity 5 (ST5), suppression of tumorigenicity 5 (ST5), suppression of tumorigenicity 14 (ST14), and Yippee-like 3 (YPEL3). The term "structural proteins" refers to proteins which confer stiffness and rigidity to otherwise-fluid biological components. Structural proteins are mostly fibrous (such as collagen and elastin) but may also be globular (such as actin and tubulin). Usually, globular proteins are soluble as monomers, but polymerize to form long, fibers which, for example, may make up the cytoskeleton. Other structural proteins are motor proteins (such as myosin, kinesin, and dynein) which are capable of generating mechanical forces, and surfactant proteins. Particular examples of structural proteins include collagen, surfactant protein A, surfactant protein B, surfactant protein C, surfactant protein D, elastin, tubulin, actin, and myosin.

The term "reprogramming factors" or "reprogramming transcription factors" relates to molecules, in particular peptides or polypeptides, which, when expressed in somatic cells optionally together with further agents such as further reprogramming factors, lead to reprogramming or de-differentiation of said somatic cells to cells having stem cell characteristics, in particular pluripotency. Particular examples of reprogramming factors include OCT4, SOX2, c-MYC, KLF4, LIN28, and NANOG.

The term "genomic engineering proteins" relates to proteins which are able to insert, delete or replace DNA in the genome of a subject. Particular examples of genomic engineering proteins include meganucleases, zinc finger nucleases (ZFNs), transcription activator-like effector nucleases (TALENs), and clustered regularly spaced short palindromic repeat-CRISPR-associated protein 9 (CRISPR-Cas9).

The term "blood proteins" relates to peptides or polypeptides which are present in blood plasma of a subject, in particular blood plasma of a healthy subject. Blood proteins have diverse functions such as transport *(e.g.,* albumin, transferrin), enzymatic activity *(e.g.,* thrombin or ceruloplasmin), blood clotting *(e.g.,* fibrinogen), defense against pathogens *(e.g.,* complement components and immunoglobulins), protease inhibitors (e.g., alpha 1-antitrypsin), etc. Particular examples of blood proteins include thrombin, serum albumin, Factor Vil, Factor VIII, insulin, Factor IX, Factor X, tissue plasminogen activator, protein C, von Willebrand factor, antithrombin III, glucocerebrosidase, erythropoietin, granulocyte colony stimulating factor (G-CSF), modified Factor VIII, and anticoagulants.

Thus, in some embodiments, the pharmaceutically active peptide or polypeptide is (i) a cytokine, preferably selected from the group consisting of erythropoietin (EPO), interleukin 4 (IL-2), and interleukin 10 (IL-11), more preferably EPO; (ii) an adhesion molecule, in particular an integrin; (iii) an immunoglobulin, in particular an antibody; (iv) an immunologically active compound, in particular an antigen, such as a viral or bacterial antigen, e.g., an antigen of SARS-CoV-2; (v) a hormone, in particular vasopressin, insulin or growth hormone; (vi) a growth factor, in particular VEGFA; (vii) a protease inhibitor, in particular alpha 1-antitrypsin; (viii) an enzyme, preferably selected from the group consisting of herpes simplex virus type 1 thymidine kinase (HSV1-TK), hexosaminidase, phenylalanine hydroxylase, pseudocholinesterase, pancreatic enzymes, and lactase; (ix) a receptor, in particular growth factor receptors; (x) an apoptosis regulator, in particular BAX; (xi) a transcription factor, in particular FOXP3; (xii) a tumor suppressor protein, in particular p53; (xiii) a structural protein, in particular surfactant protein B; (xiv) a reprogramming factor, e.g., selected from the group consisting of OCT4, SOX2, c-MYC, KLF4, LIN28 and NANOG; (xv) a genomic engineering protein, in particular

clustered regularly spaced short palindromic repeat-CRISPR-associated protein 9 (CRISPR-Cas9); and (xvi) a blood protein, in particular fibrinogen.

In some embodiments, a pharmaceutically active peptide or polypeptide comprises one or more antigens or one or more epitopes, *i.e.,* administration of the peptide or polypeptide to a subject elicits an immune response against the one or more antigens or one or more epitopes in a subject which may be therapeutic or partially or fully protective.

In some embodiments, the RNA encodes at least one epitope, e.g., at least two epitopes, at least three epitopes, at least four epitopes, at least five epitopes, at least six epitopes, at least seven epitopes, at least eight epitopes, at least nine epitopes, or at least ten epitopes.

In some embodiments, the target antigen is a tumor antigen and the antigenic sequence (e.g., an epitope) is derived from the tumor antigen. The tumor antigen may be a "standard" antigen, which is generally known to be expressed in various cancers. The tumor antigen may also be a "neo-antigen", which is specific to an individual's tumor and has not been previously recognized by the immune system. A neo-antigen or neo-epitope may result from one or more cancer-specific mutations in the genome of cancer cells resulting in amino acid changes. If the tumor antigen is a neo-antigen, the vaccine antigen preferably comprises an epitope or a fragment of said neo-antigen comprising one or more amino acid changes.

Examples of tumor antigens include, without limitation, p53, ART-4, BAGE, beta-catenin/m, Bcr-abL CAMEL, CAP-1 , CASP-8, CDC27/m, CDK4/m, CEA, the cell surface proteins of the claudin family, such as CLAUDIN-6, CLAUDIN-18.2 and CLAUDIN-12, c-MYC, CT, Cyp-B, DAM, ELF2M, ETV6-AML1, G250, GAGE, GnT-V, Gap 100, HAGE, HER-2/neu, HPV-E7, HPV-E6, HAST-2, hTERT (or hTRT), LAGE, LDLR/FUT, MAGE-A, preferably MAGE-A1 , MAGE-A2, MAGE- A3, MAGE-A4, MAGE-A5, MAGE-A6, MAGE-A7, MAGE-A8, MAGE-A9, MAGE-A 10, MAGE-A 11, or MAGE-A12, MAGE-B, MAGE-C, MART- 1 /Melan-A, MC1R, Myosin/m, MUC1, MUM-1, MUM-2, MUM-3, NA88-A, NF1 , NY-ESO-1 , NY-BR-1 , pl90 minor BCR-abL, Pml/RARa, PRAME, proteinase 3, PSA, PSM, RAGE, RU1 or RU2, SAGE, SART-1 or SART-3, SCGB3A2, SCP1, SCP2, SCP3, SSX, SURVIVIN, TEL/AML1, TPI/m, TRP-1 , TRP-2, TRP-2/INT2, TPTE, WT, and WT-1. Cancer mutations vary with each individual. Thus, cancer mutations that encode novel epitopes (neo-epitopes) represent attractive targets in the development of vaccine compositions and immunotherapies. The efficacy of tumor immunotherapy relies on the selection of cancer-specific antigens and epitopes capable of inducing a potent immune response within a host. RNA can be used to deliver patient-specific tumor epitopes to a patient. Dendritic cells (DCs) residing in the spleen represent antigen-presenting cells of particular interest for RNA expression of immunogenic epitopes or antigens such as tumor epitopes. The use of multiple epitopes has been shown to promote therapeutic efficacy in tumor vaccine compositions. Rapid sequencing of the tumor mutanome may provide multiple epitopes for individualized vaccines which can be encoded by RNA (in particular, mRNA) described herein, e.g., as a single polypeptide wherein the epitopes are optionally separated by linkers. In some embodiments of the present disclosure, the RNA (in particular, mRNA) encodes at least one epitope, at least two epitopes, at least three epitopes, at least four epitopes, at least five epitopes, at least six epitopes, at least seven epitopes, at least eight epitopes, at least nine epitopes, or at least ten epitopes. Exemplary embodiments include RNA (in particular, mRNA) that encodes at least five epitopes (termed a "pentatope") and RNA (in particular, mRNA) that encodes at least ten epitopes (termed a "decatope").

In some embodiments, the antigen or epitope is derived from a pathogen-associated antigen, in particular from a viral antigen.

In some embodiments, the antigen or epitope is derived from a coronavirus protein, an immunogenic variant thereof, or an immunogenic fragment of the coronavirus protein or the immunogenic variant thereof. Thus, in some embodiments, the mRNA used in the present disclosure encodes an amino acid sequence comprising a coronavirus protein, an immunogenic variant thereof, or an immunogenic fragment of the coronavirus protein or the immunogenic variant thereof.

In some embodiments, the antigen or epitope is derived from a coronavirus S protein, an immunogenic variant thereof, or an immunogenic fragment of the coronavirus S protein or the immunogenic variant thereof. Thus, in some embodiments, the RNA (in particular, mRNA) described in the present disclosure encodes an amino acid sequence comprising a coronavirus S protein, an immunogenic variant thereof, or an immunogenic fragment of the coronavirus S protein or the immunogenic variant thereof. In some embodiments, the coronavirus is MERS-CoV. In some embodiments, the coronavirus is SARS-CoV. In some embodiments, the coronavirus is SARS-CoV-2.

Coronaviruses are enveloped, positive-sense, single-stranded RNA ((+) ssRNA) viruses. They have the largest genomes (26-32 kb) among known RNA viruses and are phylogenetically divided into four genera ($\alpha$, $\beta$, $\gamma$, and $\delta$), with betacoronaviruses further subdivided into four lineages (A, B, C, and D). Coronaviruses infect a wide range of avian and mammalian species, including humans. Some human coronaviruses generally cause mild respiratory diseases, although severity can be greater in infants, the elderly, and the immunocompromised. Middle East respiratory syndrome coronavirus (MERS-CoV) and severe acute respiratory syndrome coronavirus (SARS-CoV), belonging to betacoronavirus lineages C and B, respectively, are highly pathogenic. Both viruses emerged into the human population from animal reservoirs within the last 15 years and caused outbreaks with high case-fatality rates. The outbreak of severe acute respiratory syndrome coronavirus-2 (SARS-CoV-2) that causes atypical pneumonia (coronavirus disease 2019; COVID-19) has raged in China since mid-December 2019, and has developed to be a public health emergency of international concern. SARS-CoV-2 (MN908947.3) belongs to betacoronavirus lineage B. It has at least 70% sequence similarity to SARS-CoV.

In general, coronaviruses have four structural proteins, namely, envelope (E), membrane (M), nucleocapsid (N), and spike (S). The E and M proteins have important functions in the viral assembly, and the N protein is necessary for viral RNA synthesis. The critical glycoprotein S is responsible for virus binding and entry into target cells. The S protein is synthesized as a single-chain inactive precursor that is cleaved by furin-like host proteases in the producing cell into two noncovalently associated subunits, S1 and S2. The S1 subunit contains the receptor-binding domain (RBD), which recognizes the host-cell receptor. The S2 subunit contains the fusion peptide, two heptad repeats, and a transmembrane domain, all of which are required to mediate fusion of the viral and host-cell membranes by undergoing a large conformational rearrangement. The S1 and S2 subunits trimerize to form a large prefusion spike.

The S precursor protein of SARS-CoV-2 can be proteolytically cleaved into S1 (685 aa) and S2 (588 aa) subunits. The S1 subunit comprises the receptor-binding domain (RBD), which mediates virus entry into sensitive cells through the host angiotensin-converting enzyme 2 (ACE2) receptor.

In some embodiments, the antigen or epitope is derived from a SARS-CoV-2 S protein, an immunogenic variant thereof, or an immunogenic fragment of the SARS-CoV-2 S protein or the immunogenic variant thereof. In some embodiments, the RNA (in particular, mRNA) described in the present disclosure encodes an amino acid sequence comprising a SARS-CoV-2 S protein, an immunogenic variant thereof, or an immunogenic fragment of the SARS-CoV-2 S protein or the immunogenic variant thereof. Thus, in some embodiments, the encoded amino acid sequence comprises an epitope of SARS-CoV-2 S protein or an immunogenic variant thereof for inducing an immune response against coronavirus S protein, in particular SARS-CoV-2 S protein in a subject. In some embodiments, RNA (in particular, mRNA) is administered to provide (following expression by appropriate target cells) antigen for induction of an immune response, e.g., antibodies and/or immune effector cells, which is targeted to target antigen (coronavirus S protein, in particular SARS-CoV-2 S protein) or a procession product thereof. In some embodiments, the immune response which is to be induced according to the present disclosure is a B cell-mediated immune response, i.e., an antibody-mediated immune response. Additionally or alternatively, in some embodiments, the immune response which is to be induced according to the present disclosure is a T cell-mediated immune response. In some embodiments, the immune response is an anti-coronavirus, in particular anti-SARS-CoV-2 immune response.

SARS-CoV-2 coronavirus full length spike (S) protein consist of 1273 amino acids and has the following amino acid sequence:

MFVFLVLLPLVSSQCVNLTTRTQLPPAYTNSFTRGVYYPDKVFRSSVLHSTQDLFLPFFSNV
TWFHAIHVSGTNGTKRFDNPVLPFNDGVYFASTEKSNIIRGWIFGTTLDSKTQSLLIVNNAT
NVVIKVCEFQFCNDPFLGVYYHKNNKSWMESEFRVYSSANNCTFEYVSQPFLMDLEGKQGNF
KNLREFVFKNIDGYFKIYSKHTPINLVRDLPQGFSALEPLVDLPIGINITRFQTLLALHRSY
LTPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSA
SFSTFKCYGVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFNCYFPLQSYG
FQPTNGVGYQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQDVNCTEV
PVAIHADQLTPTWRVYSTGSNVFQTRAGCLIGAEHVNNSYECDIPIGAGICASYQTQTNSPR
RARSVASQSIIAYTMSLGAENSVAYSNNSIAIPTNFTISVTTEILPVSMTKTSVDCTMYICG
DSTECSNLLLQYGSFCTQLNRALTGIAVEQDKNTQEVFAQVKQIYKTPPIKDFGGFNFSQIL
PDPSKPSKRSFIEDLLFNKVTLADAGFIKQYGDCLGDIAARDLICAQKFNGLTVLPPLLTDE
MIAQYTSALLAGTITSGWTFGAGAALQIPFAMQMAYRFNGIGVTQNVLYENQKLIANQFNSA
IGKIQDSLSSTASALGKLQDVVNQNAQALNTLVKQLSSNFGAISSVLNDILSRLDKVEAEVQ
IDRLITGRLQSLQTYVTQQLIRAAEIRASANLAATKMSECVLGQSKRVDFCGKGYHLMSFPQ
SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWPWYIWLGFIAGLIAIVMVTIMLCCMTSC
CSCLKGCCSCGSCCKFDEDDSEPVLKGVKLHYT

(SEQ ID NO: 11)

For purposes of the present disclosure, the above sequence is considered the wild-type SARS-CoV-2 S protein amino acid sequence. Position numberings in SARS-CoV-2 S protein given herein are in relation to the amino acid sequence according to SEQ ID NO: 11 and corresponding positions in SARS-CoV-2 S protein variants.

In specific embodiments, full length spike (S) protein according to SEQ ID NO: 11 is modified in such a way that the prototypical prefusion conformation is stabilized. Stabilization of the prefusion conformation may be obtained by introducing two consecutive proline substitutions at amino acid residues 986 and 987 in the full-length spike protein. Specifically, spike (S) protein stabilized protein variants are obtained in a way that the amino acid residue at position 986 is exchanged to proline and the amino acid residue at position 987 is also exchanged to proline. In one embodiment, a SARS-CoV-2 S protein variant wherein the prototypical prefusion conformation is stabilized comprises the following amino acid sequence:

MFVFLVLLPLVSSQCVNLTTRTQLPPAYTNSFTRGVYYPDKVFRSSVLHSTQDLFLPFFSNV
TWFHAIHVSGTNGTKRFDNPVLPFNDGVYFASTEKSNIIRGWIFGTTLDSKTQSLLIVNNAT
NVVIKVCEFQFCNDPFLGVYYHKNNKSWMESEFRVYSSANNCTFEYVSQPFLMDLEGKQGNF
KNLREFVFKNIDGYFKIYSKHTPINLVRDLPQGFSALEPLVDLPIGINITRFQTLLALHRSY
LTPGDSSSGWTAGAAAYYVGYLQPRTFLLKYNENGTITDAVDCALDPLSETKCTLKSFTVEK
GIYQTSNFRVQPTESIVRFPNITNLCPFGEVFNATRFASVYAWNRKRISNCVADYSVLYNSA
SFSTFKCYGVSPTKLNDLCFTNVYADSFVIRGDEVRQIAPGQTGKIADYNYKLPDDFTGCVI
AWNSNNLDSKVGGNYNYLYRLFRKSNLKPFERDISTEIYQAGSTPCNGVEGFNCYFPLQSYG
FQPTNGVGYQPYRVVVLSFELLHAPATVCGPKKSTNLVKNKCVNFNFNGLTGTGVLTESNKK
FLPFQQFGRDIADTTDAVRDPQTLEILDITPCSFGGVSVITPGTNTSNQVAVLYQDVNCTEV
PVAIHADQLTPTWRVYSTGSNVFQTRAGCLIGAEHVNNSYECDIPIGAGICASYQTQTNSPR
RARSVASQSIIAYTMSLGAENSVAYSNNSIAIPTNFTISVTTEILPVSMTKTSVDCTMYICG
DSTECSNLLLQYGSFCTQLNRALTGIAVEQDKNTQEVFAQVKQIYKTPPIKDFGGFNFSQIL
PDPSKPSKRSFIEDLLFNKVTLADAGFIKQYGDCLGDIAARDLICAQKFNGLTVLPPLLTDE
MIAQYTSALLAGTITSGWTFGAGAALQIPFAMQMAYRFNGIGVTQNVLYENQKLIANQFNSA
IGKIQDSLSSTASALGKLQDVVNQNAQALNTLVKQLSSNFGAISSVLNDILSRLDPPEAEVQ
IDRLITGRLQSLQTYVTQQLIRAAEIRASANLAATKMSECVLGQSKRVDFCGKGYHLMSFPQ
SAPHGVVFLHVTYVPAQEKNFTTAPAICHDGKAHFPREGVFVSNGTHWFVTQRNFYEPQIIT
TDNTFVSGNCDVVIGIVNNTVYDPLQPELDSFKEELDKYFKNHTSPDVDLGDISGINASVVN
IQKEIDRLNEVAKNLNESLIDLQELGKYEQYIKWPWYIWLGFIAGLIAIVMVTIMLCCMTSC
CSCLKGCCSCGSCCKFDEDDSEPVLKGVKLHYT

(SEQ ID NO: 12)

Those skilled in the art are aware of various spike variants, and/or resources that document them.

In some embodiments, RNA (in particular, mRNA) described herein (e.g., contained in the compositions/formulations of the present disclosure and/or used in the methods of the present disclosure) encodes an amino acid sequence which comprises, consists essentially of or consists of a spike (S) protein of SARS-CoV-2, a variant thereof, or a fragment thereof.

In some embodiments, the encoded amino acid sequence comprises the amino acid sequence of amino acids 17 to 1273 of SEQ ID NO: 11 or 12, an amino acid sequence having at least 99%, 98%, 97%, 96%, 95%, 90%, 85%, or 80% identity to the amino acid sequence of amino acids 17 to 1273 of SEQ ID NO: 11 or 12, or an immunogenic fragment of the amino acid sequence of amino acids 17 to 1273 of SEQ ID NO: 11 or 12, or the amino acid sequence having at least 99%, 98%, 97%, 96%, 95%, 90%, 85%, or 80% identity to the amino acid sequence of amino acids 17 to 1273 of SEQ ID NO: 11 or 12. In some embodiments, the encoded amino acid sequence comprises the amino acid sequence of amino acids 17 to 1273 of SEQ ID NO: 11 or 12.

In some embodiments, the encoded amino acid sequence comprises, consists essentially of or consists of SARS-CoV-2 spike S1 fragment (S1) (the S1 subunit of a spike protein (S) of SARS-CoV-2), a variant thereof, or a fragment thereof.

In some embodiments, the encoded amino acid sequence comprises the amino acid sequence of amino acids 17 to 683 of SEQ ID NO: 11, an amino acid sequence having at least 99%, 98%, 97%, 96%, 95%, 90%, 85%, or 80% identity to the amino acid sequence of amino acids 17 to 683 of SEQ ID NO: 11, or an immunogenic fragment of the amino acid sequence of amino acids 17 to 683 of SEQ ID NO: 11, or the amino acid sequence having at least 99%, 98%, 97%, 96%,

95%, 90%, 85%, or 80% identity to the amino acid sequence of amino acids 17 to 683 of SEQ ID NO: 11. In some embodiments, the encoded amino acid sequence comprises the amino acid sequence of amino acids 17 to 683 of SEQ ID NO: 11.

In some embodiments, the encoded amino acid sequence comprises the amino acid sequence of amino acids 17 to 685 of SEQ ID NO: 11, an amino acid sequence having at least 99%, 98%, 97%, 96%, 95%, 90%, 85%, or 80% identity to the amino acid sequence of amino acids 17 to 685 of SEQ ID NO: 11, or an immunogenic fragment of the amino acid sequence of amino acids 17 to 685 of SEQ ID NO: 11, or the amino acid sequence having at least 99%, 98%, 97%, 96%, 95%, 90%, 85%, or 80% identity to the amino acid sequence of amino acids 17 to 685 of SEQ ID NO: 11. In some embodiments, the encoded amino acid sequence comprises the amino acid sequence of amino acids 17 to 685 of SEQ ID NO: 11.

In some embodiments, the encoded amino acid sequence comprises, consists essentially of or consists of the receptor binding domain (RBD) of the S1 subunit of a spike protein (S) of SARS-CoV-2, a variant thereof, or a fragment thereof. The amino acid sequence of amino acids 327 to 528 of SEQ ID NO: 11, a variant thereof, or a fragment thereof is also referred to herein as "RBD" or "RBD domain".

In some embodiments, the encoded amino acid sequence comprises the amino acid sequence of amino acids 327 to 528 of SEQ ID NO: 11, an amino acid sequence having at least 99%, 98%, 97%, 96%, 95%, 90%, 85%, or 80% identity to the amino acid sequence of amino acids 327 to 528 of SEQ ID NO: 11, or an immunogenic fragment of the amino acid sequence of amino acids 327 to 528 of SEQ ID NO: 11, or the amino acid sequence having at least 99%, 98%, 97%, 96%, 95%, 90%, 85%, or 80% identity to the amino acid sequence of amino acids 327 to 528 of SEQ ID NO: 11. In some embodiments, the encoded amino acid sequence comprises the amino acid sequence of amino acids 327 to 528 of SEQ ID NO: 11.

According to certain embodiments, a signal peptide is fused, either directly or through a linker, to a SARS-CoV-2 S protein, a variant thereof, or a fragment thereof, i.e., the antigenic peptide or protein. Accordingly, in some embodiments, a signal peptide is fused to the above described amino acid sequences derived from SARS-CoV-2 S protein or immunogenic fragments thereof (antigenic peptides or proteins) comprised by the encoded amino acid sequences described above.

In some embodiments, the encoded amino acid sequence comprises the amino acid sequence of SEQ ID NO: 11 or 12, an amino acid sequence having at least 99%, 98%, 97%, 96%, 95%, 90%, 85%, or 80% identity to the amino acid sequence of SEQ ID NO: 11 or 12, or an immunogenic fragment of the amino acid sequence of SEQ ID NO: 11 or 12, or the amino acid sequence having at least 99%, 98%, 97%, 96%, 95%, 90%, 85%, or 80% identity to the amino acid sequence of SEQ ID NO: 11 or 12. In some embodiments, the encoded amino acid sequence comprises the amino acid sequence of SEQ ID NO: 11 or 12.

In some embodiments, the encoded amino acid sequence comprises the amino acid sequence of amino acids 1 to 683 of SEQ ID NO: 11, an amino acid sequence having at least 99%, 98%, 97%, 96%, 95%, 90%, 85%, or 80% identity to the amino acid sequence of amino acids 1 to 683 of SEQ ID NO: 11, or an immunogenic fragment of the amino acid sequence of amino acids 1 to 683 of SEQ ID NO: 11, or the amino acid sequence having at least 99%, 98%, 97%, 96%, 95%, 90%, 85%, or 80% identity to the amino acid sequence of amino acids 1 to 683 of SEQ ID NO: 11. In some embodiments, the encoded amino acid sequence comprises the amino acid sequence of amino acids 1 to 683 of SEQ ID NO: 11.

In some embodiments, the encoded amino acid sequence comprises the amino acid sequence of amino acids 1 to 685 of SEQ ID NO: 11, an amino acid sequence having at least 99%, 98%, 97%, 96%, 95%, 90%, 85%, or 80% identity to the amino acid sequence of amino acids 1 to 685 of SEQ ID NO: 11, or an immunogenic fragment of the amino acid sequence of amino acids 1 to 685 of SEQ ID NO: 11, or the amino acid sequence having at least 99%, 98%, 97%, 96%, 95%, 90%, 85%, or 80% identity to the amino acid sequence of amino acids 1 to 685 of SEQ ID NO: 11. In some embodiments, the encoded amino acid sequence comprises the amino acid sequence of amino acids 1 to 685 of SEQ ID NO: 11.

According to certain embodiments, a trimerization domain is fused, either directly or through a linker, e.g., a glycine/serine linker, to a SARS-CoV-2 S protein, a variant thereof, or a fragment thereof, i.e., the antigenic peptide or protein. Accordingly, in some embodiments, a trimerization domain is fused to the above described amino acid sequences derived from SARS-CoV-2 S protein or immunogenic fragments thereof (antigenic peptides or proteins) comprised by the encoded amino acid sequences described above (which may optionally be fused to a signal peptide as described above).

Such trimerization domains are preferably located at the C-terminus of the antigenic peptide or protein, without being limited thereto. Trimerization domains as defined herein preferably allow the trimerization of the antigenic peptide or protein as encoded by the RNA. Examples of trimerization domains as defined herein include, without being limited thereto, foldon, the natural trimerization domain of T4 fibritin. The C-terminal domain of T4 fibritin (foldon) is obligatory for the formation of the fibritin trimer structure and can be used as an artificial trimerization domain. In some embodiments, the trimerization domain as defined herein includes, without being limited thereto, a sequence comprising the amino acid sequence of SEQ ID NO: 13 or a functional variant thereof.

In some embodiments, a trimerization domain comprises the amino acid sequence of amino acids 3 to 29 of SEQ ID NO: 13, an amino acid sequence having at least 99%, 98%, 97%, 96%, 95%, 90%, 85%, or 80% identity to the amino acid sequence of amino acids 3 to 29 of SEQ ID NO: 13, or a functional fragment of the amino acid sequence of amino acids 3 to 29 of SEQ ID NO: 13, or the amino acid sequence having at least 99%, 98%, 97%, 96%, 95%, 90%, 85%, or 80% identity to the amino acid sequence of amino acids 3 to 29 of SEQ ID NO: 13. In some embodiments, a trimerization domain comprises the amino acid sequence of amino acids 3 to 29 of SEQ ID NO: 13.

In some embodiments, a trimerization domain comprises the amino acid sequence SEQ ID NO: 13, an amino acid sequence having at least 99%, 98%, 97%, 96%, 95%, 90%, 85%, or 80% identity to the amino acid sequence of SEQ ID NO: 13, or a functional fragment of the amino acid sequence of SEQ ID NO: 13, or the amino acid sequence having at least 99%, 98%, 97%, 96%, 95%, 90%, 85%, or 80% identity to the amino acid sequence of SEQ ID NO: 13. In some embodiments, a trimerization domain comprises the amino acid sequence of SEQ ID NO: 13. In some embodiments, the encoded amino acid sequence comprises the amino acid sequence of SEQ ID NO: 14, an amino acid sequence having at least 99%, 98%, 97%, 96%, 95%, 90%, 85%, or 80% identity to the amino acid sequence of SEQ ID NO: 14, or an immunogenic fragment of the amino acid sequence of SEQ ID NO: 14, or the amino acid sequence having at least 99%, 98%, 97%, 96%, 95%, 90%, 85%, or 80% identity to the amino acid sequence of SEQ ID NO: 14. In some embodiments, the encoded amino acid sequence comprises the amino acid sequence of SEQ ID NO: 14.

According to certain embodiments, a transmembrane domain is fused, either directly or through a linker, e.g., a glycine/serine linker, to a SARS-CoV-2 S protein, a variant thereof, or a fragment thereof, i.e., the antigenic peptide or protein. Accordingly, in some embodiments, a transmembrane domain is fused to the above described amino acid sequences derived from SARS-CoV-2 S protein or immunogenic fragments thereof (antigenic peptides or proteins) comprised by the encoded amino acid sequences described above (which may optionally be fused to a signal peptide and/or trimerization domain as described above). Such transmembrane domains are preferably located at the C-terminus of the antigenic peptide or protein, without being limited thereto. Preferably, such transmembrane domains are located at the C-terminus of the trimerization domain, if present, without being limited thereto. In some embodiments, a trimerization domain is present between the SARS-CoV-2 S protein, a variant thereof, or a fragment thereof, i.e., the antigenic peptide or protein, and the transmembrane domain. Transmembrane domains as defined herein preferably allow the anchoring into a cellular membrane of the antigenic peptide or protein as encoded by the RNA. In some embodiments, the transmembrane domain sequence as defined herein includes, without being limited thereto, the transmembrane domain sequence of SARS-CoV-2 S protein, in particular a sequence comprising the amino acid sequence of amino acids 1207 to 1254 of SEQ ID NO: 11 or a functional variant thereof. In some embodiments, a transmembrane domain sequence comprises the amino acid sequence of amino acids 1207 to 1254 of SEQ ID NO: 11, an amino acid sequence having at least 99%, 98%, 97%, 96%, 95%, 90%, 85%, or 80% identity to the amino acid sequence of amino acids 1207 to 1254 of SEQ ID NO: 11, or a functional fragment of the amino acid sequence of amino acids 1207 to 1254 of SEQ ID NO: 11, or the amino acid sequence having at least 99%, 98%, 97%, 96%, 95%, 90%, 85%, or 80% identity to the amino acid sequence of amino acids 1207 to 1254 of SEQ ID NO: 11. In some embodiments, a transmembrane domain sequence comprises the amino acid sequence of amino acids 1207 to 1254 of SEQ ID NO: 11.

In some embodiments, the encoded amino acid sequence comprises the amino acid sequence of SEQ ID NO: 15, an amino acid sequence having at least 99%, 98%, 97%, 96%, 95%, 90%, 85%, or 80% identity to the amino acid sequence of SEQ ID NO: 15, or an immunogenic fragment of the amino acid sequence of SEQ ID NO: 15, or the amino acid sequence having at least 99%, 98%, 97%, 96%, 95%, 90%, 85%, or 80% identity to the amino acid sequence of SEQ ID NO: 15. In some embodiments, the encoded amino acid sequence comprises the amino acid sequence of SEQ ID NO: 15.

In some embodiments, the encoded amino acid sequence comprises the amino acid sequence of SEQ ID NO: 16, an amino acid sequence having at least 99%, 98%, 97%, 96%, 95%, 90%, 85%, or 80% identity to the amino acid sequence of SEQ ID NO: 16, or an immunogenic fragment of the amino acid sequence of SEQ ID NO: 16, or the amino acid sequence having at least 99%, 98%, 97%, 96%, 95%, 90%, 85%, or 80% identity to the amino acid sequence of SEQ ID NO: 16. In some embodiments, the encoded amino acid sequence comprises the amino acid sequence of SEQ ID NO: 16.

In some embodiments, RNA (in particular, mRNA) described herein (e.g., contained in the compositions/formulations of the present disclosure and/or used in the methods of the present disclosure) (i) comprises the nucleotide sequence of SEQ ID NO: 17, a nucleotide sequence having at least 99%, 98%, 97%, 96%, 95%, 90%, 85%, or 80% identity to the nucleotide sequence of SEQ ID NO: 17, or a fragment of the nucleotide sequence of SEQ ID NO: 17, or the nucleotide sequence having at least 99%, 98%, 97%, 96%, 95%, 90%, 85%, or 80% identity to the nucleotide sequence of SEQ ID NO: 17; and/or (ii) encodes an amino acid sequence comprising the amino acid sequence of SEQ ID NO: 12, an amino acid sequence having at least 99%, 98%, 97%, 96%, 95%, 90%, 85%, or 80% identity to the amino acid sequence of SEQ ID NO: 12, or an immunogenic fragment of the amino acid sequence of SEQ ID NO: 12, or the amino acid sequence having at least 99%, 98%, 97%, 96%, 95%, 90%, 85%, or 80% identity to the amino acid sequence of SEQ ID NO: 12. In some embodiments, RNA (in particular, mRNA) described herein (e.g., contained in the compositions/formulations of the present disclosure and/or used in the methods of the present disclosure) (i) comprises the nucleotide sequence of SEQ ID NO: 17; and/or (ii) encodes an amino acid sequence comprising the amino acid sequence of SEQ ID NO: 12.

In some embodiments, RNA (in particular, mRNA) described herein (e.g., contained in the compositions/formulations of the present disclosure and/or used in the methods of the present disclosure) is nucleoside modified messenger RNA (modRNA) and (i) comprises the nucleotide sequence of SEQ ID NO: 17, a nucleotide sequence having at least 99%, 98%, 97%, 96%, 95%, 90%, 85%, or 80% identity to the nucleotide sequence of SEQ ID NO: 17, or a fragment of the nucleotide sequence of SEQ ID NO: 17, or the nucleotide sequence having at least 99%, 98%, 97%, 96%, 95%, 90%, 85%, or 80% identity to the nucleotide sequence of SEQ ID NO: 17; and/or (ii) encodes an amino acid sequence comprising the amino acid sequence of SEQ ID NO: 12, an amino acid sequence having at least 99%, 98%, 97%, 96%, 95%, 90%, 85%, or 80% identity to the amino acid sequence of SEQ ID NO: 12, or an immunogenic fragment of the amino acid sequence of SEQ ID NO: 12, or the amino acid sequence having at least 99%, 98%, 97%, 96%, 95%, 90%, 85%, or 80% identity to the amino acid sequence of SEQ ID NO: 12. In some embodiments, RNA (in particular, mRNA) described herein (e.g., contained in the compositions/formulations of the present disclosure and/or used in the methods of the present disclosure) is nucleoside modified messenger RNA (modRNA) and (i) comprises the nucleotide sequence of SEQ ID NO: 17; and/or (ii) encodes an amino acid sequence comprising the amino acid sequence of SEQ ID NO: 12.

In some embodiments, RNA (in particular, mRNA) described herein (e.g., contained in the compositions/formulations of the present disclosure and/or used in the methods of the present disclosure) (i) comprises the nucleotide sequence of SEQ ID NO: 18, a nucleotide sequence having at least 99%, 98%, 97%, 96%, 95%, 90%, 85%, or 80% identity to the nucleotide sequence of SEQ ID NO: 18, or a fragment of the nucleotide sequence of SEQ ID NO: 18, or the nucleotide sequence having at least 99%, 98%, 97%, 96%, 95%, 90%, 85%, or 80% identity to the nucleotide sequence of SEQ ID NO: 18; and/or (ii) encodes an amino acid sequence comprising the amino acid sequence of SEQ ID NO: 14, an amino acid sequence having at least 99%, 98%, 97%, 96%, 95%, 90%, 85%, or 80% identity to the amino acid sequence of SEQ ID NO: 14, or an immunogenic fragment of the amino acid sequence of SEQ ID NO: 14, or the amino acid sequence having at least 99%, 98%, 97%, 96%, 95%, 90%, 85%, or 80% identity to the amino acid sequence of SEQ ID NO: 14. In some embodiments, RNA (in particular, mRNA) described herein (e.g., contained in the compositions/formulations of the present disclosure and/or used in the methods of the present disclosure) (i) comprises the nucleotide sequence of SEQ ID NO: 18; and/or (ii) encodes an amino acid sequence comprising the amino acid sequence of SEQ ID NO: 14.

In some embodiments, RNA (in particular, mRNA) described herein (e.g., contained in the compositions/formulations of the present disclosure and/or used in the methods of the present disclosure) is nucleoside modified messenger RNA (modRNA) and (i) comprises the nucleotide sequence of SEQ ID NO: 18, a nucleotide sequence having at least 99%, 98%, 97%, 96%, 95%, 90%, 85%, or 80% identity to the nucleotide sequence of SEQ ID NO: 18, or a fragment of the nucleotide sequence of SEQ ID NO: 18, or the nucleotide sequence having at least 99%, 98%, 97%, 96%, 95%, 90%, 85%, or 80% identity to the nucleotide sequence of SEQ ID NO: 18; and/or (ii) encodes an amino acid sequence comprising the amino acid sequence of SEQ ID NO: 14, an amino acid sequence having at least 99%, 98%, 97%, 96%, 95%, 90%, 85%, or 80% identity to the amino acid sequence of SEQ ID NO: 14, or an immunogenic fragment of the amino acid sequence of SEQ ID NO: 14, or the amino acid sequence having at least 99%, 98%, 97%, 96%, 95%, 90%, 85%, or 80% identity to the amino acid sequence of SEQ ID NO: 14. In some embodiments, RNA (in particular, mRNA) described herein (e.g., contained in the compositions/formulations of the present disclosure and/or used in the methods of the present disclosure) is nucleoside modified messenger RNA (modRNA) and (i) comprises the nucleotide sequence of SEQ ID NO: 18; and/or (ii) encodes an amino acid sequence comprising the amino acid sequence of SEQ ID NO: 14.

In some embodiments, RNA (in particular, mRNA) described herein (e.g., contained in the compositions/formulations of the present disclosure and/or used in the methods of the present disclosure) (i) comprises the nucleotide sequence of SEQ ID NO: 19, a nucleotide sequence having at least 99%, 98%, 97%, 96%, 95%, 90%, 85%, or 80% identity to the nucleotide sequence of SEQ ID NO: 19, or a fragment of the nucleotide sequence of SEQ ID NO: 19, or the nucleotide sequence having at least 99%, 98%, 97%, 96%, 95%, 90%, 85%, or 80% identity to the nucleotide sequence of SEQ ID NO: 19; and/or (ii) encodes an amino acid sequence comprising the amino acid sequence of SEQ ID NO: 15, an amino acid sequence having at least 99%, 98%, 97%, 96%, 95%, 90%, 85%, or 80% identity to the amino acid sequence of SEQ ID NO: 15, or an immunogenic fragment of the amino acid sequence of SEQ ID NO: 15, or the amino acid sequence having at least 99%, 98%, 97%, 96%, 95%, 90%, 85%, or 80% identity to the amino acid sequence of SEQ ID NO: 15. In some embodiments, RNA (in particular, mRNA) described herein (e.g., contained in the compositions/formulations of the present disclosure and/or used in the methods of the present disclosure) (i) comprises the nucleotide sequence of SEQ ID NO: 19; and/or (ii) encodes an amino acid sequence comprising the amino acid sequence of SEQ ID NO: 15.

In some embodiments, RNA (in particular, mRNA) described herein (e.g., contained in the compositions/formulations of the present disclosure and/or used in the methods of the present disclosure) is nucleoside modified messenger RNA (modRNA) and (i) comprises the nucleotide sequence of SEQ ID NO: 19, a nucleotide sequence having at least 99%, 98%, 97%, 96%, 95%, 90%, 85%, or 80% identity to the nucleotide sequence of SEQ ID NO: 19, or a fragment of the nucleotide sequence of SEQ ID NO: 19, or the nucleotide sequence having at least 99%, 98%, 97%, 96%, 95%, 90%, 85%, or 80% identity to the nucleotide sequence of SEQ ID NO: 19; and/or (ii) encodes an amino acid sequence comprising the amino acid sequence of SEQ ID NO: 15, an amino acid sequence having at least 99%, 98%, 97%, 96%, 95%, 90%, 85%, or 80% identity to the amino acid sequence of SEQ ID NO: 15, or an immunogenic fragment of the amino

acid sequence of SEQ ID NO: 15, or the amino acid sequence having at least 99%, 98%, 97%, 96%, 95%, 90%, 85%, or 80% identity to the amino acid sequence of SEQ ID NO: 15. In some embodiments, RNA (in particular, mRNA) described herein (e.g., contained in the compositions/formulations of the present disclosure and/or used in the methods of the present disclosure) is nucleoside modified messenger RNA (modRNA) and (i) comprises the nucleotide sequence of SEQ ID NO: 19; and/or (ii) encodes an amino acid sequence comprising the amino acid sequence of SEQ ID NO: 15.

In some embodiments, RNA (in particular, mRNA) described herein (e.g., contained in the compositions/formulations of the present disclosure and/or used in the methods of the present disclosure) (i) comprises the nucleotide sequence of SEQ ID NO: 20, a nucleotide sequence having at least 99%, 98%, 97%, 96%, 95%, 90%, 85%, or 80% identity to the nucleotide sequence of SEQ ID NO: 20, or a fragment of the nucleotide sequence of SEQ ID NO: 20, or the nucleotide sequence having at least 99%, 98%, 97%, 96%, 95%, 90%, 85%, or 80% identity to the nucleotide sequence of SEQ ID NO: 20; and/or (ii) encodes an amino acid sequence comprising the amino acid sequence of SEQ ID NO: 16, an amino acid sequence having at least 99%, 98%, 97%, 96%, 95%, 90%, 85%, or 80% identity to the amino acid sequence of SEQ ID NO: 16, or an immunogenic fragment of the amino acid sequence of SEQ ID NO: 16, or the amino acid sequence having at least 99%, 98%, 97%, 96%, 95%, 90%, 85%, or 80% identity to the amino acid sequence of SEQ ID NO: 16. In some embodiments, RNA (in particular, mRNA) described herein (e.g., contained in the compositions/formulations of the present disclosure and/or used in the methods of the present disclosure) (i) comprises the nucleotide sequence of SEQ ID NO: 20; and/or (ii) encodes an amino acid sequence comprising the amino acid sequence of SEQ ID NO: 16. In some embodiments, RNA is nucleoside modified messenger RNA (modRNA) and (i) comprises the nucleotide sequence of SEQ ID NO: 20, a nucleotide sequence having at least 99%, 98%, 97%, 96%, 95%, 90%, 85%, or 80% identity to the nucleotide sequence of SEQ ID NO: 20, or a fragment of the nucleotide sequence of SEQ ID NO: 20, or the nucleotide sequence having at least 99%, 98%, 97%, 96%, 95%, 90%, 85%, or 80% identity to the nucleotide sequence of SEQ ID NO: 20; and/or (ii) encodes an amino acid sequence comprising the amino acid sequence of SEQ ID NO: 16, an amino acid sequence having at least 99%, 98%, 97%, 96%, 95%, 90%, 85%, or 80% identity to the amino acid sequence of SEQ ID NO: 16, or an immunogenic fragment of the amino acid sequence of SEQ ID NO: 16, or the amino acid sequence having at least 99%, 98%, 97%, 96%, 95%, 90%, 85%, or 80% identity to the amino acid sequence of SEQ ID NO: 16. In some embodiments, RNA (in particular, mRNA) described herein (e.g., contained in the compositions/formulations of the present disclosure and/or used in the methods of the present disclosure) is nucleoside modified messenger RNA (modRNA) and (i) comprises the nucleotide sequence of SEQ ID NO: 20; and/or (ii) encodes an amino acid sequence comprising the amino acid sequence of SEQ ID NO: 16.

In some embodiments, RNA (in particular, mRNA) described herein (e.g., contained in the compositions/formulations of the present disclosure and/or used in the methods of the present disclosure) comprises BNT162b2. BNT162b2 is an mRNA vaccine for prevention of COVID-19 and demonstrated an efficacy of 95% or more at preventing COVID-19. The vaccine is made of a 5'capped mRNA encoding for the full-length SARS-CoV-2 spike glycoprotein (S) encapsulated in lipid nanoparticles (LNPs). The RNA may be presented as a product containing BNT162b2 as active substance and other ingredients comprising: a cationically ionizable lipid (in particular, a compound of formula (I)); a polymer-conjugated lipid, preferably selected from the groups consisting of a sarcosinylated lipid and a pegylated lipid; 1,2-distearoyl-sn-glycero-3-phosphocholine (DSPC); and cholesterol. The sequence of the S protein was chosen based on the sequence for the "SARS-CoV-2 isolate Wuhan-Hu -1": GenBank: MN908947.3 (complete genome) and GenBank: QHD43416.1 (spike surface glycoprotein). The active substance consists of a single-stranded, 5'-capped codon-optimized mRNA that is translated into the spike antigen of SARS-CoV-2. The protein sequence contains two proline mutations, which ensure an antigenically optimal pre-fusion confirmation (P2 S). The RNA does not contain any uridines; instead of uridine the modified N1-methylpseudouridine is used in RNA synthesis. The RNA contains common structural elements optimized for mediating high RNA stability and translational efficiency. The LNPs protect the RNA from degradation by RNAses and enable transfection of host cells after intramuscular (IM) delivery. The mRNA is translated into the SARS-CoV-2 S protein in the host cell. The S protein is then expressed on the cell surface where it induces an adaptive immune response. The S protein is identified as a target for neutralising antibodies against the virus and is therefore considered a relevant vaccine component. BNT162b2 is administered to adults intramuscularly (IM) in two 30 $\mu$g doses given 21 days apart.

In some embodiments, the RNA (in particular, mRNA) described herein is a modified RNA, in particular a stabilized mRNA. In some embodiments, the RNA comprises a modified nucleoside in place of at least one uridine. In some embodiments, the RNA comprises a modified nucleoside in place of each uridine. In some embodiments, the modified nucleoside is independently selected from pseudouridine ($\psi$), N1-methyl-pseudouridine (m1$\psi$), and 5-methyl-uridine (m5U).

In some embodiments, the RNA (in particular, mRNA) described herein comprises a modified nucleoside in place of uridine.

In some embodiments, the modified nucleoside is selected from pseudouridine ($\psi$), N1-methyl-pseudouridine (m1$\psi$), and 5-methyl-uridine (m5U).

In some embodiments, the RNA (in particular, mRNA) described herein comprises a 5' cap. In some embodi-

ments, $m_2^{7,3'-O}$Gppp($m_1^{2'-O}$) ApG is utilized as specific capping structure at the 5'-end of the mRNA.

In some embodiments, the RNA (in particular, mRNA) encoding an amino acid sequence comprising a SARS-CoV-2 S protein, an immunogenic variant thereof, or an immunogenic fragment of the SARS-CoV-2 S protein or the immunogenic variant thereof comprises a 5' UTR comprising the nucleotide sequence of SEQ ID NO: 6, or a nucleotide sequence having at least 99%, 98%, 97%, 96%, 95%, 90%, 85%, or 80% identity to the nucleotide sequence of SEQ ID NO: 6.

In some embodiments, the RNA (in particular, mRNA) encoding an amino acid sequence comprising a SARS-CoV-2 S protein, an immunogenic variant thereof, or an immunogenic fragment of the SARS-CoV-2 S protein or the immunogenic variant thereof comprises a 3' UTR comprising the nucleotide sequence of SEQ ID NO: 7, or a nucleotide sequence having at least 99%, 98%, 97%, 96%, 95%, 90%, 85%, or 80% identity to the nucleotide sequence of SEQ ID NO: 7.

In some embodiments, the RNA (in particular, mRNA) encoding an amino acid sequence comprising a SARS-CoV-2 S protein, an immunogenic variant thereof, or an immunogenic fragment of the SARS-CoV-2 S protein or the immunogenic variant thereof comprises a poly-A sequence.

In some embodiments, the poly-A sequence comprises at least 100 nucleotides.

In some embodiments, the poly-A sequence comprises or consists of the nucleotide sequence of SEQ ID NO: 8.

In some embodiments, the RNA (in particular, mRNA) described herein is formulated or is to be formulated as a liquid, a solid, or a combination thereof.

In some embodiments, the RNA (in particular, mRNA) described herein is formulated or is to be formulated for injection.

In some embodiments, the RNA (in particular, mRNA) described herein is formulated or is to be formulated for intramuscular administration.

In some embodiments, the RNA (in particular, mRNA) described herein is formulated or is to be formulated as a composition, e.g., a pharmaceutical composition.

In some embodiments, the composition comprises a cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)).

In some embodiments, the composition comprises a cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)) and one or more additional lipids. In some embodiments, the one or more additional lipids are selected from polymer-conjugated lipids, neutral lipids, and combinations thereof. In some embodiments, the neutral lipids include phospholipids, steroid lipids, and combinations thereof. In some embodiments, the one or more additional lipids are a combination of a polymer-conjugated lipid, a phospholipid, and a steroid lipid.

In some embodiments, the composition comprises a cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)); a polymer-conjugated lipid selected from the group consisting of a polysarcosine-conjugated lipid as disclosed herein; and cholesterol.

In some embodiments, the composition comprises a cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)); a polymer-conjugated lipid selected from the group consisting of a polysarcosine-conjugated lipid as disclosed herein; cholesterol; and a phospholipid. In some embodiments, the phospholipid is DSPC. In some embodiments, the phospholipid is DOPE.

In some embodiments, the composition comprises a cationically ionizable lipid of formula (IV-1), (IV-2), or (IV-3); a polymer-conjugated lipid selected from the group consisting of DMG-PEG 2000 and C14pSar23; cholesterol; and a phospholipid. In some embodiments, the phospholipid is DSPC. In some embodiments, the phospholipid is DOPE.

In some embodiments, the RNA is mRNA or saRNA.

In some embodiments, at least a portion of (i) the RNA, (ii) the cationically ionizable lipid, and if present, (iii) the one or more additional lipids is present in particles. In some embodiments, the particles are nanoparticles, such as lipid nanoparticles (LNPs).

In some embodiments, the composition, in particular the pharmaceutical composition, is a vaccine.

In some embodiments, the composition, in particular the pharmaceutical composition, further comprises one or more pharmaceutically acceptable carriers, diluents and/or excipients.

In some embodiments, the RNA and/or the composition, in particular the pharmaceutical composition, is/are a component of a kit.

In some embodiments, the kit further comprises instructions for use of the RNA for inducing an immune response against coronavirus in a subject.

In some embodiments, the kit further comprises instructions for use of the RNA for therapeutically or prophylactically treating a coronavirus infection in a subject.

In some embodiments, the subject is a human.

In some embodiments, the coronavirus is a betacoronavirus.

In some embodiments, the coronavirus is a sarbecovirus.

In some embodiments, the coronavirus is SARS-CoV-2.

The term "immunologically equivalent" means that the immunologically equivalent molecule such as the immunologically equivalent amino acid sequence exhibits the same or essentially the same immunological properties and/or exerts the same or essentially the same immunological effects, *e.g.,* with respect to the type of the immunological effect. In the context of the present disclosure, the term "immunologically equivalent" is preferably used with respect to the immunological effects or properties of antigens or antigen variants used for immunization. For example, an amino acid sequence is immunologically equivalent to a reference amino acid sequence if said amino acid sequence when exposed to the immune system of a subject induces an immune reaction having a specificity of reacting with the reference amino acid sequence. Thus, in some embodiments, a molecule which is immunologically equivalent to an antigen exhibits the same or essentially the same properties and/or exerts the same or essentially the same effects regarding the stimulation, priming and/or expansion of T cells as the antigen to which the T cells are targeted.

In some embodiments, the RNA (in particular, mRNA), e.g., RNA encoding vaccine antigen, described in the present disclosure is non-immunogenic. RNA encoding an immunostimulant may be administered according to the present disclosure to provide an adjuvant effect. The RNA encoding an immunostimulant may be standard RNA or non-immunogenic RNA.

The term "non-immunogenic RNA" (such as "non-immunogenic mRNA") as used herein refers to RNA that does not induce a response by the immune system upon administration, e.g., to a mammal, or induces a weaker response than would have been induced by the same RNA that differs only in that it has not been subjected to the modifications and treatments that render the non-immunogenic RNA non-immunogenic, *i.e.,* than would have been induced by standard RNA (stdRNA). In certain embodiments, non-immunogenic RNA, which is also termed modified RNA (modRNA) herein, is rendered non-immunogenic by incorporating modified nucleosides suppressing RNA-mediated activation of innate immune receptors into the RNA and/or limiting the amount of double-stranded RNA (dsRNA), e.g., by limiting the formation of double-stranded RNA (dsRNA), e.g., during *in vitro* transcription, and/or by removing double-stranded RNA (dsRNA), e.g., following *in vitro* transcription. In certain embodiments, non-immunogenic RNA is rendered non-immunogenic by incorporating modified nucleosides suppressing RNA-mediated activation of innate immune receptors into the RNA and/or by removing double-stranded RNA (dsRNA), e.g., following *in vitro* transcription.

For rendering the non-immunogenic RNA (especially mRNA) non-immunogenic by the incorporation of modified nucleosides, any modified nucleoside may be used as long as it lowers or suppresses immunogenicity of the RNA. Particularly preferred are modified nucleosides that suppress RNA-mediated activation of innate immune receptors. In some embodiments, the modified nucleosides comprise a replacement of one or more uridines with a nucleoside comprising a modified nucleobase. In some embodiments, the modified nucleobase is a modified uracil. In some embodiments, the nucleoside comprising a modified nucleobase is selected from the group consisting of 3-methyl-uridine ($m^3U$), 5-methoxy-uridine ($mo^5U$), 5-aza-uridine, 6-aza-uridine, 2-thio-5-aza-uridine, 2-thio-uridine ($s^2U$), 4-thio-uridine ($s^4U$), 4-thio-pseudouridine, 2-thio-pseudouridine, 5-hydroxy-uridine ($ho^5U$), 5-aminoallyl-uridine, 5-halo-uridine (e.g., 5-iodo-uridine or 5-bromo-uridine), uridine 5-oxyacetic acid ($cmo^5U$), uridine 5-oxyacetic acid methyl ester ($mcmo^5U$), 5-carboxymethyl-uridine ($cm^5U$), 1-carboxymethyl-pseudouridine, 5-carboxyhydroxymethyl-uridine ($chm^5U$), 5-carboxyhydroxymethyl-uridine methyl ester ($mchm^5U$), 5-methoxycarbonylmethyl-uridine ($mcm^5U$), 5-methoxycarbonylmethyl-2-thio-uridine ($mcm^5s^2U$), 5-aminomethyl-2-thio-uridine ($nm^5s^2U$), 5-methylaminomethyl-uridine ($mnm^5U$), 1-ethyl-pseudouridine, 5-methylaminomethyl-2-thio-uridine ($mnm^5s^2U$), 5-methylaminomethyl-2-seleno-uridine ($mnm^5se^2U$), 5-carbamoylmethyl-uridine ($ncm^5U$), 5-carboxymethylaminomethyl-uridine ($cmnm^5U$), 5-carboxymethylaminomethyl-2-thio-uridine ($cmnm^5s^2U$), 5-propynyl-uridine, 1-propynyl-pseudouridine, 5-taurinomethyl-uridine ($\tau m^5U$), 1-taurinomethyl-pseudouridine, 5-taurinomethyl-2-thio-uridine($\tau$m5s2U), 1-taurinomethyl-4-thio-pseudouridine), 5-methyl-2-thio-uridine ($m^5s^2U$), 1-methyl-4-thio-pseudouridine ($m^1s^4\psi$), 4-thio-1-methyl-pseudouridine, 3-methyl-pseudouridine ($m^3\psi$), 2-thio-1-methyl-pseudouridine, 1-methyl-1-deaza-pseudouridine, 2-thio-1-methyl-1-deaza-pseudouridine, dihydrouridine (D), dihydropseudouridine, 5,6-dihydrouridine, 5-methyl-dihydrouridine ($m^5D$), 2-thio-dihydrouridine, 2-thio-dihydropseudouridine, 2-methoxy-uridine, 2-methoxy-4-thio-uridine, 4-methoxy-pseudouridine, 4-methoxy-2-thio-pseudouridine, N1-methyl-pseudouridine, 3-(3-amino-3-carboxypropyl)uridine ($acp^3U$), 1-methyl-3-(3-amino-3-carboxypropyl)pseudouridine ($acp^3\ \psi$), 5-(isopentenylaminomethyl)uridine ($inm^5U$), 5-(isopentenylaminomethyl)-2-thio-uridine ($inm^5s^2U$), $\alpha$-thio-uridine, 2'-O-methyl-uridine (Um), 5,2'-O-dimethyl-uridine ($m^5Um$), 2'-O-methyl-pseudouridine ($\psi$m), 2-thio-2'-O-methyl-uridine ($s^2Um$), 5-methoxycarbonylmethyl-2'-O-methyl-uridine ($mcm^5Um$), 5-carbamoylmethyl-2'-O-methyl-uridine ($ncm^5Um$), 5-carboxymethylaminomethyl-2'-O-methyl-uridine ($cmnm^5Um$), 3,2'-O-dimethyl-uridine ($m^3Um$), 5-(isopentenylaminomethyl)-2'-O-methyl-uridine ($inm^5Um$), 1-thio-uridine, deoxythymidine, 2'-F-ara-uridine, 2'-F-uridine, 2'-OH-ara-uridine, 5-(2-carbomethoxyvinyl) uridine, and 5-[3-(1-E-propenylamino)uridine. In certain embodiments, the nucleoside comprising a modified nucleobase is pseudouridine ($\psi$), N1-methyl-pseudouridine (m1$\psi$) or 5-methyl-uridine (m5U), in particular N1-methyl-pseudouridine.

In some embodiments, the replacement of one or more uridines with a nucleoside comprising a modified nucleobase comprises a replacement of at least 1%, at least 2%, at least 3%, at least 4%, at least 5%, at least 10%, at least 25%, at least 50%, at least 75%, at least 90%, at least 95%, at least 96%, at least 97%, at least 98%, at least 99% or 100% of

the uridines. During synthesis of mRNA by *in vitro* transcription (IVT) using T7 RNA polymerase significant amounts of aberrant products, including double-stranded RNA (dsRNA) are produced due to unconventional activity of the enzyme. dsRNA induces inflammatory cytokines and activates effector enzymes leading to protein synthesis inhibition. Formation of dsRNA can be limited during synthesis of mRNA by *in vitro* transcription (IVT), for example, by limiting the amount of uridine triphosphate (UTP) during synthesis. Optionally, UTP may be added once or several times during synthesis of mRNA. Also, dsRNA can be removed from RNA such as IVT RNA, for example, by ion-pair reversed phase HPLC using a non-porous or porous C-18 polystyrene-divinylbenzene (PS-DVB) matrix. Alternatively, an enzymatic based method using *E. coli* RNaseIII that specifically hydrolyzes dsRNA but not ssRNA, thereby eliminating dsRNA contaminants from IVT RNA preparations can be used. Furthermore, dsRNA can be separated from ssRNA by using a cellulose material. In some embodiments, an RNA preparation is contacted with a cellulose material and the ssRNA is separated from the cellulose material under conditions which allow binding of dsRNA to the cellulose material and do not allow binding of ssRNA to the cellulose material. Suitable methods for providing ssRNA are disclosed, for example, in WO 2017/182524.

As the term is used herein, "remove" or "removal" refers to the characteristic of a population of first substances, such as non-immunogenic RNA, being separated from the proximity of a population of second substances, such as dsRNA, wherein the population of first substances is not necessarily devoid of the second substance, and the population of second substances is not necessarily devoid of the first substance. However, a population of first substances characterized by the removal of a population of second substances has a measurably lower content of second substances as compared to the non-separated mixture of first and second substances.

In some embodiments, the amount of double-stranded RNA (dsRNA) is limited, e.g., dsRNA (especially dsmRNA) is removed from non-immunogenic RNA , such that less than 10%, less than 5%, less than 4%, less than 3%, less than 2%, less than 1%, less than 0.5%, less than 0.3%, less than 0.1%, less than 0.05%, less than 0.03%, less than 0.01%, less than 0.005%, less than 0.004%, less than 0.003%, less than 0.002%, less than 0.001%, or less than 0.0005% of the RNA in the non-immunogenic RNA composition is dsRNA. In some embodiments, the non-immunogenic RNA (especially mRNA) is free or essentially free of dsRNA. In some embodiments, the non-immunogenic RNA (especially mRNA) composition comprises a purified preparation of single-stranded nucleoside modified RNA. In some embodiments, the non-immunogenic RNA (especially mRNA) composition comprises single-stranded nucleoside modified RNA (especially mRNA) and is substantially free of double stranded RNA (dsRNA). In some embodiments, the non-immunogenic RNA (especially mRNA) composition comprises at least 90%, at least 91%, at least 92%, at least 93 %, at least 94%, at least 95%, at least 96%, at least 97%, at least 98%, at least 99%, at least 99.5%, at least 99.9%, at least 99.99%, at least 99.991%, at least 99.992%, , at least 99.993%,, at least 99.994%, , at least 99.995%, at least 99.996%, at least 99.997%, or at least 99.998% single stranded nucleoside modified RNA, relative to all other nucleic acid molecules (DNA, dsRNA, etc.).

Various methods can be used to determine the amount of dsRNA. For example, a sample may be contacted with dsRNA-specific antibody and the amount of antibody binding to RNA may be taken as a measure for the amount of dsRNA in the sample. A sample containing a known amount of dsRNA may be used as a reference.

For example, RNA may be spotted onto a membrane, e.g., nylon blotting membrane. The membrane may be blocked, e.g., in TBS-T buffer (20 mM TRIS pH 7.4, 137 mM NaCl, 0.1% (v/v) TWEEN-20) containing 5% (w/v) skim milk powder. For detection of dsRNA, the membrane may be incubated with dsRNA-specific antibody, e.g., dsRNA-specific mouse mAb (English & Scientific Consulting, Szirák, Hungary). After washing, e.g., with TBS-T, the membrane may be incubated with a secondary antibody, e.g., HRP-conjugated donkey anti-mouse IgG (Jackson ImmunoResearch, Cat #715-035-150), and the signal provided by the secondary antibody may be detected.

In some embodiments, the non-immunogenic RNA (especially mRNA) is translated in a cell more efficiently than standard RNA with the same sequence. In some embodiments, translation is enhanced by a factor of 2-fold relative to its unmodified counterpart. In some embodiments, translation is enhanced by a 3-fold factor. In some embodiments, translation is enhanced by a 4-fold factor. In some embodiments, translation is enhanced by a 5-fold factor. In some embodiments, translation is enhanced by a 6-fold factor. In some embodiments, translation is enhanced by a 7-fold factor. In some embodiments, translation is enhanced by an 8-fold factor. In some embodiments, translation is enhanced by a 9-fold factor. In some embodiments, translation is enhanced by a 10-fold factor. In some embodiments, translation is enhanced by a 15-fold factor. In some embodiments, translation is enhanced by a 20-fold factor. In some embodiments, translation is enhanced by a 50-fold factor. In some embodiments, translation is enhanced by a 100-fold factor. In some embodiments, translation is enhanced by a 200-fold factor. In some embodiments, translation is enhanced by a 500-fold factor. In some embodiments, translation is enhanced by a 1000-fold factor. In some embodiments, translation is enhanced by a 2000-fold factor. In some embodiments, the factor is 10-1000-fold. In some embodiments, the factor is 10-100-fold. In some embodiments, the factor is 10-200-fold. In some embodiments, the factor is 10-300-fold. In some embodiments, the factor is 10-500-fold. In some embodiments, the factor is 20-1000-fold. In some embodiments, the factor is 30-1000-fold. In some embodiments, the factor is 50-1000-fold. In some embodiments, the factor is 100-1000-fold. In some embodiments, the factor is 200-1000-fold. In some embodiments, translation is enhanced by any other significant amount or range of amounts.

In some embodiments, the non-immunogenic RNA (especially mRNA) exhibits significantly less innate immunogenicity than standard RNA with the same sequence. In some embodiments, the non-immunogenic RNA (especially mRNA) exhibits an innate immune response that is 2-fold less than its unmodified counterpart. In some embodiments, innate immunogenicity is reduced by a 3-fold factor. In some embodiments, innate immunogenicity is reduced by a 4-fold factor. In some embodiments, innate immunogenicity is reduced by a 5-fold factor. In some embodiments, innate immunogenicity is reduced by a 6-fold factor. In some embodiments, innate immunogenicity is reduced by a 7-fold factor. In some embodiments, innate immunogenicity is reduced by an 8-fold factor. In some embodiments, innate immunogenicity is reduced by a 9-fold factor. In some embodiments, innate immunogenicity is reduced by a 10-fold factor. In some embodiments, innate immunogenicity is reduced by a 15-fold factor. In some embodiments, innate immunogenicity is reduced by a 20-fold factor. In some embodiments, innate immunogenicity is reduced by a 50-fold factor. In some embodiments, innate immunogenicity is reduced by a 100-fold factor. In some embodiments, innate immunogenicity is reduced by a 200-fold factor. In some embodiments, innate immunogenicity is reduced by a 500-fold factor. In some embodiments, innate immunogenicity is reduced by a 1000-fold factor. In some embodiments, innate immunogenicity is reduced by a 2000-fold factor.

The term "exhibits significantly less innate immunogenicity" refers to a detectable decrease in innate immunogenicity. In some embodiments, the term refers to a decrease such that an effective amount of the non-immunogenic RNA (especially mRNA) can be administered without triggering a detectable innate immune response. In some embodiments, the term refers to a decrease such that the non-immunogenic RNA (especially mRNA) can be repeatedly administered without eliciting an innate immune response sufficient to detectably reduce production of the protein encoded by the non-immunogenic RNA. In some embodiments, the decrease is such that the non-immunogenic RNA (especially mRNA) can be repeatedly administered without eliciting an innate immune response sufficient to eliminate detectable production of the protein encoded by the non-immunogenic RNA.

"Immunogenicity" is the ability of a foreign substance, such as RNA, to provoke an immune response in the body of a human or other animal. The innate immune system is the component of the immune system that is relatively unspecific and immediate. It is one of two main components of the vertebrate immune system, along with the adaptive immune system.

**Particles**

Nucleic acids such as RNA, in particular mRNA, described herein may be present in particles comprising (i) the nucleic acid, and (ii) at least one cationic or cationically ionizable compound such as a polymer or lipid complexing the nucleic acid. Electrostatic interactions between positively charged molecules such as polymers and lipids and negatively charged nucleic acid are involved in particle formation. This results in complexation and spontaneous formation of nucleic acid particles. During the manufacturing process, introduction of an aqueous solution of RNA to an ethanolic lipid mixture containing a cationically ionizable lipid at pH of, e.g., 5 leads to an electrostatic interaction between the negatively charged RNA drug substance and the positively charged cationically ionizable lipid. This electrostatic interaction leads to particle formation coincident with efficient encapsulation of RNA drug substance. After RNA encapsulation, adjustment of the medium surrounding the resulting RNA-LNP to, e.g., pH 8 results in neutralization of the surface charge on the LNP. When all other variables are held constant, charge-neutral particles display longer *in vivo* circulation lifetimes and better delivery to hepatocytes compared to charged particles, which are cleared rapidly by the reticuloendothelial system. Upon endosomal uptake, the low pH of the endosome renders the LNP fusogenic and allows for release of the RNA into the cytosol of the target cell.

Different types of RNA containing particles have been described previously to be suitable for delivery of RNA in particulate form (cf., *e.g.,* Kaczmarek, J. C. et al., 2017, Genome Medicine 9, 60). For non-viral RNA delivery vehicles, nanoparticle encapsulation of RNA physically protects RNA from degradation and, depending on the specific chemistry, can aid in cellular uptake and endosomal escape.

In the context of the present disclosure, the term "particle" relates to a structured entity formed by molecules or molecule complexes, in particular particle forming compounds. In some embodiments, the particle contains an envelope (e.g., one or more layers or lamellas) made of one or more types of amphiphilic substances *(e.g.,* amphiphilic lipids). In this context, the expression "amphiphilic substance" means that the substance possesses both hydrophilic and lipophilic properties. The envelope may also comprise additional substances (e.g., additional lipids) which do not have to be amphiphilic. Thus, the particle may be a monolamellar or multilamellar structure, wherein the substances constituting the one or more layers or lamellas comprise one or more types of amphiphilic substances (in particular selected from the group consisting of amphiphilic lipids) optionally in combination with additional substances (e.g., additional lipids) which do not have to be amphiphilic. In some embodiments, the term "particle" relates to a micro- or nano-sized structure, such as a micro-or nano-sized compact structure. According to the present disclosure, the term "particle" includes nanoparticles.

An "RNA particle" can be used to deliver RNA to a target site of interest (e.g., cell, tissue, organ, and the like). An RNA particle may be formed from lipids comprising at least one cationic or cationically ionizable lipid. Without intending to

be bound by any theory, it is believed that the cationic or cationically ionizable lipid combines together with the RNA to form aggregates, and this aggregation results in colloidally stable particles.

RNA particles described herein include lipid nanoparticle (LNP)-based and lipoplex (LPX)-based formulations.

A lipoplex (LPX) described herein is obtainable from mixing two aqueous phases, namely a phase comprising RNA and a phase comprising a dispersion of lipids. In some embodiments, the lipid phase comprises liposomes.

In some embodiments, liposomes are self-closed unilamellar or multilamellar vesicular particles wherein the lamellae comprise lipid bilayers and the encapsulated lumen comprises an aqueous phase. A prerequisite for using liposomes for nanoparticle formation is that the lipids in the mixture as required are able to form lamellar (bilayer) phases in the applied aqueous environment.

In some embodiments, liposomes comprise unilamellar or multilamellar phospholipid bilayers enclosing an aqueous core (also referred to herein as an aqueous lumen). They may be prepared from materials possessing polar head (hydrophilic) groups and nonpolar tail (hydrophobic) groups. In some embodiments, cationic lipids employed in formulating liposomes designed for the delivery of nucleic acids are amphiphilic in nature and consist of a positively charged (cationic) amine head group linked to a hydrocarbon chain or cholesterol derivative via glycerol.

In some embodiments, lipoplexes are multilamellar liposome-based formulations that form upon electrostatic interaction of cationic liposomes with RNAs. In some embodiments, formed lipoplexes possess distinct internal arrangements of molecules that arise due to the transformation from liposomal structure into compact RNA-lipoplexes.

In some embodiments, an LPX particle comprises an amphiphilic lipid, in particular cationic or cationically ionizable amphiphilic lipid, and RNA (especially mRNA) as described herein. In some embodiments, electrostatic interactions between positively charged liposomes (made from one or more amphiphilic lipids, in particular cationic or cationically ionizable amphiphilic lipids) and negatively charged RNA (especially mRNA) results in complexation and spontaneous formation of RNA lipoplex particles. Positively charged liposomes may be generally synthesized using a cationic or cationically ionizable amphiphilic lipid, such as a cationically ionizable lipid of formula (I), DOTMA and/or DODMA, and optionally additional lipids, such as DOPE or DSPC. In some embodiments, an RNA (especially mRNA) lipoplex particle is a nanoparticle.

In general, a lipid nanoparticle (LNP) is obtainable from direct mixing of RNA in an aqueous phase with lipids in a phase comprising an organic solvent, such as ethanol. In that case, lipids or lipid mixtures can be used for particle formation, which do not form lamellar (bilayer) phases in water.

In some embodiments, LNPs comprise or consist of a cationic/cationically ionizable lipid (in particular, a cationically ionizable lipid of any one of the formulas (I), (Ia), (Ib), (Ic), (Id), (Ie), (If), (Ig), (Ih), (Ii), (Ij), (IIa), (IIb), (IIIa), (IIIb), (IV-1), (IV-2), and (IV-3) disclosed herein) and helper lipids such as phospholipids, cholesterol, and/or polymer-conjugated lipids (e.g., polyethylene glycol (PEG) lipids or sarcosinylated lipids). In some embodiments, in the RNA LNPs described herein the RNA (in particular, mRNA) is bound by cationically ionizable lipid (in particular a cationically ionizable lipid of any one of the formulas (I), (Ia), (Ib), (Ic), (Id), (Ie), (If), (Ig), (Ih), (Ii), (Ij), (IIa), (IIb), (IIIa), (IIIb), (IV-1), (IV-2), and (IV-3) disclosed herein) that occupies the central core of the LNP. In some embodiments, polymer-conjugated lipid forms the surface of the LNP, along with phospholipids. In some embodiments, the surface comprises a bilayer. In some embodiments, cholesterol and cationically ionizable lipid (in particular a cationically ionizable lipid of any one of the formulas (I), (Ia), (Ib), (Ic), (Id), (Ie), (If), (Ig), (Ih), (Ii), (Ij), (IIa), (IIb), (IIIa), (IIIb), (IV-1), (IV-2), and (IV-3) disclosed herein) in charged and uncharged forms can be distributed throughout the LNP.

In some embodiments, RNA (e.g., mRNA) described herein may be noncovalently associated with a particle as described herein. In embodiments, the RNA (especially mRNA) may be adhered to the outer surface of the particle (surface RNA (especially surface mRNA)) and/or may be contained in the particle (encapsulated RNA (especially encapsulated mRNA)).

In some embodiments, the particles (e.g., LNPs and LPXs) described herein have a size (such as a diameter) in the range of about 10 to about 2000 nm, such as at least about 15 nm (e.g., at least about 20 nm, at least about 25 nm, at least about 30 nm, at least about 35 nm, at least about 40 nm, at least about 45 nm, at least about 50 nm, at least about 55 nm, at least about 60 nm, at least about 65 nm, at least about 70 nm, at least about 75 nm, at least about 80 nm, at least about 85 nm, at least about 90 nm, at least about 95 nm, or at least about 100 nm) and/or at most about 1900 nm (e.g., at most about 1800 nm, at most about 1700 nm, at most about 1600 nm, at most about 1500 nm, at most about 1400 nm, at most about 1300 nm, at most about 1200 nm, at most about 1100 nm, at most about 1000 nm, at most about 950 nm, at most about 900 nm, at most about 850 nm, at most about 800 nm, at most about 750 nm, at most about 700 nm, at most about 650 nm, at most about 600 nm, at most about 550 nm, or at most about 500 nm), such as in the range of about 20 to about 1500 nm, such as about 30 to about 1200 nm, about 40 to about 1100 nm, about 50 to about 1000 nm, about 60 to about 900 nm, about 70 to about 800 nm, about 80 to about 700 nm, about 90 to about 600 nm, or about 50 to about 500 nm or about 100 to about 500 nm, such as in the range of 10 to 1000 nm, 15 to 500 nm, 20 to 450 nm, 25 to 400 nm, 30 to 350 nm, 40 to 300 nm, 50 to 250 nm, 60 to 200 nm, 70 to 150 nm, or 80 to 150 nm. In some embodiments, the particles (e.g., LNPs and LPXs) described herein have a size (such as a diameter) in the range of from about 40 nm to about 200 nm, such as from about 50 nm to about 180 nm, from about 60 nm to about 160 nm, from about 80 nm to about 150 nm or from about 80

nm to about 120 nm.

In some embodiments, the particles (e.g., LNPs and LPXs) described herein have an average diameter that in some embodiments ranges from about 50 nm to about 1000 nm, from about 50 nm to about 800 nm, from about 50 nm to about 700 nm, from about 50 nm to about 600 nm, from about 50 nm to about 500 nm, from about 50 nm to about 450 nm, from about 50 nm to about 400 nm, from about 50 nm to about 350 nm, from about 50 nm to about 300 nm, from about 50 nm to about 250 nm, from about 50 nm to about 200 nm, from about 100 nm to about 1000 nm, from about 100 nm to about 800 nm, from about 100 nm to about 700 nm, from about 100 nm to about 600 nm, from about 100 nm to about 500 nm, from about 100 nm to about 450 nm, from about 100 nm to about 400 nm, from about 100 nm to about 350 nm, from about 100 nm to about 300 nm, from about 100 nm to about 250 nm, from about 100 nm to about 200 nm, from about 150 nm to about 1000 nm, from about 150 nm to about 800 nm, from about 150 nm to about 700 nm, from about 150 nm to about 600 nm, from about 150 nm to about 500 nm, from about 150 nm to about 450 nm, from about 150 nm to about 400 nm, from about 150 nm to about 350 nm, from about 150 nm to about 300 nm, from about 150 nm to about 250 nm, from about 150 nm to about 200 nm, from about 200 nm to about 1000 nm, from about 200 nm to about 800 nm, from about 200 nm to about 700 nm, from about 200 nm to about 600 nm, from about 200 nm to about 500 nm, from about 200 nm to about 450 nm, from about 200 nm to about 400 nm, from about 200 nm to about 350 nm, from about 200 nm to about 300 nm, from about 200 nm to about 250 nm, or from about 80 to about 150 nm. In some embodiments, the particles (e.g., LNPs and LPXs) described herein have an average diameter that in some embodiments ranges from about 40 nm to about 200 nm, such as from about 50 nm to about 180 nm, from about 60 nm to about 160 nm, from about 80 nm to about 150 nm or from about 80 nm to about 120 nm.

In some embodiments, the particles described herein are nanoparticles. The term "nanoparticle" relates to a nano-sized particle comprising nucleic acid (especially mRNA) as described herein and at least one cationic or cationically ionizable lipid, wherein all three external dimensions of the particle are in the nanoscale, *i.e.,* at least about 1 nm and below about 1000 nm. Preferably, the size of a particle is its diameter.

RNA particles (especially mRNA particles) described herein may exhibit a polydispersity index (PDI) less than about 0.5, less than about 0.4, less than about 0.3, less than about 0.2, less than about 0.1, or less than about 0.05. By way of example, the nucleic acid particles can exhibit a polydispersity index in a range of about 0.01 to about 0.4 or about 0.1 to about 0.3. The N/P ratio gives the ratio of the nitrogen groups in the lipid to the number of phosphate groups in the nucleic acid. It is correlated to the charge ratio, as the nitrogen atoms (depending on the pH) are usually positively charged and the phosphate groups are negatively charged. The N/P ratio, where a charge equilibrium exists, depends on the pH. Lipid formulations are frequently formed at N/P ratios larger than four up to twelve, because positively charged nanoparticles are considered favorable for transfection. In that case, RNA is considered to be completely bound to nanoparticles.

RNA particles (especially mRNA particles) described herein can be prepared using a wide range of methods that may involve obtaining a colloid from at least one cationic or cationically ionizable lipid and mixing the colloid with nucleic acid to obtain nucleic acid particles.

The term "colloid" as used herein relates to a type of homogeneous mixture in which dispersed particles do not settle out. The insoluble particles in the mixture are microscopic, with particle sizes between 1 and 1000 nanometers. The mixture may be termed a colloid or a colloidal suspension. Sometimes the term "colloid" only refers to the particles in the mixture and not the entire suspension.

For the preparation of colloids comprising at least one cationic or cationically ionizable lipid methods are applicable herein that are conventionally used for preparing liposomal vesicles and are appropriately adapted. The most commonly used methods for preparing liposomal vesicles share the following fundamental stages: (i) lipids dissolution in organic solvents, (ii) drying of the resultant solution, and (iii) hydration of dried lipid (using various aqueous media). In the film hydration method, lipids are firstly dissolved in a suitable organic solvent, and dried down to yield a thin film at the bottom of the flask. The obtained lipid film is hydrated using an appropriate aqueous medium to produce a liposomal dispersion. Furthermore, an additional downsizing step may be included.

Reverse phase evaporation is an alternative method to the film hydration for preparing liposomal vesicles that involves formation of a water-in-oil emulsion between an aqueous phase and an organic phase containing lipids. A brief sonication of this mixture is required for system homogenization. The removal of the organic phase under reduced pressure yields a milky gel that turns subsequently into a liposomal suspension.

The term "ethanol injection technique" refers to a process, in which an ethanol solution comprising lipids is rapidly injected into an aqueous solution through a needle. This action disperses the lipids throughout the solution and promotes lipid structure formation, for example lipid vesicle formation such as liposome formation. Generally, the RNA (especially mRNA) lipoplex particles described herein are obtainable by adding RNA (especially mRNA) to a colloidal liposome dispersion. Using the ethanol injection technique, such colloidal liposome dispersion is, in some embodiments, formed as follows: an ethanol solution comprising lipids, such as cationic or cationically ionizable lipids (like a cationically ionizable lipid of any one of the formulas (I), (Ia), (Ib), (Ic), (Id), (Ie), (If), (Ig), (Ih), (Ii), (Ij), (IIa), (IIb), (IIIa), (IIIb), (IV-1), (IV-2), and (IV-3) disclosed herein, DOTMA and/or DODMA) and additional lipids, is injected into an aqueous solution under stirring. In some embodiments, the RNA (especially mRNA) lipoplex particles described herein are obtainable without a step of extrusion.

The term "extruding" or "extrusion" refers to the creation of particles having a fixed, cross-sectional profile. In

particular, it refers to the downsizing of a particle, whereby the particle is forced through filters with defined pores.

Other methods having organic solvent free characteristics may also be used according to the present disclosure for preparing a colloid.

In some embodiments, LNPs comprise four components: cationically ionizable lipids (in particular, a cationically ionizable lipid of any one of the formulas (I), (Ia), (Ib), (Ic), (Id), (Ie), (If), (Ig), (Ih), (Ii), (Ij), (IIa), (IIb), (IIIa), (IIIb), (IV-1), (IV-2), and (IV-3) disclosed herein), neutral lipids such as phospholipids, a steroid such as cholesterol, and a polymer-conjugated lipid. In some embodiments, LNPs may be prepared by mixing lipids dissolved in ethanol rapidly with RNA in an aqueous buffer. While RNA particles described herein may comprise polymer-conjugated lipids such as PEG lipids or sarcosinylated lipids, described herein are also RNA particles which do not comprise PEG lipids, or do not comprise sarcosinylated lipids, or do not comprise PEG lipids and sarcosinylated lipids, or do not comprise any polymer-conjugated lipids.

According to the invention, the composition comprises at least one polysarcosine-conjugated lipid.

In some embodiments, the LNPs comprising RNA and at least one cationic or cationically ionizable lipid described herein (in particular, a cationically ionizable lipid of any one of the formulas (I), (Ia), (Ib), (Ic), (Id), (Ie), (If), (Ig), (Ih), (Ii), (Ij), (IIa), (IIb), (IIIa), (IIIb), (IV-1), (IV-2), and (IV-3) disclosed herein) are prepared by (a) preparing an RNA solution containing water and a buffering system; (b) preparing an ethanolic solution comprising the cationic or cationically ionizable lipid (in particular, a cationically ionizable lipid of any one of the formulas (I), (Ia), (Ib), (Ic), (Id), (Ie), (If), (Ig), (Ih), (Ii), (Ij), (IIa), (IIb), (IIIa), (IIIb), (IV-1), (IV-2), and (IV-3) disclosed herein) and, if present, one or more additional lipids; and (c) mixing the RNA solution prepared under (a) with the ethanolic solution prepared under (b), thereby preparing the formulation comprising LNPs. After step (c) one or more steps selected from diluting and filtrating, such as tangential flow filtrating, can follow.

In some embodiments, the LNPs comprising RNA and at least one cationic or cationically ionizable lipid described herein (in particular, a cationically ionizable lipid of any one of the formulas (I), (Ia), (Ib), (Ic), (Id), (Ie), (If), (Ig), (Ih), (Ii), (Ij), (IIa), (IIb), (IIIa), (IIIb), (IV-1), (IV-2), and (IV-3) disclosed herein) are prepared by (a') preparing liposomes or a colloidal preparation of the cationic or cationically ionizable lipid and, if present, one or more additional lipids in an aqueous phase; and (b') preparing an RNA solution containing water and a buffering system; and (c') mixing the liposomes or colloidal preparation prepared under (a') with the RNA solution prepared under (b'). After step (c') one or more steps selected from diluting and filtrating, such as tangential flow filtrating, can follow.

The present disclosure describes compositions comprising RNA (especially mRNA) and at least one cationic or cationically ionizable lipid (in particular, a cationically ionizable lipid of any one of the formulas (I), (Ia), (Ib), (Ic), (Id), (Ie), (If), (Ig), (Ih), (Ii), (Ij), (IIa), (IIb), (IIIa), (IIIb), (IV-1), (IV-2), and (IV-3) disclosed herein) which associates with the RNA to form RNA particles and formulations comprising such particles. The RNA particles may comprise RNA which is complexed in different forms by non-covalent interactions to the particle. The particles described herein are not viral particles, in particular infectious viral particles, *i.e.,* they are not able to virally infect cells.

Suitable cationic or cationically ionizable lipids are those that form nucleic acid particles and are included by the term "particle forming components" or "particle forming agents". The term "particle forming components" or "particle forming agents" relates to any components which associate with nucleic acid to form nucleic acid particles. Such components include any component which can be part of nucleic acid particles.

In some embodiments, RNA particles (especially mRNA particles) comprise more than one type of RNA molecules, where the molecular parameters of the RNA molecules may be similar or different from each other, like with respect to molar mass or fundamental structural elements such as molecular architecture, capping, coding regions or other features,

In particulate formulation, it is possible that each RNA species is separately formulated as an individual particulate formulation. In that case, each individual particulate formulation will comprise one RNA species. The individual particulate formulations may be present as separate entities, e.g. in separate containers. Such formulations are obtainable by providing each RNA species separately (typically each in the form of an RNA-containing solution) together with a particle-forming agent, thereby allowing the formation of particles. Respective particles will contain exclusively the specific RNA species that is being provided when the particles are formed (individual particulate formulations). In some embodiments, a composition such as a pharmaceutical composition comprises more than one individual particle formulation. Respective pharmaceutical compositions are referred to as mixed particulate formulations. Mixed particulate formulations according to the present disclosure are obtainable by forming, separately, individual particulate formulations, followed by a step of mixing of the individual particulate formulations. By the step of mixing, a formulation comprising a mixed population of RNA-containing particles is obtainable. Individual particulate populations may be together in one container, comprising a mixed population of individual particulate formulations. Alternatively, it is possible that all RNA species of the pharmaceutical composition are formulated together as a combined particulate formulation. Such formulations are obtainable by providing a combined formulation (typically combined solution) of all RNA species together with a particle-forming agent, thereby allowing the formation of particles. As opposed to a mixed particulate formulation, a combined particulate formulation will typically comprise particles which comprise more than one RNA species. In a combined particulate composition different RNA species are typically present together in a single particle.

**Lipids**

The terms "lipid" and "lipid-like material" are broadly defined herein as molecules which comprise one or more hydrophobic moieties or groups and optionally also one or more hydrophilic moieties or groups. Molecules comprising hydrophobic moieties and hydrophilic moieties are also frequently denoted as amphiphiles. Lipids are usually insoluble or poorly soluble in water, but soluble in many organic solvents. In an aqueous environment, the amphiphilic nature allows the molecules to self-assemble into organized structures and different phases. One of those phases consists of lipid bilayers, as they are present in vesicles, multilamellar/unilamellar liposomes, or membranes in an aqueous environment. Hydrophobicity can be conferred by the inclusion of apolar groups that include, but are not limited to, long-chain saturated and unsaturated aliphatic hydrocarbon groups and such groups substituted by one or more aromatic, cycloaliphatic, or heterocyclic group(s). The hydrophilic groups may comprise polar and/or charged groups and include carbohydrates, phosphate, carboxylic, sulfate, amino, sulfhydryl, nitro, hydroxyl, and other like groups.

As used herein, the term "hydrophobic" refers to any a molecule, moiety or group which is substantially immiscible or insoluble in aqueous solution. The term hydrophobic group includes hydrocarbons having at least 6 carbon atoms. The monovalent radical of a hydrocarbon is referred to as hydrocarbyl herein. The hydrophobic group can have functional groups (e.g., ether, ester, halide, etc.) and atoms other than carbon and hydrogen as long as the group satisfies the condition of being substantially immiscible or insoluble in aqueous solution.

The term "hydrocarbon" includes non-cyclic, e.g., linear (straight) or branched, hydrocarbyl groups, such as alkyl, alkenyl, or alkynyl as defined herein. It should be appreciated that one or more of the hydrogen atoms in alkyl, alkenyl, or alkynyl may be substituted with other atoms, e.g., halogen, oxygen or sulfur. Unless stated otherwise, hydrocarbon groups can also include a cyclic (alkyl, alkenyl or alkynyl) group or an aryl group, provided that the overall polarity of the hydrocarbon remains relatively nonpolar.

The term "alkyl" refers to a saturated linear or branched monovalent hydrocarbon moiety which may have one to thirty, typically one to twenty, often six to eighteen carbon atoms. Exemplary nonpolar alkyl groups include, but are not limited to, methyl, ethyl, propyl, isopropyl, hexyl, decyl, dodecyl, tetradecyl, hexadecyl, octadecyl, and the like.

The term "alkenyl" refers to a linear or branched monovalent hydrocarbon moiety having at least one carbon-carbon double bond in which the total carbon atoms may be six to thirty, typically six to twenty often six to eighteen. Generally, the maximal number of carbon-carbon double bonds in the alkenyl group can be equal to the integer which is calculated by dividing the number of carbon atoms in the alkenyl group by 2 and, if the number of carbon atoms in the alkenyl group is uneven, rounding the result of the division down to the next integer. For example, for an alkenyl group having 9 carbon atoms, the maximum number of carbon-carbon double bonds is 4. Preferably, the alkenyl group has 1 to 6 (such as 1 to 4), i.e., 1, 2, 3, 4, 5, or 6, carbon-carbon double bonds.

The term "alkynyl" refers to a linear or branched monovalent hydrocarbon moiety having at least one carbon-carbon triple bond in which the total carbon atoms may be six to thirty, typically six to twenty, often six to eighteen. Alkynyl groups can optionally have one or more carbon-carbon double bonds. Generally, the maximal number of carbon-carbon triple bonds in the alkynyl group can be equal to the integer which is calculated by dividing the number of carbon atoms in the alkynyl group by 2 and, if the number of carbon atoms in the alkynyl group is uneven, rounding the result of the division down to the next integer. For example, for an alkynyl group having 9 carbon atoms, the maximum number of carbon-carbon triple bonds is 4. Preferably, the alkynyl group has 1 to 6 (such as 1 to 4), i.e., 1, 2, 3, 4, 5, or 6, more preferably 1 or 2 carbon-carbon triple bonds.

The term "alkylene" refers to a saturated linear or branched divalent hydrocarbon moiety which may have one to thirty, typically two to twenty, often four to twelve carbon atoms. Exemplary nonpolar alkylene groups include, but are not limited to, methylene, ethylene, trimethylene, hexamethylene, decamethylene, dodecamethylene, tetradecamethylene, hexadecamethylene, octadecmethylene, and the like.

The term "alkenylene" refers to a linear or branched divalent hydrocarbon moiety having at least one carbon-carbon double bond in which the total carbon atoms may be two to thirty, typically two to twenty, often four to twelve. Generally, the maximal number of carbon-carbon double bonds in the alkenylene group can be equal to the integer which is calculated by dividing the number of carbon atoms in the alkenylene group by 2 and, if the number of carbon atoms in the alkenylene group is uneven, rounding the result of the division down to the next integer. For example, for an alkenylene group having 9 carbon atoms, the maximum number of carbon-carbon double bonds is 4. Preferably, the alkenylene group has 1 to 6 (such as 1 to 4), i.e., 1, 2, 3, 4, 5, or 6, carbon-carbon double bonds.

The term "cycloalkyl" represents cyclic non-aromatic versions of "alkyl" and "alkenyl" with preferably 3 to 14 carbon atoms, such as 3 to 12 or 3 to 10 carbon atoms, i.e., 3, 4, 5, 6, 7, 8, 9, 10, 11, 12, 13, or 14 carbon atoms (such as 3, 4, 5, 6, 7, 8, 9, or 10 carbon atoms), more preferably 3 to 7 carbon atoms. Exemplary cycloalkyl groups include cyclopropyl, cyclopropenyl, cyclobutyl, cyclobutenyl, cyclopentyl, cyclopentenyl, cyclohexyl, cyclohexenyl, cycloheptyl, cycloheptenyl, cyclooctyl, cyclooctenyl, cyclononyl, cyclononenyl, cylcodecyl, cylcodecenyl, and adamantyl. The cycloalkyl group may consist of one ring (monocyclic), two rings (bicyclic), or more than two rings (polycyclic).

The term "aryl" refers to a monoradical of an aromatic cyclic hydrocarbon. Preferably, the aryl group contains 3 to

14 (e.g., 5, 6, 7, 8, 9, or 10, such as 5, 6, or 10) carbon atoms which can be arranged in one ring (e.g., phenyl) or two or more condensed rings (e.g., naphthyl). Exemplary aryl groups include cyclopropenylium, cyclopentadienyl, phenyl, indenyl, naphthyl, azulenyl, fluorenyl, anthryl, and phenanthryl. Preferably, "aryl" refers to a monocyclic ring containing 6 carbon atoms or an aromatic bicyclic ring system containing 10 carbon atoms. Preferred examples are phenyl and naphthyl. Aryl does not encompass fullerenes.

The term "aromatic" as used in the context of hydrocarbons means that the whole molecule has to be aromatic. For example, if a monocyclic aryl is hydrogenated (either partially or completely) the resulting hydrogenated cyclic structure is classified as cycloalkyl for the purposes of the present disclosure. Likewise, if a bi- or polycyclic aryl (such as naphthyl) is hydrogenated the resulting hydrogenated bi- or polycyclic structure (such as 1,2-dihydronaphthyl) is classified as cycloalkyl for the purposes of the present disclosure (even if one ring, such as in 1,2-dihydronaphthyl, is still aromatic).

As used herein, the term "amphiphilic" refers to a molecule having both a polar portion and a non-polar portion. Often, an amphiphilic compound has a polar head attached to a long hydrophobic tail. In some embodiments, the polar portion is soluble in water, while the non-polar portion is insoluble in water. In addition, the polar portion may have either a formal positive charge, or a formal negative charge. Alternatively, the polar portion may have both a formal positive and a negative charge, and be a zwitterion or inner salt. For purposes of the disclosure, the amphiphilic compound can be, but is not limited to, one or a plurality of natural or non-natural lipids and lipid-like compounds.

The term "lipid-like material", "lipid-like compound" or "lipid-like molecule" relates to substances, in particular amphiphilic substances, that structurally and/or functionally relate to lipids but may not be considered as lipids in a strict sense. For example, the term includes compounds that are able to form amphiphilic layers as they are present in vesicles, multilamellar/unilamellar liposomes, or membranes in an aqueous environment and includes surfactants, or synthesized compounds with both hydrophilic and hydrophobic moieties. Generally speaking, the term includes molecules, which comprise hydrophilic and hydrophobic moieties with different structural organization, which may or may not be similar to that of lipids. Examples of lipid-like compounds capable of spontaneous integration into cell membranes include functional lipid constructs such as synthetic function-spacer-lipid constructs (FSL), synthetic function-spacer-sterol constructs (FSS) as well as artificial amphipathic molecules. Lipids comprising two long alkyl chains and a polar head group are generally cylindrical. The area occupied by the two alkyl chains is similar to the area occupied by the polar head group. Such lipids have low solubility as monomers and tend to aggregate into planar bilayers that are water insoluble. Traditional surfactant monomers comprising only one linear alkyl chain and a hydrophilic head group are generally cone shaped. The hydrophilic head group tends to occupy more molecular space than the linear alkyl chain. In some embodiments, surfactants tend to aggregate into spherical or elliptoid micelles that are water soluble. While lipids also have the same general structure as surfactants - a polar hydrophilic head group and a nonpolar hydrophobic tail - lipids differ from surfactants in the shape of the monomers, in the type of aggregates formed in solution, and in the concentration range required for aggregation. As used herein, the term "lipid" is to be construed to cover both lipids and lipid-like materials unless otherwise indicated herein or clearly contradicted by context.

Generally, lipids may be divided into eight categories: fatty acids, glycerolipids, glycerophospholipids, sphingolipids, saccharolipids, polyketides (derived from condensation of ketoacyl subunits), sterol lipids and prenol lipids (derived from condensation of isoprene subunits). Although the term "lipid" is sometimes used as a synonym for fats, fats are a subgroup of lipids called triglycerides. Lipids also encompass molecules such as fatty acids and their derivatives (including tri-, di-, monoglycerides, and phospholipids), as well as steroids, *i.e.,* sterol-containing metabolites such as cholesterol or a derivative thereof. Examples of cholesterol derivatives include, but are not limited to, cholestanol, cholestanone, cholestenone, coprostanol, cholesteryl-2'-hydroxyethyl ether, cholesteryl-4'-hydroxybutyl ether, tocopherol and derivatives thereof, and mixtures thereof.

Fatty acids, or fatty acid residues are a diverse group of molecules made of a hydrocarbon chain that terminates with a carboxylic acid group; this arrangement confers the molecule with a polar, hydrophilic end, and a nonpolar, hydrophobic end that is insoluble in water. The carbon chain, typically between four and 24 carbons long, may be saturated or unsaturated, and may be attached to functional groups containing oxygen, halogens, nitrogen, and sulfur. If a fatty acid contains a double bond, there is the possibility of either a cis or trans geometric isomerism, which significantly affects the molecule's configuration. Cis-double bonds cause the fatty acid chain to bend, an effect that is compounded with more cis double bonds in the chain. Other major lipid classes in the fatty acid category are the fatty esters and fatty amides. Glycerolipids are composed of mono-, di-, and tri-substituted glycerols, the best-known being the fatty acid triesters of glycerol, called triglycerides. The word "triacylglycerol" is sometimes used synonymously with "triglyceride". In these compounds, the three hydroxyl groups of glycerol are each esterified, typically by different fatty acids. Additional subclasses of glycerolipids are represented by glycosylglycerols, which are characterized by the presence of one or more sugar residues attached to glycerol via a glycosidic linkage.

The glycerophospholipids are amphipathic molecules (containing both hydrophobic and hydrophilic regions) that contain a glycerol core linked to two fatty acid-derived "tails" by ester linkages and to one "head" group by a phosphate ester linkage. Examples of glycerophospholipids, usually referred to as phospholipids (though sphingomyelins are also classified as phospholipids) are phosphatidylcholine (also known as PC, GPCho or lecithin), phosphatidylethanolamine

(PE or GPEtn) and phosphatidylserine (PS or GPSer). Sphingolipids are a complex family of compounds that share a common structural feature, a sphingoid base backbone. The major sphingoid base in mammals is commonly referred to as sphingosine. Ceramides (N-acyl-sphingoid bases) are a major subclass of sphingoid base derivatives with an amide-linked fatty acid. The fatty acids are typically saturated or mono-unsaturated with chain lengths from 16 to 26 carbon atoms. The major phosphosphingolipids of mammals are sphingomyelins (ceramide phosphocholines), whereas insects contain mainly ceramide phosphoethanolamines and fungi have phytoceramide phosphoinositols and mannose-containing headgroups. The glycosphingolipids are a diverse family of molecules composed of one or more sugar residues linked via a glycosidic bond to the sphingoid base.

Examples of these are the simple and complex glycosphingolipids such as cerebrosides and gangliosides.

Sterol lipids, such as cholesterol and its derivatives, or tocopherol and its derivatives, are an important component of membrane lipids, along with the glycerophospholipids and sphingomyelins.

Saccharolipids describe compounds in which fatty acids are linked directly to a sugar backbone, forming structures that are compatible with membrane bilayers. In the saccharolipids, a monosaccharide substitutes for the glycerol backbone present in glycerolipids and glycerophospholipids. The most familiar saccharolipids are the acylated glucosamine precursors of the Lipid A component of the lipopolysaccharides in Gram-negative bacteria. Typical lipid A molecules are disaccharides of glucosamine, which are derivatized with as many as seven fatty-acyl chains. The minimal lipopolysaccharide required for growth in E. coli is Kdo2-Lipid A, a hexa-acylated disaccharide of glucosamine that is glycosylated with two 3-deoxy-D-manno-octulosonic acid (Kdo) residues.

Polyketides are synthesized by polymerization of acetyl and propionyl subunits by classic enzymes as well as iterative and multimodular enzymes that share mechanistic features with the fatty acid synthases. They comprise a large number of secondary metabolites and natural products from animal, plant, bacterial, fungal and marine sources, and have great structural diversity. Many polyketides are cyclic molecules whose backbones are often further modified by glycosylation, methylation, hydroxylation, oxidation, or other processes.

According to the disclosure, lipids and lipid-like materials may be cationic, anionic or neutral. Neutral lipids or lipid-like materials exist in an uncharged or neutral zwitterionic form at a selected pH.

**Cationically ionizable lipids**

The RNA compositions and formulations described herein comprise a cationically ionizable lipid comprising the structure of the following formula (I):

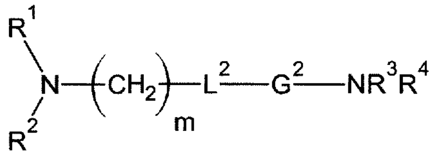

wherein

each of $R^1$ and $R^2$ is independently $R^5$ or $-G^1-L^1-R^6$, wherein at least one of $R^1$ and $R^2$ is $-G^1-L^1-R^6$;
each of $R^3$ and $R^4$ is independently selected from the group consisting of $C_{1-6}$ alkyl, $C_{2-6}$ alkenyl, aryl, and $C_{3-10}$ cycloalkyl;
each of $R^5$ and $R^6$ is independently a non-cyclic hydrocarbyl group having at least 10 carbon atoms;
each of $G^1$ and $G^2$ is independently unsubstituted $C_{1-12}$ alkylene or $C_{2-12}$ alkenylene;
each of $L^1$ and $L^2$ is independently selected from the group consisting of -O(C=O)-, -(C=O)O-, -C(=O)-, -O-, $-S(O)_x-$, -S-S-, -C(=O)S-, -SC(=O)-, $-NR^aC(=O)-$, $-C(=O)NR^a-$, $-NR^aC(=O)NR^a-$, $-OC(=O)NR^a-$ and $-NR^aC(=O)O-$;
$R^a$ is H or $C_{1-12}$ alkyl;
m is 0, 1, 2, 3, or 4; and
x is 0, 1 or 2.

In some embodiments, the cationically ionizable lipid comprises the structure of the following formula (Ia):

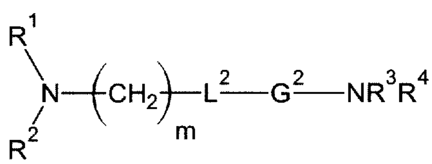

wherein $R^1$, $R^2$, $R^3$, $R^4$, $R^5$, $R^6$, $L^1$, $L^2$, $G^2$, and m are as defined above (in particular, with respect to formula (I)) or below (in particular, with respect to any one of formulas (Ib), (Ic), (Id), (Ie), (If), (Ig), (Ih), (Ii), (Ij), (IIa), (IIb), (IIIa), and (IIIb)), and each $G^1$ is independently unsubstituted $C_1$-$C_{12}$ alkylene or unsubstituted $C_{2-12}$ alkenylene, e.g., unsubstituted, straight $C_{1-12}$ alkylene or unsubstituted, straight $C_{2-12}$ alkenylene. In some embodiments, each $G^1$ is independently unsubstituted $C_{6-12}$ alkylene or unsubstituted $C_{6-12}$ alkenylene, e.g., unsubstituted, straight $C_{6-12}$ alkylene or unsubstituted, straight $C_{6-12}$ alkenylene. In some embodiments, each $G^1$ is independently unsubstituted $C_{8-12}$ alkylene or unsubstituted $C_{8-12}$ alkenylene, e.g., unsubstituted, straight $C_{8-12}$ alkylene or unsubstituted, straight $C_{8-12}$ alkenylene. In some embodiments, each $G^1$ is independently unsubstituted $C_{6-10}$ alkylene or unsubstituted $C_{6-10}$ alkenylene, e.g., unsubstituted, straight $C_{6-10}$ alkylene or unsubstituted, straight $C_{6-10}$ alkenylene. In some embodiments, each $G^1$ is independently unsubstituted alkylene having 8, 9 or 10 carbon atoms, e.g., unsubstituted, straight alkylene having 8, 9 or 10 carbon atoms. In some embodiments, where $R^1$ and $R^2$ are both independently -$G^1$-$L^1$-$R^6$, $G^1$ for $R^1$ may be different from $G^1$ for $R^2$. In some of these embodiments, for example, $G^1$ for $R^1$ is unsubstituted, straight $C_{1-12}$ alkylene and $G^1$ for $R^2$ is unsubstituted, straight $C_{2-12}$ alkenylene; or $G^1$ for $R^1$ is an unsubstituted, straight $C_{1-12}$ alkylene group and $G^1$ for $R^2$ is a different unsubstituted, straight $C_{1-12}$ alkylene group. In some embodiments, where $R^1$ and $R^2$ are both independently -$G^1$-$L^1$-$R^6$, $G^1$ for $R^1$ may be identical to $G^1$ for $R^2$. In some of these embodiments, for example, each $G^1$ is the same unsubstituted, straight $C_{8-12}$ alkylene, such as unsubstituted, straight $C_{8-10}$ alkylene, or each $G^1$ is the same unsubstituted, straight $C_{6-12}$ alkenylene.

In some embodiments, the cationically ionizable lipid comprises the structure of the following formula (Ib):

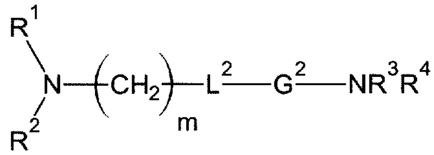

wherein $R^1$, $R^2$, $R^3$, $R^4$, $R^5$, $R^6$, $L^2$, $G^1$, $G^2$, and m are as defined above (in particular, with respect to any one of formulas (I) and (Ia)) or below (in particular, with respect to any one of formulas (Ic), (Id), (Ie), (If), (Ig), (Ih), (Ii), (Ij), (IIa), (IIb), (IIIa), and (IIIb)), and each $L^1$ is independently selected from the group consisting of -O(C=O)-, -(C=O)O-, -C(=O)S-, -SC(=O)-, -$NR^a$C(=O)-, and -C(=O)$NR^a$-. In some embodiments, $R^a$ of $L^1$ is H or $C_{1-12}$ alkyl. In some embodiments, $R^a$ of $L^1$ is H or $C_{1-6}$ alkyl, e.g., H or $C_{1-3}$ alkyl. In some embodiments, $R^a$ of $L^1$ is H, methyl, or ethyl. In some embodiments, each $L^1$ is independently selected from the group consisting of -O(C=O)-, -(C=O)O-, -C(=O)S-, and -SC(=O)-. In some embodiments, each $L^1$ is independently -O(C=O)- or -(C=O)O-. In some embodiments, where $R^1$ and $R^2$ are both independently -$G^1$-$L^1$-$R^6$, $L^1$ for $R^1$ may be different from $L^1$ for $R^2$. In some of these embodiments, for example, $L^1$ for $R^1$ is one moiety selected from the group consisting of -O(C=O)-, -(C=O)O-, -C(=O)S-, -SC(=O)-, -$NR^a$C(=O)-, and -C(=O)$NR^a$- (e.g., $L^1$ for $R^1$ is -O(C=O)-), and $L^1$ for $R^2$ is a different moiety selected from the group consisting of -O(C=O)-, -(C=O)O-, -C(=O)S-, -SC(=O)-, -$NR^a$C(=O)-, and -C(=O)$NR^a$- (e.g., $L^1$ for $R^2$ is -(C=O)O-). In some embodiments, where $R^1$ and $R^2$ are both independently -$G^1$-$L^1$-$R^6$, $L^1$ for $R^1$ may be identical to $L^1$ for $R^2$. In some of these embodiments, for example, each $L^1$ is the same moiety selected from the group consisting of -O(C=O)-, -(C=O)O-, -C(=O)S-, -SC(=O)-, -$NR^a$C(=O)-, and -C(=O)$NR^a$-, e.g., each $L^1$ is -O(C=O)- or each $L^1$ is -(C=O)O-.

In some embodiments, the cationically ionizable lipid comprises the structure of the following formula (Ic):

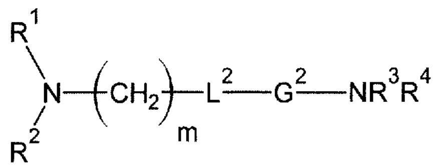

wherein $R^1$, $R^2$, $R^3$, $R^4$, $R^5$, $L^1$, $L^2$, $G^1$, $G^2$, and m are as defined above (in particular, with respect to any one of formulas (I), (Ia), and (Ib)) or below in particular, with respect to any one of formulas (Id), (Ie), (If), (Ig), (Ih), (Ii), (Ij), (IIa), (IIb), (IIIa), and (IIIb)), and each $R^6$ is independently a non-cyclic hydrocarbyl group having at least 10 carbon atoms, e.g., a straight hydrocarbyl group having at least 10 carbon atoms. In some embodiments, each $R^6$ has independently at most 30 carbon atoms, such as at most 28, at most 26, at most 24, at most 22, or at most 20 carbon atoms. In some embodiments, each $R^6$ is independently a non-cyclic hydrocarbyl group having 10 to 30 carbon atoms (such as 10 to 28, 10 to 26, 10 to 24, 10 to 22, or 10 to 20 carbon atoms), e.g, a straight hydrocarbyl group having 10 to 30 carbon atoms (such as 10 to 28, 10 to 26, 10 to 24, 10 to 22, or 10 to 20 carbon atoms). In some embodiments, each $R^6$ is attached to $L^1$ via an internal carbon atom of $R^6$. In some embodiments, each $R^6$ has independently at most 30 carbon atoms (such as at most 28, at most 26, at most 24, at most 22, or at most 20 carbon atoms), and each $R^6$ is attached to $L^1$ via an internal carbon atom of $R^6$. In some embodiments, each $R^6$ is independently a non-cyclic hydrocarbyl group having at least 10 carbon atoms, e.g., a straight hydrocarbyl group having at least 10 carbon atoms, and each $R^6$ is attached to $L^1$ via an internal carbon atom of $R^6$. In some

embodiments, each $R^6$ is independently a non-cyclic hydrocarbyl group having 10 to 30 carbon atoms (such as 10 to 28, 10 to 26, 10 to 24, 10 to 22, or 10 to 20 carbon atoms), e.g., a straight hydrocarbyl group having 10 to 30 carbon atoms (such as 10 to 28, 10 to 26, 10 to 24, 10 to 22, or 10 to 20 carbon atoms), and each $R^6$ is attached to $L^1$ via an internal carbon atom of $R^6$. In some embodiments, the hydrocarbyl group of $R^6$ is an alkyl or alkenyl group, e.g., a $C_{10-30}$ alkyl or alkenyl group. Thus, in some embodiments, each $R^6$ is independently a non-cyclic alkyl group having at least 10 carbon atoms or a non-cyclic alkenyl group having at least 10 carbon atoms, e.g., a straight alkyl group having at least 10 carbon atoms or a straight alkenyl group having at least 10 carbon atoms. In some embodiments, each $R^6$ is independently a non-cyclic alkyl group having 10 to 30 carbon atoms (such as 10 to 28, 10 to 26, 10 to 24, 10 to 22, or 10 to 20 carbon atoms) or a non-cyclic alkenyl group having 10 to 30 carbon atoms (such as 10 to 28, 10 to 26, 10 to 24, 10 to 22, or 10 to 20 carbon atoms), e.g., a straight alkyl group having 10 to 30 carbon atoms (such as 10 to 28, 10 to 26, 10 to 24, 10 to 22, or 10 to 20 carbon atoms) or a straight alkenyl group having 10 to 30 carbon atoms (such as 10 to 28, 10 to 26, 10 to 24, 10 to 22, or 10 to 20 carbon atoms). In some embodiments, each $R^6$ is independently a non-cyclic alkyl group having 11 to 19 carbon atoms (such as 11, 13, 15, 17, or 17 carbon atoms), e.g., a straight alkyl group having 11 to 19 carbon atoms (such as 11, 13, 15, 17, or 17 carbon atoms). In some embodiments, each $R^6$ is independently a non-cyclic alkyl group having 10 to 30 carbon atoms (such as 10 to 28, 10 to 26, 10 to 24, 10 to 22, or 10 to 20 carbon atoms) or a non-cyclic alkenyl group having 10 to 30 carbon atoms (such as 10 to 28, 10 to 26, 10 to 24, 10 to 22, or 10 to 20 carbon atoms), e.g., a straight alkyl group having 10 to 30 carbon atoms (such as 10 to 28, 10 to 26, 10 to 24, 10 to 22, or 10 to 20 carbon atoms) or a straight alkenyl group having 10 to 30 carbon atoms (such as 10 to 28, 10 to 26, 10 to 24, 10 to 22, or 10 to 20 carbon atoms), and each $R^6$ is attached to $L^1$ via an internal carbon atom of $R^6$. In some embodiments, each $R^6$ is independently a non-cyclic alkyl group having 11 to 19 carbon atoms (such as 11, 13, 15, 17, or 17 carbon atoms), e.g., a straight alkyl group having 11 to 19 carbon atoms (such as 11, 13, 15, 17, or 17 carbon atoms), and each $R^6$ is attached to $L^1$ via an internal carbon atom of $R^6$. The expression "internal carbon atom" means that the carbon atom of $R^6$ by which $R^6$ is attached to $L^1$ is directly bonded to at least 2 other carbon atoms of $R^6$. For example, for the following $C_{11}$ alkyl group, each carbon atom at any one of positions 2, 3, 4, 5, and 7 qualifies as "internal carbon atom" according to the present disclosure, whereas the carbon atoms at positions 1, 6, 8, 9, 10, and 11 do not.

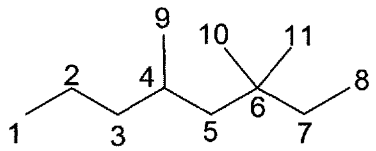

Consequently, $R^6$ being a $C_{11}$ alkyl group attached to $L^1$ via an internal carbon of $R^6$ includes the following groups:

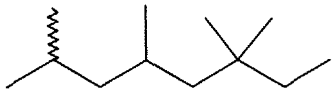
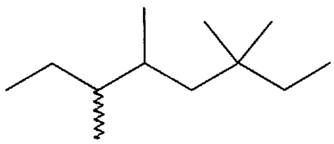
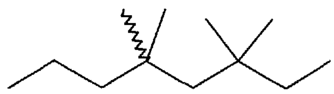

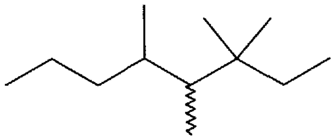
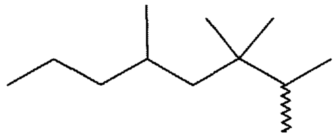

wherein 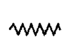 represents the bond by which $R^6$ is bound to $L^1$. Furthermore, for a straight alkyl group, e.g., a straight $C_{11}$ alkyl group, each carbon atom except for the first and last carbon atoms of the straight alkyl group (i.e, except the carbon atoms at positions 1 and 11 of the straight $C_{11}$ alkyl group) qualifies as "internal carbon atom". Thus, in some embodiments, $R^6$ being a straight alkyl group having p carbon atoms and being attached to $L^1$ via an internal carbon atom of $R^6$ means that $R^6$ is attached to $L^1$ via a carbon atom of $R^6$ at any one of positions 2 to (p-1) (thereby excluding the terminal C atoms at positions 1 and p). In some embodiments, where $R^6$ is a straight alkyl group having p' carbon atoms (wherein p' is an even number) and being attached to $L^1$ via an internal carbon atom of $R^6$, $R^6$ is attached to $L^1$ via a carbon at any one of positions (p'/2 - 1), (p'/2), and (p'/2 + 1) of $R^6$ (e.g., if p' is 10, $R^6$ is attached to $L^1$ via a carbon atom at any one of positions 4, 5, and 6 of $R^6$). In some embodiments, where $R^6$ is a straight alkyl group having p" carbon atoms (wherein p'' is an uneven number) and being attached to $L^1$ via an internal carbon atom of $R^6$, $R^6$ is attached to $L^1$ via a carbon atom at any one of positions (p" - 1)/2 and (p" + 1)/2 of $R^6$ (e.g., if p" is 11, $R^6$ is attached to $L^1$ via a carbon at any one of positions 5 and 6 of $R^6$). Generally, it is to be understood that if both $R^1$ and $R^2$ are $-G^1-L^1-R^6$ and each $R^6$ is attached to $L^1$ via an internal carbon atom of $R^6$, $R^6$ of

$R^1$ is attached to $L^1$ of $R^1$ (and not to $L^1$ of $R^2$) via an internal carbon atom of $R^6$ of $R^1$ and $R^6$ of $R^2$ is attached to $L^1$ of $R^2$ (and not to $L^1$ of $R^1$) via an internal carbon atom of $R^6$ of $R^2$. In some embodiments, each $R^6$ is independently selected from the group consisting of:

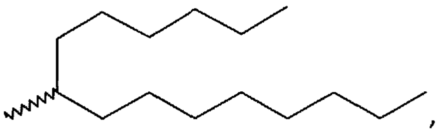,
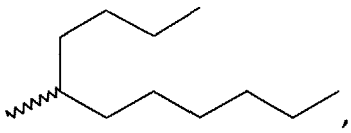,
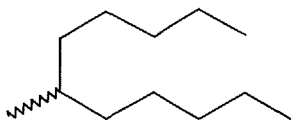,

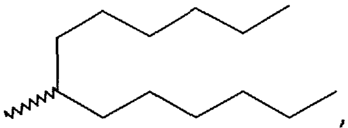,
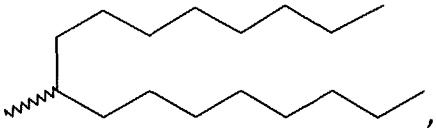,

and

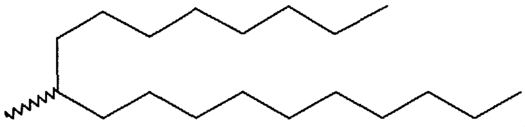,

wherein  represents the bond by which $R^6$ is bound to $L^1$. In some embodiments, where $R^1$ and $R^2$ are both independently $-G^1-L^1-R^6$, $R^6$ for $R^1$ is different from $R^6$ for $R^2$. In some of these embodiments, for example, $R^6$ for $R^1$ may be a non-cyclic, preferably straight, hydrocarbyl group having at least 10 carbon atoms (e.g., $R^6$ for $R^1$ is

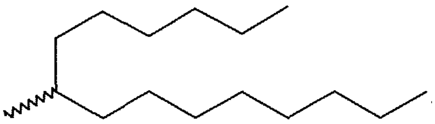

) and $R^6$ for $R^2$ may be a different non-cyclic, preferably straight, hydrocarbyl group having at least 10 carbon atoms (e.g., $R^6$ for $R^2$ is

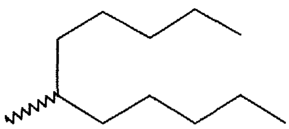

). In some embodiments, where $R^1$ and $R^2$ are both independently $-G^1-L^1-R^6$, $R^6$ for $R^1$ is identical to $R^6$ for $R^2$. In some of these embodiments, for example, each $R^6$ is the same non-cyclic, preferably straight, hydrocarbyl group having at least 10 carbon atoms (e.g., each $R^6$ is

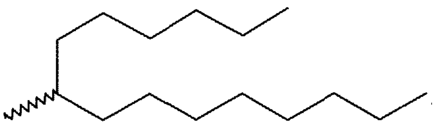

).

In some embodiments, the cationically ionizable lipid comprises the structure of the following formula (Id):

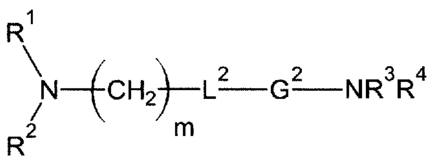

wherein $R^1$, $R^2$, $R^3$, $R^4$, $R^6$, $L^1$, $L^2$, $G^1$, $G^2$, and m are as defined above (in particular, with respect to any one of formulas (I), (Ia), (Ib), and (Ic)) or below (in particular, with respect to any one of formulas (Ie), (If), (Ig), (Ih), (Ii), (Ij), (IIa), (IIb), (IIIa), and (IIIb)), and $R^5$ is a non-cyclic hydrocarbyl group having at least 10 carbon atoms, e.g., a straight hydrocarbyl group having at least 10 carbon atoms. In some embodiments, $R^5$ is a non-cyclic hydrocarbyl group having at least 12 carbon atoms, such as at least 14, at least 16, or at least 18 carbon atoms, e.g., a straight hydrocarbyl group having at least 12, at least 14, at least 16, or at least 18 carbon atoms. In some embodiments, $R^5$ has at most 30 carbon atoms, such as at most 28, at most 26, at most 24, at most 22, or at most 20 carbon atoms. In some embodiments, $R^5$ is a non-cyclic hydrocarbyl group, e.g., a straight hydrocarbyl group, wherein each hydrocarbyl group has 10 to 30 carbon atoms (such as 10 to 28, 10 to 26, 10 to 24, 10 to 22, 10 to 20 carbon atoms, or 12 to 30, 12 to 28, 12 to 26, 12 to 24, 12 to 22, 12 to 20 carbon atoms, or 14 to 30, 14 to 28, 14 to 26, 14 to 24, 14 to 22, 14 to 20 carbon atoms, or 16 to 30, 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms). In some embodiments, the hydrocarbyl group of $R^5$ is an alkyl or alkenyl group, e.g., a $C_{10-30}$ alkyl or alkenyl group. Thus, in some embodiments, $R^5$ is a non-cyclic alkyl group having at least 10 carbon atoms (such as at least 12, at least 14, at least 16, or at least 18 carbon atoms) or a non-cyclic alkenyl group having at least 10 carbon atoms (such as at least 12, at least 14, at least 16, or at least 18 carbon atoms), e.g., a straight alkyl group having at least 10 carbon atoms (such as at least 12, at least 14, at least 16, or at least 18 carbon atoms) or a straight alkenyl group having at least 10 carbon atoms (such as at least 12, at least 14, at least 16, or at least 18 carbon atoms). In some embodiments, $R^5$ is a non-cyclic alkyl group or a non-cyclic alkenyl group, e.g., a straight alkyl group or a straight alkenyl group, wherein each of the alkyl and alkenyl groups has independently 10 to 30 carbon atoms (such as 10 to 28, 10 to 26, 10 to 24, 10 to 22, 10 to 20 carbon atoms, or 12 to 30, 12 to 28, 12 to 26, 12 to 24, 12 to 22, 12 to 20 carbon atoms, or 14 to 30, 14 to 28, 14 to 26, 14 to 24, 14 to 22, 14 to 20 carbon atoms, or 16 to 30, 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms). In some embodiments, the alkenyl group has at least 2 carbon-carbon double bonds, e.g., 2 or 3 carbon-carbon double bonds, such as 2 carbon-carbon double bonds. In some embodiments, the alkenyl group has at least 1 carbon-carbon double bond in cis configuration, e.g., 1, 2 or 3, such as 2, carbon-carbon double bonds in *cis* configuration. Thus, in some embodiments, $R^5$ is a non-cyclic alkyl group or a non-cyclic alkenyl group, e.g., a straight alkyl group or a straight alkenyl group, wherein each of the alkyl and alkenyl groups has independently 10 to 30 carbon atoms (such as 10 to 28, 10 to 26, 10 to 24, 10 to 22, 10 to 20 carbon atoms, or 12 to 30, 12 to 28, 12 to 26, 12 to 24, 12 to 22, 12 to 20 carbon atoms, or 14 to 30, 14 to 28, 14 to 26, 14 to 24, 14 to 22, 14 to 20 carbon atoms, or 16 to 30, 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms) and the alkenyl group has at least 2 carbon-carbon double bonds, e.g., 2 or 3 carbon-carbon double bonds. In some embodiments, $R^5$ is a non-cyclic alkyl group or a non-cyclic alkenyl group, e.g., a straight alkyl group or a straight alkenyl group, wherein each of the alkyl and alkenyl groups has independently 10 to 30 carbon atoms (such as 10 to 28, 10 to 26, 10 to 24, 10 to 22, 10 to 20 carbon atoms, or 12 to 30, 12 to 28, 12 to 26, 12 to 24, 12 to 22, 12 to 20 carbon atoms, or 14 to 30, 14 to 28, 14 to 26, 14 to 24, 14 to 22, 14 to 20 carbon atoms, or 16 to 30, 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms) and the alkenyl group has at least 1 carbon-carbon double bond, such as 1, 2, or 3 carbon-carbon double bonds, in *cis* configuration. In some embodiments, $R^5$ has the following structure:

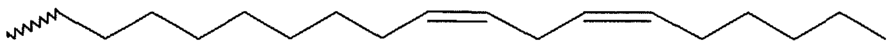,

wherein  represents the bond by which $R^5$ is bound to the remainder of the compound.

In some embodiments, the cationically ionizable lipid comprises the structure of the following formula (Ie):

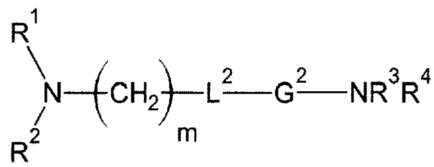

wherein $R^1$, $R^2$, $R^3$, $R^4$, $R^5$, $R^6$, $L^1$, $G^1$, $G^2$, and m are as defined above (in particular, with respect to any one of formulas (I), (Ia), (Ib), (Ic), and (Id)) or below (in particular, with respect to any one of formulas (If), (Ig), (Ih), (ii), (Ij), (IIa), (IIb), (IIIa), and (IIIb)), and $L^2$ is selected from the group consisting of -O(C=O)-, -(C=O)O-, -C(=O)-, -S-S-, -C(=O)S-, -SC(=O)-, $-NR^aC(=O)-$, $-C(=O)NR^a-$, $-NR^aC(=O)NR^a-$, $-OC(=O)NR^a-$ and $-NR^aC(=O)O-$. In some embodiments, $L^2$ is selected from the group consisting of -O(C=O)-, -(C=O)O-, -C(=O)-, -C(=O)S-, -SC(=O)-, $-NR^aC(=O)-$, and $-C(=O)NR^a-$. In some embodiments, $R^a$ of $L^2$ is H or $C_{1-12}$ alkyl. In some embodiments, $R^a$ of $L^2$ is H or $C_{1-6}$ alkyl, e.g., H or $C_{1-3}$ alkyl. In some embodiments, $R^a$ of $L^2$ is H, methyl, or ethyl. In some embodiments, $L^2$ is selected from the group consisting of -O(C=O)-, -(C=O)O-, -C(=O)S-, and -SC(=O)-. In some embodiments, $L^2$ is - O(C=O)- or -(C=O)O-.

In some embodiments, the cationically ionizable lipid comprises the structure of the following formula (If):

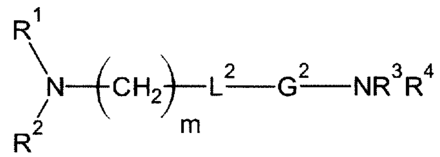

wherein $R^1$, $R^2$, $R^3$, $R^4$, $R^5$, $R^6$, $L^1$, $L^2$, $G^1$, and m are as defined above (in particular, with respect to any one of formulas (I), (Ia), (Ib), (Ic), (Id), and (Ie)) or below (in particular, with respect to any one of formulas (Ig), (Ih), (Ii), (Ij), (IIa), (IIb), (IIIa), and (IIIb)), and $G^2$ is unsubstituted $C_{1-12}$ alkylene or unsubstituted $C_{2-12}$ alkenylene, e.g., unsubstituted, straight $C_{1-12}$ alkylene or unsubstituted, straight $C_{2-12}$ alkenylene. In some embodiments, $G^2$ is unsubstituted $C_{2-10}$ alkylene or unsubstituted $C_{2-10}$ alkenylene, e.g., unsubstituted, straight $C_{2-10}$ alkylene or unsubstituted, straight $C_{2-10}$ alkenylene. In some embodiments, $G^2$ is unsubstituted $C_{2-6}$ alkylene or unsubstituted $C_{2-6}$ alkenylene, e.g., unsubstituted, straight $C_{2-6}$ alkylene or unsubstituted, straight $C_{2-6}$ alkenylene. In some embodiments, $G^2$ is unsubstituted $C_{2-4}$ alkylene or unsubstituted $C_{2-4}$ alkenylene, e.g., unsubstituted, straight $C_{2-4}$ alkylene or unsubstituted, straight $C_{2-4}$ alkenylene. In some embodiments, $G^2$ is ethylene or trimethylene. In some embodiments, the cationically ionizable lipid comprises the structure of the following formula (Ig):

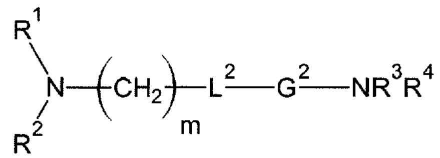

wherein $R^1$, $R^2$, $R^5$, $R^6$, $L^1$, $L^2$, $G^1$, $G^2$, and m are as defined above (in particular, with respect to any one of formulas (I), (Ia), (Ib), (Ic), (Id), (Ie), and (If)) or below (in particular, with respect to any one of formulas (Ih), (Ii), (Ij), (IIa), (IIb), (IIIa), and (IIIb)), and each of $R^3$ and $R^4$ is independently $C_{1-6}$ alkyl or $C_{2-6}$ alkenyl. In some embodiments, each of $R^3$ and $R^4$ is independently $C_{1-4}$ alkyl or $C_{2-4}$ alkenyl. In some embodiments, each of $R^3$ and $R^4$ is independently $C_{1-3}$ alkyl. In some embodiments, each of $R^3$ and $R^4$ is independently methyl or ethyl. In some embodiments, each of $R^3$ and $R^4$ is methyl.

In some embodiments, the cationically ionizable lipid comprises the structure of the following formula (Ih):

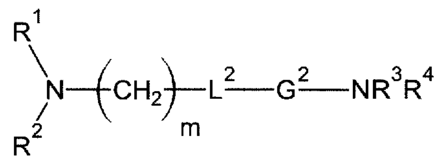

wherein $R^1$, $R^2$, $R^3$, $R^4$, $R^5$, $R^6$, $L^1$, $L^2$, $G^1$, and $G^2$ are as defined above (in particular, with respect to any one of formulas (I), (Ia), (Ib), (Ic), (Id), (Ie), (If), and (Ig)) or below (in particular, with respect to any one of formulas (Ii), (Ij), (IIa), (IIb), (IIIa), and (IIIb)), and m is 0, 1, 2 or 3. In some embodiments, m is 0 or 2. In some embodiments, m is 0. In some embodiments, m is 2.

In some embodiments, the cationically ionizable lipid comprises the structure of the following formula (Ii):

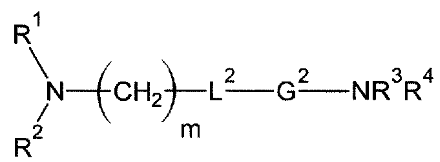

wherein one of $R^1$ and $R^2$ is $R^5$ and the other is $-G^1-L^1-R^6$, and $R^3$, $R^4$, $R^5$, $R^6$, $L^1$, $L^2$, $G^1$, $G^2$, and m are as defined above (in particular, with respect to any one of formulas (I), (Ia), (Ib), (Ic), (Id), (Ie), (If), (Ig), amd (Ih)) or below (in particular, with respect to any one of formulas (Ij), (IIa), (IIb), (IIIa), and (IIIb)). In some embodiments, $R^5$ is as defined for any one of formulas (I), (Id), (IIa), and (IIIa); $G^1$ is as defined for any one of formulas (I), (Ia), (IIa), (IIb), (IIIa), and (IIIb); $L^1$ is as defined for any one of formulas (I), (Ib), (IIa), (IIb), (IIIa), and (IIIb); and $R^6$ is as defined for any one of formulas (I), (Ic), (IIa), (IIb), (IIIa), and (IIIb). In some embodiments, $R^5$ is a non-cyclic alkyl or alkenyl group, e.g., a straight alkyl or alkenyl group, wherein each of the alkyl and alkenyl groups has at least 14 carbon atoms; $G^1$ is unsubstituted, straight $C_{4-12}$ alkylene or $C_{4-12}$ alkenylene; $L^1$ is -O(C=O)- or -(C=O)O-; and $R^6$ is (i) a straight hydrocarbyl group having at least 10 carbon atoms; or (ii) attached to $L^1$ via an internal carbon atom of $R^6$; or (iii) both, i.e., is a straight hydrocarbyl group having at least 10 carbon atoms and is attached to $L^1$ via an internal carbon atom of $R^6$.

In some embodiments, the cationically ionizable lipid comprises the structure of the following formula (Ij):

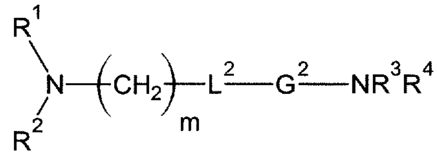

wherein each of $R^1$ and $R^2$ is independently $-G^1-L^1-R^6$, and $R^3$, $R^4$, $R^6$, $L^1$, $L^2$, $G^1$, $G^2$, and m are as defined above (in particular, with respect to any one of formulas (I), (Ia), (Ib), (Ic), (Ie), (if), (Ig), and (Ih)) or below (in particular, with respect to any one of formulas (IIa), (IIb), (IIIa), and (IIIb)). In some embodiments, each $G^1$ is independently as defined for any one of formulas (I), (Ia), (IIa), (IIb), (IIIa), and (IIIb); each $L^1$ is independently as defined for any one of formulas (I), (Ib), (IIa), (IIb), (IIIa), and (IIIb); and each $R^6$ is independently as defined for any one of formulas (I), (Ic), (IIa), (IIb), (IIIa), and (IIIb). In some embodiments, each $G^1$ is independently unsubstituted, straight $C_{4-12}$ alkylene or $C_{4-12}$ alkenylene, such as unsubstituted, straight $C_{8-12}$ alkylene or unsubstituted, straight $C_{8-12}$ alkenylene; each $L^1$ is independently -O(C=O)- or -(C=O)O-; and each $R^6$ is independently (i) a straight hydrocarbyl group having at least 10 carbon atoms; or (ii) attached to $L^1$ via an internal carbon atom of $R^6$; or (iii) both, i.e., is a straight hydrocarbyl group having at least 10 carbon atoms and is attached to $L^1$ via an internal carbon atom of $R^6$. In some embodiments, $R^6$ for $R^1$ is different from $R^6$ for $R^2$. For example, $R^6$ for $R^1$ may be a non-cyclic, preferably straight, hydrocarbyl group having at least 10 carbon atoms (e.g., $R^6$ for $R^1$ is

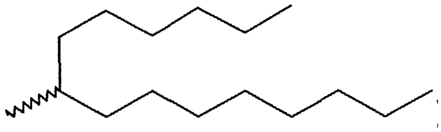

) and $R^6$ for $R^2$ may be a different non-cyclic, preferably straight, hydrocarbyl group having at least 10 carbon atoms (e.g., $R^6$ for $R^2$ is

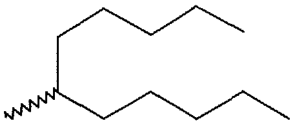

). In some embodiments, $R^6$ for $R^1$ is identical to $R^6$ for $R^2$. In some of these embodiments, for example, each $R^6$ is the same non-cyclic, preferably straight, hydrocarbyl group having at least 10 carbon atoms (e.g., each $R^6$ is

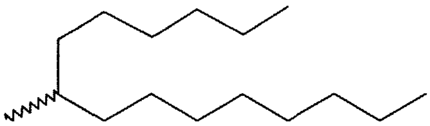

). In some embodiments, $L^1$ for $R^1$ is different from $L^1$ for $R^2$. For example, $L^1$ for $R^1$ may be -O(C=O)-, and $L^1$ for $R^2$ may be -(C=O)O-. In some embodiments, $L^1$ for $R^1$ is identical to $L^1$ for $R^2$. For example, each $L^1$ may be -O(C=O)- or each $L^1$ may be -(C=O)O-. In some embodiments, $G^1$ for $R^1$ is different from $G^1$ for $R^2$. For example, $G^1$ for $R^1$ may be unsubstituted, straight $C_{1-12}$ alkylene and $G^1$ for $R^2$ may be unsubstituted, straight $C_{2-12}$ alkenylene; or $G^1$ for $R^1$ may be an unsubstituted, straight $C_{1-12}$ alkylene group (such as an unsubstituted, straight $C_{8-12}$ alkylene group) and $G^1$ for $R^2$ may be a different unsubstituted, straight $C_{1-12}$ alkylene group. In some embodiments, $G^1$ for $R^1$ is identical to $G^1$ for $R^2$. For example, each $G^1$ is the same unsubstituted, straight $C_{4-12}$ alkylene, such as unsubstituted, straight $C_{8-12}$ alkylene, or each $G^1$ is the same unsubstituted straight $C_{8-12}$ alkenylene. In some embodiments, each $G^1$ is the same unsubstituted straight $C_{8-12}$ alkenylene; each $L^1$ is either -O(C=O)- or -(C=O)O-; and each $R^6$ is the same non-cyclic, preferably straight, hydrocarbyl group having at least 10 carbon atoms and is attached to $L^1$ via an internal carbon atom of $R^6$. In some embodiments, the cationically ionizable lipid comprises the structure of one of the following formulas (IIa) or (IIb):

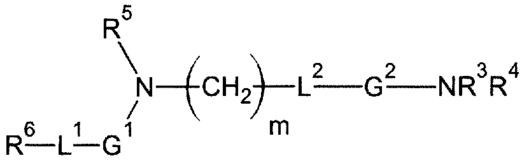
(IIa)

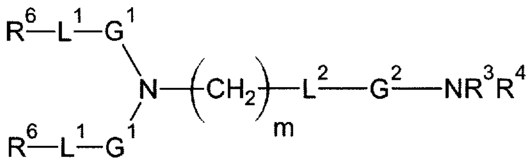

(IIb),

wherein

each of $R^3$ and $R^4$ is independently $C_1$-$C_6$ alkyl or $C_{2-6}$ alkenyl;

$R^5$ is a straight hydrocarbyl group having at least 14 carbon atoms (such as at least 16 carbon atoms), wherein the hydrocarbyl group preferably has at least 2 carbon-carbon double bonds; each $R^6$ is independently a straight hydrocarbyl group (e.g., a straight alkyl group) having at least 10 carbon atoms and/or each $R^6$ is attached to $L^1$ via an internal carbon atom of $R^6$, preferably each $R^6$ is independently a straight hydrocarbyl group (e.g., a straight alkyl group) having at least 10 carbon atoms and each $R^6$ is attached to $L^1$ via an internal carbon atom of $R^6$;

each $G^1$ is independently unsubstituted, straight $C_{4-12}$ alkylene or $C_{4-12}$ alkenylene, e.g., unsubstituted, straight $C_{6-12}$ alkylene or $C_{6-12}$ alkenylene, such as unsubstituted, straight $C_{8-12}$ alkylene or unsubstituted, straight $C_{8-12}$ alkenylene;

$G^2$ is unsubstituted $C_2$-$C_{10}$ alkylene or $C_{2-10}$ alkenylene, preferably unsubstituted $C_2$-$C_6$ alkylene or $C_{2-6}$ alkenylene;

each of $L^1$ and $L^2$ is independently -O(C=O)- or -(C=O)O-; and

m is 0, 1, 2 or 3, preferably 0 or 2.

In some embodiments of formula (IIa), $R^5$ has at most 30 carbon atoms, such as at most 28, at most 26, at most 24, at most 22, or at most 20 carbon atoms. In some embodiments of formulas (IIa), $R^5$ is a straight hydrocarbyl group having 14 to 30 carbon atoms (such as 14 to 28, 14 to 26, 14 to 24, 14 to 22, 14 to 20 carbon atoms, or 16 to 30, 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms). In some embodiments of formula (IIa), $R^5$ is a straight alkyl or alkenyl group having 14 to 30 carbon atoms (such as 14 to 28, 14 to 26, 14 to 24, 14 to 22, 14 to 20 carbon atoms, or 16 to 30, 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms). In some embodiments of formula (IIa), the alkenyl group has at least 2 carbon-carbon double bonds, e.g., 2 or 3 carbon-carbon double bonds, such as 2 carbon-carbon double bonds. In some embodiments, the alkenyl group has at least 1 carbon-carbon double bond in *cis* configuration, e.g., 1, 2 or 3, such as 2, carbon-carbon double bonds in *cis* configuration. Thus, in some embodiments of formula (IIa), $R^5$ is a straight alkyl group or a straight alkenyl group, wherein each of the alkyl and alkenyl groups has independently 14 to 30 carbon atoms (such as 14 to 28, 14 to 26, 14 to 24, 14 to 22, 14 to 20 carbon atoms, or 16 to 30, 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms) and the alkenyl group has at least 2 carbon-carbon double bonds, e.g., 2 or 3 carbon-carbon double bonds. In some embodiments of formula (IIa), $R^5$ is a straight alkyl group or a straight alkenyl group, wherein each of the alkyl and alkenyl groups has independently 14 to 30 carbon atoms (such as 14 to 28, 14 to 26, 14 to 24, 14 to 22, 14 to 20 carbon atoms, or 16 to 30, 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms) and the alkenyl group has at least 1 carbon-carbon double bond, such as 1, 2, or 3 carbon-carbon double bonds, in *cis* configuration. In some embodiments of formula (IIa), $R^5$ is a straight alkyl group or a straight alkenyl group, wherein each of the alkyl and alkenyl groups has independently 14 to 30 carbon atoms (such as 14 to 28, 14 to 26, 14 to 24, 14 to 22, 14 to 20 carbon atoms, or 16 to 30, 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms) and the alkenyl group has 2 or 3 carbon-carbon double bonds, wherein at least 1 carbon-carbon double bond, such as 1, 2, or 3 carbon-carbon double bonds, is in cis configuration. In some embodiments of formula (IIa), $R^5$ has the following structure:

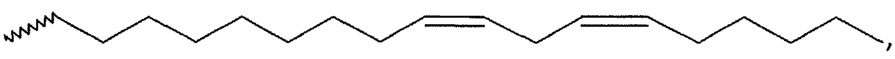,

wherein  represents the bond by which $R^5$ is bound to the remainder of the compound. In some embodiments of formula (IIa), $R^6$ has at most 30 carbon atoms, such as at most 28, at most 26, at most 24, at most 22, or at most 20 carbon atoms. In some embodiments of formula (IIa), $R^6$ is a non-cyclic hydrocarbyl group (e.g., a non-cyclic alkyl group) having 10 to 30 carbon atoms (such as 10 to 28, 10 to 26, 10 to 24, 10 to 22, or 10 to 20 carbon atoms), e.g., a straight hydrocarbyl group (e.g., a straight alkyl group) having 10 to 30 carbon atoms (such as 10 to 28, 10 to 26, 10 to 24, 10 to 22, or 10 to 20 carbon atoms). In some embodiments of formula (IIa), $R^6$ is a straight hydrocarbyl group (e.g., a straight alkyl group) having at least 10 carbon atoms and $R^6$ is attached to $L^1$ via an internal carbon atom of $R^6$. In some embodiments of formula (IIa), $R^6$ is a non-cyclic hydrocarbyl group (e.g., a non-cyclic alkyl group) having 10 to 30 carbon atoms (such as 10 to 28, 10 to 26, 10 to 24, 10 to 22, or 10 to 20 carbon atoms), e.g., a straight hydrocarbyl group (e.g., a straight alkyl group) having 10 to

30 carbon atoms (such as 10 to 28, 10 to 26, 10 to 24, 10 to 22, or 10 to 20 carbon atoms), and $R^6$ is attached to $L^1$ via an internal carbon atom of $R^6$. In some embodiments of formula (IIa), $G^1$ is independently unsubstituted, straight $C_{4-12}$ alkylene or $C_{4-12}$ alkenylene, e.g., unsubstituted, straight $C_{6-12}$ alkylene or $C_{6-12}$ alkenylene. In some embodiments of formula (IIa), $R^5$ is a straight hydrocarbyl group, e.g., a straight alkenyl group, having at least 14 carbon atoms (such as 14 to 30 carbon atoms) and 2 or 3 carbon-carbon double bonds; $R^6$ is a straight hydrocarbyl group (e.g., a straight alkyl group) having at least 10 carbon atoms (e.g., having 10 to 30 carbon atoms) and $R^6$ is attached to $L^1$ via an internal carbon atom of $R^6$; and $G^1$ is independently unsubstituted, straight $C_{4-12}$ alkylene or $C_{4-12}$ alkenylene, e.g., unsubstituted, straight $C_{6-12}$ alkylene or $C_{6-12}$ alkenylene.

In some embodiments of formula (IIb), each $R^6$ has independently at most 30 carbon atoms, such as at most 28, at most 26, at most 24, at most 22, or at most 20 carbon atoms. In some embodiments of formula (IIb), each $R^6$ is independently a straight hydrocarbyl group (e.g., a straight alkyl group) having 10 to 30 carbon atoms (such as 10 to 28, 10 to 26, 10 to 24, 10 to 22, or 10 to 20 carbon atoms, or 11 to 19 carbon atoms, such as 11, 13, 15, 17, or 17 carbon atoms). In some embodiments of formula (IIb), each $R^6$ is independently a straight hydrocarbyl group (e.g., a straight alkyl group) having 10 to 30 carbon atoms (such as 10 to 28, 10 to 26, 10 to 24, 10 to 22, or 10 to 20 carbon atoms, or 11 to 19 carbon atoms, such as 11, 13, 15, 17, or 17 carbon atoms) and each $R^6$ is attached to $L^1$ via an internal carbon atom of $R^6$. In some embodiments of formula (IIb), each $R^6$ is independently selected from the group consisting of:

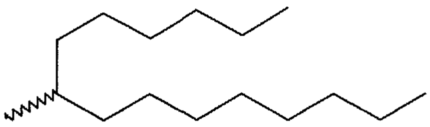,
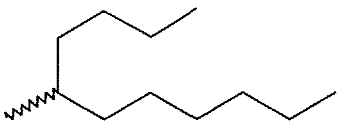,
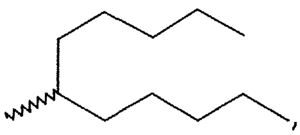,
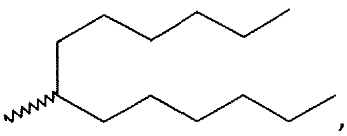,
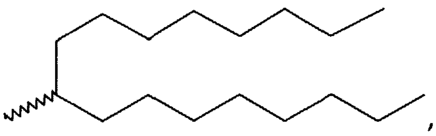,

and

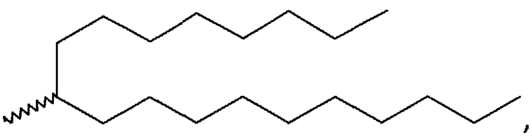,

wherein 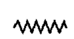 represents the bond by which $R^6$ is bound to $L^1$. In some embodiments of formula (IIb), each $G^1$ is independently unsubstituted, straight $C_{6-12}$ alkylene or $C_{6-12}$ alkenylene. In some embodiments of formula (IIb), each $G^1$ is independently unsubstituted, straight $C_{8-12}$ alkylene or $C_{8-12}$ alkenylene. In some embodiments of formula (IIb), each $R^6$ is independently a straight hydrocarbyl group (e.g., a straight alkyl group) having at least 10 carbon atoms (such as 10 to 28, 10 to 26, 10 to 24, 10 to 22, or 10 to 20 carbon atoms, or 11 to 19 carbon atoms, such as 11, 13, 15, 17, or 17 carbon atoms) and is attached to $L^1$ via an internal carbon atom of $R^6$; and each $G^1$ is independently unsubstituted, straight $C_{8-12}$ alkylene or $C_{8-12}$ alkenylene.

In some embodiments, the cationically ionizable lipid comprises the structure of one of the following formulas (IIIa) or (IIIb):

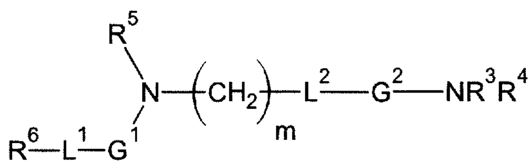

**(IIIa)**

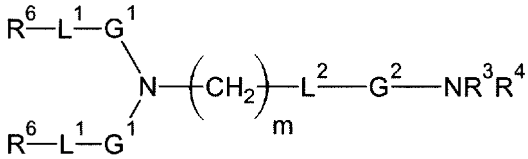

**(IIIb),**

wherein

each of $R^3$ and $R^4$ is independently $C_{1-4}$ alkyl or $C_{2-4}$ alkenyl, more preferably $C_{1-3}$ alkyl, such as methyl or ethyl;
$R^5$ is a straight alkyl or alkenyl group having at least 16 carbon atoms, wherein the alkenyl group preferably has at least 2 carbon-carbon double bonds;
each $R^6$ is independently a straight hydrocarbyl group having at least 10 carbon atoms, wherein $R^6$ is attached to $L^1$ via an internal carbon atom of $R^6$;
each $G^1$ is independently unsubstituted, straight $C_{6-12}$ alkylene or unsubstituted, straight $C_{6-12}$ alkenylene, e.g., unsubstituted, straight $C_{8-12}$ alkylene or unsubstituted, straight $C_{8-12}$ alkenylene, such as unsubstituted, straight $C_{8-10}$ alkylene or unsubstituted, straight $C_{8-10}$ alkenylene, such as unsubstituted, straight $C_8$ alkylene;
$G^2$ is unsubstituted $C_{2-6}$ alkylene or $C_{2-6}$ alkenylene, preferably unsubstituted $C_{2-4}$ alkylene or $C_{2-4}$ alkenylene, such as ethylene or trimethylene;
each of $L^1$ and $L^2$ is independently -O(C=O)- or -(C=O)O-; and
m is 0, 1, 2 or 3, preferably 0 or 2.

In some embodiments of formula (IIIa), $R^5$ has at most 30 carbon atoms, such as at most 28, at most 26, at most 24, at most 22, or at most 20 carbon atoms. In some embodiments of formulas (IIIa), $R^5$ is a straight alkyl or alkenyl group having 16 to 30 carbon atoms (such as 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms). In some embodiments of formula (IIIa), the alkenyl group has at least 2 carbon-carbon double bonds, e.g., 2 or 3 carbon-carbon double bonds, such as 2 carbon-carbon double bonds. In some embodiments, the alkenyl group has at least 1 carbon-carbon double bond in *cis* configuration, e.g., 1, 2 or 3, such as 2, carbon-carbon double bonds in *cis* configuration. Thus, in some embodiments of formula (IIIa), $R^5$ is a straight alkyl group or a straight alkenyl group, wherein each of the alkyl and alkenyl groups has independently 16 to 30 carbon atoms (such as 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms) and the alkenyl group has at least 2 carbon-carbon double bonds, e.g., 2 or 3 carbon-carbon double bonds. In some embodiments of formula (IIIa), $R^5$ is a straight alkyl group or a straight alkenyl group, wherein each of the alkyl and alkenyl groups has independently 16 to 30 carbon atoms (such as 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms) and the alkenyl group has at least 1 carbon-carbon double bond, such as 1, 2, or 3 carbon-carbon double bonds, in *cis* configuration. In some embodiments of formula (IIIa), $R^5$ is a straight alkyl group or a straight alkenyl group, wherein each of the alkyl and alkenyl groups has independently 16 to 30 carbon atoms (such as 16 to 28, 16 to 26, 16 to 24, 16 to 22, 16 to 20 carbon atoms, or 18 to 30, 18 to 28, 18 to 26, 18 to 24, 18 to 22, or 18 to 20 carbon atoms) and the alkenyl group has 2 or 3 carbon-carbon double bonds, wherein at least 1 carbon-carbon double bond, such as 1, 2, or 3 carbon-carbon double bonds, is in cis configuration. In some embodiments of formula (IIIa), $R^5$ has the following structure:

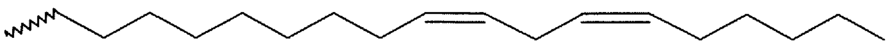,

wherein  represents the bond by which $R^5$ is bound to the remainder of the compound. In some embodiments of formula (IIIa), $R^6$ has at most 30 carbon atoms, such as at most 28, at most 26, at most 24, at most 22, or at most 20 carbon atoms. In some embodiments of formula (IIIa), $R^6$ is a straight hydrocarbyl group (e.g., a straight alkyl group) having 10 to 30 carbon atoms (such as 10 to 28, 10 to 26, 10 to 24, 10 to 22, or 10 to 20 carbon atoms) and $R^6$ is attached to $L^1$ via an internal carbon atom of $R^6$. In some embodiments of formula (IIIa), $R^6$ is a straight alkyl group having 10 to 30 carbon atoms (such as 10 to 28, 10 to 26, 10 to 24, 10 to 22, or 10 to 20 carbon atoms) and $R^6$ is attached to $L^1$ via an internal carbon atom of $R^6$. In some embodiments of formula (IIIa), $G^1$ is independently unsubstituted, straight $C_{4-12}$ alkylene or $C_{4-12}$ alkenylene, e.g., unsubstituted, straight $C_{6-12}$ alkylene or $C_{6-12}$ alkenylene. In some embodiments of formula (IIIa), $R^5$ is a straight hydrocarbyl group, e.g., a straight alkenyl group, having at least 16 carbon atoms (such as 16 to 30 carbon atoms) and 2 or 3 carbon-carbon double bonds; $R^6$ is a straight hydrocarbyl group (e.g., a straight alkyl group) having at least 10 carbon atoms (e.g., having 10 to 30 carbon atoms) and $R^6$ is attached to $L^1$ via an internal carbon atom of $R^6$; and $G^1$ is independently unsubstituted, straight $C_{4-12}$ alkylene or $C_{4-12}$ alkenylene, e.g., unsubstituted, straight $C_{6-12}$ alkylene or $C_{6-12}$ alkenylene.

In some embodiments of formula (IIIb), each $R^6$ has independently at most 30 carbon atoms, such as at most 28, at most 26, at most 24, at most 22, or at most 20 carbon atoms. In some embodiments of formula (IIIb), each $R^6$ is independently a straight hydrocarbyl group (e.g., a straight alkyl group) having 10 to 30 carbon atoms (such as 10 to 28, 10 to 26, 10 to 24, 10 to 22, or 10 to 20 carbon atoms, or 11 to 19 carbon atoms, such as 11, 13, 15, 17, or 17 carbon atoms) and each $R^6$ is attached to $L^1$ via an internal carbon atom of $R^6$. In some embodiments of formula (IIIb), each $R^6$ is attached to $L^1$ via an internal carbon atom of $R^6$ and is independently selected from the group consisting of:

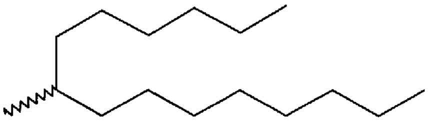,
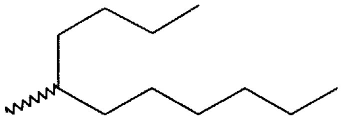,
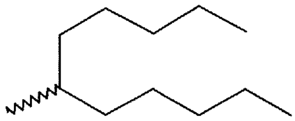,

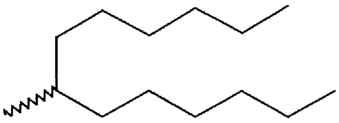,
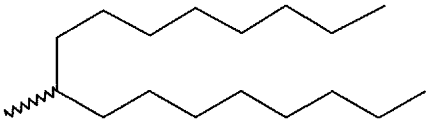,

and

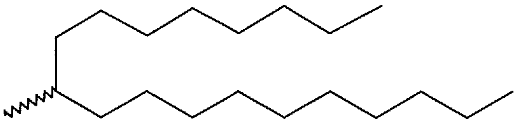,

wherein  represents the bond by which $R^6$ is bound to $L^1$. In some embodiments of formula (IIIb), each $G^1$ is independently unsubstituted, straight $C_{8-12}$ alkylene or $C_{8-12}$ alkenylene, e.g., unsubstituted, straight $C_{8-10}$ alkylene or $C_{8-10}$ alkenylene. In some embodiments of formula (IIIb), each $R^6$ is independently a straight hydrocarbyl group (e.g., a straight alkyl group) having at least 10 carbon atoms (such as 10 to 28, 10 to 26, 10 to 24, 10 to 22, or 10 to 20 carbon atoms, or 11 to 19 carbon atoms, such as 11, 13, 15, 17, or 17 carbon atoms) and is attached to $L^1$ via an internal carbon atom of $R^6$; and each $G^1$ is independently unsubstituted, straight $C_{8-12}$ alkylene or $C_{8-12}$ alkenylene, e.g., unsubstituted, straight $C_{8-10}$ alkylene or $C_{8-10}$ alkenylene.

In some embodiments, the cationically ionizable lipid comprises one of the following formulas (IV-1), (IV-2), and (IV-3):

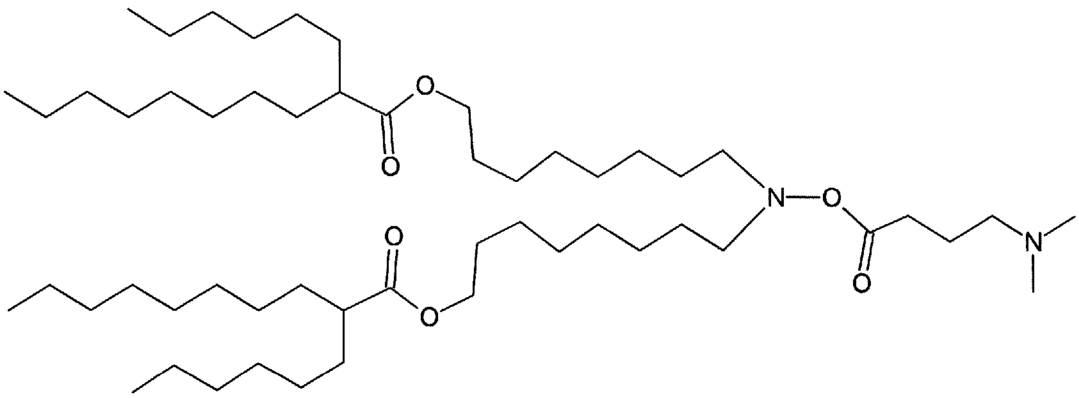

(IV-1);

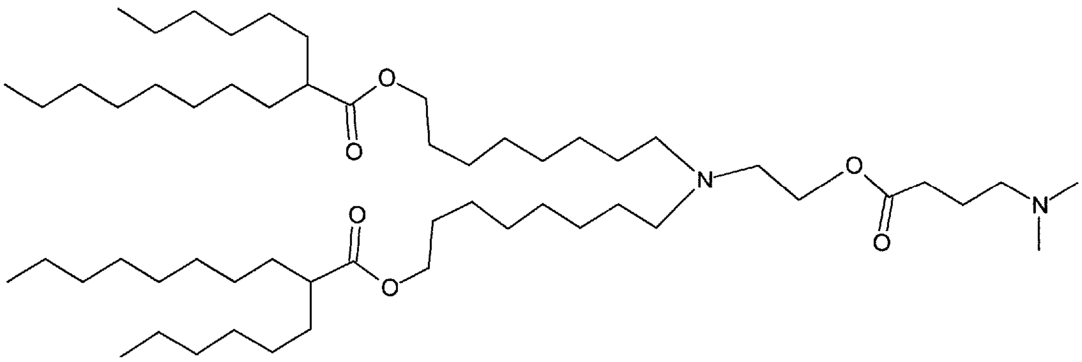

(IV-2);

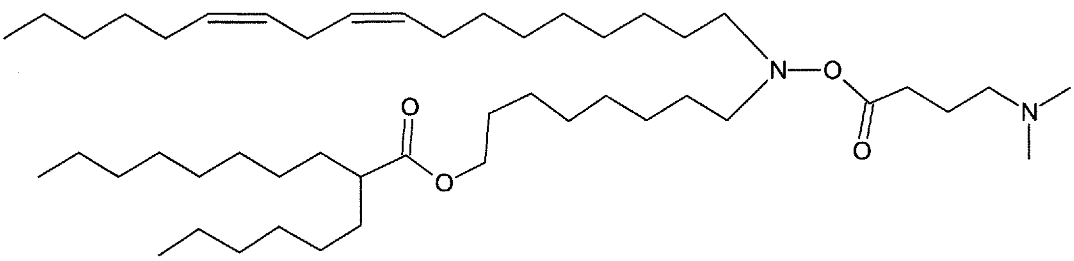

(IV-3).

The cationically ionizable lipids of formulas (IV-1), (IV-2), (IV-3) are also referred to herein as "HY-405", "EA-405", and "HY-501", respectively.

It is to be understood that any reference to a cationically ionizable lipid of any one of formulas (I), (Ia), (Ib), (Ic), (Id), (Ie), (If), (Ig), (Ih), (Ii), (Ij), (IIa), (IIb), (IIIa), (IIIb), (IV-1), (IV-2), and (IV-3) also includes the salts (in particular pharmaceutically acceptable salts), tautomers, stereoisomers, solvates (e.g., hydrates), and isotopically labeled forms thereof.

In some embodiments, the RNA compositions/formulations described herein comprise the cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)) as particle forming agent. The cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)) can be associated with nucleic acid (in particular RNA, such as mRNA), e.g., by forming complexes with the RNA or forming vesicles in which the RNA is enclosed or encapsulated.

In some embodiments, the cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)) may comprise from about 10 mol % to about 95 mol %, from about 20 mol % to about 95 mol %, from about 20 mol % to about 90 mol %, from about 30 mol % to about 90 mol %, from about 40 mol % to about 90 mol %, or from about 40 mol % to about 80 mol % of the total lipid present in the composition, such as from about 20 mol % to about 80 mol %, from about 25 mol % to about 70 mol %, from about 30 mol % to about 60 mol %, or from about 30 mol % to about 50 mol %, such as from about 40 mol % to about 60 mol %, from about 40 mol % to about 50 mol %, or from about 55 mol % to about 65 mol % of the total lipid present in the composition/formulation.

In some embodiments, where at least a portion of the cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)) is associated with at least a portion of the RNA to form particles (e.g., nanoparticles, such as LNPs), the cationically ionizable lipid may comprise from about 10 mol % to about 95 mol %, from about 20 mol % to about 95 mol %, from about 20 mol % to about 90 mol %, from about 30 mol % to about 90 mol %, from about 40 mol % to about 90 mol %, or from about 40 mol % to about 80 mol % of the total lipid present in the composition, such as from about 20 mol % to about 80 mol %, from about 25 mol % to about 70 mol %, from about 30 mol % to about 60 mol %, or from about 30 mol % to about 50 mol %, such as from about 40 mol % to about 60 mol %, from about 40 mol % to about 50 mol %, or from about 55 mol % to about 65 mol % of the total lipid present in the particles.

In some embodiments, the N/P value is at least about 4. In some embodiments, the N/P value ranges from 4 to 20, 4 to 12, 4 to 10, 4 to 8, or 5 to 7. In some embodiments, the N/P value is about 6. In some embodiments, the N/P value is about 8. In some embodiments, the N/P value is about 12.

**Additional cationic/cationically ionizable lipids**

In some embodiments, the RNA compositions/formulations described herein comprise, in addition to the cationically ionizable lipid of formula (I) (or of any one of the formulas (Ia), (Ib), (Ic), (Id), (Ie), (If), (Ig), (1h), (Ii), (Ij), (IIa), (IIb), (IIIa), (IIIb), (IV-1), (IV-2), and (IV-3)), one or more additional cationic or cationically ionizable lipids as particle forming agent. The additional one or more cationic or cationically ionizable lipids include any cationic or cationically ionizable lipids (including lipid-like materials) which are able to electrostatically bind nucleic acid. In some embodiments, the additional one or more cationic or cationically ionizable lipids can be associated with nucleic acid, *e.g.,* by forming complexes with the nucleic acid or forming vesicles in which the nucleic acid is enclosed or encapsulated.

As used herein, a "cationic lipid" refers to a lipid or lipid-like material having a net positive charge. Cationic lipids bind negatively charged nucleic acid by electrostatic interaction. Generally, cationic lipids possess a lipophilic moiety, such as a sterol, an acyl chain, a diacyl or more acyl chains, and the head group of the lipid typically carries the positive charge.

In some embodiments, a cationic lipid has a net positive charge only at certain pH, in particular acidic pH, while it has preferably no net positive charge, preferably has no charge, *i.e.,* it is neutral, at a different, preferably higher pH such as physiological pH. This ionizable behavior is thought to enhance efficacy through helping with endosomal escape and reducing toxicity as compared with particles that remain cationic at physiological pH.

As used herein, a "cationically ionizable lipid" refers to a lipid or lipid-like material which has a net positive charge

or is neutral, *i.e.,* which is not permanently cationic. Thus, depending on the pH of the composition in which the cationically ionizable lipid is solved, the cationically ionizable lipid is either positively charged or neutral. For purposes of the present disclosure, cationically ionizable lipids are covered by the term "cationic lipid" unless contradicted by the circumstances.

In some embodiments, the additional one or more cationic or cationically ionizable lipids comprise a head group which includes at least one nitrogen atom (N) which is positive charged or capable of being protonated, e.g., under physiological conditions.

Examples of the additional one or more cationic or cationically ionizable lipids include, but are not limited to N,N-dimethyl-2,3-dioleyloxypropylamine (DODMA), 1,2-dioleoyl-3-trimethylammonium propane (DOTAP), 4-((di((9Z,12Z)-octadeca-9,12-dien-1-yl)amino)oxy)-N,N-dimethyl-4-oxobutan-1-amine (DPL14), 1,2-di-O-octadecenyl-3-trimethylammonium propane (DOTMA), 3-(N-(N',N'-dimethylaminoethane)-carbamoyl)cholesterol (DC-Chol), dimethyldioctadecylammonium (DDAB); 1,2-dioleoyl-3-dimethylammonium-propane (DODAP); 1,2-diacyloxy-3-dimethylammonium propanes; 1,2-dialkyloxy-3-dimethylammonium propanes; dioctadecyldimethyl ammonium chloride (DODAC), 1,2-distearyloxy-N,N-dimethyl-3-aminopropane (DSDMA), 2,3-di(tetradecoxy)propyl-(2-hydroxyethyl)-dimethylazanium (DMRIE), 1,2-dimyristoyl-sn-glycero-3-ethylphosphocholine (DMEPC), l,2-dimyristoyl-3-trimethylammonium propane (DMTAP), 1,2-dioleyloxypropyl-3-dimethyl-hydroxyethyl ammonium bromide (DORIE), and 2,3-dioleoyloxy-N-[2(spermine carboxamide)ethyl]-N,N-dimethyl-l-propanamium trifluoroacetate (DOSPA), 1,2-dilinoleyloxy-N,N-dimethylaminopropane (DLinDMA), 1,2-dilinolenyloxy-N,N-dimethylaminopropane (DLenDMA), dioctadecylamidoglycyl spermine (DOGS), 3-dimethylamino-2-(cholest-5-en-3-beta-oxybutan-4-oxy)-1-(cis,cis-9,12-oc-tadecadienoxy)propane (CLinDMA), 2-[5'-(cholest-5-en-3-beta-oxy)-3'-oxapentoxy)-3-dimethyl-1-(cis,cis-9',12'-octadecadienoxy)propane (CpLinDMA), N,N-dimethyl-3,4-dioleyloxybenzylamine (DMOBA), 1,2-N,N'-dioleylcarbamyl-3-dimethylaminopropane (DOcarbDAP), 2,3-Dilinoleoyloxy-N,N-dimethylpropylamine (DLinDAP), 1,2-N,N'-Dilinoleylcarbamyl-3-dimethylaminopropane (DLincarbDAP), 1,2-Dilinoleoylcarbamyl-3-dimethylaminopropane (DLinCDAP), 2,2-dilinoleyl-4-dimethylaminomethyl-[1,3]-dioxolane (DLin-K-DMA), 2,2-dilinoleyl-4-dimethylaminoethyl-[1,3]-dioxolane (DLin-K-XTC2-DMA), 2,2-dilinoleyl-4-(2-dimethylaminoethyl)-[1,3]-dioxolane (DLin-KC2-DMA), heptatriaconta-6,9,28,31-tetraen-19-yl-4-(dimethylamino)butanoate (DLin-MC3-DMA), N-(2-Hydroxyethyl)-N,N-dimethyl-2,3-bis(tetradecyloxy)-1-propanaminium bromide (DMRIE), (±)-N-(3-aminopropyl)-N,N-dimethyl-2,3-bis(cis-9-tetradecenyloxy)-1-propanaminium bromide (GAP-DMORIE), (±)-N-(3-aminopropyl)-N,N-dimethyl-2,3-bis(dodecyloxy)-1-propanaminium bromide (GAP-DLRIE), (±)-N-(3-aminopropyl)-N,N-dimethyl-2,3-bis(tetradecyloxy)-1-propanaminium bromide (GAP-DMRIE), N-(2-Aminoethyl)-N,N-dimethyl-2,3-bis(tetradecyloxy)-1-propanaminium bromide (βAE-DMRIE), N-(4-carboxybenzyl)-N,N-dimethyl-2,3-bis(oleoyloxy)propan-1-aminium (DOBAQ), 2-({8-[(3β)-cholest-5-en-3-yloxy)octyl}oxy)-N,N-dimethyl-3-[(9Z,12Z)-octadeca-9,12-dien-1-yloxy]propan-1-amine (Octyl-CLinDMA), 1,2-dimyristoyl-3-dimethylammonium-propane (DMDAP), 1,2-dipalmitoyl-3-dimethylammonium-propane (DPDAP), N1-[2-((1S)-1-[(3-aminopropyl)amino]-4-[di(3-amino-propyl)amino]butylcarboxamido)ethyl]-3,4-di[oleyloxy]-benzamide (MVLS), 1,2-dioleoyl-sn-glycero-3-ethylphosphocholine (DOEPC), 2,3-bis(dodecyloxy)-N-(2-hydroxyethyl)-N,N-dimethylpropan-1-amonium bromide (DLRIE), N-(2-aminoethyl)-N,N-dimethyl-2,3-bis(tetradecyloxy)propan-1-aminium bromide (DMORIE), di((Z)-non-2-en-1-yl) 8,8'-((((2(dimethylamino)ethyl)thio)carbonyl)azanediyl)dioctanoate (ATX), N,N-dimethyl-2,3-bis(dodecyloxy)propan-1-amine (DLDMA), N,N-dimethyl-2,3-bis(tetradecyloxy)propan-1-amine (DMDMA), Di((Z)-non-2-en-1-yl)-9-((4-(dimethylaminobutanoyl)oxy)heptadecanedioate (L319), N-Dodecyl-3-((2-dodecylcarbamoyl-ethyl)-{2-[(2-dodecylcarbamoyl-ethyl)-2-{(2-dodecylcarbamoyl-ethyl)-[2-(2-dodecylcarbamoyl-ethylamino)-ethyl]-amino}-ethylamino)propionamide (lipidoid $98N_{12}$-5), 1-[2-[bis(2-hydroxydodecyl)amino]ethyl-[2-[4-[2-[bis(2 hydroxydodecyl)amino]ethyl]piperazin-1-yl]ethyl]amino]dodecan-2-ol (lipidoid C12-200).

In some embodiments, the additional cationic or cationically ionizable lipid is DOTMA. In some embodiments, the additional cationic or cationically ionizable lipid is DODMA. In some embodiments, the additional cationic or cationically ionizable lipid is DPL14.

DOTMA is a cationic lipid with a quaternary amine headgroup. The structure of DOTMA may be represented as follows:

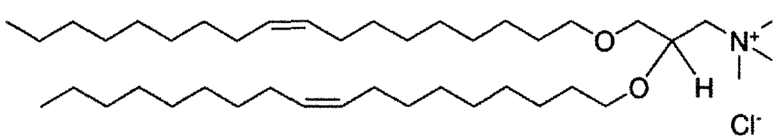

DODMA is an ionizable cationic lipid with a tertiary amine headgroup. The structure of DODMA may be represented as follows:

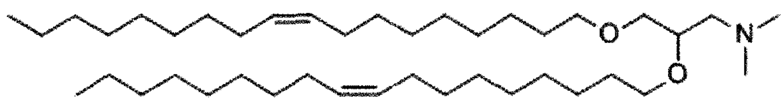

DPL14 is a cationic lipid with a tertiary amine headgroup. The structure of DPL14 may be represented as follows:

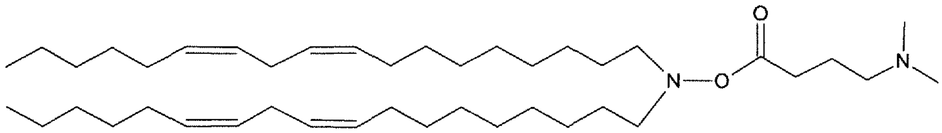

In some embodiments, the additional one or more cationic or cationically ionizable lipids may replace from 0 mol % to about 50 mol %, such as from 0 mol % to about 40 mol %, from about 1 mol % to about 30 mol %, from about 2 mol % to about 20 mol %, or from about 5 mol % to about 10 mol %, of the cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)). In some embodiments, the additional one or more cationic or cationically ionizable lipids may comprise from 0 mol % to about 20 mol %, from 0 mol % to 15 mol %, from about 0.5 mol % to about 10 mol %, or from about 1 mol % to about 5 mol % of the total lipid present in the RNA compositions/formulations described herein.

In some embodiments, where at least a portion of the cationically ionizable lipid of formula (I) together with at least a portion of the additional one or more cationic or cationically ionizable lipids are associated with at least a portion of the RNA to form particles (e.g., nanoparticles, such as LNPs), the additional one or more cationic or cationically ionizable lipids may comprise from 0 mol % to about 20 mol %, from 0 mol % to 15 mol %, from about 0.5 mol % to about 10 mol %, or from about 1 mol % to about 5 mol % of the total lipid present in the particles. In some embodiments, besides the cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)), the RNA compositions/formulations described herein do not comprise any additional cationic or cationically ionizable lipid (in other words, the total amount of additional cationic or cationically ionizable lipids besides the cationically ionizable lipid of formula (I) in the RNA compositions/formulations described herein is below detection limits or about 0 mol% .

Similarly, in some embodiments, where at least a portion of the cationically ionizable lipid of formula (I) is associated with at least a portion of the RNA to form particles (e.g., nanoparticles, such as LNPs), these particles do not contain any additional cationic or cationically ionizable lipids besides the cationically ionizable lipid of formula (I).

Additional lipids

The RNA compositions/formulations described herein may also comprise lipids (including lipid-like materials) other than cationic or cationically ionizable lipids (also collectively referred to herein as cationic lipids), *i.e.,* non-cationic lipids (including non-cationic or non-cationically ionizable lipids or lipid-like materials). Collectively, anionic and neutral lipids or lipid-like materials are referred to herein as non-cationic lipids. Optimizing the formulation of nucleic acid particles by addition of other hydrophobic moieties, such as cholesterol and lipids, in addition to a cationic or cationically ionizable lipid may enhance composition and/or particle stability and efficacy of RNA delivery.

One or more additional lipids may or may not affect the overall charge of the nucleic acid particles. In some embodiments, the or more additional lipids are a non-cationic lipid or lipid-like material. The non-cationic lipid may comprise, e.g., one or more anionic lipids and/or neutral lipids. As used herein, an "anionic lipid" refers to any lipid that is negatively charged at a selected pH. As used herein, a "neutral lipid" refers to any of a number of lipid species that exist either in an uncharged or neutral zwitterionic form at a selected pH.

In some embodiments, the RNA compositions/formulations described herein comprise the cationically ionizable lipid of formula (I) (or of any one of the formulas (IIa), (Ib), (Ic), (Id), (Ie), (If), (Ig), (Ih), (Ii), (Ij), (Ia), (Iib), (IIIa), (IIIb), (IV-1), (IV-2), and (IV-3)) and one or more additional lipids.

Without wishing to be bound by theory, the amount of the cationically ionizable lipid of formula (I) (or of any one of the formulas (Ia), (Ib), (Ic), (Id), (Ie), (If), (Ig), (Ih), (Ii), (Il), (IIa), (IIb), (IIIa), (IIIb), (IV-1), (IV-2), and (IV-3)) compared to the amount of the one or more additional lipids may affect important characteristics of RNA particles contained in the RNA compositions/formulations, such as charge, particle size, stability, tissue selectivity, and bioactivity of the nucleic acid. Accordingly, in some embodiments, the molar ratio of the cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)) to the one or more additional lipids is from about 10:0 to about 1:9, about 4:1 to about 1:2, about 4:1 to about 1:1, about 3:1 to about 1:1, or about 3:1 to about 2:1, or about 2:1 to about 3:2.

In some embodiments, the one or more additional lipids comprised in the RNA compositions/formulations

described herein comprise one or more of the following: neutral lipids, steroids, and combinations thereof.

In some embodiments, the one or more additional lipids comprise a neutral lipid which is a phospholipid. In some embodiments, the phospholipid is selected from the group consisting of phosphatidylcholines, phosphatidylethanolamines, phosphatidylglycerols, phosphatidic acids, phosphatidylserines and sphingomyelins. Specific phospholipids that can be used include, but are not limited to, phosphatidylcholines, phosphatidylethanolamines, phosphatidylglycerols, phosphatidic acids, phosphatidylserines or sphingomyelin. Such phospholipids include in particular diacylphosphatidylcholines, such as distearoylphosphatidylcholine (DSPC), dioleoylphosphatidylcholine (DOPC), dimyristoylphosphatidylcholine (DMPC), dipentadecanoylphosphatidylcholine, dilauroylphosphatidylcholine, dipalmitoylphosphatidylcholine (DPPC), diarachidoylphosphatidylcholine (DAPC), dibehenoylphosphatidylcholine (DBPC), ditricosanoylphosphatidylcholine (DTPC), dilignoceroylphatidylcholine (DLPC), palmitoyloleoyl-phosphatidylcholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC) and phosphatidylethanolamines, in particular diacylphosphatidylethanolamines, such as dioleoylphosphatidylethanolamine (DOPE), distearoyl-phosphatidylethanolamine (DSPE), dipalmitoyl-phosphatidylethanolamine (DPPE), dimyristoyl-phosphatidylethanolamine (DMPE), dilauroyl-phosphatidylethanolamine (DLPE), diphytanoyl-phosphatidylethanolamine (DPyPE), 1,2-di-(9Z-octadecenoyl)-sn-glycero-3-phosphocholine (DOPG), 1,2-dipalmitoyl-sn-glycero-3-phospho-(1'-rac-glycerol) (DPPG), 1-palmitoyl-2-oleoyl-sn-glycero-3-phosphoethanolamine (POPE), N-palmitoyl-D-erythro-sphingosylphosphorylcholine (SM), and further phosphatidylethanolamine lipids with different hydrophobic chains. In some embodiments, the neutral lipid is selected from the group consisting of DSPC, DOPC, DMPC, DPPC, POPC, DOPE, DOPG, DPPG, POPE, DPPE, DMPE, DSPE, and SM. In some embodiments, the neutral lipid is selected from the group consisting of DSPC, DPPC, DMPC, DOPC, POPC, DOPE and SM. In some embodiments, the neutral lipid is DSPC. In some embodiments, the neutral lipid is DOPE.

In some embodiments, the additional lipid comprises one of the following: (1) a phospholipid, (2) cholesterol or a derivative thereof; or (3) a mixture of a phospholipid and cholesterol or a derivative thereof. Examples of cholesterol derivatives include, but are not limited to, cholestanol, cholestanone, cholestenone, coprostanol, cholesteryl-2'-hydroxyethyl ether, cholesteryl-4'-hydroxybutyl ether, tocopherol and derivatives thereof, and mixtures thereof. Thus, in some embodiments, the RNA compositions/formulations described herein comprise (1) the cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)), and a phospholipid such as DSPC or DOPE, or (2) the cationically ionizable lipid of formula (I) (or of any one of the formulas (Ia), (Ib), (Ic), (Id), (Ie), (If), (Ig), (1h), (Ii), (Ij), (IIa), (IIb), (IIIa), (IIIb), (IV-1), (IV-2), and (IV-3)), a phospholipid such as DSPC or DOPE, and cholesterol.

In some embodiments, the RNA compositions/formulations described herein comprise (1) the cationically ionizable lipid of formula (IV-1) and DSPC, (2) the cationically ionizable lipid of formula (IV-1), DSPC, and cholesterol, (3) the cationically ionizable lipid of formula (IV-2) and DSPC, (4) the cationically ionizable lipid of formula (IV-2), DSPC, and cholesterol, (5) the cationically ionizable lipid of formula (IV-3) and DSPC, (6) the cationically ionizable lipid of formula (IV-3), DSPC, and cholesterol, (7) the cationically ionizable lipid of formula (IV-1) and DOPE, (8) the cationically ionizable lipid of formula (IV-1), DOPE, and cholesterol, (9) the cationically ionizable lipid of formula (IV-2) and DOPE, (10) the cationically ionizable lipid of formula (IV-2), DOPE, and cholesterol, (11) the cationically ionizable lipid of formula (IV-3) and DOPE, or (12) the cationically ionizable lipid of formula (IV-3), DOPE, and cholesterol.

DSPC is a neutral phospholipid. The structure of DSPC may be represented as follows:

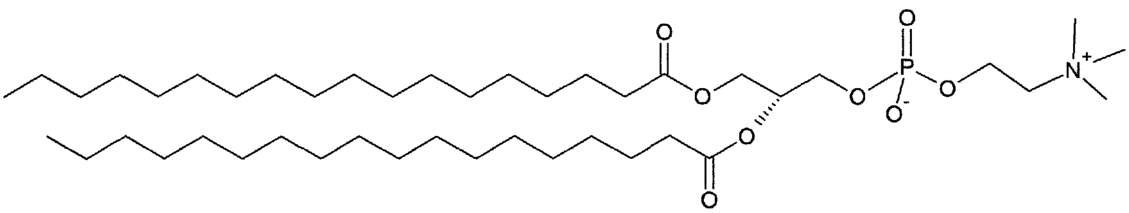

DOPE is a neutral phospholipid. The structure of DOPE may be represented as follows:

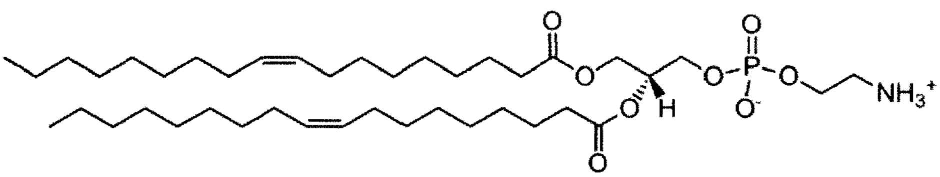

The structure of cholesterol may be represented as follows:

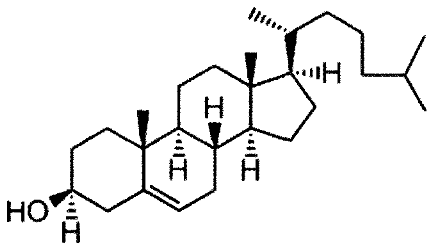

In some embodiments, the additional lipid (e.g., one or more phospholipids and/or cholesterol) may comprise from about 0 mol % to about 60 mol %, from about 2 mol % to about 55 mol %, from about 5 mol % to about 50 mol %, from about 5 mol % to about 45 mol %, from about 10 mol % to about 45 mol %, from about 15 mol % to about 40 mol %, or from about 20 mol % to about 40 mol % of the total lipid present in the RNA compositions/formulations described herein. In some embodiments, the additional lipid (e.g., one or more phospholipids and/or cholesterol) comprises about 15 mol %, about 20 mol %, about 25 mol %, about 30 mol %, or about 35 mol % of the total lipid present in the RNA compositions/formulations described herein.

In some embodiments, the additional lipid comprises a mixture of: (i) a phospholipid such as DSPC or DOPE; and (ii) cholesterol or a derivative thereof. In some embodiments, the molar ratio of the phospholipid, such as DSPC or DOPE, to the cholesterol or a derivative thereof is from about 9:0 to about 1:10, about 2:1 to about 1:4, about 1:1 to about 1:4, or about 1:1 to about 1:3.

In some embodiments, the phospholipid may comprise from about 5 mol % to about 40 mol %, preferably from about 5 mol % to about 20 mol %, more preferably from about 5 mol % to about 15 mol % of the total lipid present in the RNA compositions/formulations described herein.

In some embodiments, the steroid (in particular, cholesterol) comprises from about 10 mol % to about 65 mol %, preferably from about 20 mol % to about 60 mol %, more preferably from about 30 mol % to about 50 mol % or from about 25 mol % to about 35 mol % of the total lipid present in the formulation/composition.

In some embodiments, where at least a portion of (i) the RNA, (ii), the cationically ionizable lipid of formula (I), and (iii) the additional lipid form particles (e.g., nanoparticles, such as LNPs), the additional lipid (e.g., one or more phospholipids and/or cholesterol) may comprise from about 0 mol % to about 60 mol %, from about 2 mol % to about 55 mol %, from about 5 mol % to about 50 mol %, from about 5 mol % to about 45 mol %, from about 10 mol % to about 45 mol %, from about 15 mol % to about 40 mol %, or from about 20 mol % to about 40 mol % of the total lipid present in the particles.

In some embodiments, the phospholipid may comprise from about 5 mol % to about 40 mol %, preferably from about 5 mol % to about 20 mol %, more preferably from about 5 mol % to about 15 mol % of the total lipid present in the particles.

In some embodiments, the steroid (in particular, cholesterol) comprises from about 10 mol % to about 65 mol %, preferably from about 20 mol % to about 60 mol %, more preferably from about 30 mol % to about 50 mol % or from about 25 mol % to about 35 mol % of the total lipid present in the particles.

Polymer-conjugated lipids

The RNA compositions/formulations described herein comprise at least one polymer-conjugated lipid. A polymer-conjugated lipid is typically a molecule comprising a lipid portion and a polymer portion conjugated thereto. In some embodiments, a polymer-conjugated lipid is a PEG-conjugated lipid, also referred to herein as pegylated lipid or PEG-lipid. The term "pegylated lipid" refers to a molecule comprising both a lipid portion and a polyethylene glycol portion. Pegylated lipids are known in the art. In some embodiments, a polymer-conjugated lipid is a polysarcosine-conjugated lipid, also referred to herein as sarcosinylated lipid or pSar-lipid. The term "sarcosinylated lipid" refers to a molecule comprising both a lipid portion and a polysarcosine portion.

According to the invention, the composition comprises at least one polysarcosine-conjugated lipid.

A "polymer," as used herein, is given its ordinary meaning, i.e., a molecular structure comprising one or more repeat units (monomers), connected by covalent bonds. The repeat units can all be identical, or in some cases, there can be more than one type of repeat unit present within the polymer. In some cases, the polymer is biologically derived, i.e., a biopolymer such as a protein. In some cases, additional moieties can also be present in the polymer, for example targeting moieties. If more than one type of repeat unit is present within the polymer, then the polymer is said to be a "copolymer." The repeat units forming the copolymer can be arranged in any fashion. For example, the repeat units can be arranged in a random order, in an alternating order, or as a "block" copolymer, i.e., comprising one or more regions each comprising a first repeat unit (e.g., a first block), and one or more regions each comprising a second repeat unit (e.g., a second block), etc. Block copolymers can have two (a diblock copolymer), three (a triblock copolymer), or more numbers of distinct blocks.

In some embodiments, a polymer-conjugated lipid is designed to sterically stabilize a lipid particle by forming a protective hydrophilic layer that shields the hydrophobic lipid layer. In some embodiments, a polymer-conjugated lipid can reduce its association with serum proteins and/or the resulting uptake by the reticuloendothelial system when such lipid particles are administered *in vivo.*

In some embodiments, the RNA compositions/formulations described herein do not include a pegylated lipid.

**Polyethyleneglycol (PEG)-conjugated lipids**

In some embodiments, RNA compositions/formulations described herein comprise a cationically ionizable lipid of formula (I) (or of any one of the formulas (Ia), (Ib), (Ic), (Id), (Ie), (If), (Ig), (1h), (Ii), (Ij), (IIa), (IIb), (IIIa), (IIIb), (IV-1), (IV-2), and (IV-3)) and a PEG-conjugated lipid. In some embodiments, RNA compositions/formulations described herein may further comprise a neutral lipid, e.g., a phospholipid, cholesterol or a derivative thereof, or a combination of a neutral lipid, e.g., a phospholipid, and cholesterol or a derivative thereof. In some embodiments, RNA compositions/formulations described herein comprise a cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)), a PEG-conjugated lipid, a neutral lipid, e.g., a phospholipid, and cholesterol or a derivative thereof. In some embodiments, the phospholipid is DSPC. In some embodiments, the phospholipid is DOPE. In some embodiments, RNA compositions/formulations described herein comprise a cationically ionizable lipid of formula (I) (or of any one of the formulas (la), (Ib), (Ic), (Id), (Ie), (If), (Ig), (Ih), (Ii), (II), (IIa), (IIb), (IIIa), (IIIb), (IV-1), (IV-2), and (IV-3)), a PEG-conjugated lipid, DOPE, and cholesterol or a derivative thereof.

In some embodiments, RNA compositions/formulations described herein comprise a cationically ionizable lipid of formula (IV-3), a PEG-conjugated lipid, DSPC, and cholesterol. In some embodiments, RNA compositions/formulations described herein comprise a cationically ionizable lipid of formula (IV-3), a PEG-conjugated lipid, DOPE, and cholesterol. In some embodiments, RNA compositions/formulations described herein comprise a cationically ionizable lipid of formula (IV-2), a PEG-conjugated lipid, DSPC, and cholesterol. In some embodiments, RNA compositions/formulations described herein comprise a cationically ionizable lipid of formula (IV-2), a PEG-conjugated lipid, DOPE, and cholesterol.

In some embodiments, the PEG-conjugated lipid (pegylated lipid) is a lipid having the structure of the following general formula (VIII):

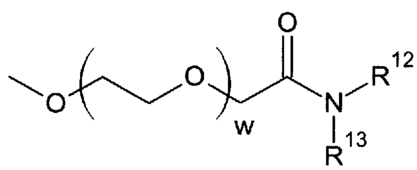

or a pharmaceutically acceptable salt, tautomer or stereoisomer thereof, wherein:

each of $R^{12}$ and $R^{13}$ is each independently a straight or branched, alkyl or alkenyl chain containing from 10 to 30 carbon atoms, wherein the alkyl/alkenyl chain is optionally interrupted by one or more ester bonds; and w has a mean value ranging from 30 to 60.

In some embodiments of formula (VIII), each of $R^{12}$ and $R^{13}$ is independently a straight alkyl chain containing from 10 to 18 carbon atoms, preferably from 12 to 16 carbon atoms.

In some embodiments of formula (VIII), $R^{12}$ and $R^{13}$ are identical. In some embodiments, each of $R^{12}$ and $R^{13}$ is a straight alkyl chain containing 12 carbon atoms. In some embodiments, each of $R^{12}$ and $R^{13}$ is a straight alkyl chain containing 14 carbon atoms. In some embodiments, each of $R^{12}$ and $R^{13}$ is a straight alkyl chain containing 16 carbon atoms.

In some embodiments of formula (VIII), $R^{12}$ and $R^{13}$ are different. In some embodiments, one of $R^{12}$ and $R^{13}$ is a straight alkyl chain containing 12 carbon atoms and the other of $R^{12}$ and $R^{13}$ is a straight alkyl chain containing 14 carbon atoms.

In some embodiments of formula (VIII), z has a mean value ranging from 40 to 50, such as a mean value of 45.

In some embodiments of formula (VIII), z is within a range such that the PEG portion of the pegylated lipid of formula (VIII) has an average molecular weight of from about 400 to about 6000 g/mol, such as from about 1000 to about 5000 g/mol, from about 1500 to about 4000 g/mol, or from about 2000 to about 3000 g/mol.

Various PEG-conjugated lipids are known in the art and include, but are not limited to pegylated diacylglycerol (PEG-DAG) such as 1-(monomethoxy-polyethyleneglycol)-2,3-dimyristoylglycerol (PEG-DMG), a pegylated phosphatidylethanoloamine (PEG-PE), a PEG succinate diacylglycerol (PEG-S-DAG) such as 4-O-(2' ,3 '-di(tetradecanoyloxy) propyl-1-O-(ω-methoxy(polyethoxy)ethyl)butanedioate (PEG-S-DMG), a pegylated ceramide (PEG-cer), or a PEG dialkoxypropylcarbamate such as ω-methoxy(polyethoxy)ethyl-N-(2,3-di(tetradecanoxy)propyl)carbamate or 2,3-di(tetradecanoxy)propyl-N-(ω methoxy(polyethoxy)ethyl)carbamate, and the like.

In some embodiments, the PEG-conjugated lipid (pegylated lipid) is or comprises 2-[(polyethylene glycol)-2000]-N,N-ditetradecylacetamide. In some embodiments, the pegylated lipid has the following structure:

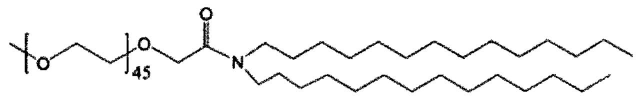

In some embodiments, the PEG-conjugated lipid (pegylated lipid) is DMG-PEG 2000, e.g., having the following structure:

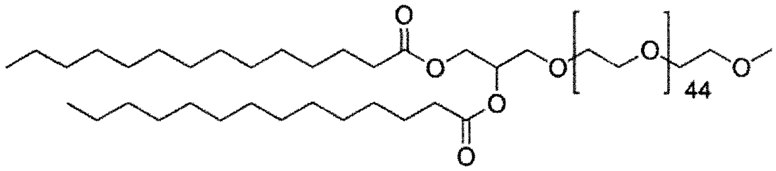

Thus, in some embodiments, RNA compositions/formulations described herein comprise a cationically ionizable lipid of formula (IV-3), DMG-PEG 2000, DSPC, and cholesterol. In some embodiments, RNA compositions/formulations described herein comprise a cationically ionizable lipid of formula (IV-3), DMG-PEG 2000, DOPE, and cholesterol. In some embodiments, RNA compositions/formulations described herein comprise a cationically ionizable lipid of formula (IV-2), DMG-PEG 2000, DSPC, and cholesterol. In some embodiments, RNA compositions/formulations described herein comprise a cationically ionizable lipid of formula (IV-2), DMG-PEG 2000, DOPE, and cholesterol.

In some embodiments, the PEG-conjugated lipid (pegylated lipid) has the following structure:

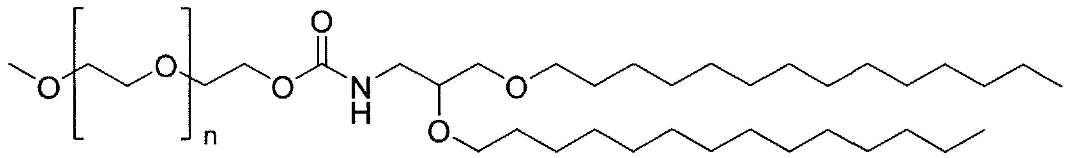

wherein n has a mean value ranging from 30 to 60, such as about 50. In one embodiment, the PEG-conjugated lipid (pegylated lipid) is $PEG_{2000}$-C-DMA which preferably refers to 3-N-[(ω-methoxy poly(ethylene glycol)2000)carbamoyl]-1,2-dimyristyloxy-propylamine (MPEG-(2 kDa)-C-DMA) or methoxy-polyethylene glycol-2,3-bis(tetradecyloxy) propylcarbamate (2000). $PEG_{2000}$-C-DMA may be selected in order to provide optimum delivery of RNA to the liver. It has been found that by modulating the alkyl chain length of the PEG lipid anchor, the pharmacology of encapsulated nucleic acid can be controlled in a predictable manner. In a vial, i.e., prior to administration, the particles retain a full complement of $PEG_{2000}$-C-DMA. In the blood compartment, i.e., after administration, $PEG_{2000}$-C-DMA dissociates from the RNA particle over time, revealing a more fusogenic particle that is more readily taken up by cells, ultimately leading to release of the RNA payload.

In some embodiments, RNA compositions/formulations described herein may comprise one or more PEG-conjugated lipids or pegylated lipids as described in WO 2017/075531 and WO 2018/081480.

In some embodiments, the pegylated lipid comprises from about 1 mol % to about 10 mol %, preferably from about 1 mol % to about 5 mol %, more preferably from about 1 mol % to about 2.5 mol % of the total lipid present in the RNA compositions/formulations described herein.

In some embodiments, where at least a portion of (i) the RNA, (ii), the cationically ionizable lipid of formula (I), and (iii) the pegylated lipid form particles (e.g., nanoparticles, such as LNPs), the pegylated lipid may comprise from about 1 mol % to about 10 mol %, preferably from about 1 mol % to about 5 mol %, more preferably from about 1 mol % to about 2.5 mol % of the total lipid present in the particles.

In some embodiments, RNA compositions/formulations described herein comprise the cationically ionizable lipid of formula (I) (in particular, of any one of the formulas (I), (Ia), (Ib), (Ic), (Id), (Ie), (If), (Ig), (Ih), (Ii), (Ij), (IIa), (IIb), (IIIa), (IIIb), wherein m is 0, (IV-1), and (IV-3)), a pegylated lipid, a phospholipid, and cholesterol, wherein the cationically ionizable lipid comprises from 40 to 50 mol percent of the total lipid present in the composition/formulation, the pegylated lipid comprises from 1 to 5 mol percent, such as from 1 to 2.5 mol percent, of the total lipid present in the composition/formulation, the phospholipid comprises from 5 to 15 mol percent of the total lipid present in the composition/formulation, and the cholesterol comprises from 30 to 50 mol percent of the total lipid present in the composition/formulation. In some embodiments, the phospholipid is DOPE. In some embodiments, the phospholipid is DSPC. In some embodiments, the

pegylated lipid is DMG-PEG 2000. In some embodiments, the cationically ionizable lipid is the lipid of formula (IV-1). In some embodiments, the cationically ionizable lipid is the lipid of formula (IV-3). In some embodiments, the cationically ionizable lipid is the lipid of formula (IV-3); the phospholipid is DSPC; and the pegylated lipid is DMG-PEG 2000. In some embodiments, the cationically ionizable lipid is the lipid of formula (IV-3); the phospholipid is DOPE; and the pegylated lipid is DMG-PEG 2000. In some embodiments, the N/P value ranges from 4 to 20, 4 to 12, 4 to 10, 4 to 8, or 5 to 7. In some embodiments, the N/P value is about 6. In some embodiments, the N/P value is about 8. In some embodiments, the N/P value is about 12.

In some embodiments, RNA compositions/formulations described herein comprise the cationically ionizable lipid of formula (I) (in particular, of any one of the formulas (I), (Ia), (Ib), (Ic), (Id), (Ie), (If), (Ig), (Ih), (Ii), (Ij), (IIa), (IIb), (IIIa), (IIIb), wherein m is 2, and (IV-2)), a pegylated lipid, a phospholipid, and cholesterol, wherein the cationically ionizable lipid comprises from 55 to 65 mol percent of the total lipid present in the composition/formulation, the pegylated lipid comprises from 1 to 5 mol percent, such as from 1 to 2.5 mol percent, of the total lipid present in the composition/formulation, the phospholipid comprises from 5 to 15 mol percent of the total lipid present in the composition/formulation, and the cholesterol comprises from 25 to 35 mol percent of the total lipid present in the composition/formulation. In some embodiments, the phospholipid is DOPE. In some embodiments, the phospholipid is DSPC. In some embodiments, the pegylated lipid is DMG-PEG 2000. In some embodiments, the cationically ionizable lipid is the lipid of formula (IV-2). In some embodiments, the cationically ionizable lipid is the lipid of formula (IV-2); the phospholipid is DSPC; and the pegylated lipid is DMG-PEG 2000. In some embodiments, the cationically ionizable lipid is the lipid of formula (IV-2); the phospholipid is DOPE; and the pegylated lipid is DMG-PEG 2000. In some embodiments, the N/P value ranges from 4 to 20, 4 to 12, 4 to 10, 4 to 8, or 5 to 7. In some embodiments, the N/P value is about 6. In some embodiments, the N/P value is about 8. In some embodiments, the N/P value is about 12.

Polysarcosin (pSar)-conjugated lipids

RNA compositions/formulations described herein comprise a cationically ionizable lipid of formula (I) (or of any one of the formulas (Ia), (Ib), (Ic), (Id), (Ie), (If), (Ig), (Ih), (Ii), (Il), (IIa), (IIb), (IIIa), (IIIb), (IV-1), (IV-2), and (IV-3)) and a pSar-conjugated lipid. In some embodiments, RNA compositions/formulations described herein may further comprise a neutral lipid, e.g., a phospholipid, cholesterol or a derivative thereof, or a combination of a neutral lipid, e.g., a phospholipid, and cholesterol or a derivative thereof. In some embodiments, RNA compositions/formulations described herein comprise a cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)), a pSar-conjugated lipid, a neutral lipid, e.g., a phospholipid, and cholesterol or a derivative thereof. In some embodiments, the phospholipid is DSPC. In some embodiments, the phospholipid is DOPE. In some embodiments, RNA compositions/formulations described herein, comprise a cationically ionizable lipid of formula (I) (or of any one of the formulas (Ia), (Ib), (Ic), (Id), (Ie), (If), (Ig), (Ih), (Ii), (Il), (IIa), (IIb), (IIIa), (IIIb), (IV-1), (IV-2), and (IV-3)), a pSar-conjugated lipid, DSPC, and cholesterol or a derivative thereof. In some embodiments, RNA compositions/formulations described herein, comprise a cationically ionizable lipid of formula (IV-3), a pSar-conjugated lipid, DSPC, and cholesterol. In some embodiments, RNA compositions/formulations described herein, comprise a cationically ionizable lipid of formula (IV-3), a pSar-conjugated lipid, DOPE, and cholesterol. In some embodiments, RNA compositions/formulations described herein, comprise a cationically ionizable lipid of formula (IV-2), a pSar-conjugated lipid, DSPC, and cholesterol. In some embodiments, RNA compositions/formulations described herein, comprise a cationically ionizable lipid of formula (IV-2), a pSar-conjugated lipid, DOPE, and cholesterol.

In some embodiments, the pSar-conjugated lipid comprises between 2 and 200 sarcosine units, such as between 5 and 100 sarcosine units, between 10 and 50 sarcosine units, between 15 and 40 sarcosine units, e.g., about 23 sarcosine units.

In some embodiments, the pSar-conjugated lipid comprises the structure of the following general formula (V):

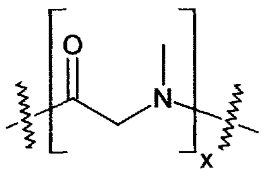

wherein x is the number of sarcosine units.

In some embodiments, the pSar-conjugated lipid comprises the structure of the following general formula (VI):

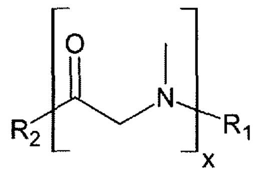

wherein one of $R_1$ and $R_2$ comprises a hydrophobic group and the other is H, a hydrophilic group or a functional group optionally comprising a targeting moiety; and x is the number of sarcosine units.

In some embodiments of formula (VI), $R_1$ is H, a hydrophilic group or a functional group optionally comprising a targeting moiety; and $R_2$ comprises one or two straight alkyl or alkenyl groups each having at least 12 carbon atoms, such as at least 14 carbon atoms. In some embodiments, each of the straight alkyl and alkenyl groups has at most 30 carbon atoms, such as at most 28, at most 26, at most 24, at most 22, at most 20, or at most 18 carbon atoms. In some embodiments, $R_2$ comprises one or two straight alkyl or alkenyl groups each having 12 to 30 carbon atoms (such as 12 to 28 carbon atoms, 12 to 26 carbon atoms, 12 to 24 carbon atoms, 12 to 22 carbon atoms, 12 to 20 carbon atoms, or 12 to 18 carbon atoms).

In some embodiments, the pSar-conjugated lipid has the structure of the following general formula (VII):

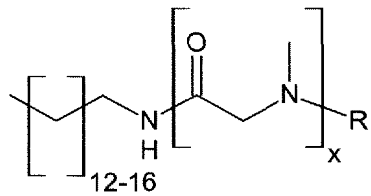

wherein R is H, a hydrophilic group or a functional group optionally comprising a targeting moiety; and x is the number of sarcosine units.

In some embodiments, the pSar-conjugated lipid has the structure of the following formula (VII-1):

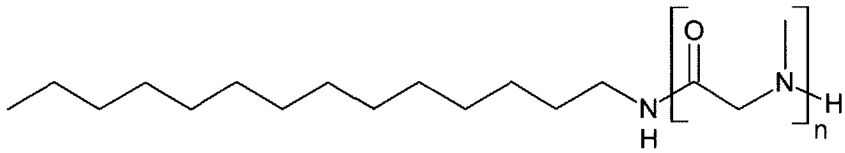,

wherein n is 23. The pSar-conjugated lipid of formula (VII-1) is also referred to herein as "C14pSar23".

In some embodiments, the pSar-conjugated lipid comprises from about 0.1 mol % to about 5 mol %, preferably from about 0.5 mol % to about 4.5 mol %, more preferably from about 1 mol % to about 4 mol %, such as from about 3 to about 4 mol%, of the total lipid present in the RNA compositions/formulations described herein.

In some embodiments, where at least a portion of (i) the RNA, (ii), the cationically ionizable lipid of formula (I), and (iii) the pSar-conjugated lipid form particles (e.g., nanoparticles, such as LNPs), the pSar-conjugated lipid may comprise from about 0.1 mol % to about 5 mol %, preferably from about 0.5 mol % to about 4.5 mol %, more preferably from about 1 mol % to about 4 mol %, such as from about 3 to about 4 mol%, of the total lipid present in the particles. In some embodiments, RNA compositions/formulations described herein comprise the cationically ionizable lipid of formula (I) (in particular, of any one of the formulas (I), (Ia), (Ib), (Ic), (Id), (Ie), (If), (Ig), (Ih), (Ii), (Ij), (IIa), (IIb), (IIIa), (IIIb), wherein m is 0, (IV-1), and (IV-3)), a pSar-conjugated lipid, a phospholipid, and cholesterol, wherein the cationically ionizable lipid comprises from 40 to 50 mol percent of the total lipid present in the composition/formulation, the pSar-conjugated lipid comprises from 1 to 4.5 mol percent, such as from 3 to 4 mol percent, of the total lipid present in the composition/formulation, the phospholipid comprises from 5 to 15 mol percent of the total lipid present in the composition/formulation, and the cholesterol comprises from 30 to 50 mol percent of the total lipid present in the composition/formulation. In some embodiments, the phospholipid is DOPE. In some embodiments, the phospholipid is DSPC. In some embodiments, the pSar-conjugated lipid is C14pSar23. In some embodiments, the cationically ionizable lipid is the lipid of formula (IV-1). In some embodiments, the cationically ionizable lipid is the lipid of formula (IV-3). In some embodiments, the cationically ionizable lipid is the lipid of formula (IV-3); the pSar-conjugated lipid is C14pSar23; and the phospholipid is DSPC. In some embodiments, the cationically ionizable lipid is the lipid of formula (IV-3) and comprises from 40 to 50 mol percent of the total lipid present in the composition/formulation; the pSar-conjugated lipid is C14pSar23 and comprises from 3 to 4 mol percent of the total lipid present in the composition/formulation; the phospholipid is DSPC and comprises from 5 to 15 mol percent of the total lipid present in the composition/formulation; and cholesterol comprises from 35 to 45 mol percent of the

total lipid present in the composition/formulation. In some embodiments, the cationically ionizable lipid is the lipid of formula (IV-3); the pSar-conjugated lipid is C14pSar23; and the phospholipid is DOPE. In some embodiments, the cationically ionizable lipid is the lipid of formula (IV-3) and comprises from 40 to 50 mol percent of the total lipid present in the composition/formulation; the pSar-conjugated lipid is C14pSar23 and comprises from 3 to 4 mol percent of the total lipid present in the composition/formulation; the phospholipid is DOPE and comprises from 5 to 15 mol percent of the total lipid present in the composition/formulation; and cholesterol comprises from 35 to 45 mol percent of the total lipid present in the composition/formulation. In some of the above embodiments, the N/P value in the composition/formulation is at least about 4. For example, the N/P value may range from 4 to 20, 4 to 12, 4 to 10, 4 to 8, or 5 to 7. In some of the above embodiments, the N/P value is about 6. In some of the above embodiments, the N/P value is about 8. In some of the above embodiments, the N/P value is about 12.

In some embodiments, RNA compositions/formulations described herein comprise the cationically ionizable lipid of formula (I) (in particular, of any one of the formulas (I), (Ia), (Ib), (Ic), (Id), (Ie), (If), (Ig), (Ih), (Ii), (Ij), (IIa), (IIb), (IIIa), (IIIb), wherein m is 2, and (IV-2)), a pSar-conjugated lipid, a phospholipid, and cholesterol, wherein the cationically ionizable lipid comprises from 55 to 65 mol percent of the total lipid present in the composition/formulation, the pSar-conjugated lipid comprises from 1 to 4.5 mol percent, such as from 3 to 4 mol percent, of the total lipid present in the composition/formulation, the phospholipid comprises from 5 to 15 mol percent of the total lipid present in the composition/formulation, and the cholesterol comprises from 25 to 35 mol percent of the total lipid present in the composition/formulation. In some embodiments, the phospholipid is DOPE. In some embodiments, the phospholipid is DSPC. In some embodiments, the pSar-conjugated lipid is C14pSar23. In some embodiments, the cationically ionizable lipid is the lipid of formula (IV-2). In some embodiments, the cationically ionizable lipid is the lipid of formula (IV-2); the pSar-conjugated lipid is C14pSar23; and the phospholipid is DSPC. In some embodiments, the cationically ionizable lipid is the lipid of formula (IV-2) and comprises from 55 to 65 mol percent of the total lipid present in the composition/formulation; the pSar-conjugated lipid is C14pSar23 and comprises from 3 to 4 mol percent of the total lipid present in the composition/formulation; the phospholipid is DSPC and comprises from 5 to 15 mol percent of the total lipid present in the composition/formulation; and cholesterol comprises from 25 to 35 mol percent of the total lipid present in the composition/formulation. In some embodiments, the cationically ionizable lipid is the lipid of formula (IV-2); the pSar-conjugated lipid is C14pSar23; and the phospholipid is DOPE. In some embodiments, the cationically ionizable lipid is the lipid of formula (IV-2) and comprises from 55 to 65 mol percent of the total lipid present in the composition/formulation; the pSar-conjugated lipid is C14pSar23 and comprises from 3 to 4 mol percent of the total lipid present in the composition/formulation; the phospholipid is DOPE and comprises from 5 to 15 mol percent of the total lipid present in the composition/formulation; and cholesterol comprises from 25 to 35 mol percent of the total lipid present in the composition/formulation. In some of the above embodiments, the N/P value in the composition/formulation is at least about 4. For example, the N/P value may range from 4 to 20, 4 to 12, 4 to 10, 4 to 8, or 5 to 7. In some of the above embodiments, the N/P value is about 6. In some of the above embodiments, the N/P value is about 8. In some of the above embodiments, the N/P value is about 12.

Embodiments of Lipoplex Particles

In some embodiments of the present disclosure, at least a portion of (i) the RNA described herein, (ii) the cationically ionizable lipid described herein (i.e., a cationically ionizable lipid of formula (I) or of any one of the formulas (Ia), (Ib), (Ic), (Id), (Ie), (If), (Ig), (Ih), (Ii), (Il), (IIa), (IIb), (IIIa), (IIIb), (IV-1), (IV-2), and (IV-3)), and, if present, (iii) the one or more additional lipids described herein may be present in RNA lipoplex particles.

Lipoplexes (LPX) are electrostatic complexes which are generally formed by mixing preformed cationic lipid liposomes with anionic RNA. Formed lipoplexes possess distinct internal arrangements of molecules that arise due to the transformation from liposomal structure into compact RNA-lipoplexes.

In certain embodiments, the RNA lipoplex particles include at least a portion of (i) RNA, (ii) the cationically ionizable lipid of formula (I) (or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)) and (iii) the one or more additional (non-cationic) lipids. In some embodiments, the cationically ionizable lipid is the lipid of any one of the formulas (IV-1), (IV-2), and (IV-3) and the additional (non-cationic) lipid is DOPE. In some embodiments, the cationically ionizable lipid is the lipid of any one of the formulas (IV-1), (IV-2), and (IV-3) and the additional (non-cationic) lipid is DSPC.

In some embodiments, the molar ratio of the cationically ionizable lipid of formula (I) to the one or more additional (non-cationic) lipids is from about 10:0 to about 1:9, about 4:1 to about 1:2, or about 3:1 to about 1:1. In some embodiments, the molar ratio may be about 3:1, about 2.75:1, about 2.5:1, about 2.25:1, about 2:1, about 1.75:1, about 1.5:1, about 1.25:1, or about 1:1. In some embodiments, the molar ratio of the at least one cationically ionizable lipid of formula (I) to the one or more additional (non-cationic) lipids is about 2:1.

RNA lipoplex particles described herein have an average diameter that in some embodiments ranges from about 200 nm to about 1000 nm, from about 200 nm to about 800 nm, from about 250 to about 700 nm, from about 400 to about 600 nm, from about 300 nm to about 500 nm, or from about 350 nm to about 400 nm. In specific embodiments, the RNA

lipoplex particles have an average diameter of about 200 nm, about 225 nm, about 250 nm, about 275 nm, about 300 nm, about 325 nm, about 350 nm, about 375 nm, about 400 nm, about 425 nm, about 450 nm, about 475 nm, about 500 nm, about 525 nm, about 550 nm, about 575 nm, about 600 nm, about 625 nm, about 650 nm, about 675 nm, about 700 nm, about 725 nm, about 750 nm, about 775 nm, about 800 nm, about 825 nm, about 850 nm, about 875 nm, about 900 nm, about 925 nm, about 950 nm, about 975 nm, or about 1000 nm. In some embodiments, the RNA lipoplex particles have an average diameter that ranges from about 250 nm to about 700 nm. In some embodiments, the RNA lipoplex particles have an average diameter that ranges from about 300 nm to about 500 nm. In an exemplary embodiment, the RNA lipoplex particles have an average diameter of about 400 nm.

The RNA lipoplex particles and RNA compositions/formulations comprising RNA lipoplex particles described herein are useful for delivery of RNA to a target tissue after parenteral administration, in particular after intravenous administration.

Spleen targeting RNA lipoplex particles are described in WO 2013/143683.

It has been found that RNA lipoplex particles having a net negative charge may be used to preferentially target spleen tissue or spleen cells such as antigen-presenting cells, in particular dendritic cells. Accordingly, following administration of the RNA lipoplex particles, RNA accumulation and/or RNA expression in the spleen occurs. Thus, RNA lipoplex particles of the disclosure may be used for expressing RNA in the spleen. In an embodiment, after administration of the RNA lipoplex particles, no or essentially no RNA accumulation and/or RNA expression in the lung and/or liver occurs. In some embodiments, after administration of the RNA lipoplex particles, RNA accumulation and/or RNA expression in antigen presenting cells, such as professional antigen presenting cells in the spleen occurs. Thus, RNA lipoplex particles of the disclosure and RNA compositions/formulations comprising such RNA lipoplex particles may be used for targeting RNA, e.g., RNA encoding an antigen or at least one epitope, to the lymphatic system, in particular secondary lymphoid organs, more specifically spleen. Targeting the lymphatic system, in particular secondary lymphoid organs, more specifically spleen is in particular preferred if the RNA administered is RNA encoding vaccine antigen. In some embodiments, the target cell is a spleen cell. In some embodiments, the target cell is an antigen presenting cell such as a professional antigen presenting cell in the spleen. In some embodiments, the target cell is a dendritic cell in the spleen.

The electric charge of the RNA lipoplex particles of the present disclosure is the sum of the electric charges present in the cationically ionizable lipid and the electric charges present in the RNA and further ionic moieties, if present. The charge ratio is the ratio of the positive charges present in the cationically ionizable lipid to the negative charges present in the RNA. The charge ratio of the positive charges present in the cationically ionizable lipid to the negative charges present in the RNA is calculated by the following equation: charge ratio=[(cationically ionizable lipid concentration (mol)) * (the total number of positive charges in the cationically ionizable lipid)] / [(RNA concentration (mol)) * (the total number of negative charges in RNA)]. The concentration of RNA and the cationically ionizable lipid amount can be determined using routine methods by one skilled in the art.

In some embodiments, at physiological pH the charge ratio of positive charges to negative charges in the RNA lipoplex particles is from about 1.6:2 to about 1:2, or about 1.6:2 to about 1.1:2. In specific embodiments, the charge ratio of positive charges to negative charges in the RNA lipoplex particles at physiological pH is about 1.6:2.0, about 1.5:2.0, about 1.4:2.0, about 1.3:2.0, about 1.2:2.0, about 1.1:2.0, or about 1:2.0.

In some embodiments, the N/P value is at least about 4. In some embodiments, the N/P value ranges from 4 to 20, 4 to 12, 4 to 10, 4 to 8, or 5 to 7. In some embodiments, the N/P value is about 6.

Embodiments of Lipid nanoparticles (LNPs)

In some embodiments, at least a portion of (i) the RNA described herein, (ii) the cationically ionizable lipid described herein (i.e., a cationically ionizable lipid of formula (I) or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3)), and, if present, (iii) the one or more additional lipids described herein is present in the form of lipid nanoparticles (LNPs). The LNP may comprise any lipid capable of forming a particle to which the one or more nucleic acid molecules are attached, or in which the one or more nucleic acid molecules are encapsulated.

LNPs typically comprise four components: cationically ionizable lipids, neutral lipids such as phospholipids, a steroid such as cholesterol, and a polymer-conjugated lipid such as PEG-lipid or a pSar-conjugated lipid. LNPs may be prepared by mixing lipids dissolved in ethanol with nucleic acid in an aqueous buffer.

In some embodiments, in the RNA LNPs described herein the RNA is bound by ionizable lipid (in particular cationically ionizable lipid of formula (I)) that occupies the central core of the LNP. The polymer-conjugated lipid forms the surface of the LNP, along with phospholipids. In some embodiments, the surface comprises a bilayer. In some embodiments, cholesterol and the cationically ionizable lipid in charged and uncharged forms can be distributed throughout the LNP.

In some embodiments, the LNP comprises one or more cationic lipids, and one or more stabilizing lipids. Stabilizing lipids include neutral lipids and polymer-conjugated lipids.

In some embodiments, the LNP comprises a cationically ionizable lipid of formula (I) or of any one of the formulas (Ia), (Ib), (Ic), (Id), (Ie), (If), (Ig), (Ih), (Ii), (Ij), (IIa), (IIb), (IIIa), (IIIb), (IV-1), (IV-2), and (IV-3), a neutral lipid, a steroid, a polymer-conjugated lipid, and the RNA, encapsulated within or associated with the lipid nanoparticle.

In some embodiments, the LNP comprises from 40 to 55 mol percent, from 40 to 50 mol percent, from 41 to 50 mol percent, from 42 to 50 mol percent, from 43 to 50 mol percent, from 44 to 50 mol percent, from 45 to 50 mol percent, from 46 to 50 mol percent, or from 46 to 49 mol percent of the cationically ionizable lipid. In some embodiments, the LNP comprises from 55 to 65 mol percent of the cationically ionizable lipid, in particular of the cationically ionizable lipid of formula (IV-2).

In some embodiments, the N/P value is at least about 4. In some embodiments, the N/P value ranges from 4 to 20, 4 to 12, 4 to 10, 4 to 8, or 5 to 7. In some embodiments, the N/P value is about 6. In some of the above embodiments, the N/P value is about 8. In some of the above embodiments, the N/P value is about 12.

In some embodiments, the neutral lipid is present in a concentration ranging from 5 to 15 mol percent, from 7 to 13 mol percent, or from 9 to 11 mol percent.

In some embodiments, the steroid is present in a concentration ranging from 20 to 50 mol percent, from 30 to 45 mol percent or from 38 to 43 mol percent. In some embodiments, the steroid is present in a concentration ranging from 25 to 35 mol percent, in particular if the cationically ionizable lipid is the lipid of formula (IV-2).

In some embodiments, the LNP comprises from 0.1 to 10 mol percent, from 0.5 to 5 mol percent, from 1 to 4.5 mol percent, such as from 3 to 4 mol percent or from 1 to 2.5 mol percent of the polymer-conjugated lipid. In some embodiments, the LNP comprises from 1 mol % to about 10 mol %, preferably from about 1 mol % to about 5 mol %, more preferably from about 1 mol % to about 2.5 mol % of the PEG-lipid. In some embodiments, the LNP comprises from 0.1 mol % to about 5 mol %, preferably from about 0.5 mol % to about 4.5 mol %, more preferably from about 1 mol % to about 4 mol %, such as from about 3 mol % to about 4 mol %, of pSar-conjugated lipid.

In some embodiments, the LNP comprises from 40 to 50 mol percent of a cationically ionizable lipid of formula (I) (in particular, of any one of the formulas (I), (Ia), (Ib), (Ic), (Id), (Ie), (If), (Ig), (Ih), (Ii), (Ij), (IIa), (IIb), (IIIa), (IIIb), wherein m is 0, (IV-1), and (IV-3)); from 5 to 15 mol percent of a neutral lipid; from 30 to 50 mol percent of a steroid; from 1 to 5 mol percent, such as from about 3 mol % to about 4 mol %, of a polymer-conjugated lipid; and the RNA, encapsulated within or associated with the lipid nanoparticle.

In some embodiments, the LNP comprises from 55 to 65 mol percent of a cationically ionizable lipid of formula (I) (in particular, of any one of the formulas (I), (Ia), (Ib), (Ic), (Id), (Ie), (If), (Ig), (Ih), (Ij), (II), (IIa), (IIIa), wherein m is 2, and (IV-2)); from 5 to 15 mol percent of a neutral lipid; from 25 to 35 mol percent of a steroid; from 1 to 5 mol percent, such as from about 1 mol % to about 2.5 mol %, of a polymer-conjugated lipid; and the RNA, encapsulated within or associated with the lipid nanoparticle.

In some embodiments, the mol percent is determined based on total mol of lipid present in the lipid nanoparticle. In some embodiments, the mol percent is determined based on total mol of cationically ionizable lipid, neutral lipid, steroid and polymer-conjugated lipid present in the lipid nanoparticle.

In some embodiments, the neutral lipid is selected from the group consisting of DSPC, DPPC, DMPC, DOPC, POPC, DOPE, DOPG, DPPG, POPE, DPPE, DMPE, DSPE, and SM. In some embodiments, the neutral lipid is selected from the group consisting of DSPC, DPPC, DMPC, DOPC, POPC, DOPE and SM. In some embodiments, the neutral lipid is DSPC. In some embodiments, the neutral lipid is DOPE.

In some embodiments, the steroid is cholesterol.

In some embodiments, the polymer-conjugated lipid is a pegylated lipid. In some embodiments, the pegylated lipid has the general formula (VIII) shown above or a pharmaceutically acceptable salt, tautomer or stereoisomer thereof. In some embodiments of formula (VIII), $R^{12}$ and $R^{13}$ are each independently straight alkyl chains containing from 12 to 16 carbon atoms. In some embodiments of formula (VIII), w has a mean value ranging from 40 to 55. In some embodiments of formula (VIII), the average w is about 45. In some embodiments of formula (VIII), $R^{12}$ and $R^{13}$ are each independently a straight alkyl chain containing about 14 carbon atoms, and w has a mean value of about 45.

In some embodiments, the pegylated lipid is or comprises 2-[(polyethylene glycol)-2000]-N,N-ditetradecylacetamide. In some embodiments, the pegylated lipid has the following structure:

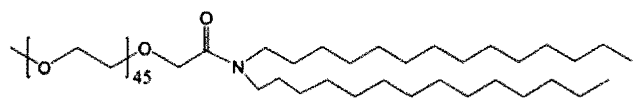

In some embodiments, the pegylated lipid is DMG-PEG 2000, e.g., having the following structure:

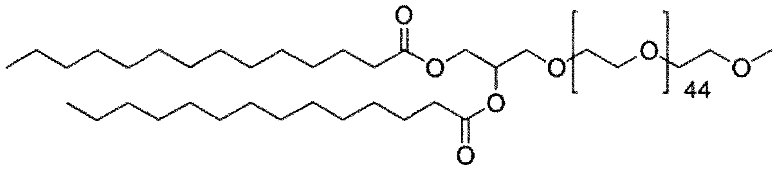

In some embodiments, the polymer conjugated lipid is a pSar-conjugated lipid, such as a pSar-conjugated lipid as specified above, e.g., a pSar-conjugated lipid comprising between 2 and 200 sarcosine units, such as between 5 and 100 sarcosine units, between 10 and 50 sarcosine units, between 15 and 40 sarcosine units, e.g., about 23 sarcosine units.

In some embodiments, the pSar-conjugated lipid comprises the structure of the general formula (V) shown above. In some embodiments, the pSar-conjugated lipid comprises the structure of the general formula (VI) shown above. In some embodiments of formula (VI), $R_1$ is H, a hydrophilic group or a functional group optionally comprising a targeting moiety; and $R_2$ comprises one or two straight alkyl or alkenyl groups each having at least 12 carbon atoms, such as at least 14 carbon atoms. In some embodiments, the pSar-conjugated lipid has the structure of the general formula (VII) shown above. In some embodiments, the pSar-conjugated lipid has the structure of the following formula (VII-1):

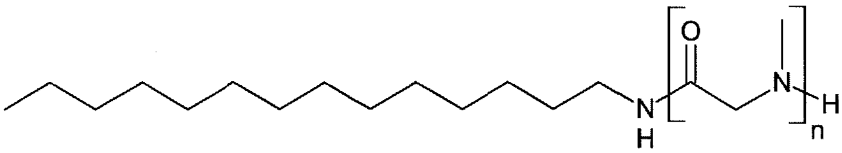,

wherein n is 23.

In some embodiments, the cationically ionizable lipid component of the LNPs has the structure of formula (I) (or of any one of the Ia), (Ib), (Ic), (Id), (Ie), (If), (Ig), (Ih), (Ii), (Ij), (IIa), (IIb), (IIIa), (IIIb), (IV-1), (IV-2), and (IV-3)) shown above. In some embodiments, the cationically ionizable lipid of formula (I) comprises one of the structures (IV-1), (IV-2), and (IV-3). In some embodiments, the cationically ionizable lipid is the lipid of formula (IV-1). In some embodiments, the cationically ionizable lipid is the lipid of formula (IV-2). In some embodiments, the cationically ionizable lipid is the lipid of formula (IV-3).

Various lipids (including, e.g., cationic lipids, neutral lipids, and polymer-conjugated lipids) are known in the art and can be used herein to form lipid nanoparticles, e.g., lipid nanoparticles targeting a specific cell type (e.g., liver cells). In some embodiments, a neutral lipid may be or comprise a phospholipid or derivative thereof (e.g., 1,2-Distearoyl-sn-glycero-3-phosphocholine (DPSC)) and/or cholesterol. In some embodiments, a polymer-conjugated lipid may be a PEG-conjugated lipid (e.g., DMG-PEG 2000 or 2-[(polyethylene glycol)-2000]-N,N-ditetradecylacetamide or a derivative thereof) or a pSar-conjugated lipid (e.g., the pSar-conjugated lipid of formula (VII-1)).

In some embodiments, the LNP comprises a cationically ionizable lipid of formula (I) (or of any one of the Ia), (Ib), (Ic), (Id), (Ie), (If), (Ig), (Ih), (Ii), (Ij), (IIa), (IIb), (IIIa), (IIIb), (IV-1), (IV-2), and (IV-3)), RNA, a neutral lipid (in particular, a phospholipid), a steroid and a pegylated lipid. In some embodiments, the neutral lipid is DOPE or DSPC. In some embodiments, the neutral lipid is DOPE. In some embodiments, the steroid is cholesterol. In some embodiments, the pegylated lipid is DMG-PEG 2000. In some embodiments, the cationically ionizable lipid is a lipid of formula (IV-3); the neutral lipid is DSPC; the pegylated lipid DMG-PEG 2000; and the steroid is cholesterol. In some embodiments, the cationically ionizable lipid is a lipid of formula (IV-3); the neutral lipid is DOPE; the pegylated lipid DMG-PEG 2000; and the steroid is cholesterol. In some embodiments, the cationically ionizable lipid is a lipid of formula (IV-2); the neutral lipid is DSPC; the pegylated lipid DMG-PEG 2000; and the steroid is cholesterol. In some embodiments, the cationically ionizable lipid is a lipid of formula (IV-2); the neutral lipid is DOPE; the pegylated lipid DMG-PEG 2000; and the steroid is cholesterol.

In some embodiments, the LNP comprises from 40 to 50 mol percent of the cationically ionizable lipid of formula (I) (in particular, the cationically ionizable lipid of formula (IV-3)); from 5 to 15 mol percent of a neutral lipid (in particular, DSPC or DOPE); from 30 to 50 mol percent of a steroid (in particular cholesterol); from 1 to 5 mol percent, such as from about 1 mol % to about 2.5 mol %, of the pegylated lipid (in particular DMG-PEG 2000); and the RNA, encapsulated within or associated with the lipid nanoparticle.

In some embodiments, the LNP comprises from 55 to 65 mol percent of the cationically ionizable lipid of formula (I) (in particular, the cationically ionizable lipid of formula (IV-2)); from 5 to 15 mol percent of a neutral lipid (in particular, DSPC or DOPE); from 25 to 35 mol percent of a steroid (in particular cholesterol); from 1 to 5 mol percent, such as from about 1 mol % to about 2.5 mol %, of the pegylated lipid (in particular DMG-PEG 2000); and the RNA, encapsulated within or associated with the lipid nanoparticle.

In some embodiments, the LNP comprises a cationically ionizable lipid of formula (I) (or of any one of the Ia), (Ib), (Ic), (Id), (Ie), (If), (Ig), (Ih), (Ii), (Ij), (IIa), (IIb), (IIIa), (IIIb), (IV-1), (IV-2), and (IV-3)), RNA, a neutral lipid (in particular, a phospholipid), a steroid and a pSar-conjugated lipid. In some embodiments, the neutral lipid is DSPC or DOPE. In some

embodiments, the neutral lipid is DSPC. In some embodiments, the steroid is cholesterol. In some embodiments, the pSar-conjugated lipid has the structure of of formula (VII-1). In some embodiments, the cationically ionizable lipid is a lipid of formula (IV-3); the neutral lipid is DSPC; the pSar-conjugated lipid has the structure of of formula (VII-1); and the steroid is cholesterol. In some embodiments, the cationically ionizable lipid is a lipid of formula (IV-3); the neutral lipid is DOPE; the pSar-conjugated lipid has the structure of of formula (VII-1); and the steroid is cholesterol. In some embodiments, the cationically ionizable lipid is a lipid of formula (IV-2); the neutral lipid is DSPC; the pSar-conjugated lipid has the structure of formula (VII-1); and the steroid is cholesterol. In some embodiments, the cationically ionizable lipid is a lipid of formula (IV-2); the neutral lipid is DOPE; the pSar-conjugated lipid has the structure of of formula (VII-1); and the steroid is cholesterol.

In some embodiments, the LNP comprises from 40 to 50 mol percent of the cationically ionizable lipid of formula (I) (in particular, the cationically ionizable lipid of formula (IV-3)); from 5 to 15 mol percent of a neutral lipid (in particular, DSPC or DOPE); from 30 to 50 mol percent of a steroid (in particular cholesterol); from 1 to 5 mol percent, such as from about 3 mol % to about 4 mol %, of the pSar-conjugated lipid (in particular the pSar-conjugated lipid of formula (VII-1)); and the RNA, encapsulated within or associated with the lipid nanoparticle. In some embodiments, the LNP comprises from 55 to 65 mol percent of the cationically ionizable lipid of formula (I) (in particular, the cationically ionizable lipid of formula (IV-2)); from 5 to 15 mol percent of a neutral lipid (in particular, DSPC or DOPE); from 25 to 35 mol percent of a steroid (in particular cholesterol); from 1 to 5 mol percent, such as from about 3 mol % to about 4 mol %, of the pSar-conjugated lipid (in particular the pSar-conjugated lipid of formula (VII-1)); and the RNA, encapsulated within or associated with the lipid nanoparticle. In some embodiments, the LNPs have a size of from about 30 nm to about 500 nm, such as from about 40 nm to about 200 nm, from about 50 nm to about 180 nm, from about 60 nm to about 160 nm, from about 80 nm to about 150 nm or from about 80 nm to about 120 nm.

**Additional treatments**

In certain embodiments, additional treatments may be administered to a patient in combination with the treatments described herein. Such additional treatments include classical cancer therapy, e.g., radiation therapy, surgery, hyperthermia therapy and/or chemotherapy. Furthermore, such additional treatments include treatments involving immune checkpoint modulators.

Chemotherapy is a type of cancer treatment that uses one or more anti-cancer drugs (chemotherapeutic agents), usually as part of a standardized chemotherapy regimen. The term chemotherapy has come to connote non-specific usage of intracellular poisons to inhibit mitosis. The connotation excludes more selective agents that block extracellular signals (signal transduction). The development of therapies with specific molecular or genetic targets, which inhibit growth-promoting signals from classic endocrine hormones (primarily estrogens for breast cancer and androgens for prostate cancer) are now called hormonal therapies. By contrast, other inhibitions of growth-signals like those associated with receptor tyrosine kinases are referred to as targeted therapy.

Importantly, the use of drugs (whether chemotherapy, hormonal therapy or targeted therapy) constitutes systemic therapy for cancer in that they are introduced into the blood stream and are therefore in principle able to address cancer at any anatomic location in the body. Systemic therapy is often used in conjunction with other modalities that constitute local therapy (i.e. treatments whose efficacy is confined to the anatomic area where they are applied) for cancer such as radiation therapy, surgery or hyperthermia therapy.

Traditional chemotherapeutic agents are cytotoxic by means of interfering with cell division (mitosis) but cancer cells vary widely in their susceptibility to these agents. To a large extent, chemotherapy can be thought of as a way to damage or stress cells, which may then lead to cell death if apoptosis is initiated.

Chemotherapeutic agents include alkylating agents, antimetabolites, anti-microtubule agents, topoisomerase inhibitors, and cytotoxic antibiotics.

Alkylating agents have the ability to alkylate many molecules, including proteins, RNA and DNA. The subtypes of alkylating agents are the nitrogen mustards, nitrosoureas, tetrazines, aziridines, cisplatins and derivatives, and non-classical alkylating agents. Nitrogen mustards include mechlorethamine, cyclophosphamide, melphalan, chlorambucil, ifosfamide and busulfan. Nitrosoureas include N-Nitroso-N-methylurea (MNU), carmustine (BCNU), lomustine (CCNU) and semustine (MeCCNU), fotemustine and streptozotocin. Tetrazines include dacarbazine, mitozolomide and temozolomide. Aziridines include thiotepa, mytomycin and diaziquone (AZQ). Cisplatin and derivatives include cisplatin, carboplatin and oxaliplatin. They impair cell function by forming covalent bonds with the amino, carboxyl, sulfhydryl, and phosphate groups in biologically important molecules. Non-classical alkylating agents include procarbazine and hexamethylmelamine. In one particularly preferred embodiment, the alkylating agent is cyclophosphamide.

Anti-metabolites are a group of molecules that impede DNA and RNA synthesis. Many of them have a similar structure to the building blocks of DNA and RNA. Anti-metabolites resemble either nucleobases or nucleosides, but have altered chemical groups. These drugs exert their effect by either blocking the enzymes required for DNA synthesis or becoming incorporated into DNA or RNA. Subtypes of the anti-metabolites are the anti-folates, fluoropyrimidines,

deoxynucleoside analogues and thiopurines. The anti-folates include methotrexate and pemetrexed. The fluoropyrimidines include fluorouracil and capecitabine. The deoxynucleoside analogues include cytarabine, gemcitabine, decitabine, azacitidine, fludarabine, nelarabine, cladribine, clofarabine, and pentostatin. The thiopurines include thioguanine and mercaptopurine.

Anti-microtubule agents block cell division by preventing microtubule function. The vinca alkaloids prevent the formation of the microtubules, whereas the taxanes prevent the microtubule disassembly. Vinca alkaloids include vinorelbine, vindesine, and vinflunine. Taxanes include docetaxel (Taxotere) and paclitaxel (Taxol).

Topoisomerase inhibitors are drugs that affect the activity of two enzymes: topoisomerase I and topoisomerase II and include irinotecan, topotecan, camptothecin, etoposide, doxorubicin, mitoxantrone, teniposide, novobiocin, merbarone, and aclarubicin.

The cytotoxic antibiotics are a varied group of drugs that have various mechanisms of action. The common theme that they share in their chemotherapy indication is that they interrupt cell division. The most important subgroup is the anthracyclines (e.g., doxorubicin, daunorubicin, epirubicin, idarubicin pirarubicin, and aclarubicin) and the bleomycins; other prominent examples include mitomycin C, mitoxantrone, and actinomycin.

In some embodiments, e.g., prior to administration of immune effector cells, a lymphodepleting treatment may be applied, e.g., by administering cyclophosphamide and fludarabine. Such treatment may increase cell persistence and the incidence and duration of clinical responses.

"Immune checkpoint" refers to regulators of the immune system, and, in particular, co-stimulatory and inhibitory signals that regulate the amplitude and quality of T cell activity. In certain embodiments, the immune checkpoint is an inhibitory signal. In certain embodiments, the inhibitory signal is the interaction between PD-1 and PD-L1 and/or PD-L2.

The "Programmed Death-1 (PD-1)" receptor refers to an immuno-inhibitory receptor belonging to the CD28 family. PD-1 is expressed predominantly on previously activated T cells *in vivo,* and binds to two ligands, PD-L1 and PD-L2. The term "PD-1" as used herein includes human PD-1 (hPD-1), variants, isoforms, and species homologs of hPD-1, and analogs having at least one common epitope with hPD-1. "Programmed Death Ligand-1 (PD-L1)" is one of two cell surface glycoprotein ligands for PD-1 (the other being PD-L2) that downregulates T cell activation and cytokine secretion upon binding to PD-1. The term "PD-L1" as used herein includes human PD-L1 (hPD-L1), variants, isoforms, and species homologs of hPD-L1, and analogs having at least one common epitope with hPD-L1. The term "PD-L2" as used herein includes human PD-L2 (hPD-L2), variants, isoforms, and species homologs of hPD-L2, and analogs having at least one common epitope with hPD-L2. The ligands of PD-1 (PD-L1 and PD-L2) are expressed on the surface of antigen-presenting cells, such as dendritic cells or macrophages, and other immune cells. Binding of PD-1 to PD-L1 or PD-L2 results in downregulation of T cell activation. Cancer cells expressing PD-L1 and/or PD-L2 are able to switch off T cells expressing PD-1 which results in suppression of the anticancer immune response. The interaction between PD-1 and its ligands results in a decrease in tumor infiltrating lymphocytes, a decrease in T cell receptor mediated proliferation, and immune evasion by the cancerous cells. Immune suppression can be reversed by inhibiting the local interaction of PD-1 with PD-L1, and the effect is additive when the interaction of PD-1 with PD-L2 is blocked as well.

Many of the immune checkpoints are regulated by interactions between specific receptor and ligand pairs, such as those described above. Thus, immune checkpoint proteins mediate immune checkpoint signaling. For example, checkpoint proteins directly or indirectly regulate T cell activation, T cell proliferation and/or T cell function. Cancer cells often exploit these checkpoint pathways to protect themselves from being attacked by the immune system. Hence, the function of checkpoint proteins, which is modulated according to the present disclosure is typically the regulation of T cell activation, T cell proliferation and/or T cell function. Immune checkpoint proteins thus regulate and maintain self-tolerance and the duration and amplitude of physiological immune responses.

As used herein, the term "immune checkpoint modulator" or "checkpoint modulator" refers to a molecule or to a compound that modulates the function of one or more checkpoint proteins. Immune checkpoint modulators are typically able to modulate self-tolerance and/or the amplitude and/or the duration of the immune response. Preferably, the immune checkpoint modulator modulates the function of one or more human checkpoint proteins and is, thus, a "human checkpoint modulator". Specifically, the human checkpoint modulator is an immune checkpoint inhibitor.

As used herein, "immune checkpoint inhibitor" or "checkpoint inhibitor" refers to a molecule that totally or partially reduces, inhibits, interferes with or negatively modulates one or more checkpoint proteins or that totally or partially reduces, inhibits, interferes with or negatively modulates expression of one or more checkpoint proteins. In certain embodiments, the immune checkpoint inhibitor binds to one or more checkpoint proteins. In certain embodiments, the immune checkpoint inhibitor binds to one or more molecules regulating checkpoint proteins.

In certain embodiments, the immune checkpoint inhibitor prevents inhibitory signals associated with the immune checkpoint. In certain embodiments, the immune checkpoint inhibitor is an antibody, or fragment thereof that disrupts inhibitory signaling associated with the immune checkpoint. In certain embodiments, the immune checkpoint inhibitor is a small molecule inhibitor that disrupts inhibitory signaling. In certain embodiments, the immune checkpoint inhibitor is a peptide-based inhibitor that disrupts inhibitory signaling.

In certain embodiments, the immune checkpoint inhibitor is an antibody, fragment thereof, or antibody mimic, that

prevents the interaction between checkpoint blocker proteins.

In some embodiments, inhibiting or blocking of inhibitory immune checkpoint signaling, as described herein, results in preventing or reversing immune-suppression and establishment or enhancement of T cell immunity. In some embodiments, inhibition of immune checkpoint signaling, as described herein, reduces or inhibits dysfunction of the immune system. In some embodiments, inhibition of immune checkpoint signaling, as described herein, renders dysfunctional immune cells less dysfunctional. In some embodiments, inhibition of immune checkpoint signaling, as described herein, renders a dysfunctional T cell less dysfunctional.

In certain embodiments, the inhibitory immunoregulator (immune checkpoint blocker) is a component of the PD-1/PD-L1 or PD-1/PD-L2 signaling pathway.

In certain embodiments, the inhibitory immunoregulator (immune checkpoint blocker) is a PD-1 axis binding antagonist.

The term "PD-1 axis binding antagonist" refers to a molecule that inhibits the interaction of a PD-1 axis binding partner with either one or more of its binding partners, so as to remove T-cell dysfunction resulting from signaling on the PD-1 signaling axis - with a result being to restore or enhance T-cell function (e.g., proliferation, cytokine production, target cell killing). As used herein, a PD-1 axis binding antagonist includes a PD-1 binding antagonist, a PD-L1 binding antagonist and a PD-L2 binding antagonist.

The term "PD-1 binding antagonist" refers to a molecule that decreases, blocks, inhibits, abrogates or interferes with signal transduction resulting from the interaction of PD-1 with one or more of its binding partners, such as PD-L1, PD-L2. In some embodiments, the PD-1 binding antagonist is a molecule that inhibits the binding of PD-1 to one or more of its binding partners. In a specific aspect, the PD-1 binding antagonist inhibits the binding of PD-1 to PD-L1 and/or PD-L2. For example, PD-1 binding antagonists include anti-PD-1 antibodies, antigen binding fragments thereof, immunoadhesins, fusion proteins, oligopeptides and other molecules that decrease, block, inhibit, abrogate or interfere with signal transduction resulting from the interaction of PD-1 with PD-L1 and/or PD-L2. In some embodiments, a PD-1 binding antagonist reduces the negative co-stimulatory signal mediated by or through cell surface proteins expressed on T lymphocytes mediated signaling through PD-1 so as render a dysfunctional T-cell less dysfunctional (e.g., enhancing effector responses to antigen recognition). In some embodiments, the PD-1 binding antagonist is an anti-PD-1 antibody.

The term "PD-L1 binding antagonist" refers to a molecule that decreases, blocks, inhibits, abrogates or interferes with signal transduction resulting from the interaction of PD-L1 with either one or more of its binding partners, such as PD-1, B7-1. In some embodiments, a PD-L1 binding antagonist is a molecule that inhibits the binding of PD-L1 to one or more of its binding partners. In a specific aspect, the PD-L1 binding antagonist inhibits binding of PD-L1 to PD-1 and/or B7-1. In some embodiments, the PD-L1 binding antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1, B7-1. In some embodiments, a PD-L1 binding antagonist reduces the negative co-stimulatory signal mediated by or through cell surface proteins expressed on T lymphocytes mediated signaling through PD-L1 so as to render a dysfunctional T-cell less dysfunctional (e.g., enhancing effector responses to antigen recognition). In some embodiments, a PD-L1 binding antagonist is an anti-PD-L1 antibody.

The term "PD-L2 binding antagonist" refers to a molecule that decreases, blocks, inhibits, abrogates or interferes with signal transduction resulting from the interaction of PD-L2 with either one or more of its binding partners, such as PD-1. In some embodiments, a PD-L2 binding antagonist is a molecule that inhibits the binding of PD-L2 to one or more of its binding partners. In a specific aspect, the PD-L2 binding antagonist inhibits binding of PD-L2 to PD-1. In some embodiments, the PD-L2 antagonists include anti-PD-L2 antibodies, antigen binding fragments thereof, immunoadhesins, fusion proteins, oligopeptides and other molecules that decrease, block, inhibit, abrogate or interfere with signal transduction resulting from the interaction of PD-L2 with either one or more of its binding partners, such as PD-1. In some embodiments, a PD-L2 binding antagonist reduces the negative co-stimulatory signal mediated by or through cell surface proteins expressed on T lymphocytes mediated signaling through PD-L2 so as render a dysfunctional T-cell less dysfunctional (e.g., enhancing effector responses to antigen recognition). In some embodiments, a PD-L2 binding antagonist is an immunoadhesin.

In some embodiments, a PD-1 axis binding antagonist includes a PD-1 binding antagonist, a PD-L1 binding antagonist and a PD-L2 binding antagonist. Alternative names for "PD-1" include CD279 and SLEB2. Alternative names for "PD-L1" include B7-H1, B7-4, CD274, and B7-H. Alternative names for "PD-L2" include B7-DC, Btdc, and CD273. In some embodiments, PD-1, PD-L1, and PD-L2 are human PD-1, PD-L1 and PD-L2.

In some embodiments, the PD-1 binding antagonist is a molecule that inhibits the binding of PD-1 to its ligand binding partner(s). In a specific aspect the PD-1 ligand binding partners are PD-L1 and/or PD-L2.

In some embodiments, the PD-L1 binding antagonist is a molecule that inhibits the binding of PD-L1 to its binding partner(s). In a specific aspect, PD-L1 binding partner(s) are PD-1 and/or B7-1.

In some embodiments, the PD-L2 binding antagonist is a molecule that inhibits the binding of PD-L2 to its binding partner(s). In a specific aspect, a PD-L2 binding partner is PD-1.

The antagonist may be an antibody, an antigen binding fragment thereof, an immunoadhesin, a fusion protein, or oligopeptide.

In some embodiments, the PD-1 binding antagonist is an anti-PD-1 antibody (e.g., a human antibody, a humanized antibody, or a chimeric antibody).

In some embodiments, the PD-L1 binding antagonist is an anti-PD-L1 antibody.

PD-1 axis binding antagonists such as anti-PD-1 antibodies and anti-PD-L1 antibodies may be administered in any manner and by any route known in the art. The mode and route of administration will depend on the type of PD-1 axis binding antagonist to be used.

PD-1 axis binding antagonists may be administered in the form of any suitable pharmaceutical composition as described herein.

PD-1 axis binding antagonists such as anti-PD-1 antibodies and anti-PD-L1 antibodies may be administered in the form of nucleic acid, such DNA or RNA, encoding a PD-1 axis binding antagonist such as anti-PD-1 antibody or anti-PD-L1 antibody. For example, antibodies can be delivered encoded in expressing nucleic acids, as described herein. Nucleic acid molecules can be delivered as such, e.g., in the form of a plasmid or mRNA molecule, or complexed with a delivery vehicle, e.g., a liposome, lipoplex or any other nucleic-acid particle such as nucleic-acid lipid particle. PD-1 axis binding antagonists such as anti-PD-1 antibodies and anti-PD-L1 antibodies may also be administered via an oncolytic virus comprising an expression cassette encoding the PD-1 axis binding antagonist.

Compositions comprising nucleic acid

A composition comprising one or more RNAs described herein, e.g., in the form of RNA particles, may comprise salts, buffers, or other components as further described below.

In some embodiments, a salt for use in the compositions described herein comprises sodium chloride. Without wishing to be bound by theory, sodium chloride functions as an ionic osmolality agent for preconditioning RNA prior to mixing with lipids. In some embodiments, the compositions described herein may comprise alternative organic or inorganic salts. Alternative salts include, without limitation, potassium chloride, dipotassium phosphate, monopotassium phosphate, potassium acetate, potassium bicarbonate, potassium sulfate, disodium phosphate, monosodium phosphate, sodium acetate, sodium bicarbonate, sodium sulfate, lithium chloride, magnesium chloride, magnesium phosphate, calcium chloride, and sodium salts of ethylenediaminetetraacetic acid (EDTA).

Generally, compositions for storing RNA particles such as for freezing RNA particles comprise low sodium chloride concentrations, or comprises a low ionic strength. In some embodiments, the sodium chloride is at a concentration from 0 mM to about 50 mM, from 0 mM to about 40 mM, or from about 10 mM to about 50 mM.

According to the present disclosure, the RNA particle compositions described herein have a pH suitable for the stability of the RNA particles and, in particular, for the stability of the RNA. Without wishing to be bound by theory, the use of a buffer system maintains the pH of the particle compositions described herein during manufacturing, storage and use of the compositions. In some embodiments of the present disclosure, the buffer system may comprise a solvent (in particular, water, such as deionized water, in particular water for injection) and a buffering substance. The buffering substance may be selected from 2-[4-(2-hydroxyethyl)piperazin-1-yl]ethanesulfonic acid (HEPES), 2-amino-2-(hydroxymethyl)propane-1,3-diol (Tris), acetate, and histidine. In some embodiments, the buffering substance is HEPES. In some embodiments, the buffering substance is Tris. Compositions (in particular, RNA compositions/formulations) described herein may also comprise a cryoprotectant and/or a surfactant as stabilizer to avoid substantial loss of the product quality and, in particular, substantial loss of RNA activity during storage, freezing, and/or lyophilization, for example to reduce or prevent aggregation, particle collapse, RNA degradation and/or other types of damage.

In some embodiments, the cryoprotectant is a carbohydrate. The term "carbohydrate", as used herein, refers to and encompasses monosaccharides, disaccharides, trisaccharides, oligosaccharides and polysaccharides.

In some embodiments, the cryoprotectant is a monosaccharide. The term "monosaccharide", as used herein refers to a single carbohydrate unit (e.g., a simple sugar) that cannot be hydrolyzed to simpler carbohydrate units. Exemplary monosaccharide cryoprotectants include glucose, fructose, galactose, xylose, ribose and the like.

In some embodiments, the cryoprotectant is a disaccharide. The term "disaccharide", as used herein refers to a compound or a chemical moiety formed by 2 monosaccharide units that are bonded together through a glycosidic linkage, for example through 1-4 linkages or 1-6 linkages. A disaccharide may be hydrolyzed into two monosaccharides. Exemplary disaccharide cryoprotectants include sucrose, trehalose, lactose, maltose and the like. In some embodiments, the cryoprotectant is sucrose.

The term "trisaccharide" means three sugars linked together to form one molecule. Examples of a trisaccharides include raffinose and melezitose.

In some embodiments, the cryoprotectant is an oligosaccharide. The term "oligosaccharide", as used herein refers to a compound or a chemical moiety formed by 3 to about 15, such as 3 to about 10 monosaccharide units that are bonded together through glycosidic linkages, for example through 1-4 linkages or 1-6 linkages, to form a linear, branched

or cyclic structure. Exemplary oligosaccharide cryoprotectants include cyclodextrins, raffinose, melezitose, maltotriose, stachyose, acarbose, and the like. An oligosaccharide can be oxidized or reduced. In an embodiment, the cryoprotectant is a cyclic oligosaccharide. The term "cyclic oligosaccharide", as used herein refers to a compound or a chemical moiety formed by 3 to about 15, such as 6, 7, 8, 9, or 10 monosaccharide units that are bonded together through glycosidic linkages, for example through 1-4 linkages or 1-6 linkages, to form a cyclic structure. Exemplary cyclic oligosaccharide cryoprotectants include cyclic oligosaccharides that are discrete compounds, such as $\alpha$ cyclodextrin, $\beta$ cyclodextrin, or $\gamma$ cyclodextrin.

Other exemplary cyclic oligosaccharide cryoprotectants include compounds which include a cyclodextrin moiety in a larger molecular structure, such as a polymer that contains a cyclic oligosaccharide moiety. A cyclic oligosaccharide can be oxidized or reduced, for example, oxidized to dicarbonyl forms. The term "cyclodextrin moiety", as used herein refers to cyclodextrin (e.g., an $\alpha$, $\beta$, or $\gamma$ cyclodextrin) radical that is incorporated into, or a part of, a larger molecular structure, such as a polymer. A cyclodextrin moiety can be bonded to one or more other moieties directly, or through an optional linker. A cyclodextrin moiety can be oxidized or reduced, for example, oxidized to dicarbonyl forms.

Carbohydrate cryoprotectants, e.g., cyclic oligosaccharide cryoprotectants, can be derivatized carbohydrates. For example, in an embodiment, the cryoprotectant is a derivatized cyclic oligosaccharide, *e.g.,* a derivatized cyclodextrin, *e.g.,* 2-hydroxypropyl-$\beta$-cyclodextrin, *e.g.,* partially etherified cyclodextrins (*e.g.,* partially etherified $\beta$ cyclodextrins).

An exemplary cryoprotectant is a polysaccharide. The term "polysaccharide", as used herein refers to a compound or a chemical moiety formed by at least 16 monosaccharide units that are bonded together through glycosidic linkages, for example through 1-4 linkages or 1-6 linkages, to form a linear, branched or cyclic structure, and includes polymers that comprise polysaccharides as part of their backbone structure. In backbones, the polysaccharide can be linear or cyclic. Exemplary polysaccharide cryoprotectants include glycogen, amylase, cellulose, dextran, maltodextrin and the like.

In some embodiments, RNA particle compositions may include sucrose. Without wishing to be bound by theory, sucrose functions to promote cryoprotection of the compositions, thereby preventing RNA (especially mRNA) particle aggregation and maintaining chemical and physical stability of the composition. In some embodiments, RNA particle compositions may include alternative cryoprotectants to sucrose. Alternative stabilizers include, without limitation, trehalose and glucose. In a specific embodiment, an alternative stabilizer to sucrose is trehalose or a mixture of sucrose and trehalose.

A preferred cryoprotectant is selected from the group consisting of sucrose, trehalose, glucose, and a combination thereof, such as a combination of sucrose and trehalose. In a preferred embodiment, the cryoprotectant is sucrose.

Some embodiments of the present disclosure contemplate the use of a chelating agent in an RNA composition described herein. Chelating agents refer to chemical compounds that are capable of forming at least two coordinate covalent bonds with a metal ion, thereby generating a stable, water-soluble complex. Without wishing to be bound by theory, chelating agents reduce the concentration of free divalent ions, which may otherwise induce accelerated RNA degradation in the present disclosure. Examples of suitable chelating agents include, without limitation, ethylenediaminetetraacetic acid (EDTA), a salt of EDTA, desferrioxamine B, deferoxamine, dithiocarb sodium, penicillamine, pentetate calcium, a sodium salt of pentetic acid, succimer, trientine, nitrilotriacetic acid, trans-diaminocyclohexanetetraacetic acid (DCTA), diethylenetriaminepentaacetic acid (DTPA), and bis(aminoethyl)glycolether-N,N,N',N'-tetraacetic acid. In some embodiments, the chelating agent is EDTA or a salt of EDTA. In some embodiments, the chelating agent is EDTA disodium dihydrate. In some embodiments, the EDTA is at a concentration from about 0.05 mM to about 5 mM, from about 0.1 mM to about 2.5 mM or from about 0.25 mM to about 1 mM. In an alternative embodiment, the RNA particle compositions described herein do not comprise a chelating agent.

## Pharmaceutical compositions

The agents described herein may be administered in pharmaceutical compositions or medicaments and may be administered in the form of any suitable pharmaceutical composition. In some embodiments, the pharmaceutical composition is for therapeutic or prophylactic treatments, e.g., for use in treating or preventing a disease involving an antigen such as a cancer disease or an infectious disease.

The term "pharmaceutical composition" relates to a composition comprising a therapeutically effective agent, preferably together with pharmaceutically acceptable carriers, diluents and/or excipients. Said pharmaceutical composition is useful for treating, preventing, or reducing the severity of a disease by administration of said pharmaceutical composition to a subject.

The pharmaceutical compositions of the present disclosure may comprise one or more adjuvants or may be administered with one or more adjuvants. The term "adjuvant" relates to a compound which prolongs, enhances or accelerates an immune response. Adjuvants comprise a heterogeneous group of compounds such as oil emulsions (e.g., Freund's adjuvants), mineral compounds (such as alum), bacterial products (such as Bordetella pertussis toxin), or

immune-stimulating complexes. Examples of adjuvants include, without limitation, LPS, GP96, CpG oligodeoxynucleotides, growth factors, and cytokines, such as monokines, lymphokines, interleukins, chemokines. The chemokines may be IL-1, IL-2, IL-3, IL-4, IL-5, IL-6, IL-7, IL-8, IL-9, IL-10, IL-12, INFa, INF-y, GM-CSF, LT-a. Further known adjuvants are aluminum hydroxide, Freund's adjuvant or oil such as Montanide® ISA51. Other suitable adjuvants for use in the present disclosure include lipopeptides, such as Pam3Cys, as well as lipophilic components, such as saponins, trehalose-6,6-dibehenate (TDB), monophosphoryl lipid-A (MPL), monomycoloyl glycerol (MMG), or glucopyranosyl lipid adjuvant (GLA).

The pharmaceutical compositions of the present disclosure may be in a storable form (e.g., in a frozen or lyophilized/freeze-dried form) or in a "ready-to-use form" (i.e., in a form which can be immediately administered to a subject, e.g., without any processing such as diluting). Thus, prior to administration of a storable form of a pharmaceutical composition, this storable form has to be processed or transferred into a ready-to-use or administrable form. *E.g.,* a frozen pharmaceutical composition has to be thawed, or a freeze-dried pharmaceutical composition has to be reconstituted, e.g. by using a suitable solvent (*e.g.,* deionized water, such as water for injection) or liquid (e.g., an aqueous solution).

The pharmaceutical compositions according to the present disclosure are generally applied in a "pharmaceutically effective amount" and in "a pharmaceutically acceptable preparation". The term "pharmaceutically acceptable" refers to the non-toxicity of a material which does not interact with the action of the active component of the pharmaceutical composition. The term "pharmaceutically effective amount" refers to the amount which achieves a desired reaction or a desired effect alone or together with further doses. In some embodiments relating to the treatment of a particular disease, the desired reaction may relate to inhibition of the course of the disease. This comprises slowing down the progress of the disease and, in some embodiments, interrupting or reversing the progress of the disease. The desired reaction in a treatment of a disease may also be delay of the onset or a prevention of the onset of said disease or said condition. An effective amount of the pharmaceutical compositions described herein will depend on the condition to be treated, the severeness of the disease, the individual parameters of the patient, including age, physiological condition, size and weight, the duration of treatment, the type of an accompanying therapy (if present), the specific route of administration and similar factors. Accordingly, the doses administered of the pharmaceutical compositions described herein may depend on various of such parameters. In the case that a reaction in a patient is insufficient with an initial dose, higher doses (or effectively higher doses achieved by a different, more localized route of administration) may be used.

The pharmaceutical compositions of the present disclosure may contain buffers, preservatives, and optionally other therapeutic agents. In some embodiments, the pharmaceutical compositions of the present disclosure comprise one or more pharmaceutically acceptable carriers, diluents and/or excipients.

Suitable preservatives for use in the pharmaceutical compositions of the present disclosure include, without limitation, benzalkonium chloride, chlorobutanol, paraben and thimerosal.

The term "excipient" as used herein refers to a substance which may be present in a pharmaceutical composition of the present disclosure but is not an active ingredient. Examples of excipients, include without limitation, carriers, binders, diluents, lubricants, thickeners, surface active agents, preservatives, stabilizers, emulsifiers, buffers, flavoring agents, or colorants

The term "diluent" relates a diluting and/or thinning agent. Moreover, the term "diluent" includes any one or more of fluid, liquid or solid suspension and/or mixing media. Examples of suitable diluents include ethanol, glycerol and water.

The term "carrier" refers to a component which may be natural, synthetic, organic, inorganic in which the active component is combined in order to facilitate, enhance or enable administration of the pharmaceutical composition. A carrier as used herein may be one or more compatible solid or liquid fillers, diluents or encapsulating substances, which are suitable for administration to subject. Suitable carriers include, without limitation, sterile water, Ringer, Ringer lactate, sterile sodium chloride solution, isotonic saline, polyalkylene glycols, hydrogenated naphthalenes and, in particular, biocompatible lactide polymers, lactide/glycolide copolymers or polyoxyethylene/polyoxy-propylene copolymers. In some embodiments, the pharmaceutical composition of the present disclosure includes isotonic saline.

Pharmaceutically acceptable carriers, excipients or diluents for therapeutic use are well known in the pharmaceutical art, and are described, for example, in Remington's Pharmaceutical Sciences, Mack Publishing Co. (A. R Gennaro edit. 1985).

Pharmaceutical carriers, excipients or diluents can be selected with regard to the intended route of administration and standard pharmaceutical practice.

**Routes of administration of pharmaceutical compositions**

In some embodiments, the pharmaceutical compositions described herein may be administered intravenously, intraarterially, subcutaneously, intradermally, dermally, intranodally, intramuscularly, intratumorally, or peritumorally. In some embodiments, the pharmaceutical compositions described herein may be administered intramuscularly. In some embodiments, the pharmaceutical composition is formulated for local administration or systemic administration. Systemic administration may include enteral administration, which involves absorption through the gastrointestinal tract, or

parenteral administration. As used herein, "parenteral administration" refers to the administration in any manner other than through the gastrointestinal tract, such as by intravenous injection. In some embodiments, the pharmaceutical compositions are formulated for systemic administration. In some embodiments, the systemic administration is by intravenous administration. In some embodiments, the pharmaceutical compositions are formulated for intrmuscular administration.

Use of compositions

Compositions described herein may be used in the therapeutic or prophylactic treatment of various diseases, in particular diseases in which provision of a peptide or polypeptide, e.g., vaccine antigen, to a subject results in a therapeutic or prophylactic effect, e.g., a disease characterized by the presence of diseased cells expressing an antigen such as cancer diseases or infectious diseases. For example, provision of an antigen or epitope which is derived from a virus may be useful in the treatment of a viral disease caused by said virus. Provision of a tumor antigen or epitope may be useful in the treatment of a cancer disease wherein cancer cells express said tumor antigen. Provision of a functional protein or enzyme may be useful in the treatment of genetic disorder characterized by a dysfunctional protein, for example in lysosomal storage diseases (*e.g.,* mucopolysaccharidoses) or factor deficiencies. Provision of a cytokine or a cytokine-fusion may be useful to modulate tumor microenvironment.

The term "disease" (also referred to as "disorder" herein) refers to an abnormal condition that affects the body of an individual. A disease is often construed as a medical condition associated with specific symptoms and signs. A disease may be caused by factors originally from an external source, such as infectious disease, or it may be caused by internal dysfunctions, such as autoimmune diseases. In humans, "disease" is often used more broadly to refer to any condition that causes pain, dysfunction, distress, social problems, or death to the individual afflicted, or similar problems for those in contact with the individual. In this broader sense, it sometimes includes injuries, disabilities, disorders, syndromes, infections, isolated symptoms, deviant behaviors, and atypical variations of structure and function, while in other contexts and for other purposes these may be considered distinguishable categories. Diseases usually affect individuals not only physically, but also emotionally, as contracting and living with many diseases can alter one's perspective on life, and one's personality.

The term "disease involving an antigen" refers to any disease which implicates an antigen, e.g. a disease which is characterized by the presence of an antigen. The disease involving an antigen can be an infectious disease, or a cancer disease or simply cancer. The antigen may be a disease-associated antigen, such as a tumor-associated antigen, a viral antigen, or a bacterial antigen. In some embodiments, a disease involving an antigen is a disease involving cells expressing an antigen, and preferably presenting the antigen on the cell surface, e.g., in the context of MHC.

The term "infectious disease" refers to any disease which can be transmitted from individual to individual or from organism to organism, and is caused by a microbial agent (e.g. common cold). Infectious diseases are known in the art and include, for example, a viral disease, a bacterial disease, or a parasitic disease, which diseases are caused by a virus, a bacterium, and a parasite, respectively. In this regard, the infectious disease can be, for example, hepatitis, sexually transmitted diseases (e.g. chlamydia or gonorrhea), tuberculosis, HIV/acquired immune deficiency syndrome (AIDS), diphtheria, hepatitis B, hepatitis C, cholera, severe acute respiratory syndrome (SARS), the bird flu, and influenza.

The terms "cancer disease" or "cancer" refer to or describe the physiological condition in an individual that is typically characterized by unregulated cell growth. Examples of cancers include, but are not limited to, carcinoma, lymphoma, blastoma, sarcoma, and leukemia. More particularly, examples of such cancers include bone cancer, blood cancer lung cancer, liver cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, prostate cancer, uterine cancer, carcinoma of the sexual and reproductive organs, Hodgkin's Disease, cancer of the esophagus, cancer of the small intestine, cancer of the endocrine system, cancer of the thyroid gland, cancer of the parathyroid gland, cancer of the adrenal gland, sarcoma of soft tissue, cancer of the bladder, cancer of the kidney, renal cell carcinoma, carcinoma of the renal pelvis, neoplasms of the central nervous system (CNS), neuroectodermal cancer, spinal axis tumors, glioma, meningioma, and pituitary adenoma. The term "cancer" according to the disclosure also comprises cancer metastases.

In the present context, the term "treatment", "treating" or "therapeutic intervention" relates to the management and care of a subject for the purpose of combating a condition such as a disease. The term is intended to include the full spectrum of treatments for a given condition from which the subject is suffering, such as administration of the therapeutically effective compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of an individual for the purpose of combating the disease, condition or disorder and includes the administration of the active compounds to prevent the onset of the symptoms or complications.

The term "therapeutic treatment" relates to any treatment which improves the health status and/or prolongs (increases) the lifespan of an individual. Said treatment may eliminate the disease in an individual, arrest or slow the

development of a disease in an individual, inhibit or slow the development of a disease in an individual, decrease the frequency or severity of symptoms in an individual, and/or decrease the recurrence in an individual who currently has or who previously has had a disease.

The terms "prophylactic treatment" or "preventive treatment" relate to any treatment that is intended to prevent a disease from occurring in an individual. The terms "prophylactic treatment" or "preventive treatment" are used herein interchangeably.

The terms "individual" and "subject" are used herein interchangeably. They refer to a human or another mammal (e.g., mouse, rat, rabbit, dog, cat, cattle, swine, sheep, horse or primate), or any other non-mammal-animal, including birds (chicken), fish or any other animal species that can be afflicted with or is susceptible to a disease (e.g., cancer, infectious diseases) but may or may not have the disease, or may have a need for prophylactic intervention such as vaccination, or may have a need for interventions such as by protein replacement. In many embodiments, the individual is a human being. Unless otherwise stated, the terms "individual" and "subject" do not denote a particular age, and thus encompass adults, elderlies, children, and newborns. In some embodiments of the present disclosure, the "individual" or "subject" is a "patient".

The term "patient" means an individual or subject for treatment, in particular a diseased individual or subject.

Nucleic acid, in particular RNA may be administered to a subject for delivering the nucleic acid to cells of the subject.

Nucleic acid, in particular RNA may be administered to a subject for delivering a therapeutic or prophylactic peptide or polypeptide (e.g., a pharmaceutically active peptide or polypeptide) to the subject, wherein the nucleic acid encodes a therapeutic or prophylactic peptide or polypeptide.

Nucleic acid, in particular RNA may be administered to a subject for treating or preventing a disease in a subject, wherein delivering the nucleic acid to cells of the subject is beneficial in treating or preventing the disease.

Nucleic acid, in particular RNA may be administered to a subject for treating or preventing a disease in a subject, wherein the nucleic acid encodes a therapeutic or prophylactic peptide or polypeptide and wherein delivering the therapeutic or prophylactic peptide or polypeptide to the subject is beneficial in treating or preventing the disease.

In some embodiments, the nucleic acid is present in a composition as described herein.

In some embodiments, the nucleic acid is administered in a pharmaceutically effective amount.

In some embodiments, the subject is a mammal. In some embodiments, the mammal is a human.

In some embodiments of the disclosure, the aim is to induce an immune response by providing a vaccine.

A person skilled in the art will know that one of the principles of immunotherapy and vaccination is based on the fact that an immunoprotective reaction to a disease is produced by immunizing a subject with an antigen or an epitope, which is immunologically relevant with respect to the disease to be treated. Accordingly, nucleic acids (in particular RNAs) described herein are applicable for inducing or enhancing an immune response. Nucleic acids described herein are thus useful in a prophylactic and/or therapeutic treatment of a disease involving an antigen or epitope.

In some embodiments of the disclosure, the aim is to provide an immune response against diseased cells expressing an antigen such as cancer cells expressing a tumor antigen, and to treat a disease such as a cancer disease involving cells expressing an antigen such as a tumor antigen.

In some embodiments of the disclosure, the aim is to treat cancer by vaccination.

In some embodiments of the disclosure, the aim is to provide an immune response against cancer cells expressing a tumor antigen and to treat a cancer disease involving cells expressing a tumor antigen.

In some embodiments of the disclosure, the aim is to provide protection against an infectious disease by vaccination.

In some embodiments of the disclosure, the aim is to provide secreted therapeutic proteins, such as antibodies, bispecific antibodies, cytokines, cytokine fusion proteins, enzymes, to a subject, in particular a subject in need thereof.

In some embodiments of the disclosure, the aim is to provide a protein replacement therapy, such as production of erythropoietin, Factor VII, Von Willebrand factor, β-galactosidase, alpha-N-acetylglucosaminidase, to a subject, in particular a subject in need thereof.

In some embodiments of the disclosure, the aim is to modulate/reprogram immune cells in the blood.

In some embodiments of the disclosure, the aim is to provide one or more cytokines or cytokine fusions which modulate tumor microenvironment to a subject, in particular a subject in need thereof.

In some embodiments of the disclosure, the aim is to provide one or more cytokines or cytokine fusions which have antitumoral activity to a subject, in particular a subject in need thereof.

Citation of documents and studies referenced herein is not intended as an admission that any of the foregoing is pertinent prior art. All statements as to the contents of these documents are based on the information available to the applicants and do not constitute any admission as to the correctness of the contents of these documents.

The description (including the following examples) is presented to enable a person of ordinary skill in the art to make and use the various embodiments. Descriptions of specific devices, techniques, and applications are provided only as examples. Various modifications to the examples described herein will be readily apparent to those of ordinary skill in

the art, and the general principles defined herein may be applied to other examples and applications without departing from the spirit and scope of the various embodiments. Thus, the various embodiments are not intended to be limited to the examples described herein and shown, but are to be accorded the scope consistent with the claims.

## Examples

### Materials and Methods

*Materials*

The PEG lipids N-palmitoyl-sphingosine-1-{succinyl[methoxy(polyethylene glycol)2000]} (C16 PEG2000-ceramide) or (2-hexyldecanoate),2-[(polyethylene glycol)-2000]-N,N-ditetradecylacetamide, the phospholipid 1,2-distearoyl-sn-glycero-3-phosphocholine (DPSC) and cholesterol (plant derived) were purchased from Avanti Polar Lipids (Alabaster, AL). DODMA was purchased by Merck. DPL14, EA-405, HY-405 and HY-501 were either synthesized by the Dan Peer Lab (University of Tel Aviv, Israel) or purchased from NucleoSyn (Olivet, France). The polysarcosine (pSar) lipids were either synthesized by the Matthias Barz group (Johannes-Gutenberg Universität Mainz) or from PTS (Valencia, Spain). The luciferase mRNA was synthesized in house using 1-methyl-pseudouridine instead of uridine, using internal protocols. Ethanol absolute was purchased by Merck Millipore. Acetic acid glacial (Reag. USP, Ph. Eur.) was purchased from AppliChem. HEPES buffer was purchased from Life Technologies. UltraPure ™ 0.5M EDTA, pH 8.0 was purchased from Invitrogen. OmniPur® Water (WFI Quality, sterile purified water) was purchased from Merck Millipore. The RNase-free TE buffer concentrate, 20X TE buffer used for the RiboGreen® RNA quantitation assays, as well as the Ribogreen dye were purchased from Invitrogen. Triton X-100 was purchased from VWR International GmbH, Darmstadt, Germany. All materials in contact with the solutions and liposome preparations were sterile. All methods including the use of kits and reagents are carried out according to the manufacturers' information unless specifically indicated.

*Lipid nanoparticles (LNPs) preparation*

Lipid nanoparticles were prepared by mixing an organic phase containing dissolved lipids with an aqueous phase containing RNA using a microfluidic device (NanoAssemblr® Benchtop, Precision NanoSystems, Vancouver, BC, Canada). The lipid mixture (13.5 mM or 17.05 mM total concentration) was composed of a cationically ionizable lipid (such as EA-405, HY-501, HY-405, DODMA, DPL14), a phospholipid (such as DOPE or DSPC), cholesterol and a polymer-conjugated lipid (such as 1,2-dimyristoyl-rac-glycero-3-methoxypolyethylene glycol-2000 (DMG-PEG 2000 also referred to as "PEG-DMG" in the following) or C14pSar23) dissolved in ethanol at molar ratios of 40-47.5:37.5-48:10:1.5-5, respectively, unless otherwise specified in the respective example. The aqueous phase composed of mRNA diluted in 100 mM citrate buffer (pH 4.0) and mixed with the ethanol phase at a volume ratio of 3:1 (aqueous: ethanol). The ratio of nitrogen present in the cationically ionizable lipid to phosphate groups in the mRNA (N/P) was set to 4 for each formulation in initial experiments (screening) and varied in optimization phase from (4, 6, 8 and 12). Obtained formulations were dialyzed against Phosphate-buffered saline (PBS, pH 7.4) (Gibco™ Fisher Scientific GmbH, Schwerte, Germany) in Slide-A-Lyzer dialysis cassettes of 10K MWCO (Thermo Fisher Scientific, Waltham, MA, USA). Formulations were concentrated using Amicon® Ultra Centrifugal Filters of 100K MWCO (Merck Chemicals GmbH/ Millipore, Darmstadt, Germany) if required, filtered through a 0.22 μm polyethersulfone (PES) filter and stored at 4°C.

Formulations were characterized in terms of size, size distribution, polydispersity, charge (zeta potential), RNA encapsulation efficiency, RNA content, RNA integrity and free RNA after preparation. Afterwards, formulations were evaluated in terms of cytotoxicity and transfection efficiency *in vitro* using different cell lines and/or *in vivo* in an animal model (mouse) via intramuscular route (IM).

*Size determination*

Particle sizes were determined by dynamic light scattering (DLS) using DynaPro Plate Reader II (Wyatt, Dernbach, Germany). Measurements were conducted in 96-well plate with LNP samples diluted to 0.005 mg/mL (RNA concentration) in 1x PBS (pH 7.4) and measured in at least duplicates at 23°C. A refractive index of 1.33, dispersant viscosity of 1.019 and a run of 10 acquisitions were used as settings for the measurement. Average size and size distribution expressed as polydispersity index (PDI) were obtained using Dynamics software 7.8.1.3 (Wyatt Technology, Santa Barbara, CA, USA).

*Zeta potential determination*

Surface charges of the particles were assessed by photon correlation spectroscopy using Nicomp® 380

DLS/ZLS System (Anasysta e. k., Mülheim, Germany). LNPs were diluted to 0.002 mg/mL (RNA concentration) using 0.1x PBS (pH 7.4) in a single use plastic cuvette. Measurements were conducted with an electric field strength of 4 V/cm during 180 sec at 23°C.

*Encapsulation efficiency and RNA content determination*

The encapsulation efficiency of mRNA in LNPs was quantified using Invitrogen™ Quant-iT RiboGreen assay (Fisher Scientific GmbH, Schwerte, Germany). Samples of 0.05 mg/mL (RNA concentration) were diluted 100-fold in 1x Tris-HCl-EDTA buffer (TE Buffer 20x, Thermo Fisher Scientific, Waltham, MA, USA) to determine RNA accessibility and/or free RNA and diluted in 1X TE buffer containing 0.5% Triton X-100 (Merck Chemicals GmbH/ Millipore, Darmstadt, Germany) to determine the total RNA in LNPs. RNA standards (0.1 to 1.5 μg/mL) were also prepared in 1x TE buffer and 0.5% Triton X-100 to account for any variation in fluorescence. Samples were loaded in black 96-well plate and RiboGreen reagent was added to all samples as well as standards. Fluorescence signal is measured after 5 min incubation in dark using microplate reader (Tecan Infinite F200PRO, Maennedorf, Switzerland) at an excitation of 485 nm and emission at 528 nm.

*Free RNA determination*

Agarose gel electrophoresis technique was performed to determine free RNA percentage in LNP formulations. The agarose gel (1%) was prepared by dissolving 1 g agarose in 100 mL of 1x Tris-acetate-EDTA buffer (ROTIPHORESE®50x TAE Buffer, Carl Roth, Karlsruhe, Germany). In addition, 1 mL of 5% sodium hypochlorite (NaOCl) and 10 μL of GelRed® Nucleic acid Stain (Biotium, Hayward, CA, USA) were added to the mixture and heated in the microwave for 1 min (3 cycles to ensure complete dissolution). Using appropriate casting tray and combs, the agarose solution is poured at room temperature and the gel allowed to solidify for approximately 20-25 min. Samples are prepared either directly (only LNPs to determine free RNA) or diluted 1:2 with 2% Triton X-100 and incubated at 40°C for 7 min (to determine total RNA), before adding the loading dye (6X DNA Gel Loading Dye, Thermo Fisher Scientific, Waltham, MA, USA). An electrophoresis chamber containing 1x TAE running buffer (pH 7.4) was used to run the gel at 80 V for 40 min, and GEL Doc EZ System was used to take gel images (Bio-Rad Laboratories GmbH, Postfach, Germany).

*Hemolysis*

A hemolysis assay was performed to study the hemolytic properties of the formulations. To this end human red blood cells (RBCs) were separated from the whole blood and washed three times with 1×PBS and diluted in 1×PBS to a 10% vol/vol RBC solution. In a 96-round bottom plate, 100 μL LNPs or LPX diluted at an equivalent concentration of 0.05 mg mRNA/mL were added to 100 μL RBC solution in PBS and incubated at 37 °C for 1 h (triplicates). After incubation, the plate was centrifuged at 23°C at 500 × g for 5 min. Then, the supernatant was transferred into a clear 96-well plate and UV absorption was read at 540 nm using Tecan Pro200 Plate Reader. Positive and negative controls were carried out with 0.2% Triton-X and buffer alone, respectively.

*Complement activation*

*In vitro* SC5b-9 and Bb levels were determined by by Microvue SC5b-9 Plus ELISA and Microvue Bb Plus ELISA kits (Quidel Co., SanDiego, CA, USA), respectively. Briefly, samples and positive (Cremophor El, Merck, Darmstadt, Germany; Cobra venom factor (CVF); Quidel Co., SanDiego, CA, USA) and negative (1x DPBS and Intralipid) controls were incubated with Normal Human Serum Complement (Innovative Research, Michigan, USA) at the ratio of 20:80 (Specimen: Serum) for 1 h at 37 °C. LNPs or LPX formulations were incubated with human serum at a final mRNA concentration of 0.01 mg/ml, the theoretical concentration (corresponding to a dose of 0.49 mg/kg). SC5b-9 and Bb EIA kits (Quidel, San Diego, USA) were used according to manufactures protocol.

*Determination of Cholesterol content by enzymatic colorimetry*

To determine the cholesterol content of the liposomal formulations, and indirectly calculate the concentration of the ionizable lipid in liposomes, an enzymatic method based on cholesterol oxidase was employed (Cholesterol E-Test Wako KIT (Wako Catalog No. 439-17501)). This fast method is based on the hydrolysis of the cholesterol esters in the liposomes by cholesterol ester hydrolase and their oxidosis by cholesterol oxidase that generates hydrogen peroxide. The formed hydrogen peroxide participates then in a quantitative oxidative condensation between DAOS reagent and 4-aminoantipyrine, resulting in a blue pigment, which can be detected by measurement of absorbance at 595 nm. Following the manufacturer's instructions, aqueous liposomal samples were dissolved to 0.6 mM total lipid concentration in water

(30-fold dilution), followed by 11-fold dilution in a surfactant solution containing an enzyme/substrate system. The mix produced a colored product in the presence of cholesterol after a 20-min incubation at 37°C. The amount of colored product was determined by absorbance at 595 nm and was directly related to the amount of cholesterol in the sample through a linear calibration curve based on a cholesterol working standard (0-18 μg/mL).

*HPLC analysis of lipids*

Agilent HPLC system (Agilent Technologies 1260 Infinity) was used for the quantification of C14pSar23 and cholesterol in the LNPs. Waters Xbridge Protein BEH C4 300A 2.5 μm 2.1 x 100 mm (Waters GmbH, Eschborn, Germany) was used for the separation of C14pSar23 and cholesterol from the other lipids. The column temperature was set at 40°C. The autosampler thermostat was set at 15°C. A gradient system consisting of eluent [A] (water + 0.1% trifluoroacetic acid), eluent [B] (acetonitrile + 0.075% trifluoroacetic acid) and eluent [C] (methanol) was used as shown in Table 2. The LNP formulations were diluted (x20) with ethanol/water (1:1) (50 μL of LNP + 950 μL of ethanol/water). The diluted samples were vortexed for 3 seconds and analyzed by HPLC. The injection was 10 μL. For simultaneous quantification of C14-pSar-23 and cholesterol, the UV absorption at 205 nm was used. Additionally, for quantification of C14-pSar-23, the UV absorption at 217.5 nm was used to eliminate the baseline drifting observed at 205 nm due to acetonitrile in the eluent system. The LOD and LOQ of the method were determined to be 0.3 μM and 1.0 μM, respectively. The calibration ranges (concentration vs peak area) of 0.0063 - 0.0190 mM of C14-pSar-23 and 0.090 - 0.269 mM cholesterol were used with linearity ($R^2$) of greater than 0.999.

**Table 2.** Gradient used for single chain C14-PSAR-23 quantification method

| Time [min] | A [%] ($H2O$ + 0.1%TFA) | B [%] (ACN + 0.075%TFA) | C [%] (MeOH) | Flow [mL/min] |
|---|---|---|---|---|
| 0.00 | 70.0 | 30.0 | 0.0 | 0.40 |
| 1.00 | 70.0 | 30.0 | 0.0 | |
| 14.00 | 20.0 | 80.0 | 0.0 | |
| 17.00 | 5.0 | 95.0 | 0.0 | |
| 19.00 | 5.0 | 95.0 | 0.0 | |
| 21.00 | 0.0 | 0.0 | 100.0 | 0.25 |
| 26.00 | 0.0 | 0.0 | 100.0 | 0.25 |
| 27.00 | 70.0 | 30.0 | 0.0 | 0.40 |
| 30.00 | 70.0 | 30.0 | 0.0 | 0.40 |

*Cells and cell lines*

HEK 293T-17 cells were seeded into 96-well plates (136101, Thermo Fisher Scientific GmbH) 24 h prior to transfection. Cells were transfected with increasing amounts of formulations (0.01, 0.025, 0.1, 0.25, 0.75 and 2 μg/well). Samples were diluted with sample-buffer to enable comparable conditions. 24h after transfection, viability and luciferase expression were determined with the use of the ONE-Glo TOX luciferase and cell viability assay kit (E7110 Promega). The assay was performed according to the manufacturers protocol. Fluorescence (Ex: 430 nm, Em: 510 nm) and luminescence were determined with the help of a tecan reader (INF-FPLEX, Tecan Trading AG). For the calculation of the cell viability, measured values for untreated cells was set to 100%. For the determination of RLU values (luciferase expression), background (culturing media) was subtracted. To guarantee plate-comparability, a benchmark (BM) was prepared on each plate.

*Animal Experiments*

Healthy female Balb/C mice were obtained from Charles River. All mice were kept following the federal and state policies on animal research at BioNTech SE. Group size was 3 per test item and 3 for negative control (PBS). The total number of test groups was 11. For biodistribution studies, lipid nanoparticles were injected intramuscular (i.m.) in a volume of 20 μL (2 μg mRNA) into the right and left hind legs. Biological activity of luciferase-encoding mRNA was evaluated by dorsal bioluminescence imaging using an IVIS spectrum imaging system (Caliper Life Sciences, Alameda, CA, USA) Bin 4 (1 min) and Bin 8 (1 min). Readout was performed after 0 h, 6 h and 24 h. Bioluminescence was presented as colour-scaled images superimposed on grayscale photos. Quantification resulted from the average radiance (quantified as photons/sec/cm2 /sr) from each pixel inside the regions of interest (ROIs)

*In vitro evaluation (cell transfection)*

Immortalized mouse myoblast cell line (C2C12), immortalized human hepatocytes carcinoma cells (HepG2) and murine macrophage-like cell line (RAW 264.7) obtained from TRON (Mainz, Germany) were cultured using Dulbecco's modified Eagle's medium (DMEM) for C2C12/RAW cells and Eagle's Minimum Essential Medium (EMEM) for HepG2 both obtained from Gibco™ Fisher Scientific GmbH (Schwerte, Germany). Media were supplemented with 10% Fetal Calf Serum (FCS, Biochrom, Berlin, Germany) and cells were incubated at 37°C and 5% $CO_2$ (HepG2 and RAW cells) and 7.5% $CO_2$ (C2C12), media were exchanged every second day. In Nunc™ MicroWell™ 96-Flat-Bottom Microplate, C2C12, HepG2 and RAW cells were platted at 5 x $10^3$, 2 x $10^4$, and 2.5 x $10^4$ cells/well respectively, and allowed for growth 24 h prior transfection. LNP formulations were added at mRNA final dose of 12.5, 25 and 50 ng (in triplicates) in a total volume per well of 100 μL fresh medium and incubated for 24 h. Cells were then subjected to Cell Titer-Glo™ assay (Promega, Madison, WI, USA) and Bright-Glo™ Luciferase assay (Promega, Madison, WI, USA) to determine cell viability and luciferase expression respectively.

*In vivo evaluation (Animal studies)*

Balb/C mice (female, 8 weeks) were purchased from Charles River (Charles River Laboratories, MA, USA). All animals were handled in accordance with federal and state policies on animal research at BioNTech SE. For Bioluminescence studies, mice (*n* = *3*) were injected intramuscularly (IM) in both hind leg quadriceps muscles with 1 μg/leg of mRNA-coding for luciferase. Bioluminescence imaging (BLI) were performed - after injecting mice with intraperitoneally with D-Luciferin Bioluminescent Substrate (IVISbrite potassium salt XenoLight, Perkin Elmer, MA, USA), and then anesthetized - using an *In Vivo* Imaging System (IVIS Spectrum imaging, Perkin Elmer, MA, USA), equipped with imaging software (LivingImage®, Perkin Elmer, MA, USA). The signals from each injection site or specific organs either *in vivo* (lung, liver, and spleen) or *ex vivo* after euthanizing animal and organ collection (heart, lungs, liver, spleen, kidneys, and lymph nodes) were quantified by regions of interest (ROIs) at 6-, 24- and 48-hours post-injection.

For immunogenicity studies, animals (n = 5) were injected IM into one hind leg with 1 μg mRNA-coding for model antigen at day 0 (prime) and boosted with the identical formulations after 3 weeks. Tail bleedings were collected prior to each vaccination and after 2 and 4 weeks after the boost injections.

Serum was harvested after blood collection/centrifugation and stored at -20°C. ELISA for serum samples and ELISpot were performed to determine antibody titers and T cell responses in total splenocytes respectively.

## Example 1

The objective of this study was to explore the capacity of pSar lipid C14pSar23 for particle engineering of LNPs comprising different ionizable lipids with otherwise identical composition, manufacturing process and further conditions. A fixed pSar fraction of 5mol% was used for all experiments. In this example EA-405, HY-405 and HY-501 were used as ionizabe lipids in the formulations. Ionizable lipid, helper lipid DSPC, cholesterol and C14pSar23 at respective molar fractions either 40:45:10:5 or 47.5:37.5:10:5 were used. Different N/P ratios, between 6 and 12, were selected for the experiments. The particles in the formulations were characterized by a size around 80 nm. Virtually no free RNA was detected in the formulations, but a certain fraction of (dye) accessible was determined. In contrary to formulations using PEG lipids as the grafted moiety, the zeta potential was slightly positive, and not negative as with PEG. All formulations showed activity in cell culture models using RNA coding for reporter genes (luciferase). On intramuscular injection *in vivo* (luciferase coding RNA), the formulations displayed high activity in the region of the injection site, but low or no activity in the liver. In that aspect, the pSar formulations differed from other formulations with PEGylated lipids, which showed such liver expression to a certain extend. With these characteristics, the described formulations appear particularly suitable for application for vaccination against infectious diseases by intramuscular injection. A summary of all the formulations used in this study is given in Table 3. For all formulations, the same RNA buffer and the same RNA construct was used resulting in a final concentration of 0.05 mg/mL. The safety of the formulations is demonstrated by results from complement activation and hemolysis assays.

**Table 3.** Composition of LNPs using different cationic lipids.

| LNP number | LNP name | Cationic lipid type | Formulation composition /mol % | N/P |
|---|---|---|---|---|
| LNP#01 | EA-405_pSar | EA-405 | EA-405/CHOL/DSPC/C14pSar23 40/45/10/5 | 8 |
| LNP#02 | EA-405-NP6_47.5% | | EA-405/CHOL/DSPC/C14pSar23 47.5/37.5/10/5 | 6 |

(continued)

| LNP number | LNP name | Cationic lipid type | Formulation composition /mol % | N/P |
|---|---|---|---|---|
| LNP#03 | HY-405_p5ar | HY-405 | HY-405/CHOL/DSPC/C14pSar23 40/45/10/5 | 12 |
| LNP#04 | HY-405-NP6_47.5% | | HY-405/CHOL/DSPC/C14pSar23 47.5/37.5/10/5 | 6 |
| LNP#05 | HY-501_pSar | HY-501 | HY-501/CHOL/DSPC/C14pSar23 40/45/10/5 | 6 |
| LNP#06 | HY-501-NP6_47.5% | | HY-501/CHOL/DSPC/C14pSar23 47.5/37.5/10/5 | 6 |

The size and size distribution of LNPs containing different cationically ionizable lipids are plotted in Figure 1A. As can be seen, independent to the type and concentration of cationically ionizable lipids as well as the N/P ratio used in the formulations, small particles with the size below 100 nm with homogeneous size distribution were observed for all the formulations. These results confirm the ability of the tested lipids in the preparation of small, stable and homogeneous LNPs.

Figure 1B shows the zeta-potential of all the LNPs of Example 1 measured in 0.1 x PBS at pH 7. The results show that slightly positive to positive charge was measured with all tested ionizable lipids in the LNPs containing C14pSar23. In that aspect, the pSar LNP are clearly different from PEGylated LNPs, which typically have a slightly negative zeta potential.

The accessibility of RNA and the amount of free RNA in the LNP formulations was measured by Ribogreen Assay and agarose gel electrophoresis methods, respectively. Figure 1C presents the RNA accessibility of the formulations. As can be seen, the accessibility of RNA in the formulations is dependent on the type and concentration of cationic lipid used in the formulation. On the other hand, the agarose gel electrophoresis images show almost no free RNA in the formulations (Figure 1D). These results from the agarose gel measurements show that virtually all of RNA in the formulations is bound to the particles, where, as derived from the accessibility measurements, different fractions of the bound RNA may be accessible to water-soluble dye molecules.

To investigate the pSar LNP potency, mRNA expression was evaluated *in vitro* using a skeletal muscle cell line (C2C12), a hepatocarcinoma cell line (HepG2) and a murine macrophage cell line (Raw cell). For this purpose, the LNP formulations comprising firefly luciferase-encoding mRNA were incubated with the cells at three dosages, 12.5, 25 and 50 ng per well and luciferase expression was assessed 24 h post-incubation. Figure 2A shows that independent of the cationic lipid concentration, the luciferase expression of formulations containing HY-501 was slightly higher than HY-405 and EA-405 containing LNPs in C2C12 cell lines. On the other hand, no significant difference was observed in the amount of luciferase expression in HepG2 cell lines for all the formulations. Interestingly, the luciferase expression of the formulations containing 40 mol% of cationic lipids was higher than the formulations containing 47.5 mol% in the raw cell line. Moreover, it is worth mentioning that the no-to-low cytotoxicity was observed for all the LNP formulations in the tested dosages (Figure 2B).

To investigate the ability of LNPs to protect the mRNA and mediate efficient and safe *in vivo* mRNA delivery, Balb/C mice were intramuscularly injected with 1 $\mu$g of luciferase-encoding mRNA formulated into LNP formulations. Whole animal and extracted organs were analyzed for bioluminescence. As shown in Figures 3A and 3B, 6h post-injection, all LNPs mediated high mRNA expression in the muscle region and low to no signal was observed in the liver region. Bioluminescence imaging of mice 24h post-injection shows still high luciferase signal in the muscle region and no luciferase signal was observed in the liver region (Figures 3A and 3B). Complement activation of the formulations were studied by *in vitro* quantification of SC5b-9 level using Microvue SC5b-9 Plus ELISA kit (Quidel Co., SanDiego, CA, USA). Figure 4A shows the terminal complement complex (SCSb-9) formation after incubation of human serum with LNPs containing different cationically ionizable lipids. The concentration of formed SC5b-9 complex corresponding to the control items are also included. As can be seen, no complement activation was observed for all the formulations. Figure 4B presents the hemolysis analysis after incubation (in neutral pH condition) of human red blood cells with LNPs containing different cationically ionizable lipids. The hemolysis analysis of control items are also included. Negligible complement activation was observed for all the formulations.

## Example 2

The objective of this study was the investigation of C14pSar23 lipid as stealth lipid for engineering mRNA nanoparticles using the LNP technology comprising HY501 as a cationically ionizable lipid. The pSar containing LNPs were prepared by mixing the RNA aqueous phase and the lipid organic phase using a microfluidic instrument. Different

mole fractions of C14pSar23 lipid (2.5-5.0 %) were used for LNP formulations. The ionizable lipid HY501, cholesterol, the helper lipid DSPC, and C14pSar23 at respective molar fractions 47.5:(42.5 - x):10:x were used, where x is the fraction of the pSar lipid. N/P ratio of 6 was selected for the experiments.

It was found that relatively low pSar fractions are best suitable to obtain particles with good biological activity on intramuscular injection for application for vaccination against infectious diseases. All particles have a slightly positive zeta potential. pSar fractions of 4 mol% and below showed best biological activity. Interestingly, particles with a size as big as 120 nm (obtained at 3 mol% of pSar) showed very good signals in the potency assay, although frequently smaller particles, in the size range of 80 nm are considered ideal for such application. For the current combination of pSar23 and HY501, particles with a pSar fraction of 3.5 to 4 mol% and below, and a particle size of 120 nm and below are considered best for application for vaccination through the i.m. injection route.

A summary of all the formulations used in this study is given in Table 4. For all formulations, the same RNA buffer and the same RNA construct was used resulting in a final concentration of 0.05 mg/mL.

**Table 4.** HY501-containing LNPs tested in Example 2.

| LNP number | Formulation composition /mol % | N/P |
|---|---|---|
| LNP#01 | HY501/CHOL/DSPC/C14pSar23<br>47.5/40/10/2.5 | 6 |
| LNP#02 | HY501/CHOL/DSPC/C14pSar23<br>47.5/39.5/10/3 | 6 |
| LNP#03 | HY501/CHOL/DSPC/C14pSar23<br>47.5/39/10/3.5 | 6 |
| LNP#04 | HY501/CHOL/DSPC/C14pSar23<br>47.5/38.5/10/4 | 6 |
| LNP#05 | HY501/CHOL/DSPC/C14pSar23<br>47.5/38/10/4.5 | 6 |
| LNP#06 | HY501/CHOL/DSPC/C14pSar23<br>47.5/37.5/10/5 | 6 |

The evolution of size and size distribution of C14pSar23 containing LNPs (based on HY-501) manufactured with different concentration of C14pSar23 lipid in the formulation are shown in Figure 5A. As can be seen, the particle sizes decreased monotonously with the increased amounts of C14pSar23 lipid from 2.5 to 4 mol% in LNP compositions and then a plateau was observed for the LNPs manufactured with 4, 4.5 and 5 mol% C14pSar23 in the composition. Moreover, it was observed that small particles ($d \leq 100$ nm) was obtained for the LNP formulations containing 3.5-5 mol% of C14pSar23 in the composition. On the other hand, the PDI of the LNPs increased slightly with increasing C14pSar23 concentration and all the formulations exhibited a PDI inferior to 0.25.

Figure 5B shows the zeta-potential of the samples from Example 2 measured in 0.1 x PBS at pH 7. For all pSar fractions, the zeta-potential was in the positive range, around 10 mV. In that aspect, the pSar LNP are different from PEGylated LNPs, which typically have a slightly negative zeta potential.

The accessibility of RNA and the amount of free RNA in the LNP formulations was measured by Ribogreen Assay and Agarose gel electrophoresis methods, respectively. Figure 5C presents the RNA accessibility of the formulations. As can be seen, the accessibility of RNA in the formulations is almost independent of the concentration of C14pSar23 lipid in the formulation and high level of inaccessible RNA was measured for all formulations. On the other hand, the agarose gel electrophoresis images show almost no free RNA in the formulations (Figure 5D). These results from the agarose gel measurements show that virtually all of RNA in the formulations is bound to the particles.

Complement activation of the formulations were studied by *in vitro* quantification of SC5b-9 level using Microvue SC5b-9 Plus ELISA kit (Quidel Co., SanDiego, CA, USA). Figure 6A shows the terminal complement complex (SC5b-9) formation after incubation of human serum with LNP formulations. The concentration of formed SC5b-9 complex corresponding to the control items are also included. As can be seen no complement activation was observed for all the formulations.

Figure 6B presents the hemolysis analysis after incubation (in neutral pH condition) of the whole human blood with LNP formulations. The hemolysis analysis of control items is also included. As can be seen negligible hemolysis was observed for all the formulation.

Figure 7 represents the S1 protein expression in HEK 293T-17 cells after incubation with HY501 LNPs containing increasing mole-fractions of C14pSar23 (2.5-5.0 mol%). Mean fluorescent intensities (MFI; Figure 7A) and cell viability

(Figure 7B) were investigated by FACS (Fluorescent Activated Cell Sorting) analysis. As can be seen in Figure 7A, in terms of *in vitro* performance, the best mole fraction is around 3 mol% C14pSar23.

**Example 3**

The objective of this study was the investigation of C14pSar23 lipid as stealth moiety for engineering mRNA nanoparticles using the LNP technology comprising HY405 as an ionizable lipid. The pSar containing LNPs were prepared by mixing the RNA aqueous phase and the lipid organic phase using a microfluidic instrument. C14pSar23 contain a single aliphatic carbon chain and a secondary amine as an endgroup with an average length of 23 sarcosine repeat units. Different mole fraction of C14pSar23 lipids (1.5-5.0 %) have been used in the LNP formulations. Cationically ionizable lipid (HY405), cholesterol, the helper lipid DSPC, and C14pSar23 at respective molar fractions 47.5:(42.5 - x):10:x were used, where x is the fraction of the pSar lipid. N/P ratio of 6 was selected for the experiments. A summary of all the formulations used in this study is given in Table 5. For all formulations, the same RNA buffer and the same RNA construct was used resulting in a final concentration of 0.05 mg/mL.

**Table 5.** HY405-containing LNPs tested in example 3.

| LNP number | Formulation composition /mol % | N/P |
|---|---|---|
| LNP#01 | HY405/CHOL/DSPC/C14pSar23 47.5/41/10/1.5 | 6 |
| LNP#02 | HY405/CHOL/DSPC/C14pSar23 47.5/40.5/10/2 | 6 |
| LNP#03 | HY405/CHOL/DSPC/C14pSar23 47.5/40/10/2.5 | 6 |
| LNP#04 | HY405/CHOL/DSPC/C14pSar23 47.5/39.5/10/3 | 6 |
| LNP#05 | HY405/CHOL/DSPC/C14pSar23 47.5/39/10/3.5 | 6 |
| LNP#06 | HY405/CHOL/DSPC/C14pSar23 47.5/38.5/10/4 | 6 |
| LNP#07 | HY405/CHOL/DSPC/C14pSar23 47.5/38/10/4.5 | 6 |
| LNP#08 | HY405/CHOL/DSPC/C14pSar23 47.5/37.5/10/5 | 6 |

The evolution of size and size distribution of C14pSar23 containing LNPs (based on HY-405) manufactured with different concentration of C14pSar23 lipid in the formulation are shown in Figure 8A. As can be seen, the particle sizes decreased monotonously with the increased amounts of C14pSar23 lipid from 1.5 to 3 mol% in LNP compositions and then a plateau was observed for the LNPs manufactured with 3, 3.5, 4 and 5 mol% C14pSar23 in the composition.

Moreover, it was observed that small particles (d ≤ 100 nm) was obtained for the LNP formulations containing 2-5 mol% of C14pSar23 in the composition. On the other hand, all the formulations exhibited a PDI inferior to 0.25.

Figure 8B shows the zeta-potential of C14pSar23 containing LNPs in Example 3 measured in 0.1 x PBS at pH 7. The results show that slightly positive to positive charge was measured with all tested ionizable lipids in the LNPs containing C14pSar23. In that aspect, the pSar LNP are different from PEGylated LNPs, which typically have a slightly negative zeta potential.

The accessibility of RNA and the amount of free RNA in the LNP formulations was measured by Ribogreen Assay and agarose gel electrophoresis methods, respectively. Figure 8C presents the RNA accessibility of the formulations. As can be seen, the accessibility of RNA in the formulations is almost independent of the concentration of C14pSar23 lipid in the formulation and high level of inaccessible RNA was measured for all formulations. On the other hand, the agarose gel electrophoresis images show almost no free RNA in the formulations (Figure 8D). These results from the agarose gel measurements show that virtually all of RNA in the formulations is bound to the particles.

Complement activation of the formulations were studied by *in vitro* quantification of SCSb-9 level using Microvue SC5b-9 Plus ELISA kit (Quidel Co., SanDiego, CA, USA). Figures 9A shows the terminal complement complex (SC5b-9) formation after incubation of human serum with LNP formulations (ContL-C14pSar23_4: LNP formulation containing a

control lipid and 4 mol % of C14pSar23). The concentration of formed SC5b-9 complex corresponding to the control items are also included. As can be seen no complement activation was observed for all the formulations.

Figure 9B presents the hemolysis analysis after incubation (in neutral pH condition) of whole human blood with LNP formulations. The hemolysis analysis of control items is also included. As can be seen negligible hemolysis was observed for all the formulation.

## Example 4

The objective of this study was the *in vivo* evaluation of immunoligicity of nanoparticle formulations containing a cationically ionizable lipid. In this study the lipid nanoparticles were prepared at small scale under R&D conditions as mentioned before (see Example 3). For immunological study, 5 mice per group were immunized twice at day 0 and day 21 with 0.2 μg modified mRNA encoding the P2 S protein of the SARS-CoV-2 formulated with HY501 and HY405 as cationically ionizable lipids. PBS was used as negative control in this study. A summary of the formulations is given in Table 6. For all formulations (with the exception of the control group 1), the same RNA buffer and the same RNA construct was used resulting in a final concentration of 0.05 mg/mL.

**Table 6.** LNPs tested in example 4.

| Group | Formulation composition /mol % | N/P |
|---|---|---|
| 1 | Buffer (PBS) | -- |
| 2 | HY501/CHOL/DSPC/C14pSar23 47.5/38.5/10/4 | 6 |
| 3 | HY405/CHOL/DSPC/C14pSar23 47.5/38.5/10/4 | 6 |

The characteristic properties of the formulations including size and size distribution, pH and osmolality of the formulations used in the first and second injections are summarized in Tables 7 and 8, respectively.

**Table 7. Characterization properties of** LNPs tested in Example 4 (first injection).

| Group | Formulation composition /mol % | Size [d, nm] | PDI | pH | Osmolatity [mOsmol/kg] | Zeta (mV) |
|---|---|---|---|---|---|---|
| 1 | Buffer (PBS) | -- | --- | --- | --- | --- |
| 2 | HY501/CHOL/DSPC/C14pSar23 47.5/38.5/10/4 | 70.6 | 0.15 | 6.96 | 271 | 9.68 |
| 3 | HY405/CHOL/DSPC/C14pSar23 47.5/38.5/10/4 | 70.0 | 0.14 | 7.03 | 273 | 13.44 |

**Table 8. Characterization properties of** LNPs tested in Example 4 (second injection).

| Group | Formulation composition /mol % | Size [d, nm] | PDI | pH | Osmolality [mOsmol/kg] | Zeta (mV) |
|---|---|---|---|---|---|---|
| 1 | Buffer (PBS) | -- | --- | --- | --- | --- |
| 2 | HY501/CHOL/DSPC/C14pSar23 47.5/38.5/10/4 | 73.8 | 0.10 | 7.0 | 307 | 11.60 |
| 3 | HY405/CHOL/DSPC/C14pSar23 47.5/38.5/10/4 | 73.0 | 0.18 | 7.0 | 305 | 14.21 |

The accessibility of RNA and the amount of free RNA in the LNP formulations was measured by Ribogreen Assay and agarose gel electrophoresis methods, respectively. Figures 10 and 11 presents the RNA accessibility and agarose gel electrophoresis images of the formulations injected for the first and second immunizations, respectively. As can be seen, the accessibility of RNA in the formulations is almost independent of the cationic lipid in the formulation and high level of inaccessible RNA was measured for all formulations. On the other hand, the agarose gel electrophoresis images show almost no free RNA in the formulations. These results from the agarose gel measurements show that virtually all of RNA in the formulations is bound to the particles.

Figure 12 represents the S1 protein expression in HEK 293T-17 cells after incubation with LNP formulations used

in the first injection (ContL: control lipid). Mean fluorescent intensities (MFI) and cell viability were investigated by FACS (Fluorescent Activated Cell Sorting) analysis (UT: untreated). The *in vitro* performance of the HY501-pSar formulation was higher than the HY405-pSar formulations. On the other hand, high level of cell viability (>90%) was obtained for both formulations.

Figure 13 represents the S1 protein expression in HEK 293T-17 cells after incubation with LNP formulations used in the second injection. Mean fluorescent intensities (MFI) and cell viability were investigated by FACS (Fluorescent Activated Cell Sorting) analysis. As can be seen, the *in vitro* performance of the HY501-pSar formulation was higher than the HY405-pSar formulations. On the other hand, high level of cell viability (>80%) was obtained for both formulations.

After first immunization, animals were bled weekly and serological analysis were performed, namely ELISA (Figure 14) to detect SARS-CoV-2 S protein specific antibodies and pVNT (Figure 15) to test for virus neutralizing antibodies. At the final study 56 days after first immunization, animals were sacrificed and splenocytes were tested for IFNy-secreting after restimulation with an overlapping peptide mix specific for the S protein (Figure 16).

Both formulation candidates delivering the modRNA encoding for SARS-CoV-2 P2 S induced a high and comparable antigen-specific IgG antibody response (Figure 14). IgG antibodies were detectable as early as 7 days after first immunization increasing up to day 21. After boost, there was a strong increase of antibodies in the serum samples of immunized animals. The titers stayed high till the final day of study, day 56 after first immunization (day 28 after second immunization).

In alignment, we have tested for neutralizing antibodies, starting at day 21. After one immunization, only some animals immunized in the second group (receiving the HY501/CHOL/DSPC/C14pSar23 [47.5/38.5/10/4] formulated modRNA/ group 2) developed neutralizing antibodies. After a second immunization, all immunized developed neutralizing antibodies. In group 2, the titer of neutralizing antibody was slightly higher compared to group 3 (namely HY405/-CHOL/DSPC/C14pSar23 [47.5/38.5/10/4] formulated modRNA).

At the end of the study, splenocytes were generated and tested for IFNγ secretion after restimulation with an overlapping peptide mix (15mer peptides with an 11 amino acid overlap) of the full-length S protein or receptor-binding domain (RBD; Figure 16). IFNy secretion in this assay is induced by either CD8+ T cells or CD4+ Th1 cells that were primed and activated during the immunization phase. For both immunized groups, a high IFNy spot count against the S protein specific peptide pool was observed with no significant difference between the groups; the same was observed for RBD-peptide specific T cells.

According to the data, both test items are suitable formulation candidates for vaccine development with the HY501-containing formulation resulting in a faster functional antibody response.

## Example 5

*In vivo* potency assessment was performed after pre-selection from *in vitro* screening in different cell lines. LNPs were prepared as described in methods part with one of the ionizable lipids (EA-405, HY-501 or HY-405), cholesterol, DOPE, and PEG-DMG at molar fractions (40:48:10:2) at N/P 4 (see Figure 17). Particles of smaller size (60 nm) were observed for HY-405 and HY-501 formulations, while LNPs of approximately 90 nm were observed with EA-405. These LNPs showed a neutral surface charge, an encapsulation efficiency >80% and no free RNA. LNPs were formulated with mRNA-coding for luciferase and injected to the mice IM (1 μg/leg). Bioluminescence imaging (BLI) *in vivo* either dorsal to visualize luciferase expression at injection sites (see Figure 18A) or ventral to visualize the extended luciferase expression in liver (see Figure 18B), as well as *ex-vivo* (see Figure 18C) to enable specific organs imaging (heart, lung, liver, spleen and kidneys), was performed. The imaging of luciferase expression was conducted at 6h, 24 h and 48h (*in vivo* and *ex vivo*). As can be seen from Figure 18C, higher luciferase signal in the sites of injections and more draining to the liver compared to benchmark (BM) was observed. Quantification of the luciferase signal was performed to evaluate the potency of the formulations in comparison to the BM in muscle, liver regions (*in vivo*) and spleen and multiple organs (*ex vivo*) as shown in Figure 19. This data set of Figure 19 indicates the potency of the three formulations tested to induce high luciferase expression levels in muscle (site of injection), liver as well as in spleen compared to benchmark (BM) formulation. *Ex vivo* BLI shows that the main accumulation of the formulation after reaching the systemic circulation is the liver.

## Example 6

Optimization of the three formulations tested in Example 5 was performed initially by varying the N/P ratio of the formulations from 3 to 12. LNPs were characterized in terms of physicochemical properties including size, charge, encapsulation efficiency and free RNA (see Figure 20). Moreover, these formulations were tested *in vitro* in 3 different cell lines projecting different organs C2C12 (muscle cells), HepG2 (hepatocytes, liver cells) and RAW (macrophages, antigen presenting cells) (see Figure 21), to confirm that all the prepared formulations with different N/P ratios lead to the delivery and expression of the luciferase-encoding mRNA and enable a selection of optimal N/P ratios to be tested *in vivo* in mice.

As can be seen from Figure 20, the size of the formulations tested tends to decrease by increasing the N/P ratio for the EA-405 and HY-501 LNP formulations, while no major changes were observed with the HY-405 formulation. Similar observations can be made in terms of LNPs surface charge, wherein a switch from slightly negative to neutral or slightly positive zeta potential values for both lipids EA-405 and HY-501, and similar values for HY-405 with all the different N/P ratios were observed. RNA encapsulation efficiency determined by RiboGreen assay increased by increasing the N/P ratio for all 3 LNPs. However, more free RNA was detected with the EA-405 formulations compared to the HY-501 and HY-405 formulations. The results presented in Figure 21 indicate the efficiency of the three formulations tested to transfect a variety of cell lines using different N/P ratios (3-12 for EA-405 and HY-501 and 3-10 for HY-405), with a dose escalation profile.

Animal study was then performed with 3 different N/P ratios/formulation (4, 6 and 12). Mice were injected with 1 μg/leg luciferase-encoding mRNA via IM route, and BLI *in vivo* dorsal (Figure 22A), ventral (Figure 22B) and *ex vivo* (Figure 22C) was performed at 6h, 24h and 48h. Luciferase expression was quantified based on BLI and data were expressed as total flux (photons/sec) in Figure 23.

High local luciferase expression at injection sites of the three formulations were observed with all N/P ratios (Figure 22). According to the results presented in Figure 23, N/P ratio may be formulation dependent as well as target dependent. In muscle region, there are no major differences within the N/P range tested; though, for optimal expression at a specific organ such as liver, spleen, and lymph nodes (LNs), a specific N/P ratio should be selected. For EA-405 and HY-501 formulations, an increment of the N/P ratio above 6 led to an increase of luciferase expression in liver and spleen. While for HY-405 formulation, an N/P 6 seems to be the optimal especially for targeting LNs. Similar observations were made for HY-501 (N/P12), where 30% of the total signal was detected in lymph nodes (LNs).

No major impact of the N/P ratio in local expression was observed, while increment of the N/P ratio led to an increase of the luciferase signal in liver for the three formulations (Figure 23). However, the higher N/P ratio for the HY-501 formulation might have induced a shutdown of the expression in spleen, in contrast to the EA-405 and HY-405 formulations (N/P 12 led to highest expression of the reporter). A signal of approximately 35% and 40% in lymph nodes (LNs) from total signal intensity was detected in LNs with HY-501 (N/P 12) and HY-405 (N/P 6) respectively.

## Example 7

The potency of the formulations tested was assessed with reporter (luciferase) in previous studies; therefore, the aim of this study was to evaluate the immunogenicity of these LNP formulations with model antigen. Formulations were formulated with optimized N/P ratio: EA-405 (N/P 8), HY-501 (N/P 12) and HY-405 (N/P 6). Mice were injected with 1 μg/mouse of model antigen via IM route at day 0 as prime and then injected with the boost after 3 weeks. Mice were monitored over time and blood collection for serum analysis before injection, at day 14, day 28 and day 42. Splenocytes were collected at day 42 and harvested to perform ELISpot analysis. Antibody levels determined by ELISA at day 14 showed higher titers compared to BM, and similar observations were made at day 28 and day 42 (Figure 24a-c), indicating the potency of the formulations. However, T cell responses determined by ELISpot-IFN-y of HY-501 and HY-405 formulations were lower than BM, while EA-405 showed T cell responses equivalent to BM (Figure 24d), indicating that these formulations may require further optimization to improve the T cell responses.

## Example 8

The testing of LNP formulations (EA-405, HY-501 and HY-405) with model antigen showed a significant superiority to the BM in inducing antibodies; however, the T cell responses may require further optimization (see Example 7 and Figure 24). To this end, LNP formulations were formulated with EA-405 using different molar fractions of the cationically ionizable lipid (50% and 60%) vs. the standard molar ratio 40% (40-60:48-28:10:2/cationically ionizable lipid: cholesterol: DOPE: PEG-DMG). EA-405 formulations were used with N/P 8 and total lipid of 13.5 mM. In this study, LNP formulations containing ≥50% EA-405 had the following properties: a size of approximately 100 nm (PDI: ~0.2) and ≥90% encapsulation efficiency (neutral charge and no free RNA) (Figure 25). These LNP formulations showed high luciferase expression in muscle (Figure 26A) and lower drainage to the liver (Figure 26B). All formulations tested gave higher luciferase signal at the sites of injections and improved T cell responses (Figure 27) compared to standard formulations containing 40% of EA-405. The results presented in Figure 27 indicate no significant differences between 40% and 50% expression at site of injections, while 60% EA-405 containing LNP formulations showed a slight decrease after 24 h compared to 6 h. In terms of liver signal, LNP formulations containing ≥ 50% EA-405 led to lower signal in the liver compared to the standard molar ratio (40%). However, increasing the molar ratio of EA-405 induced higher T cell responses compared to 40% (3 times higher spots), indicating a successful improvement of the formulation to generate - besides higher antibody titers - optimal T cell responses.

## Claims

**1.** A composition comprising:

(i) RNA;

(ii) a cationically ionizable lipid comprising the structure of the following formula (I):

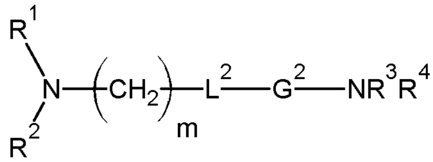

wherein
each of $R^1$ and $R^2$ is independently $R^5$ or $-G^1-L^1-R^6$, wherein at least one of $R^1$ and $R^2$ is $-G^1-L^1-R^6$;
each of $R^3$ and $R^4$ is independently selected from the group consisting of $C_{1-6}$ alkyl, $C_{2-6}$ alkenyl, aryl, and $C_{3-10}$ cycloalkyl;
each of $R^5$ and $R^6$ is independently a non-cyclic hydrocarbyl group having at least 10 carbon atoms;
each of $G^1$ and $G^2$ is independently unsubstituted $C_{1-12}$ alkylene or $C_{2-12}$ alkenylene; each of $L^1$ and $L^2$ is independently selected from the group consisting of -O(C=O)-, -(C=O)O-, -C(=O)-, -O-, $-S(O)_x-$, -S-S-, -C(=O)S-, -SC(=O)-, $-NR^aC(=O)-$, $-C(=O)NR^a-$, $-NR^aC(=O)NR^a-$, $-OC(=O)NR^a-$ and $-NR^aC(=O)O-$;
$R^a$ is H or $C_{1-12}$ alkyl;
m is 0, 1, 2, 3, or 4; and
x is 0, 1 or 2; and

(iii) at least one polysarcosine-conjugated lipid.

**2.** The composition of claim 1, wherein

(i) each $L^1$ is independently selected from the group consisting of -O(C=O)-, -(C=O)O-, -C(=O)S-, -SC(=O)-, $-NR^aC(=O)-$, and $-C(=O)NR^a-$, preferably each $L^1$ is independently -O(C=O)- or -(C=O)O-;
(ii) $L^2$ is selected from the group consisting of -O(C=O)-, -(C=O)O-, -C(=O)-, -C(=O)S-, -SC(=O)-, $-NR^aC(=O)-$, and $-C(=O)NR^a-$, preferably $L^2$ is -O(C=O)- or -(C=O)O-;
(iii) $R^5$ is a straight alkyl or alkenyl group having at least 10 carbon atoms, preferably at least 14 carbon atoms, more preferably at least 16 carbon atoms;
(iv) $R^5$ is a straight alkyl group or a straight alkenyl group having at least 2 carbon-carbon double bonds;
(v) $R^5$ has the following structure:

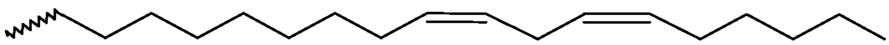 ,

wherein represents the bond by which $R^5$ is bound to the remainder of the compound;
(vi) each $G^1$ is independently unsubstituted straight $C_{1-12}$ alkylene or $C_{2-12}$ alkenylene, preferably unsubstituted, straight $C_{6-12}$ alkylene or $C_{6-12}$ alkenylene, more preferably unsubstituted, straight $C_{8-12}$ alkylene or $C_{8-12}$ alkenylene, or unsubstituted straight $C_{6-10}$ alkylene or $C_{6-10}$ alkenylene, such as unsubstituted straight $C_8$ alkylene;
(vii) each $R^6$ is independently a straight hydrocarbyl group having at least 10 carbon atoms;
(viii) each $R^6$ is attached to $L^1$ via an internal carbon atom of $R^6$;
(ix) each $R^6$ is independently selected from the group consisting of:

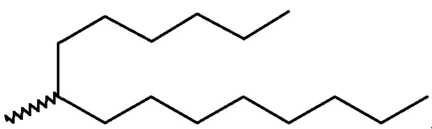 , 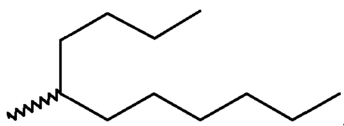 , 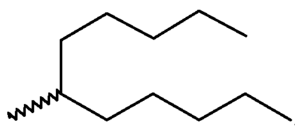 ,

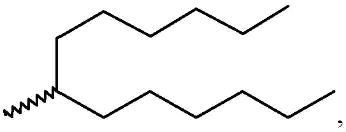, 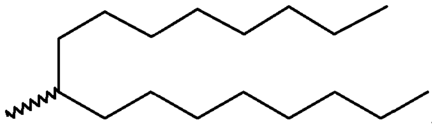,

and

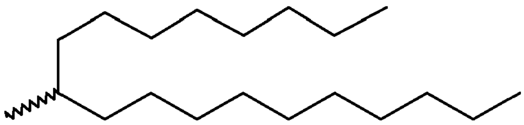,

wherein represents the bond by which $R^6$ is bound to $L^1$;

(x) one of $R^1$ and $R^2$ is $R^5$ and the other is $-G^1-L^1-R^6$, or each of $R^1$ and $R^2$ is independently $-G^1-L^1-R^6$;

(xi) each of $R^3$ and $R^4$ is independently $C_{1-6}$ alkyl or $C_{2-6}$ alkenyl, preferably $C_{1-4}$ alkyl or $C_{2-4}$ alkenyl, more preferably $C_{1-3}$ alkyl, such as methyl or ethyl;

(xii) $G^2$ is unsubstituted $C_{2-10}$ alkylene or $C_{2-10}$ alkenylene, preferably unsubstituted $C_{2-6}$ alkylene or $C_{2-6}$ alkenylene, more preferably unsubstituted $C_{2-4}$ alkylene or $C_{2-4}$ alkenylene, such as ethylene or trimethylene; and/or

(xiii) m is 0, 1, 2 or 3, preferably 0 or 2.

**3.** The composition of claim 1 or 2, wherein

(i) the cationically ionizable lipid comprises the structure of one of the following formulas (IIIa) or (IIIb):

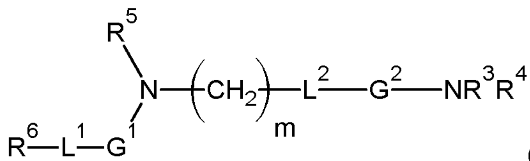

(IIIa)

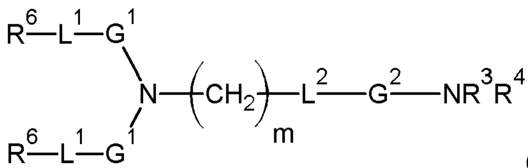

(IIIb),

wherein

each of $R^3$ and $R^4$ is independently $C_{1-4}$ alkyl or $C_{2-4}$ alkenyl, more preferably $C_{1-3}$ alkyl, such as methyl or ethyl;
$R^5$ is a straight alkyl or alkenyl group having at least 16 carbon atoms, wherein the alkenyl group preferably has at least 2 carbon-carbon double bonds;
each $R^6$ is independently a straight hydrocarbyl group having at least 10 carbon atoms, wherein $R^6$ is attached to $L^1$ via an internal carbon atom of $R^6$;
each $G^1$ is independently unsubstituted, straight $C_{6-12}$ alkylene or $C_{6-12}$ alkenylene, e.g., unsubstituted, straight $C_{8-10}$ alkylene or $C_{8-10}$ alkenylene, such as unsubstituted, straight $C_8$ alkylene;
$G^2$ is unsubstituted $C_{2-6}$ alkylene or $C_{2-6}$ alkenylene, preferably unsubstituted $C_{2-4}$ alkylene or $C_{2-4}$ alkenylene, such as ethylene or trimethylene;
each of $L^1$ and $L^2$ is independently -O(C=O)- or -(C=O)O-; and
m is 0, 1, 2 or 3, preferably 0 or 2;

(ii) the cationically ionizable lipid comprises one of the following structures (IV-1), (IV-2), and (IV-3):

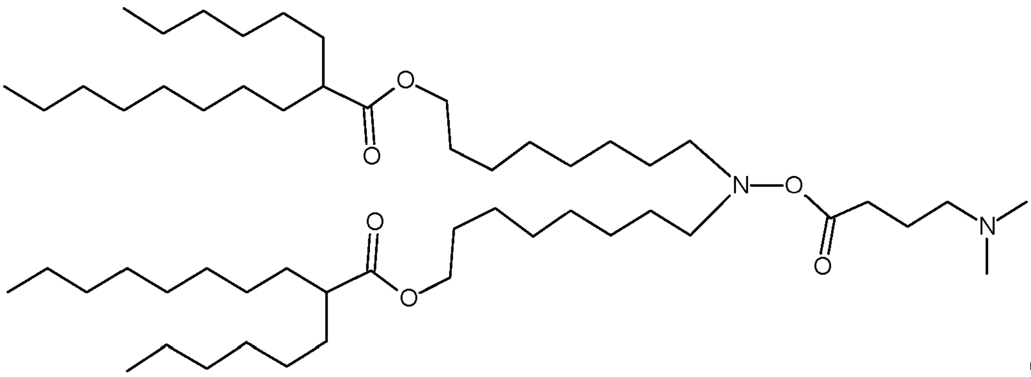

(IV-1);

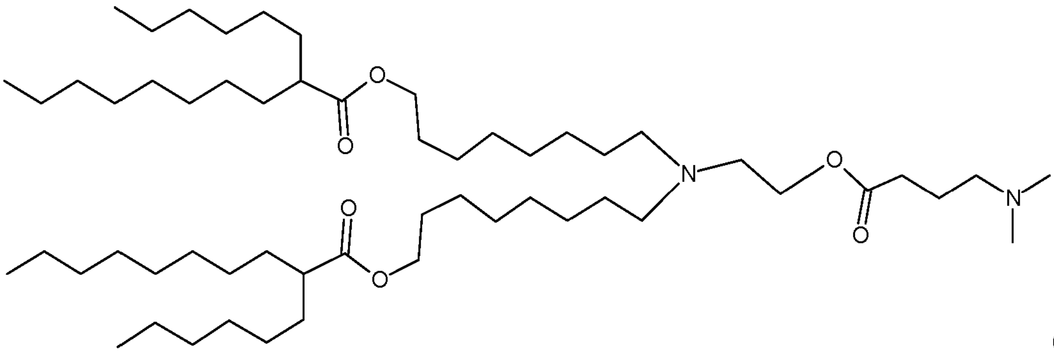

(IV-2);

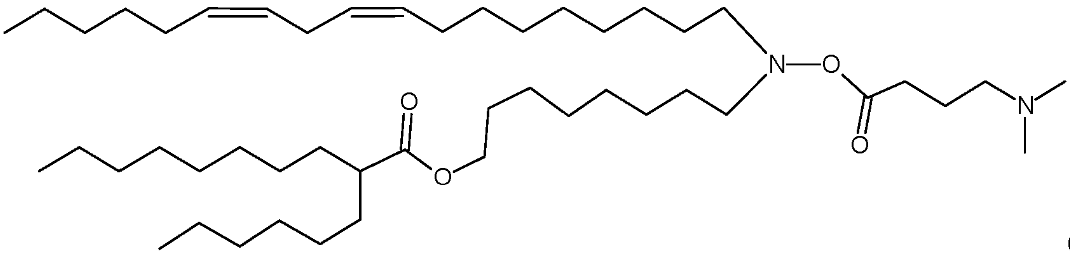

(IV-3);

and/or
(iii) the cationically ionizable lipid comprises from 20 mol % to 80 mol %, preferably from 25 mol % to 65 mol %, more preferably from 30 mol % to 50 mol %, such as from 40 mol % to 50 mol %, or from 55 mol % to 65 mol % of the total lipid present in the composition.

**4.** The composition of any one of claims 1 to 3, wherein

(i) the polysarcosine comprises between 2 and 200 sarcosine units, preferably between 5 and 100 sarcosine units, more preferably between 10 and 50 sarcosine units, more preferably between 15 and 40 sarcosine units, more preferably about 23 sarcosine units;
(ii) the polysarcosine-conjugated lipid comprises the structure of the following general formula (V):

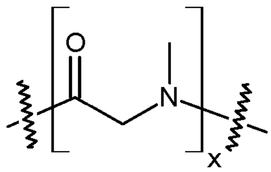

wherein x is the number of sarcosine units;
(iii) the polysarcosine-conjugated lipid has the structure of the following general formula (VI):

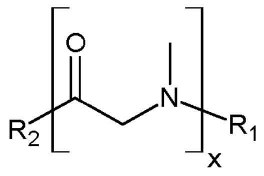

wherein one of $R_1$ and $R_2$ comprises a hydrophobic group and the other is H, a hydrophilic group or a functional group optionally comprising a targeting moiety; and x is the number of sarcosine units, wherein, optionally, $R_1$ is H, a hydrophilic group or a functional group optionally comprising a targeting moiety; and $R_2$ comprises one or two straight alkyl or alkenyl groups each having at least 12 carbon atoms, preferably at least 14 carbon atoms;

(iv) the polysarcosine-conjugated lipid has the structure of the following general formula (VII):

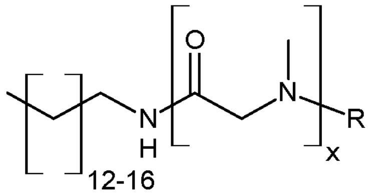

wherein R is H, a hydrophilic group or a functional group optionally comprising a targeting moiety; and x is the number of sarcosine units;
(v) the polysarcosine-conjugated lipid has the structure of the following formula:

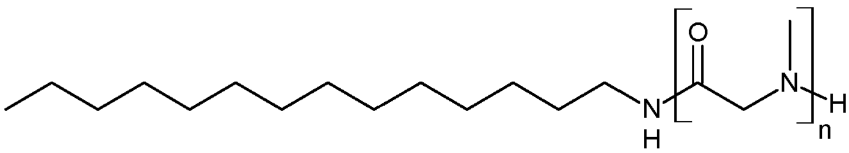,

wherein n is 23; and/or
(vi) the polysarcosine-conjugated lipid comprises from 0.1 mol % to 5 mol %, preferably from 0.5 mol % to 4.5 mol %, more preferably from 1 mol % to 4 mol %, such as 3 mol % to 4 mol %, of the total lipid present in the composition.

**5.** The composition of any one of claims 1 to 4, wherein the composition further comprises one or more additional lipids, preferably selected from the group consisting of phospholipids, steroids, and combinations thereof, more preferably the composition comprises the cationically ionizable lipid; a polymer-conjugated lipid, preferably selected from a polysarcosine-conjugated lipid and a pegylated lipid; a phospholipid; and a steroid.

**6.** The composition of claim 5, wherein

(i) the phospholipid is selected from the group consisting of phosphatidylcholines, phosphatidylethanolamines, phosphatidylglycerols, phosphatidic acids, phosphatidylserines and sphingomyelins, more preferably selected from the group consisting of distearoylphosphatidylcholine (DSPC), dioleoylphosphatidylcholine (DOPC), dimyristoylphosphatidylcholine (DMPC), dipentadecanoylphosphatidylcholine, dilauroylphosphatidylcholine, dipalmitoylphosphatidylcholine (DPPC), diarachidoylphosphatidylcholine (DAPC), dibehenoylphosphatidylcholine (DBPC), ditricosanoylphosphatidylcholine (DTPC), dilignoceroylphatidylcholine (DLPC), palmitoyloleoylphosphatidylcholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), dioleoylphosphatidylethanolamine (DOPE), distearoyl-phosphatidylethanolamine (DSPE), dipalmitoyl-phosphatidylethanolamine (DPPE), dimyristoyl-phosphatidylethanolamine (DMPE), dilauroyl-phosphatidylethanolamine (DLPE), and diphytanoyl-phosphatidylethanolamine (DPyPE);
(ii) the phospholipid comprises from 5 mol % to 40 mol %, preferably from 5 mol % to 20 mol %, more preferably from 5 mol % to 15 mol % of the total lipid present in the composition;

(iii) the steroid comprises a sterol such as cholesterol;
(iv) the steroid comprises from 10 mol % to 65 mol %, preferably from 20 mol % to 60 mol %, more preferably from 30 mol % to 50 mol % or from 25 mol % to 35 mol % of the total lipid present in the composition; and/or
(v) wherein the composition comprises the cationically ionizable lipid, a polysarcosine-conjugated lipid, a phospholipid, and a steroid, wherein the cationically ionizable lipid comprises from 30 mol % to 50 mol %, such as from 40 mol % to 50 mol %, of the total lipid present in the composition; the polysarcosine-conjugated lipid comprises from 1 mol % to 4.5 mol % of the total lipid present in the composition; the phospholipid comprises from 5 mol % to 15 mol % of the total lipid present in the composition; and the steroid comprises from 30 mol % to 50 mol % of the total lipid present in the composition; or
the composition comprises the cationically ionizable lipid, a polysarcosine-conjugated lipid, a phospholipid, and a steroid, wherein the cationically ionizable lipid comprises from 55 mol % to 65 mol % of the total lipid present in the composition; the polysarcosine-conjugated lipid comprises from 1 mol % to 4.5 mol % of the total lipid present in the composition; the phospholipid comprises from 5 mol % to 15 mol % of the total lipid present in the composition; and the steroid comprises from 25 mol % to 35 mol % of the total lipid present in the composition.

**7.** The composition of any one of claims 1 to 6, wherein

(i) at least a portion of (i) the RNA, (ii) the cationically ionizable lipid, and, if present, (iii) the one or more additional lipids is present in nanoparticles, such as lipid nanoparticles (LNPs),
wherein, optionally, the nanoparticles have a size of from 30 nm to 500 nm;
(ii) the RNA is mRNA;
(iii) the RNA (a) comprises a modified nucleoside in place of uridine, wherein the modified nucleoside is preferably selected from pseudouridine ($\psi$), N1-methyl-pseudouridine (m1$\psi$), and 5-methyl-uridine (m5U); (b) has a coding sequence which is codon-optimized; and/or (c) has a coding sequence whose G/C content is increased compared to the wild-type coding sequence; and/or
(iv) the RNA comprises at least one of the following, preferably all of the following: a 5' cap; a 5' UTR; a 3' UTR; and a poly-A sequence,
wherein, optionally,

(a) the poly-A sequence comprises at least 100 A nucleotides, wherein the poly-A sequence preferably is an interrupted sequence of A nucleotides; and/or
(b) the 5' cap is a cap1 or cap2 structure.

**8.** The composition of any one of claims 1 to 7, wherein the RNA encodes one or more peptides or proteins, wherein preferably the one or more peptides or proteins are pharmaceutically active peptides or proteins and/or comprise an epitope for inducing an immune response against an antigen in a subject,
wherein, optionally,

(i) the pharmaceutically active peptide or protein and/or the antigen or epitope is derived from or is a SARS-CoV-2 spike (S) protein, an immunogenic variant thereof, or an immunogenic fragment of the SARS-CoV-2 S protein or the immunogenic variant thereof; and/or
(ii) the RNA comprises an open reading frame (ORF) encoding an amino acid sequence comprising a SARS-CoV-2 S protein, an immunogenic variant thereof, or an immunogenic fragment of the SARS-CoV-2 S protein or the immunogenic variant thereof,
wherein, optionally,

(a) the SARS-CoV-2 S protein variant has proline residue substitutions at positions 986 and 987 of SEQ ID NO: 11;
(b) the SARS-CoV-2 S protein variant has at least 80% identity to the amino acid sequence of amino acids 17 to 1273 of SEQ ID NO: 11 or the amino acid sequence of amino acids 17 to 1273 of SEQ ID NO: 12;
(c) the fragment comprises the receptor binding domain (RBD) of the SARS-CoV-2 S protein; or
(d) the fragment of (i) the SARS-CoV-2 S protein or (ii) the immunogenic variant of the SARS-CoV-2 S protein has at least 80% identity to the amino acid sequence of amino acids 327 to 528 of SEQ ID NO: 11.

**9.** The composition of any one of claims 1 to 8 for use in therapy.

**10.** The composition of any one of claims 1 to 8 for use in a method for delivering RNA to cells of a subject, the method comprising administering the composition to a subject.

**11.** The composition of any one of claims 1 to 8 for use in a method for delivering a pharmaceutically active peptide or protein to a subject, the method comprising administering the composition to a subject, wherein the RNA encodes the pharmaceutically active peptide or protein.

**12.** The composition of any one of claims 1 to 8 for use in a method for treating or preventing a disease or disorder in a subject, the method comprising administering the composition to a subject, wherein delivering the RNA to cells of the subject is beneficial in treating or preventing the disease or disorder.

**13.** The composition of any one of claims 1 to 8 for use in a method for treating or preventing a disease or disorder in a subject, the method comprising administering the composition to a subject, wherein the RNA encodes a pharmaceutically active peptide or protein and wherein delivering the pharmaceutically active peptide or protein to the subject is beneficial in treating or preventing the disease or disorder.

**14.** The composition of any one of claims 1 to 8 for use in inducing an immune response in a subject.

**15.** The composition for use of any one of claims 10 to 13, wherein the subject is a mammal, and wherein, optionally, the mammal is a human.

## Patentansprüche

**1.** Zusammensetzung, umfassend:

(i) RNA;
(ii) ein kationisch ionisierbares Lipid, umfassend die Struktur der nachstehenden Formel (I):

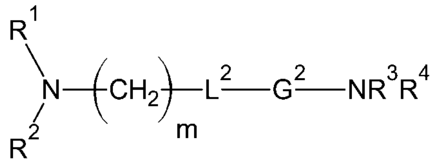

worin

jedes von $R^1$ und $R^2$ unabhängig $R^5$ oder $-G^1-L^1-R^6$ ist, wobei mindestens eines von $R^1$ und $R^2$ $-G^1-L^1-R^6$ ist;
jedes von $R^3$ und $R^4$ unabhängig ausgewählt ist aus der Gruppe, bestehend aus $C_{1-6}$-Alkyl-, $C_{2-6}$-Alkenyl-, Aryl- und $C_{3-10}$-Cycloalkylgruppe;
jedes von $R^5$ und $R^6$ unabhängig eine nicht-cyclische Hydrocarbylgruppe mit mindestens 10 Kohlenstoffatomen ist;
jedes von $G^1$ und $G^2$ unabhängig eine unsubstituierte $C_{1-12}$-Alkylen- oder $C_{2-12}$-Alkenylen-gruppe ist;
jedes von $L^1$ und $L^2$ unabhängig ausgewählt ist aus der Gruppe, bestehend aus -O(C=O)-, -(C=O)O-, -C(=O)-, -O-, $-S(O)_x-$, -S-S-, -C(=O)S-, -SC(=O)-, $-NR^aC(=O)-$, $-C(=O)NR^a-$, $-NR^aC(=O)NR^a-$, $-OC(=O)NR^a-$ und $-NR^aC(=O)O-$;
$R^a$ H oder eine $C_{1-12}$-Alkylgruppe ist;
m 0, 1, 2, 3 oder 4 ist; und
x 0, 1 oder 2 ist; und

(iii) mindestens ein Polysarcosin-konjugiertes Lipid.

**2.** Zusammensetzung nach Anspruch 1, wobei

(i) jedes $L^1$ unabhängig ausgewählt ist aus der Gruppe, bestehend aus -O(C=O)-, -(C=O)O-, -C(=O)S-, -SC(=O)-, $-NR^aC(=O)-$ und $-C(=O)NR^a-$, vorzugsweise jedes $L^1$ unabhängig -O(C=O)- oder -(C=O)O- ist;
(ii) $L^2$ aus der Gruppe ausgewählt ist, bestehend aus -O(C=O)-, -(C=O)O-, -C(=O)-, -C(=O)S-, -SC(=O)-, $-NR^aC(=O)-$ und $-C(=O)NR^a-$, vorzugsweise $L^2$ -O(C=O)- oder -(C=O)O- ist;
(iii) $R^5$ eine gerade Alkyl- oder Alkenylgruppe mit mindestens 10 Kohlenstoffatomen, vorzugsweise mindestens 14 Kohlenstoffatomen, mehr bevorzugt mindestens 16 Kohlenstoffatomen ist;
(iv) $R^5$ eine gerade Alkylgruppe oder eine gerade Alkenylgruppe mit mindestens 2 Kohlenstoff-Kohlenstoff-

Doppelbindungen ist;

(v) $R^5$ die nachstehende Struktur aufweist:

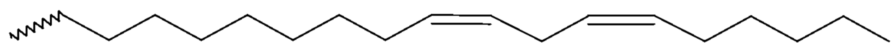
,

worin die Bindung repräsentiert, durch die $R^5$ an den Rest der Verbindung gebunden ist;

(vi) jedes $G^1$ unabhängig eine unsubstituierte gerade $C_{1-12}$-Alkylen- oder $C_{2-12}$-Alkenylen-gruppe, vorzugsweise eine unsubstituierte gerade $C_{6-12}$-Alkylen- oder $C_{6-12}$-Alkenylen-gruppe, mehr bevorzugt eine unsubstituierte gerade $C_{8-12}$-Alkylen- oder $C_{8-12}$-Alkeny-lengruppe oder eine unsubstituierte gerade $C_{6-10}$-Alkylen- oder $C_{6-10}$-Alkenylengruppe, wie eine unsubstituierte gerade $C_8$-Alkylengruppe ist;

(vii) jedes $R^6$ unabhängig eine gerade Hydrocarbylgruppe mit mindestens 10 Kohlenstoffatomen ist;

(viii) jedes $R^6$ an $L^1$ über ein internes Kohlenstoffatom von $R^6$ gebunden ist;

(ix) jedes $R^6$ unabhängig ausgewählt ist aus der Gruppe, bestehend aus:

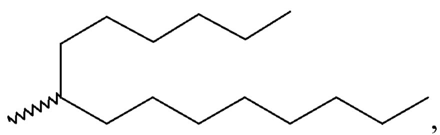
,
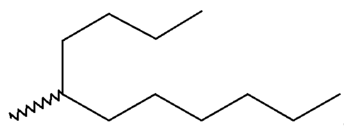
,
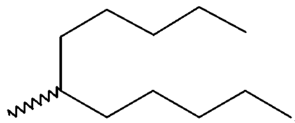
,
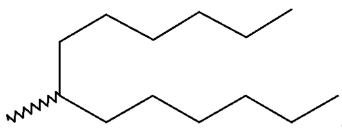
,
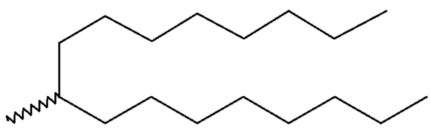
und
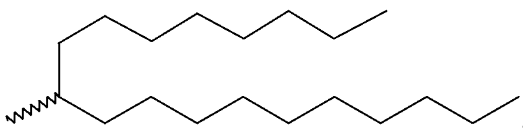
,

worin die Bindung repräsentiert, durch die $R^6$ an $L^1$ gebunden ist;

(x) eines von $R^1$ und $R^2$ $R^5$ ist und das andere $-G^1-L^1-R^6$ ist oder jedes von $R^1$ und $R^2$ unabhängig $-G^1-L^1-R^6$ ist;

(xi) jedes von $R^3$ und $R^4$ unabhängig eine $C_{1-6}$-Alkyl- oder $C_{2-6}$-Alkenylgruppe, vorzugsweise eine $C_{1-4}$-Alkyl- oder $C_{2-4}$-Alkenylgruppe, mehr bevorzugt eine $C_{1-3}$-Alkylgruppe, wie eine Methyl- oder Ethylgruppe ist;

(xii) $G^2$ eine unsubstituierte $C_{2-10}$-Alkylen- oder $C_{2-10}$-Alkenylengruppe, vorzugsweise eine unsubstituierte $C_{2-6}$-Alkylen- oder $C_{2-6}$-Alkenylengruppe, mehr bevorzugt eine unsubstituierte $C_{2-4}$-Alkylen- oder $C_{2-4}$-Alkenylengruppe, wie eine Ethylen- oder Trimethylengruppe ist; und/oder

(xiii) m 0, 1, 2 oder 3, vorzugsweise 0 oder 2 ist.

**3.** Zusammensetzung nach Anspruch 1 oder 2, wobei

(i) das kationisch ionisierbare Lipid die Struktur einer der nachstehenden Formeln (IIIa) oder (IIIb) umfasst:

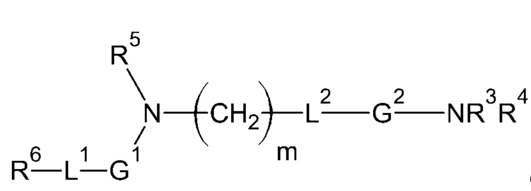
(IIIa)

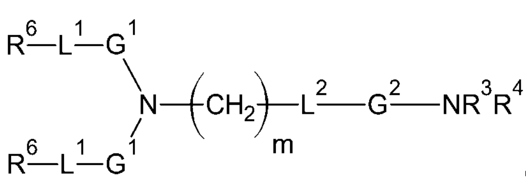
(IIIb),

worin

jedes von $R^3$ und $R^4$ unabhängig eine $C_{1-4}$-Alkyl- oder $C_{2-4}$-Alkenylgruppe, mehr bevorzugt eine $C_{1-3}$-Alkylgruppe, wie eine Methyl- oder Ethylgruppe ist;
$R^5$ eine gerade Alkyl- oder Alkenylgruppe mit mindestens 16 Kohlenstoffatomen ist, wobei die Alkenylgruppe vorzugsweise mindestens 2 Kohlenstoff-Kohlenstoff-Doppelbindungen aufweist;
jedes $R^6$ unabhängig eine gerade Hydrocarbylgruppe mit mindestens 10 Kohlenstoffatomen ist, wobei $R^6$ an $L^1$ über ein internes Kohlenstoffatom von $R^6$ gebunden ist;
jedes $G^1$ unabhängig eine unsubstituierte gerade $C_{6-12}$-Alkylen- oder $C_{6-12}$-Alkenylengruppe, z.B. eine unsubstituierte gerade $C_{8-10}$-Alkylen- oder $C_{8-10}$-Alkenylengruppe, wie eine unsubstituierte gerade $C_8$-Alkylengruppe ist;
$G^2$ eine unsubstituierte $C_{2-6}$-Alkylen- oder $C_{2-6}$-Alkenylengruppe, vorzugsweise eine unsubstituierte $C_{2-4}$-Alkylen- oder $C_{2-4}$-Alkenylengruppe, wie eine Ethylen- oder Trimethylengruppe ist;
jedes von $L^1$ und $L^2$ unabhängig -O(C=O)- oder -(C=O)O- ist; und
m 0, 1, 2 oder 3, vorzugsweise 0 oder 2 ist;

(ii) das kationisch ionisierbare Lipid eine der nachstehenden Strukturen (IV-1), (IV-2) und (IV-3) aufweist:

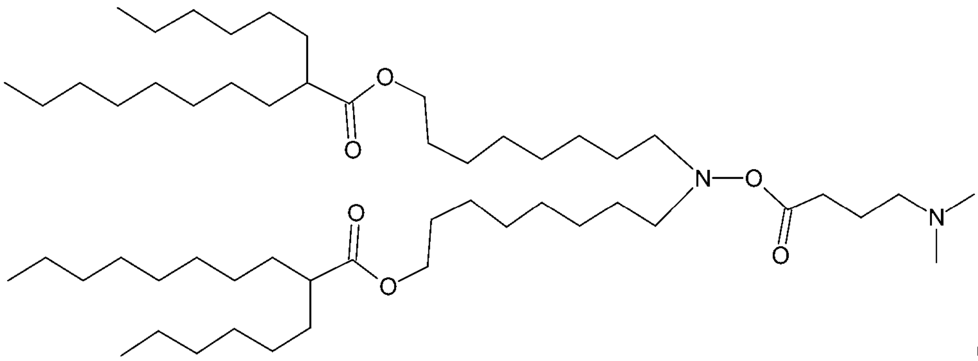

(IV-1);

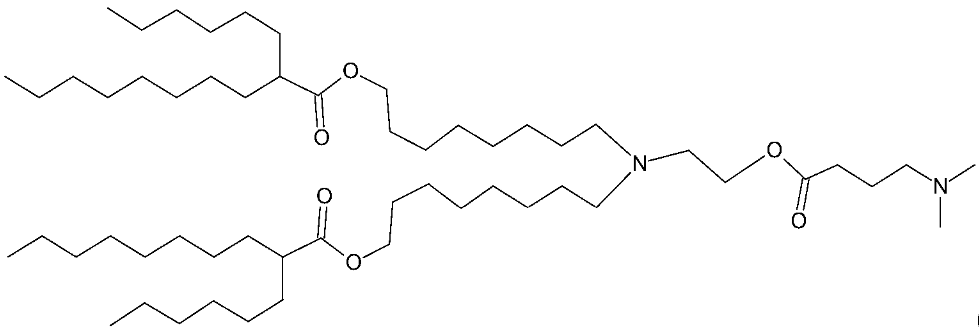

(IV-2);

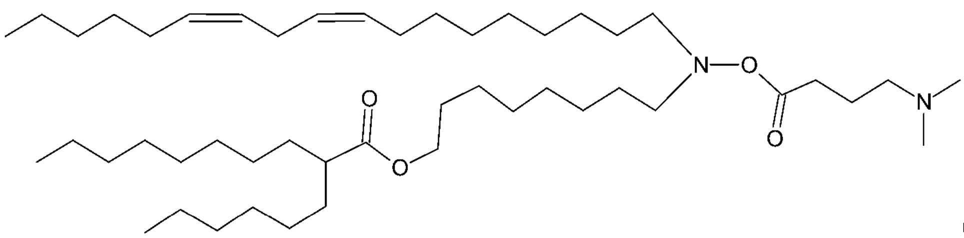

(IV-3);

und/oder
(iii) das kationisch ionisierbare Lipid 20 Mol-% bis 80 Mol-%, vorzugsweise 25 Mol-% bis 65 Mol-%, mehr bevorzugt 30 Mol-% bis 50 Mol-%, wie 40 Mol-% bis 50 Mol-%, oder 55 Mol-% bis 65 Mol-% des in der Zusammensetzung vorhandenen Gesamtlipids umfasst.

**4.** Zusammensetzung nach einem jeglichen der Ansprüche 1 bis 3, wobei

(i) das Polysarcosin zwischen 2 und 200 Sarcosin-Einheiten, vorzugsweise zwischen 5 und 100 Sarcosin-Einheiten, mehr bevorzugt zwischen 10 und 50 Sarcosin-Einheiten, mehr bevorzugt zwischen 15 und 40 Sarcosin-Einheiten, mehr bevorzugt etwa 23 Sarcosin-Einheiten umfasst;

(ii) das Polysarcosin-konjugierte Lipid die Struktur der nachstehenden allgemeinen Formel (V) umfasst:

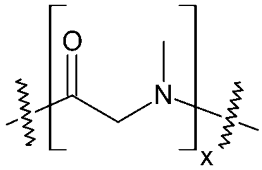

worin x die Anzahl von Sarcosin-Einheiten ist;

(iii) das Polysarcosin-konjugierte Lipid die Struktur der nachstehenden allgemeinen Formel (VI) aufweist:

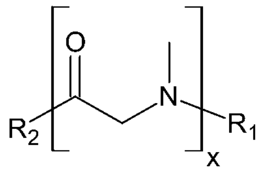

worin eines von $R_1$ und $R_2$ eine hydrophobe Gruppe umfasst und das andere H, eine hydrophile Gruppe oder eine funktionelle Gruppe, die optional eine Zielbindungsgruppe umfasst, ist; und x die Anzahl von Sarcosin-Einheiten ist,

wobei optional $R_1$ H, eine hydrophile Gruppe oder eine funktionelle Gruppe, die optional eine Zielbindungsgruppe umfasst, ist; und $R_2$ eine oder zwei gerade Alkyl- oder Alkenylgruppen umfasst, die jeweils mindestens 12 Kohlenstoffatome, vorzugsweise mindestens 14 Kohlenstoffatome aufweisen;

(iv) das Polysarcosin-konjugierte Lipid die Struktur der nachstehenden allgemeinen Formel (VII) aufweist:

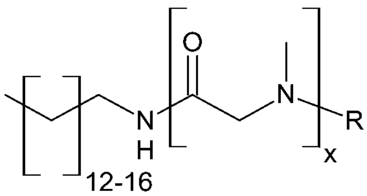

worin R H, eine hydrophile Gruppe oder eine funktionelle Gruppe, die optional eine Zielbindungsgruppe umfasst, ist; und x die Anzahl von Sarcosin-Einheiten ist;

(v) das Polysarcosin-konjugierte Lipid die Struktur der nachstehenden Formel aufweist:

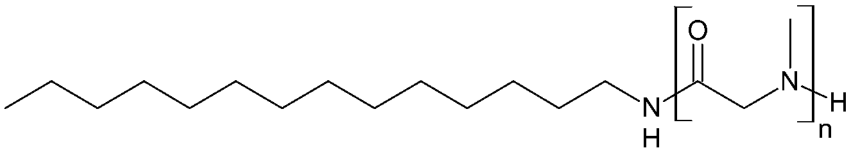,

worin n 23 ist; und/oder

(vi) das Polysarcosin-konjugierte Lipid 0,1 Mol-% bis 5 Mol-%, vorzugsweise 0,5 Mol-% bis 4,5 Mol-%, mehr bevorzugt 1 Mol-% bis 4 Mol-%, wie 3 Mol-% bis 4 Mol-% des in der Zusammensetzung vorhandenen Gesamtlipids umfasst.

**5.** Zusammensetzung nach einem jeglichen der Ansprüche 1 bis 4, wobei die Zusammensetzung ferner ein oder mehrere zusätzliche Lipide, vorzugsweise ausgewählt aus der Gruppe, bestehend aus Phospholipiden, Steroiden und Kombinationen davon, umfasst, mehr bevorzugt die Zusammensetzung das kationisch ionisierbare Lipid; ein Polymer-konjugiertes Lipid, vorzugsweise ausgewählt aus einem Polysarcosin-konjugiertem Lipid und einem pegyliertem Lipid; ein Phospholipid; und ein Steroid umfasst.

6. Zusammensetzung nach Anspruch 5, wobei

(i) das Phospholipid ausgewählt ist aus der Gruppe, bestehend aus Phosphatidylcholinen, Phosphatidylethanolaminen, Phosphatidylglycerinen, Phosphatidsäuren, Phosphatidylserinen und Sphingomyelinen, mehr bevorzugt ausgewählt ist aus der Gruppe, bestehend aus Distearoylphosphatidylcholin (DSPC), Dioleoylphosphatidylcholin (DOPC), Dimyristoylphosphatidylcholin (DMPC), Dipentadecanoylphosphatidylcholin, Dilauroylphosphatidylcholin, Dipalmitoylphosphatidylcholin (DPPC), Diarachidoylphosphatidylcholin (DAPC), Dibehenoylphosphatidylcholin (DBPC), Ditricosanoylphosphatidylcholin (DTPC), Dilignoceroylphatidylcholin (DLPC), Palmitoyloleoyl-phosphatidylcholin (POPC), 1,2-Di-O-octadecenyl-sn-glycero-3-phosphocholin (18:0 Diether-PC), 1-Oleoyl-2-cholesterylhemisuccinoyl-sn-glycero-3-phosphocholin (OChemsPC), 1-Hexadecyl-sn-glycero-3-phosphocholin (C16 Lyso-PC), Dioleoylphosphatidylethanolamin (DOPE), Distearoyl-phosphatidylethanolamin (DSPE), Dipalmitoyl-phosphatidylethanolamin (DPPE), Dimyristoyl-phosphatidylethanolamin (DMPE), Dilauroyl-phosphatidylethanolamin (DLPE) und Diphytanoyl-phosphatidylethanolamin (DPyPE);
(ii) das Phospholipid 5 Mol-% bis 40 Mol-%, vorzugsweise 5 Mol-% bis 20 Mol-%, mehr bevorzugt 5 Mol-% bis 15 Mol-% des in der Zusammensetzung vorhandenen Gesamtlipids umfasst;
(iii) das Steroid ein Sterol, wie Cholesterin umfasst;
(iv) das Steroid 10 Mol-% bis 65 Mol-%, vorzugsweise 20 Mol-% bis 60 Mol-%, mehr bevorzugt 30 Mol-% bis 50 Mol-% oder 25 Mol-% bis 35 Mol-% des in der Zusammensetzung vorhandenen Gesamtlipids umfasst; und/oder
(v) wobei die Zusammensetzung das kationisch ionisierbare Lipid, ein Polysarcosin-konjugiertes Lipid, ein Phospholipid und ein Steroid umfasst, wobei das kationisch ionisierbare Lipid 30 Mol-% bis 50 Mol-%, wie 40 Mol-% bis 50 Mol-%, des in der Zusammensetzung vorhandenen Gesamtlipids umfasst; das Polysarcosin-konjugierte Lipid 1 Mol-% bis 4,5 Mol-% des in der Zusammensetzung vorhandenen Gesamtlipids umfasst; das Phospholipid 5 Mol-% bis 15 Mol-% des in der Zusammensetzung vorhandenen Gesamtlipids umfasst; und das Steroid 30 Mol-% bis 50 Mol-% des in der Zusammensetzung vorhandenen Gesamtlipids umfasst; oder
die Zusammensetzung das kationisch ionisierbare Lipid, ein Polysarcosin-konjugiertes Lipid, ein Phospholipid und ein Steroid umfasst, wobei das kationisch ionisierbare Lipid 55 Mol-% bis 65 Mol-% des in der Zusammensetzung vorhandenen Gesamtlipids umfasst; das Polysarcosin-konjugierte Lipid 1 Mol-% bis 4,5 Mol-% des in der Zusammensetzung vorhandenen Gesamtlipids umfasst; das Phospholipid 5 Mol-% bis 15 Mol-% des in der Zusammensetzung vorhandenen Gesamtlipids umfasst; und das Steroid 25 Mol-% bis 35 Mol-% des in der Zusammensetzung vorhandenen Gesamtlipids umfasst.

7. Zusammensetzung nach einem jeglichen der Ansprüche 1 bis 6, wobei

(i) mindestens ein Teil (i) der RNA, (ii) des kationisch ionisierbaren Lipids, und, falls vorhanden, (iii) des einen oder der mehreren zusätzlichen Lipide in Nanopartikeln, wie Lipidnanopartikeln (LNPs), vorhanden ist,
wobei optional die Nanopartikel eine Größe von 30 nm bis 500 nm aufweisen;
(ii) die RNA mRNA ist;
(iii) die RNA (a) ein modifiziertes Nukleosid anstelle von Uridin umfasst, wobei das modifizierte Nukleosid vorzugsweise ausgewählt ist aus Pseudouridin ($\psi$), N1-Methyl-pseudouridin (m1$\psi$) und 5-Methyl-uridin (m5U); (b) eine kodierende Sequenz aufweist, die codonoptimiert ist; und/oder (c) eine kodierende Sequenz aufweist, deren G/C-Gehalt im Vergleich zu der kodierenden Sequenz des Wildtyps erhöht ist;
und/oder
(iv) die RNA mindestens eines der nachstehenden, vorzugsweise alle der nachstehenden umfasst: eine 5'-Kappe; eine 5'-UTR; eine 3'-UTR; und eine Poly-A Sequenz, wobei optional

(a) die Poly-A-Sequenz mindestens 100 A-Nukleotide umfasst, wobei die Poly-A-Sequenz vorzugsweise eine unterbrochene Sequenz von A-Nukleotiden ist; und/oder
(b) die 5'-Kappe eine Kappen1- oder Kappen2-Struktur ist.

8. Zusammensetzung nach einem jeglichen der Ansprüche 1 bis 7, wobei die RNA ein oder mehrere Peptide oder Proteinen kodiert, wobei vorzugsweise das eine oder die mehreren Peptide oder Proteine pharmazeutisch aktive Peptide oder Proteine sind und/oder ein Epitop zum Induzieren einer Immunantwort gegen ein Antigen in einem Subjekt umfassen,
wobei optional

(i) das pharmazeutisch aktive Peptid oder Protein und/oder das Antigen oder Epitop von einem SARS-CoV-2-Spike(S)-Protein, einer immunogenen Variante davon oder einem immunogenen Fragment des SARS-CoV-2-S-Proteins oder der immunogenen Variante davon abstammt oder ein SARS-CoV-2-Spike(S)-Protein, eine

immunogene Variante davon oder ein immunogenes Fragment des SARS-CoV-2-S-Proteins oder der immunogenen Variante davon ist; und/oder
(ii) die RNA einen offenen Leserahmen (ORF) umfasst, der eine Aminosäuresequenz kodiert, die ein SARS-CoV-2-S-Protein, eine immunogene Variante davon oder ein immunogenes Fragment des SARS-CoV-2-S-Proteins oder der immunogenen Variante davon umfasst,
wobei optional

(a) die SARS-CoV-2-S-Proteinvariante Prolinrest-Substitutionen an den Positionen 986 und 987 von SEQ ID NO: 11 aufweist;
(b) die SARS-CoV-2-S-Proteinvariante mindestens 80% Identität zu der Aminosäuresequenz der Aminosäuren 17 bis 1273 von SEQ ID NO: 11 oder der Aminosäuresequenz der Aminosäuren 17 bis 1273 von SEQ ID NO: 12 aufweist;
(c) das Fragment die Rezeptorbindungsdomäne (RBD) des SARS-CoV-2-S-Proteins umfasst; oder
(d) das Fragment (i) des SARS-CoV-2-S-Proteins oder (ii) der immunogenen Variante des SARS-CoV-2-S-Proteins mindestens 80% Identität zu der Aminosäuresequenz der Aminosäuren 327 bis 528 von SEQ ID NO: 11 aufweist.

**9.** Zusammensetzung nach einem jeglichen der Ansprüche 1 bis 8 zur Verwendung in der Therapie.

**10.** Zusammensetzung nach einem jeglichen der Ansprüche 1 bis 8 zur Verwendung in einem Verfahren zum Zuführen von RNA an Zellen eines Subjekts, wobei das Verfahren ein Verabreichen der Zusammensetzung an ein Subjekt umfasst.

**11.** Zusammensetzung nach einem jeglichen der Ansprüche 1 bis 8 zur Verwendung in einem Verfahren zum Zuführen eines pharmazeutisch aktiven Peptids oder Proteins an ein Subjekt, wobei das Verfahren ein Verabreichen der Zusammensetzung an ein Subjekt umfasst, wobei die RNA das pharmazeutisch aktive Peptid oder Protein kodiert.

**12.** Zusammensetzung nach einem jeglichen der Ansprüche 1 bis 8 zur Verwendung in einem Verfahren zum Behandeln oder Verhindern einer Krankheit oder Störung in einem Subjekt, wobei das Verfahren ein Verabreichen der Zusammensetzung an ein Subjekt umfasst, wobei ein Zuführen der RNA an Zellen des Subjekts vorteilhaft beim Behandeln oder Verhindern der Krankheit oder Störung ist.

**13.** Zusammensetzung nach einem jeglichen der Ansprüche 1 bis 8 zur Verwendung in einem Verfahren zum Behandeln oder Verhindern einer Krankheit oder Störung in einem Subjekt, wobei das Verfahren ein Verabreichen der Zusammensetzung an ein Subjekt umfasst, wobei die RNA ein pharmazeutisch aktives Peptid oder Protein kodiert und wobei ein Zuführen des pharmazeutisch aktiven Peptids oder Proteins an das Subjekt vorteilhaft beim Behandeln oder Verhindern der Krankheit oder Störung ist.

**14.** Zusammensetzung nach einem jeglichen der Ansprüche 1 bis 8 zur Verwendung beim Induzieren einer Immunantwort in einem Subjekt.

**15.** Zusammensetzung zur Verwendung nach einem jeglichen der Ansprüche 10 bis 13, wobei das Subjekt ein Säuger ist, und wobei optional der Säuger ein Mensch ist.

## Revendications

**1.** Composition comprenant :

(i) de l'ARN ;
(ii) un lipide cationiquement ionisable comprenant la structure de la formule (I) suivante :

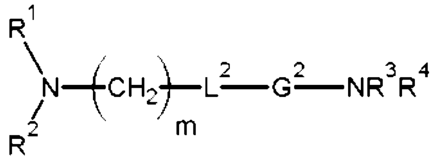

,

dans laquelle

chacun des groupes $R^1$ et $R^2$ représente indépendamment un groupe $R^5$ ou $-G^1-L^1-R^6$, au moins l'un des groupes $R^1$ et $R^2$ représentant $-G^1-L^1-R^6$ ;
chacun des groupes $R^3$ et $R^4$ est indépendamment choisi dans le groupe constitué par un groupe $C_{1-6}$ alkyle, un groupe $C_{2-6}$ alcényle, un groupe aryle et un groupe $C_{3-10}$ cycloalkyle ;
chacun des groupes $R^5$ et $R^6$ représente indépendamment un groupe hydrocarbyle non cyclique comportant au moins 10 atomes de carbone ;
chacun des groupes $G^1$ et $G^2$ représente indépendamment un groupe $C_{1-12}$ alkylène ou $C_{2-12}$ alcénylène non substitué ;
chacun des groupes $L^1$ et $L^2$ est indépendamment choisi dans le groupe constitué par les groupes -O(C=O)-, -(C=O)O-, -C(=O)-, -O-, $-S(O)_x-$, -S-S-, -C(=O)S-, -SC(=O)-, $-NR^aC(=O)-$, $- C(=O)NR^a-$, $-NR^aC(=O)NR^a-$, $-OC(=O)NR^a-$ et $-NR^aC(=O)O-$ ;
$R^a$ représente un atome H ou un groupe $C_{1-12}$ alkyle ;
m vaut 0, 1, 2, 3 ou 4 ; et
x vaut 0, 1 ou 2 ; et

(iii) au moins un lipide conjugué à la polysarcosine.

**2.** Composition selon la revendication 1,

(i) chaque groupe $L^1$ étant indépendamment choisi dans le groupe constitué par les groupes -O(C=O)-, -(C=O)O-, -C(=O)S-, -SC(=O)-, $-NR^aC(=O)-$ et $-C(=O)NR^a-$, de préférence chaque groupe $L^1$ représentant indépendamment un groupe -O(C=O)- ou -(C=O)O- ;
(ii) $L^2$ étant choisi dans le groupe constitué par un groupe -O(C=O)-, -(C=O)O-, - C(=O)-, -C(=O)S-, -SC(=O)-, $-NR^aC(=O)-$ et $-C(=O)NR^a-$, de préférence $L^2$ représentant un groupe -O(C=O)- ou -(C=O)O- ;
(iii) $R^5$ représentant un groupe alkyle ou alcényle linéaire comportant au moins 10 atomes de carbone, de préférence au moins 14 atomes de carbone, plus préférablement au moins 16 atomes de carbone ;
(iv) $R^5$ représentant un groupe alkyle linéaire ou un groupe alcényle linéaire comportant au moins 2 doubles liaisons carbone-carbone ;
(v) $R^5$ présentant la structure suivante :

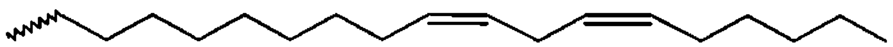,

dans laquelle représente la liaison par laquelle $R^5$ est lié au reste du composé ;
(vi) chaque groupe $G^1$ représentant indépendamment un groupe $C_{1-12}$ alkylène ou $C_{2-12}$ alcénylène linéaire non substitué, de préférence un groupe $C_{6-12}$ alkylène ou $C_{6-12}$ alcénylène linéaire non substitué, plus préférablement un groupe $C_{8-12}$ alkylène ou $C_{8-12}$ alcénylène linéaire non substitué, ou un groupe $C_{6-10}$ alkylène ou $C_{6-10}$ alcénylène linéaire non substitué, tel qu'un groupe $C_8$ alkylène linéaire non substitué ;
(vii) chaque groupe $R^6$ représentant indépendamment un groupe hydrocarbyle linéaire comportant au moins 10 atomes de carbone ;
(viii) chaque groupe $R^6$ étant lié à $L^1$ par l'intermédiaire d'un atome de carbone interne de $R^6$ ;
(ix) chaque groupe $R^6$ étant indépendamment choisi dans le groupe constitué par :

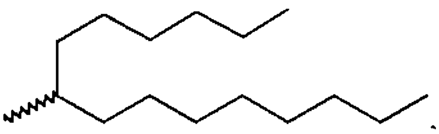, 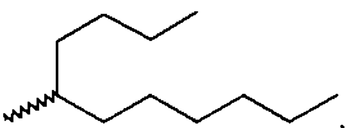, 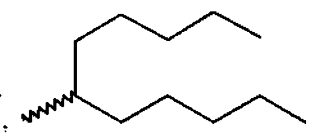,

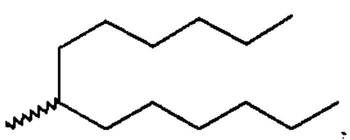, 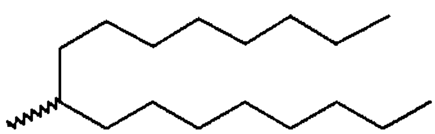,

et

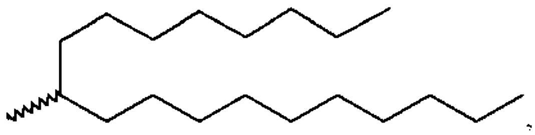

dans lesquelles  représente la liaison par laquelle $R^6$ est lié à $L^1$ ;

(x) l'un des groupes $R^1$ et $R^2$ représentant $R^5$ et l'autre représentant un groupe $-G^1-L^1-R^6$, ou chacun des groupes $R^1$ et $R^2$ représentant indépendamment un groupe $-G^1-L^1-R^6$ ;

(xi) chacun des groupes $R^3$ et $R^4$ représentant indépendamment un groupe $C_{1-6}$ alkyle ou $C_{2-6}$ alcényle, de préférence un groupe $C_{1-4}$ alkyle ou $C_{2-4}$ alcényle, plus préférablement un groupe $C_{1-3}$ alkyle, tel qu'un groupe méthyle ou éthyle ;

(xii) $G^2$ représentant un groupe $C_{2-10}$ alkylène ou $C_{2-10}$ alcénylène non substitué, de préférence un groupe $C_{2-6}$ alkylène ou $C_{2-6}$ alcénylène non substitué, plus préférablement un groupe $C_{2-4}$ alkylène ou $C_{2-4}$ alcénylène non substitué, tel qu'un groupe éthylène ou triméthylène ; et/ou

(xiii) m vaut 0, 1, 2 ou 3, de préférence 0 ou 2.

**3.** Composition selon l'une des revendications 1 ou 2,

(i) ledit lipide cationiquement ionisable comprenant la structure de l'une des formules (IIIa) ou (IIIb) suivantes :

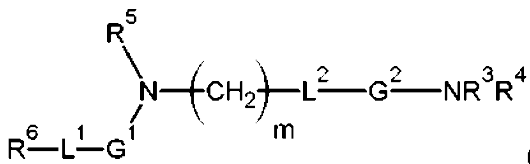

(IIIa)

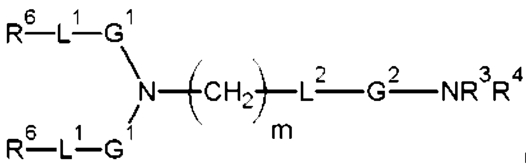

(IIIb),

dans lesquelles

chacun des groupes $R^3$ et $R^4$ représente indépendamment un groupe $C_{1-4}$ alkyle ou $C_{2-4}$ alcényle, de préférence un groupe $C_{1-3}$ alkyle, tel qu'un groupe méthyle ou éthyle ;

$R^5$ représente un groupe alkyle ou alcényle linéaire comportant au moins 16 atomes de carbone, ledit groupe alcényle comportant de préférence au moins 2 doubles liaisons carbone-carbone ;

chaque groupe $R^6$ représente indépendamment un groupe hydrocarbyle linéaire comportant au moins 10 atomes de carbone, $R^6$ étant lié à $L^1$ par l'intermédiaire d'un atome de carbone interne de $R^6$ ;

chaque groupe $G^1$ représente indépendamment un groupe $C_{6-12}$ alkylène ou $C_{6-12}$ alcénylène linéaire non substitué, par exemple un groupe $C_{8-10}$ alkylène ou $C_{8-10}$ alcénylène linéaire non substitué, tel qu'un groupe $C_3$ alkylène linéaire non substitué ;

$G^2$ représente un groupe $C_{2-6}$ alkylène ou $C_{2-6}$ alcénylène non substitué, de préférence un groupe $C_{2-4}$ alkylène ou $C_{2-4}$ alcénylène non substitué, tel qu'un groupe éthylène ou triméthylène ;

chacun des groupes $L^1$ et $L^2$ représente indépendamment un groupe -O(C=O)- ou - (C=O)O- ; et

m vaut 0, 1, 2 ou 3, de préférence 0 ou 2 ;

(ii) ledit lipide cationiquement ionisable comprenant l'une des structures (IV-1), (IV-2) et (IV-3) suivantes :

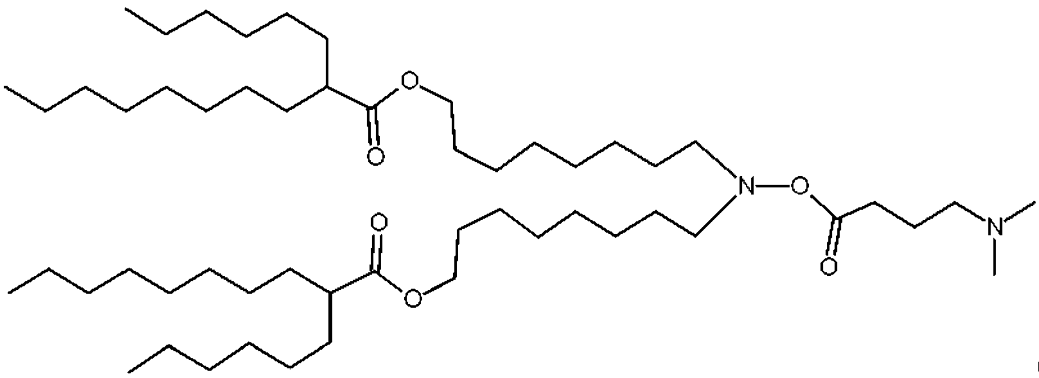

(IV-1);

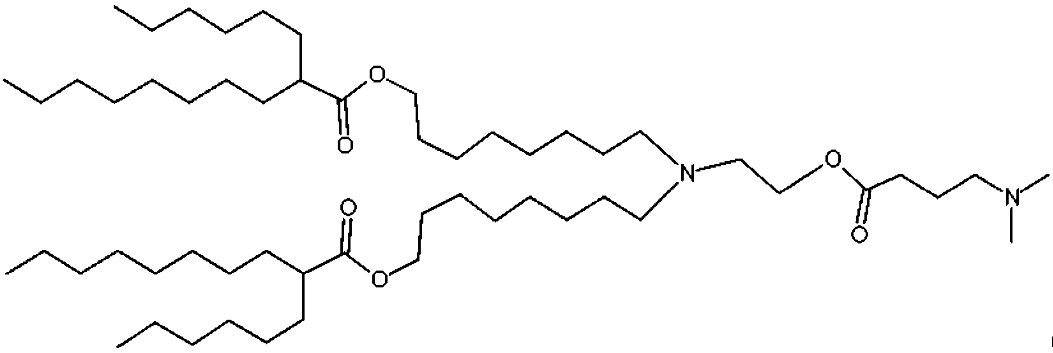

(IV-2);

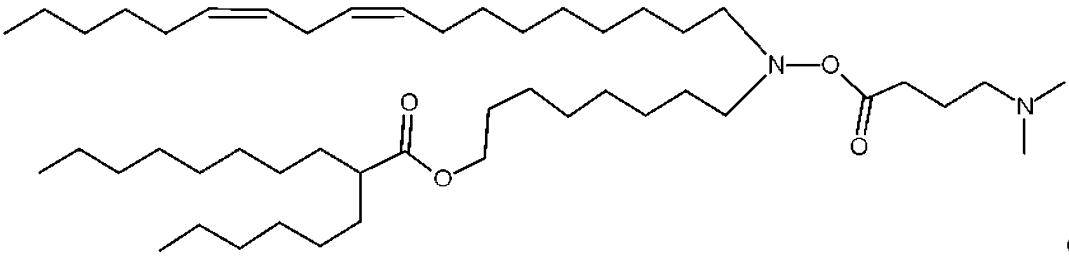

(IV-3);

et/ou

(iii) ledit lipide cationiquement ionisable comprenant de 20 % en moles à 80 % en moles, de préférence de 25 % en moles à 65 % en moles, plus préférablement de 30 % en moles à 50 % en moles, tel que de 40 % en moles à 50 % en moles, ou de 55 % en moles à 65 % en moles des lipides totaux présents dans la composition.

**4.** Composition selon l'une quelconque des revendications 1 à 3,

(i) ladite polysarcosine comprenant entre 2 et 200 motifs de sarcosine, de préférence entre 5 et 100 motifs de sarcosine, plus préférablement entre 10 et 50 motifs de sarcosine, plus préférablement entre 15 et 40 motifs de sarcosine, plus préférablement environ 23 motifs de sarcosine ;

(ii) ledit lipide conjugué à la polysarcosine comprenant la structure de la formule générale (V) suivante :

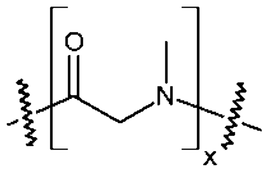

,

dans laquelle x représente le nombre de motifs de sarcosine ;

(iii) ledit lipide conjugué à la polysarcosine présentant la structure de la formule générale (VI) suivante :

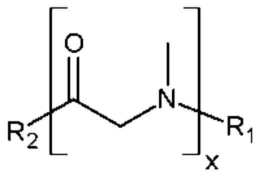

,

dans laquelle l'un des groupes $R_1$ et $R_2$ comprend un groupe hydrophobe et l'autre représente un atome H, un groupe hydrophile ou un groupe fonctionnel comprenant éventuellement un groupement de ciblage ; et x représente le nombre de motifs de sarcosine,

dans laquelle, éventuellement, $R_1$ représente un atome H, un groupe hydrophile ou un groupe fonctionnel comprenant éventuellement un groupement de ciblage ; et $R_2$ comprend un ou deux groupes alkyle ou alcényle linéaires comportant chacun au moins 12 atomes de carbone, de préférence au moins 14 atomes de carbone ;

(iv) ledit lipide conjugué à la polysarcosine présentant la structure de la formule générale (VII) suivante :

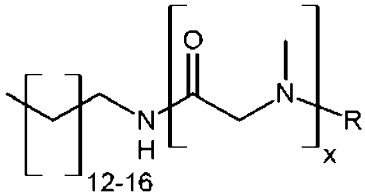

,

dans laquelle R représente un atome H, un groupe hydrophile ou un groupe fonctionnel comprenant éventuellement un groupement de ciblage ; et x représente le nombre de motifs de sarcosine ;

(v) ledit lipide conjugué à la polysarcosine présentant la structure de la formule suivante :

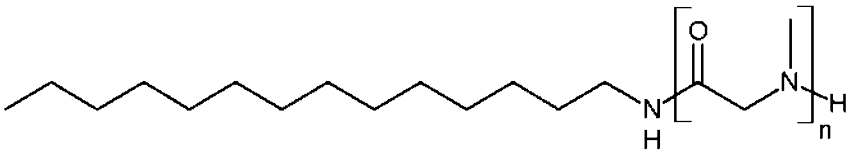

,

dans laquelle n vaut 23 ; et/ou

(vi) ledit lipide conjugué à la polysarcosine comprenant de 0,1 % en moles à 5 % en moles, de préférence de 0,5 % en moles à 4,5 % en moles, plus préférablement de 1 % en moles à 4 % en moles, tel que de 3 % en moles à 4 % en moles, des lipides totaux présents dans la composition.

**5.** Composition selon l'une quelconque des revendications 1 à 4, ladite composition comprenant en outre un ou plusieurs lipides supplémentaires, de préférence choisis dans le groupe constitué par les phospholipides, les stéroïdes et les combinaisons de ceux-ci, plus préférablement ladite composition comprenant le lipide cationiquement ionisable ; un lipide conjugué à un polymère, de préférence choisi parmi un lipide conjugué à la polysarcosine et un lipide pégylé ; un phospholipide ; et un stéroïde.

**6.** Composition selon la revendication 5,

(i) ledit phospholipide étant choisi dans le groupe constitué par les phosphatidylcholines, les phosphatidyléthanolamines, les phosphatidylglycérols, les acides phosphatidiques, les phosphatidylsérines et les sphingomyélines, plus préférablement choisi dans le groupe constitué par la distéaroylphosphatidylcholine (DSPC), la dioléoylphosphatidylcholine (DOPC), la dimyristoylphosphatidylcholine (DMPC), la dipentadécanoylphosphatidylcholine, la dilauroylphosphatidylcholine, la dipalmitoylphosphatidylcholine (DPPC), la diarachidoylphosphatidylcholine (DAPC), la dibéhénoylphosphatidylcholine (DBPC), la ditricosanoylphosphatidylcholine (DTPC), la dilignocéroylphatidylcholine (DLPC), la palmitoyloléoyl-phosphatidylcholine (POPC), la 1,2-di-O-octadécényl-sn-glycéro-3-phosphocholine (18:0 diéther PC), la 1-oléoyl-2-cholestérylhémisuccinoyl-sn-glycéro-3-phosphocholine (OChemsPC), la 1-hexadécyl-sn-glycéro-3-phosphocholine (C16 Lyso PC), la dioléoylphosphatidyléthanolamine (DOPE), la distéaroyl-phosphatidyléthanolamine (DSPE), la dipalmitoylphosphatidyléthanolamine (DPPE), la dimyristoyl-phosphatidyléthanolamine (DMPE), la dilauroyl-phosphatidyléthanolamine (DLPE) et la diphytanoyl-phosphatidyléthanolamine (DPyPE) ;
(ii) ledit phospholipide comprenant de 5 % en moles à 40 % en moles, de préférence de 5 % en moles à 20 % en moles, plus préférablement de 5 % en moles à 15 % en moles des lipides totaux présents dans la composition ;
(iii) ledit stéroïde comprenant un stérol tel que le cholestérol ;
(iv) ledit stéroïde comprenant de 10 % en moles à 65 % en moles, de préférence de 20 % en moles à 60 % en moles, plus préférablement de 30 % en moles à 50 % en moles ou de 25 % en moles à 35 % en moles des lipides totaux présents dans la composition ; et/ou
(v) ladite composition comprenant ledit lipide cationiquement ionisable, un lipide conjugué à la polysarcosine, un phospholipide et un stéroïde, ledit lipide cationiquement ionisable comprenant de 30 % en moles à 50 % en moles, tel que de 40 % en moles à 50 % en moles, des lipides totaux présents dans la composition ; ledit lipide conjugué à la polysarcosine comprenant de 1 % en moles à 4,5 % en moles des lipides totaux présents dans la composition ; ledit phospholipide comprenant de 5 % en moles à 15 % en moles des lipides totaux présents dans la composition ; et ledit stéroïde comprenant de 30 % en moles à 50 % en moles des lipides totaux présents dans la composition ; ou
ladite composition comprenant le lipide cationiquement ionisable, un lipide conjugué à la polysarcosine, un phospholipide et un stéroïde, ledit lipide cationiquement ionisable comprenant de 55 % en moles à 65 % en moles des lipides totaux présents dans la composition ; ledit lipide conjugué à la polysarcosine comprenant de 1 % en moles à 4,5 % en moles des lipides totaux présents dans la composition ; ledit phospholipide comprenant de 5 % en moles à 15 % en moles des lipides totaux présents dans la composition ; et ledit stéroïde comprenant de 25 % en moles à 35 % en moles des lipides totaux présents dans la composition.

**7.** Composition selon l'une quelconque des revendications 1 à 6,

(i) au moins une partie de (i) l'ARN, (ii) du lipide cationiquement ionisable, et, s'ils sont présents, (iii) desdits un ou plusieurs lipides supplémentaires étant présente dans des nanoparticules, telles que des nanoparticules lipidiques (LNP),
éventuellement, lesdites nanoparticules présentant une taille allant de 30 nm à 500 nm ;
(ii) ledit ARN étant de l'ARNm ;
(iii) ledit ARN (a) comprenant un nucléoside modifié à la place de l'uridine, ledit nucléoside modifié étant de préférence choisi parmi la pseudo-uridine (Ψ), la N1-méthyl-pseudo-uridine (m1Ψ) et la 5-méthyl-uridine (m5U) ; (b) présentant une séquence codante qui est à codons optimisés ; et/ou (c) présentant une séquence codante dont la teneur en G/C est augmentée par rapport à la séquence codante de type sauvage ; et/ou
(iv) ledit ARN comprenant au moins l'un des éléments suivants, de préférence tous les éléments suivants : une coiffe en 5' ; une région 5' UTR ; une région 3' UTR ; et une séquence poly-A,
éventuellement,

(a) ladite séquence poly-A comprenant au moins 100 nucléotides A, ladite séquence poly-A étant de préférence une séquence interrompue de nucléotides A ; et/ou
(b) ladite coiffe en 5' étant une structure de coiffe1 ou de coiffe2.

**8.** Composition selon l'une quelconque des revendications 1 à 7, ledit ARN codant un ou plusieurs peptides ou une ou plusieurs protéines, de préférence, lesdits un ou plusieurs peptides ou lesdites une ou plusieurs protéines étant des peptides ou des protéines pharmaceutiquement actifs et/ou comprenant un épitope pour induire une réponse immunitaire contre un antigène chez un sujet,

éventuellement,

(i) ledit peptide ou ladite protéine pharmaceutiquement actif et/ou ledit antigène ou ledit épitope étant dérivé de ou étant une protéine spike (S) de SARS-CoV-2, un variant immunogène de celle-ci, ou un fragment immunogène de la protéine S de SARS-CoV-2 ou du variant immunogène de celle-ci ; et/ou
(ii) ledit ARN comprenant un cadre de lecture ouvert (ORF) codant une séquence d'acides aminés comprenant une protéine S de SARS-CoV-2, un variant immunogène de celle-ci ou un fragment immunogène de la protéine S de SARS-CoV-2 ou du variant immunogène de celle-ci,

éventuellement,

(a) ledit variant de la protéine S de SARS-CoV-2 présentant des substitutions de résidus proline aux positions 986 et 987 de SEQ ID n° : 11 ;
(b) ledit variant de la protéine S de SARS-CoV-2 présentant une identité de séquence d'au moins 80 % avec la séquence d'acides aminés des acides aminés 17 à 1273 de SEQ ID n° : 11 ou la séquence d'acides aminés des acides aminés 17 à 1273 de SEQ ID n° : 12 ;
(c) ledit fragment comprenant le domaine de liaison au récepteur (RBD) de la protéine S de SARS-CoV-2 ; ou
(d) ledit fragment de (i) la protéine S de SARS-CoV-2 ou (ii) du variant immunogène de la protéine S de SARS-CoV-2 présentant une identité de séquence d'au moins 80 % avec la séquence d'acides aminés des acides aminés 327 à 528 de SEQ ID n° : 11.

**9.** Composition selon l'une quelconque des revendications 1 à 8, destinée à être utilisée en thérapie.

**10.** Composition selon l'une quelconque des revendications 1 à 8 destinée à être utilisée dans une méthode d'administration d'ARN aux cellules d'un sujet, la méthode comprenant l'administration de la composition à un sujet.

**11.** Composition selon l'une quelconque des revendications 1 à 8 destinée à être utilisée dans une méthode d'administration d'un peptide ou d'une protéine pharmaceutiquement actif à un sujet, la méthode comprenant l'administration de la composition à un sujet, ledit ARN codant le peptide ou la protéine pharmaceutiquement actif.

**12.** Composition selon l'une quelconque des revendications 1 à 8, destinée à être utilisée dans une méthode de traitement ou de prévention d'une maladie ou d'un trouble chez un sujet, la méthode comprenant l'administration de la composition à un sujet, ladite administration de l'ARN aux cellules du sujet étant bénéfique dans le traitement ou la prévention de la maladie ou du trouble.

**13.** Composition selon l'une quelconque des revendications 1 à 8 destinée à être utilisée dans une méthode de traitement ou de prévention d'une maladie ou d'un trouble chez un sujet, la méthode comprenant l'administration de la composition à un sujet, ledit ARN codant un peptide ou une protéine pharmaceutiquement actif et ladite administration du peptide ou de la protéine pharmaceutiquement actif au sujet étant bénéfique dans le traitement ou la prévention de la maladie ou du trouble.

**14.** Composition selon l'une quelconque des revendications 1 à 8 destinée à être utilisée pour induire une réponse immunitaire chez un sujet.

**15.** Composition destinée à être utilisée selon l'une quelconque des revendications 10 à 13, ledit sujet étant un mammifère et, éventuellement, ledit mammifère étant un humain.

Fig. 1

A

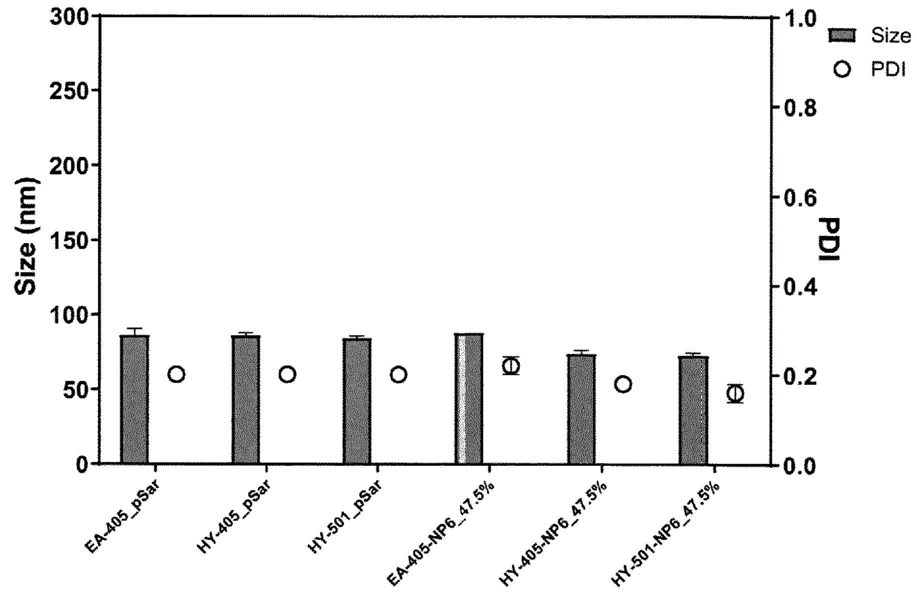
Size
PDI
Size (nm)
PDI
300
250
200
150
100
50
0
1.0
0.8
0.6
0.4
0.2
0.0
EA-405_pSar
HY-405_pSar
HY-501_pSar
EA-405-NP6_47.5%
HY-405-NP6_47.5%
HY-501-NP6_47.5%

B

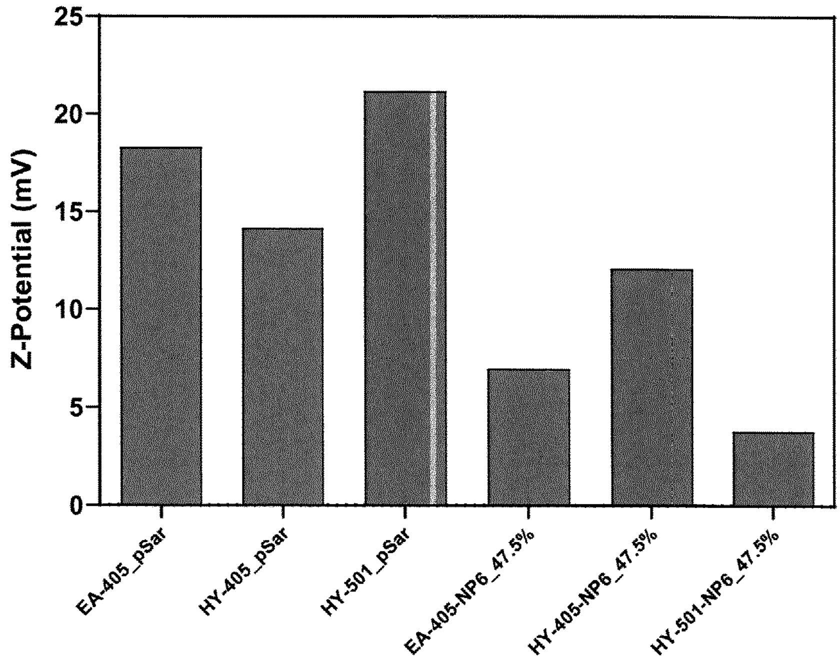
Z-Potential (mV)
25
20
15
10
5
0
EA-405_pSar
HY-405_pSar
HY-501_pSar
EA-405-NP6_47.5%
HY-405-NP6_47.5%
HY-501-NP6_47.5%

Fig. 1 (contin.)

C

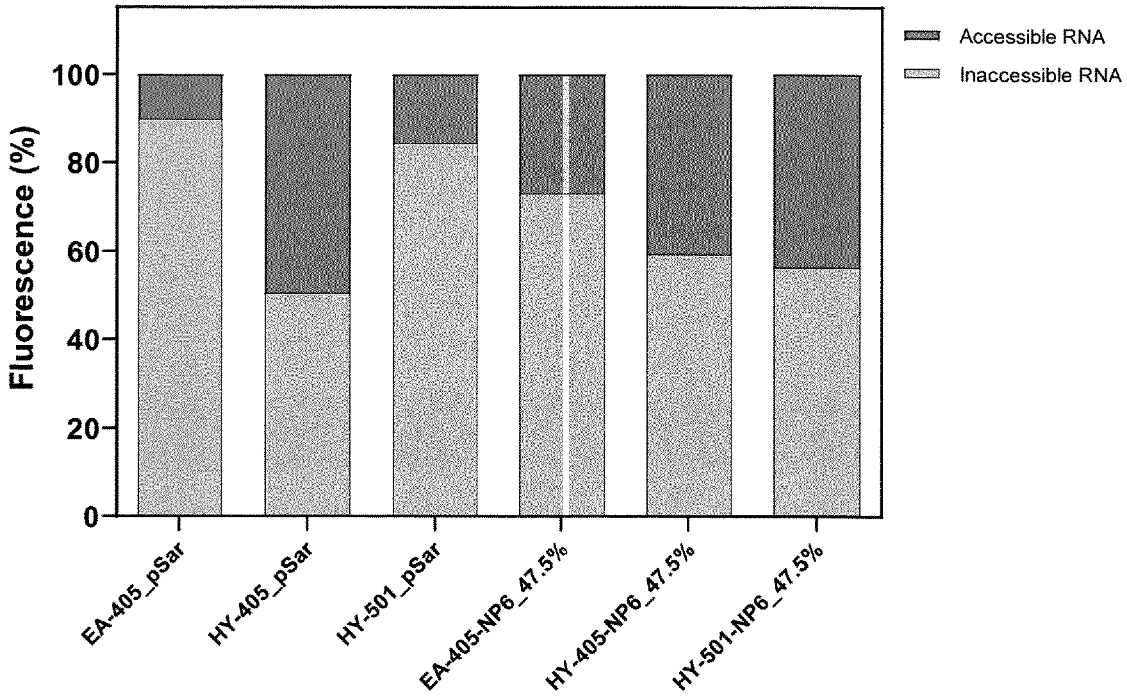


D

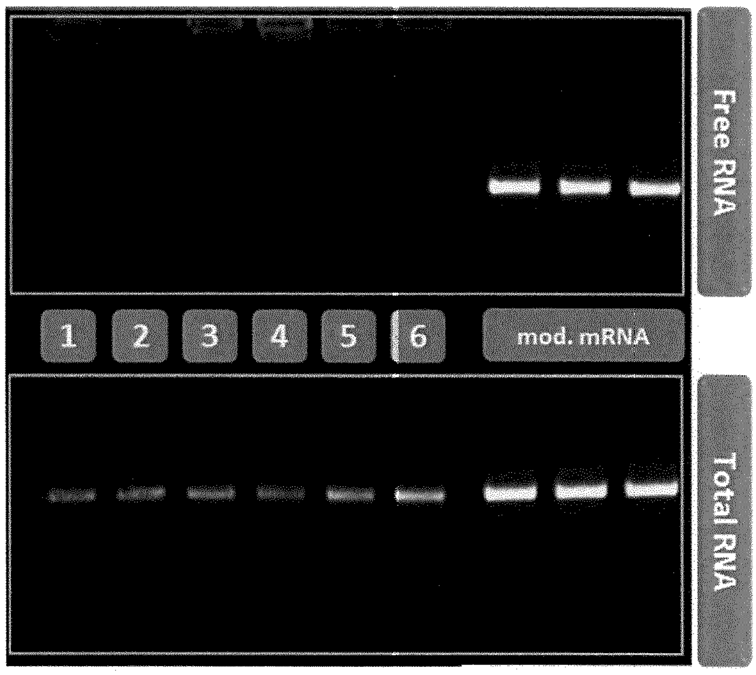

Fig. 2

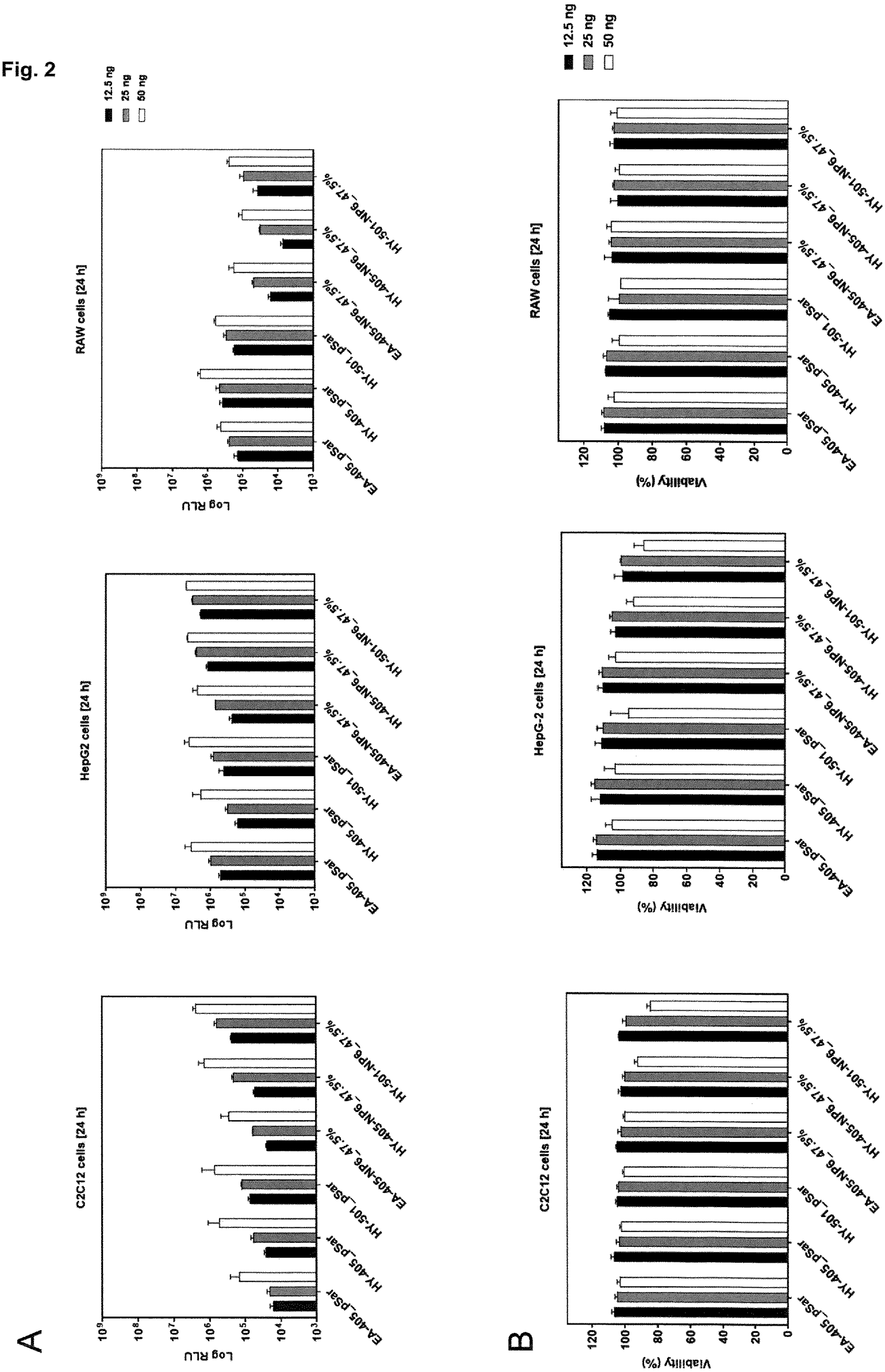
A
C2C12 cells [24 h]
HepG2 cells [24 h]
RAW cells [24 h]
Log RLU
12.5 ng
25 ng
50 ng
EA-405_pSar
HY-405_pSar
HY-501_pSar
EA-405-NP6_47.5%
HY-405-NP6_47.5%
HY-501-NP6_47.5%
B
HepG-2 cells [24 h]
Viability (%)

Fig. 3

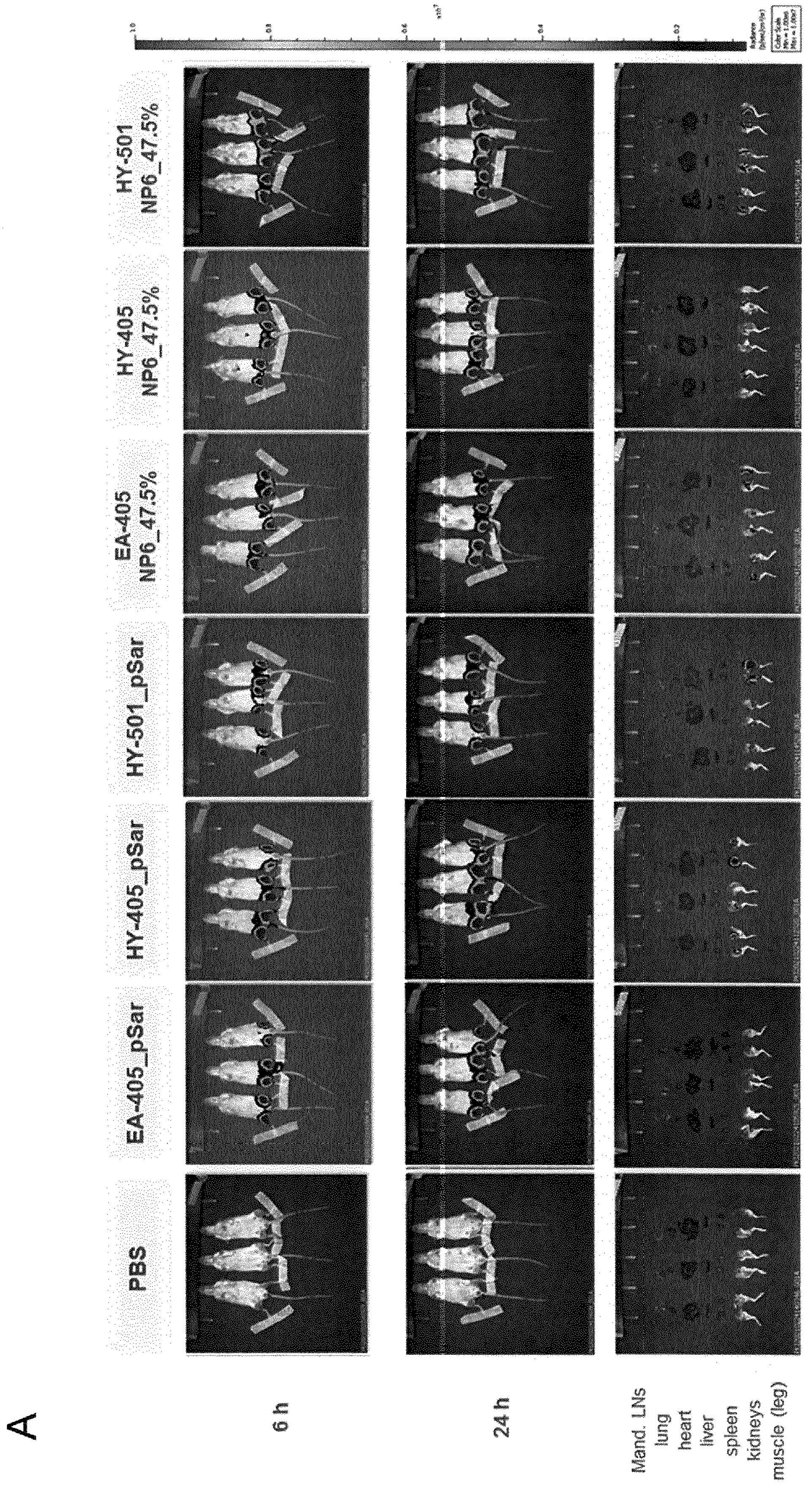
A
PBS
EA-405_pSar
HY-405_pSar
HY-501_pSar
EA-405 NP6_47.5%
HY-405 NP6_47.5%
HY-501 NP6_47.5%
6 h
24 h
Mand. LNs
lung
heart
liver
spleen
kidneys
muscle (leg)

Fig. 3 (contin.)

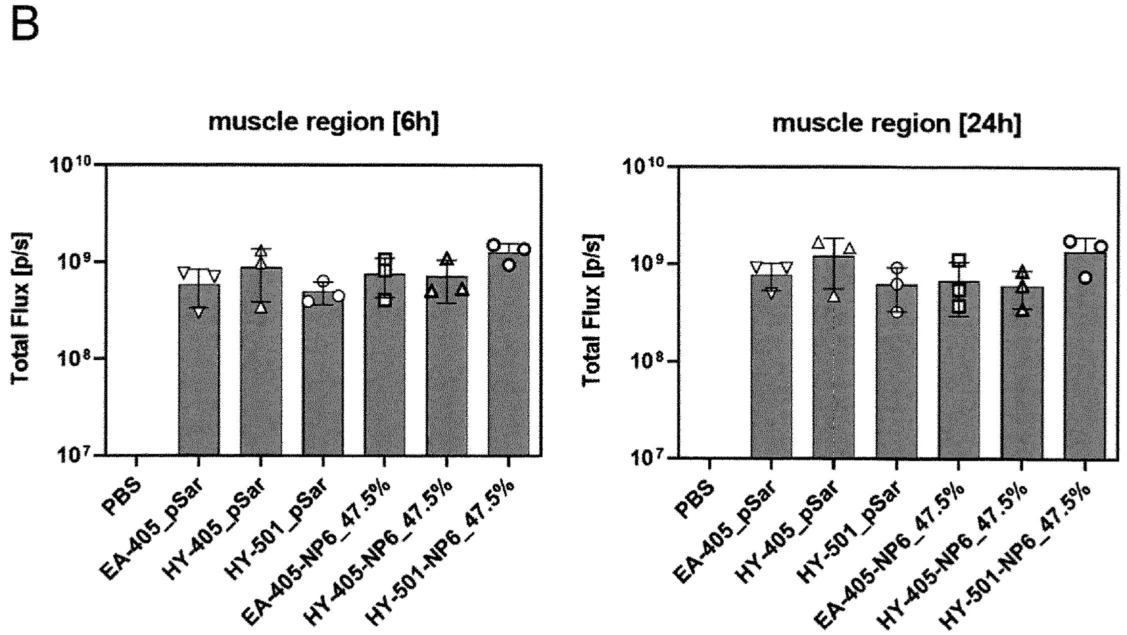


Fig. 4

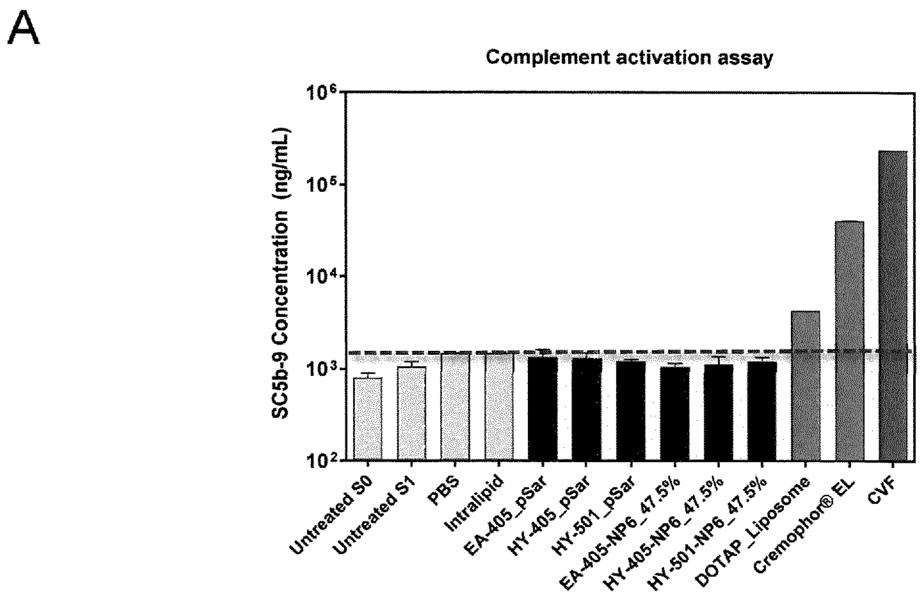

Fig. 4 (contin.)

B

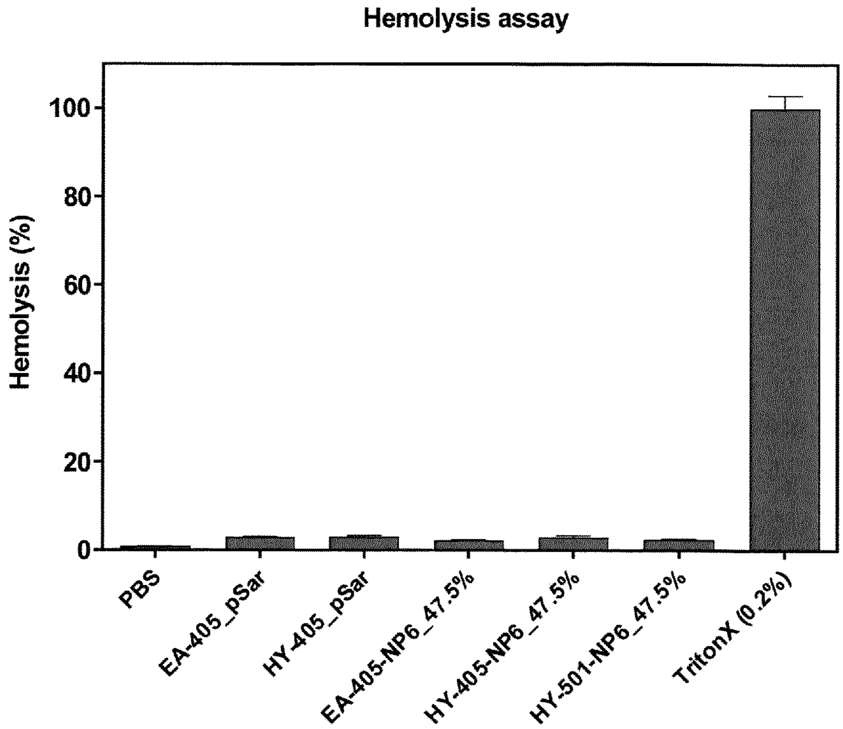


Fig. 5

A

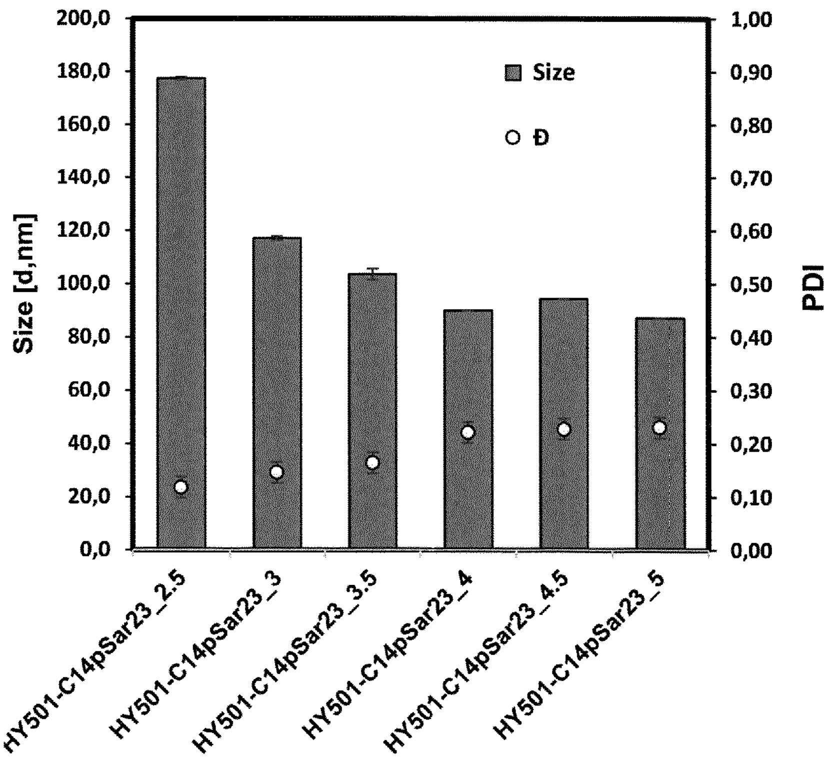

Fig. 5 (contin.)

B

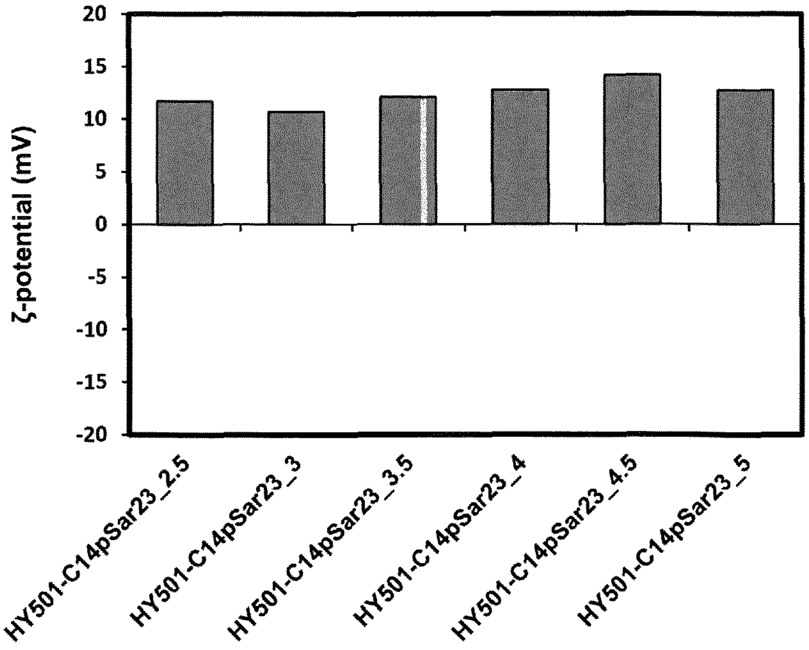


C

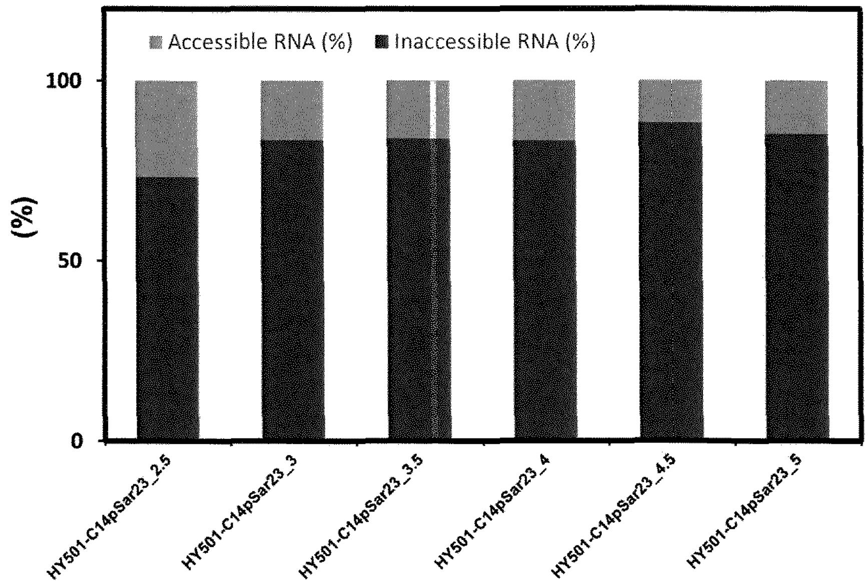

Fig. 5 (contin.)

D

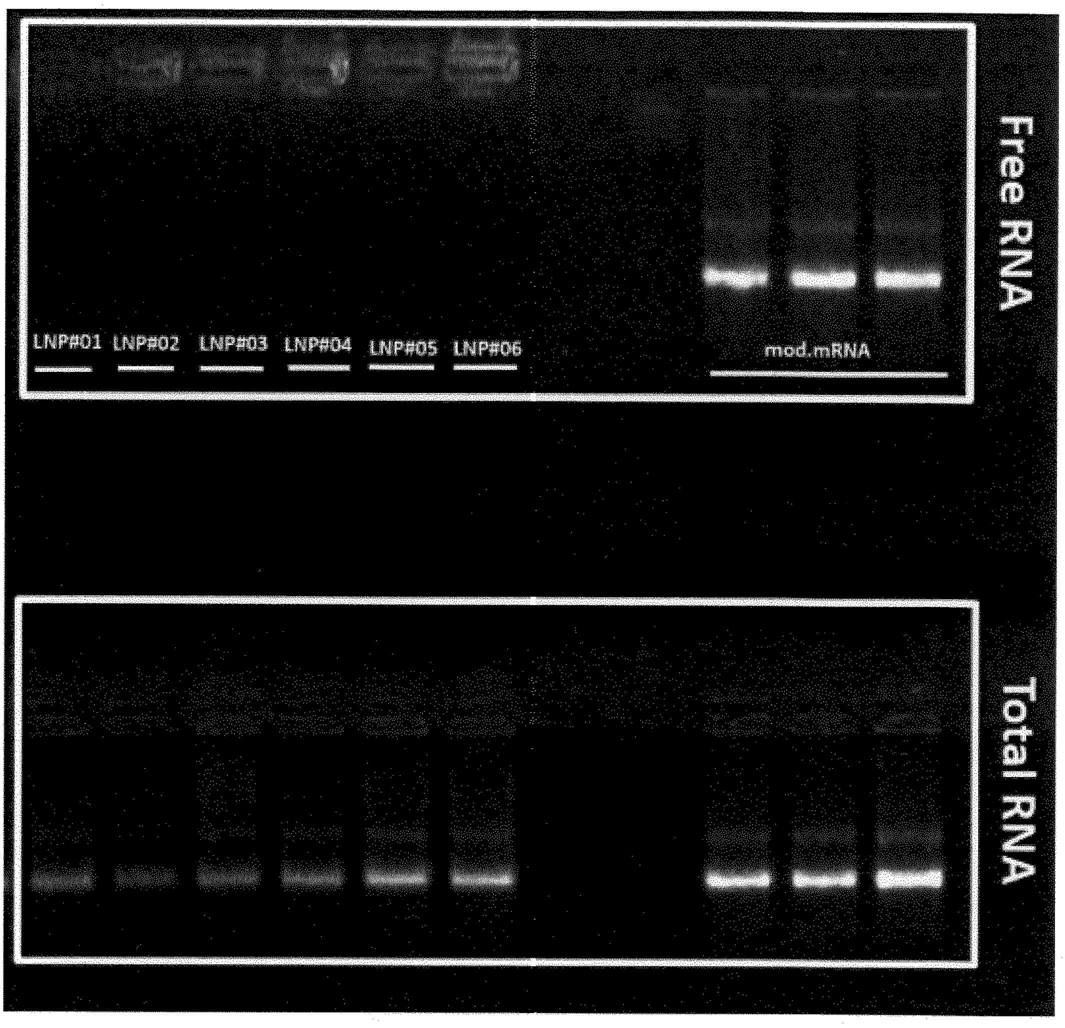

Fig. 6

A

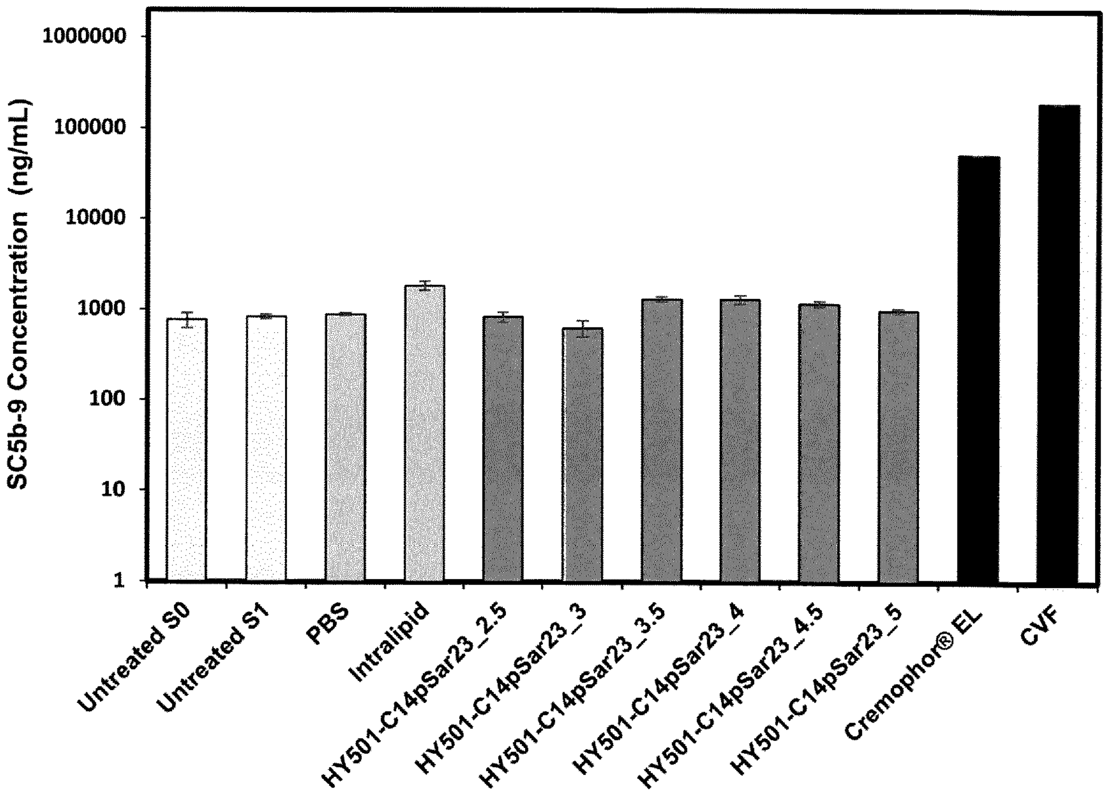
SC5b-9 Concentration (ng/mL)
1000000
100000
10000
1000
100
10
1
Untreated S0
Untreated S1
PBS
Intralipid
HY501-C14pSar23_2.5
HY501-C14pSar23_3
HY501-C14pSar23_3.5
HY501-C14pSar23_4
HY501-C14pSar23_4.5
HY501-C14pSar23_5
Cremophor® EL
CVF

B

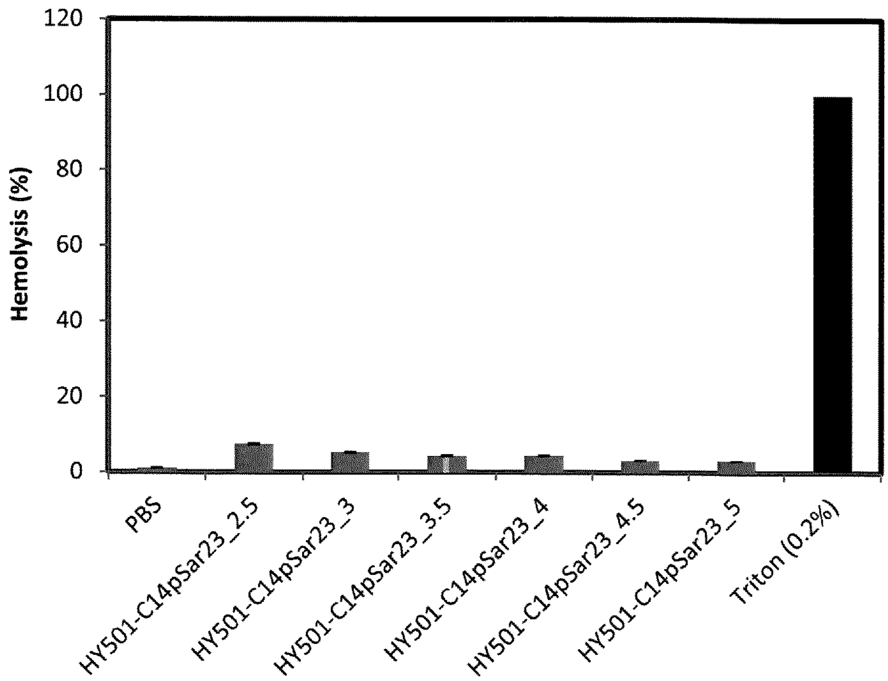
Hemolysis (%)
120
100
80
60
40
20
0
PBS
HY501-C14pSar23_2.5
HY501-C14pSar23_3
HY501-C14pSar23_3.5
HY501-C14pSar23_4
HY501-C14pSar23_4.5
HY501-C14pSar23_5
Triton (0.2%)

Fig. 7

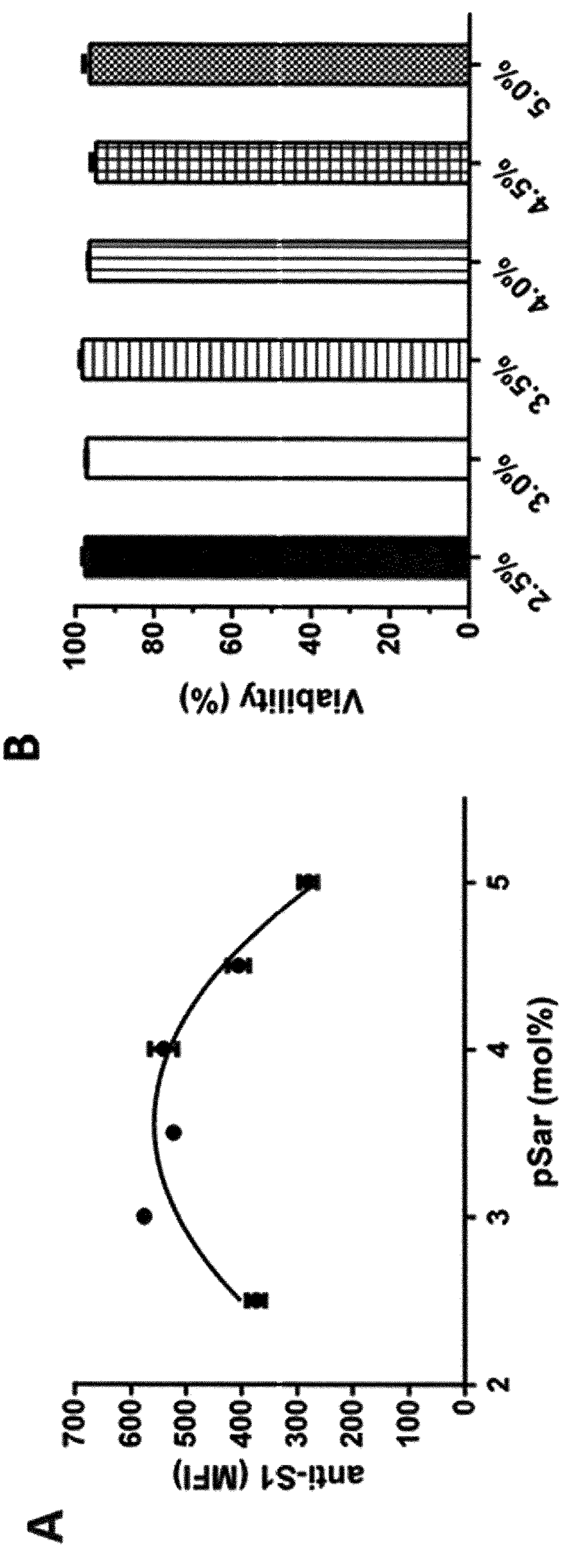
A
anti-S1 (MFI)
700
600
500
400
300
200
100
0
2
3
4
5
pSar (mol%)
B
Viability (%)
100
80
60
40
20
0
2.5%
3.0%
3.5%
4.0%
4.5%
5.0%

Fig. 8

A

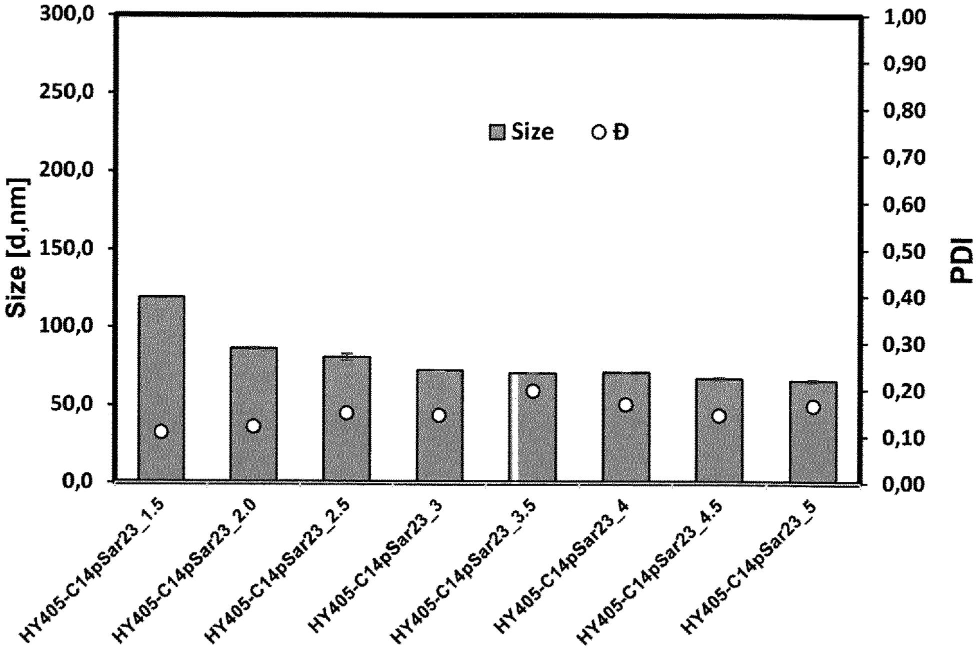
Size
Ð
Size [d,nm]
PDI
300,0
250,0
200,0
150,0
100,0
50,0
0,0
1,00
0,90
0,80
0,70
0,60
0,50
0,40
0,30
0,20
0,10
0,00
HY405-C14pSar23_1.5
HY405-C14pSar23_2.0
HY405-C14pSar23_2.5
HY405-C14pSar23_3
HY405-C14pSar23_3.5
HY405-C14pSar23_4
HY405-C14pSar23_4.5
HY405-C14pSar23_5

B

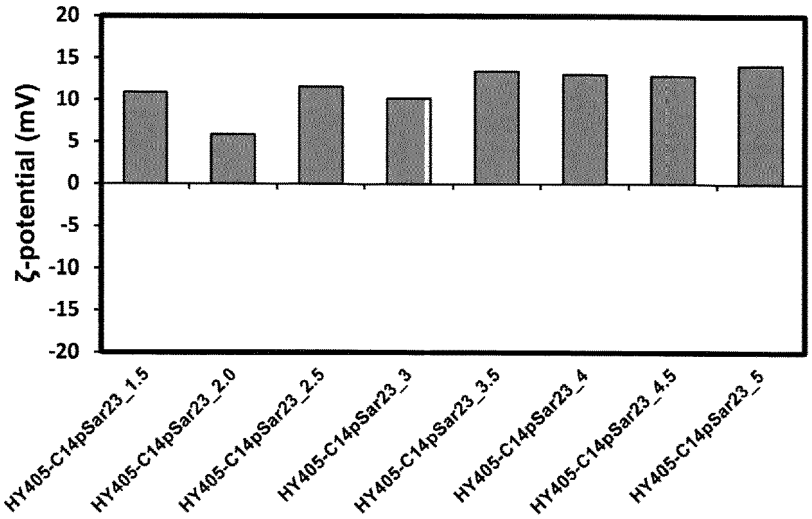
ζ-potential (mV)
20
15
10
5
0
-5
-10
-15
-20
HY405-C14pSar23_1.5
HY405-C14pSar23_2.0
HY405-C14pSar23_2.5
HY405-C14pSar23_3
HY405-C14pSar23_3.5
HY405-C14pSar23_4
HY405-C14pSar23_4.5
HY405-C14pSar23_5

Fig. 8 (continued)

C

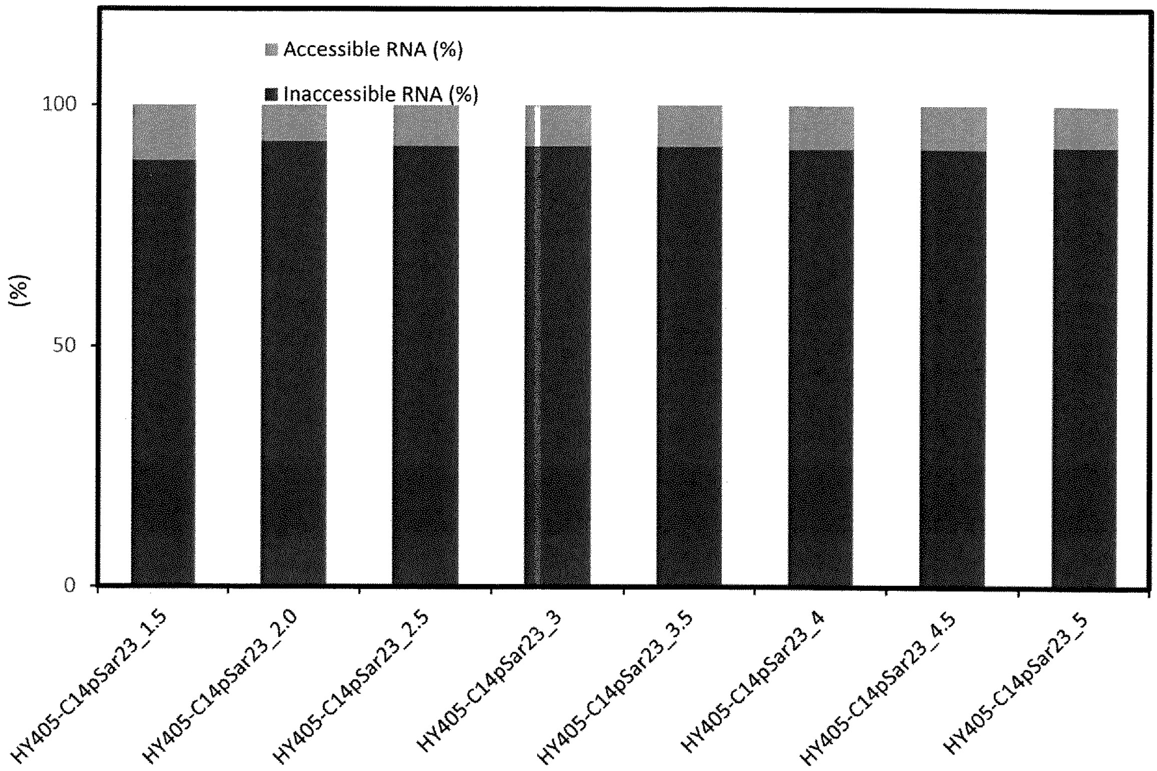


D

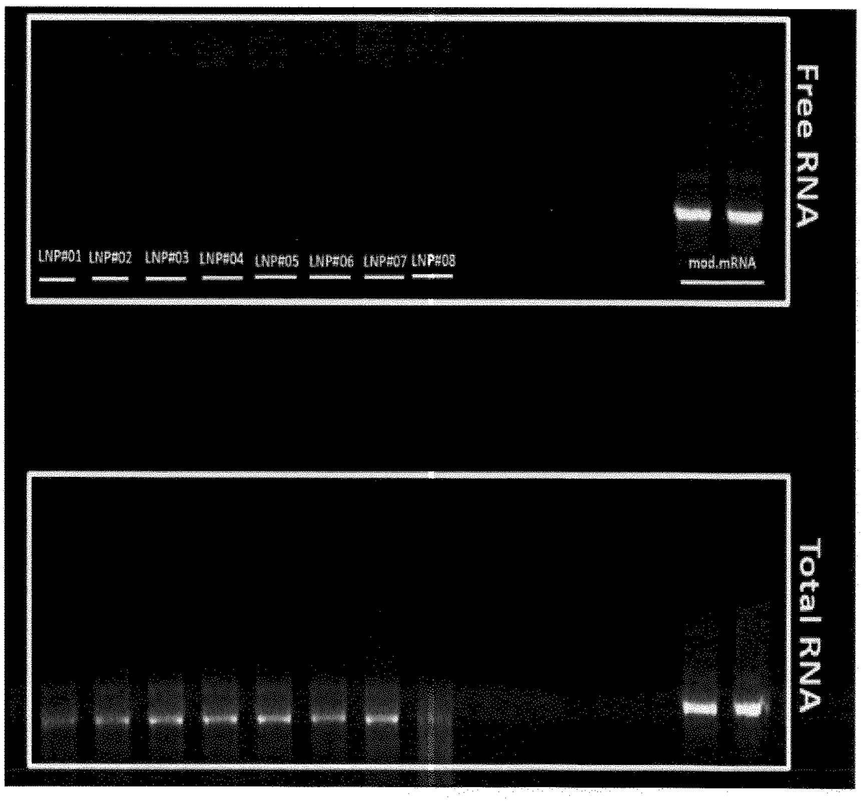

Fig. 9

A

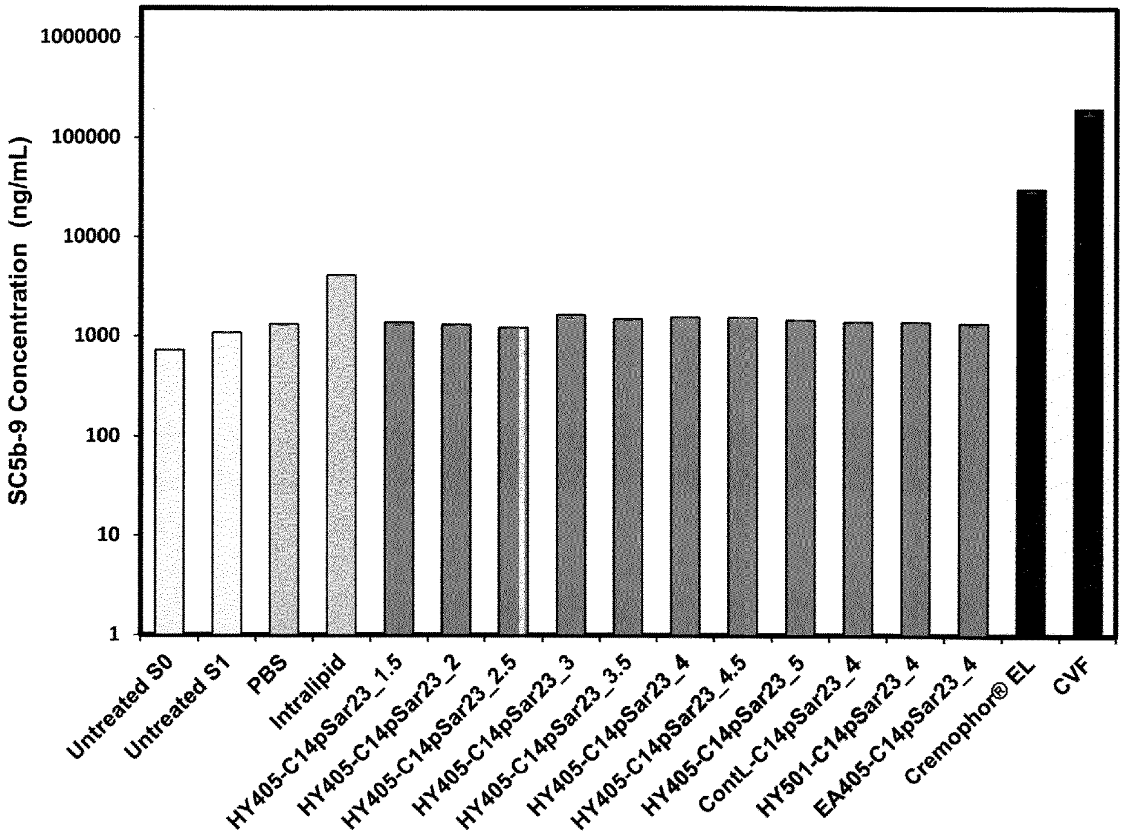
SC5b-9 Concentration (ng/mL)
1000000
100000
10000
1000
100
10
1
Untreated S0
Untreated S1
PBS
Intralipid
HY405-C14pSar23_1.5
HY405-C14pSar23_2
HY405-C14pSar23_2.5
HY405-C14pSar23_3
HY405-C14pSar23_3.5
HY405-C14pSar23_4
HY405-C14pSar23_4.5
HY405-C14pSar23_5
ContL-C14pSar23_4
HY501-C14pSar23_4
EA405-C14pSar23_4
Cremophor® EL
CVF

B

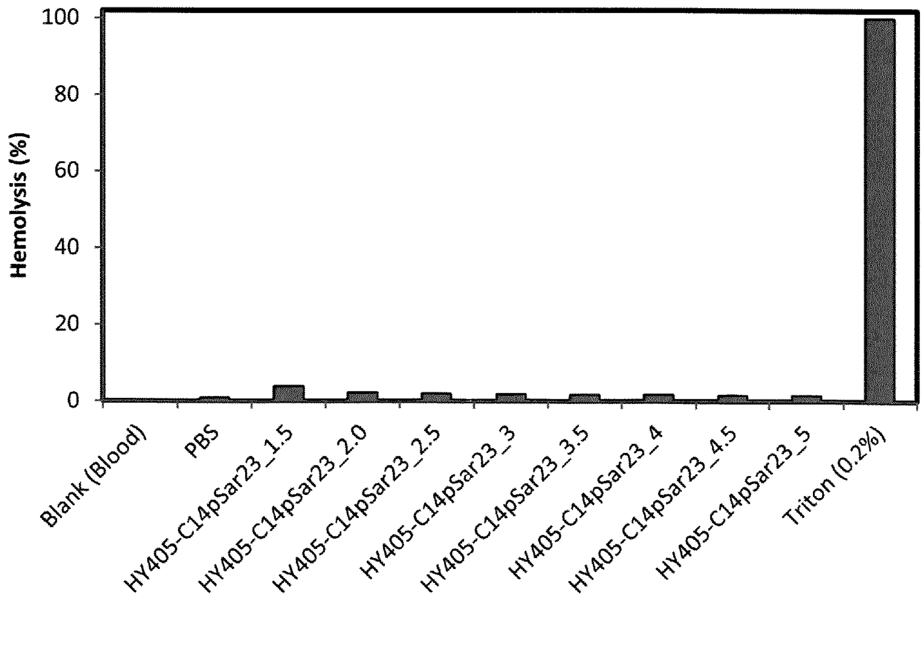
Hemolysis (%)
100
80
60
40
20
0
Blank (Blood)
PBS
HY405-C14pSar23_1.5
HY405-C14pSar23_2.0
HY405-C14pSar23_2.5
HY405-C14pSar23_3
HY405-C14pSar23_3.5
HY405-C14pSar23_4
HY405-C14pSar23_4.5
HY405-C14pSar23_5
Triton (0.2%)

Fig. 10

A

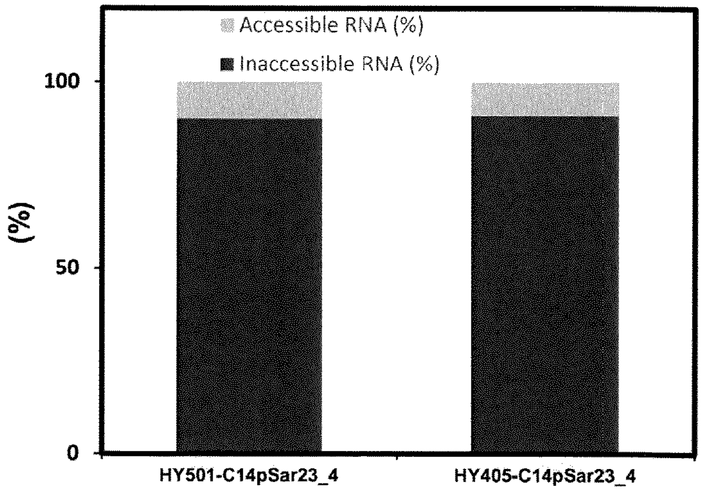
Accessible RNA (%)
Inaccessible RNA (%)
100
50
0
(%)
HY501-C14pSar23_4
HY405-C14pSar23_4

B

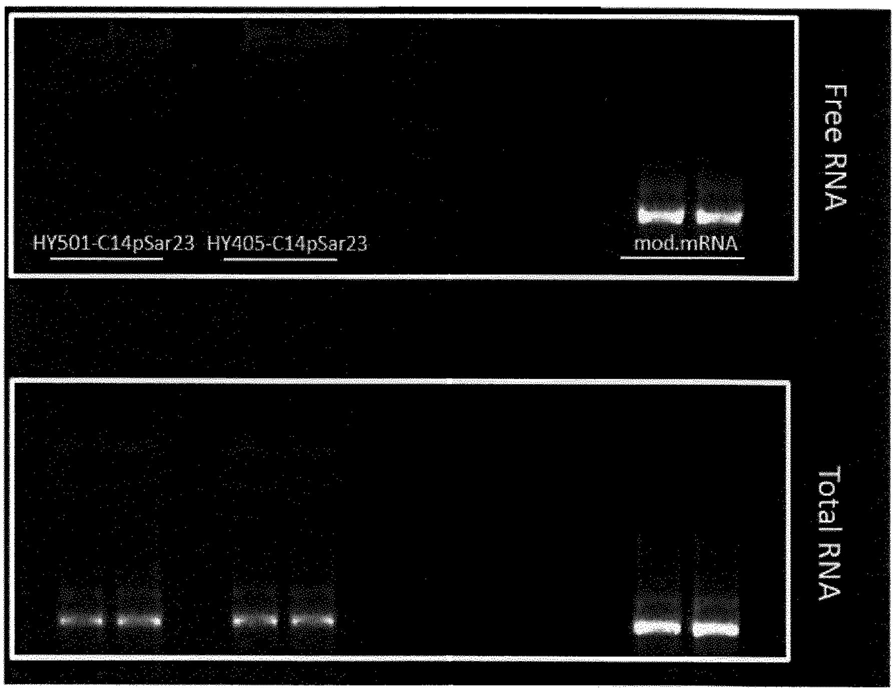
HY501-C14pSar23
HY405-C14pSar23
mod.mRNA
Free RNA
Total RNA

Fig. 11

A

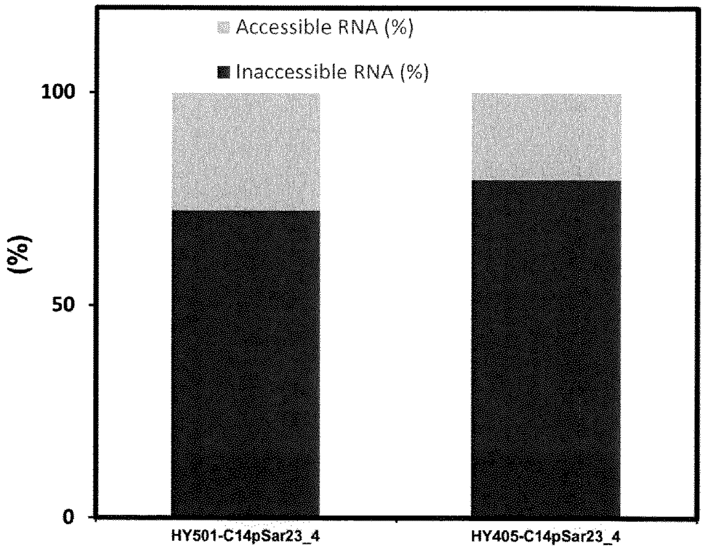
Accessible RNA (%)
Inaccessible RNA (%)
100
50
0
(%)
HY501-C14pSar23_4
HY405-C14pSar23_4

B

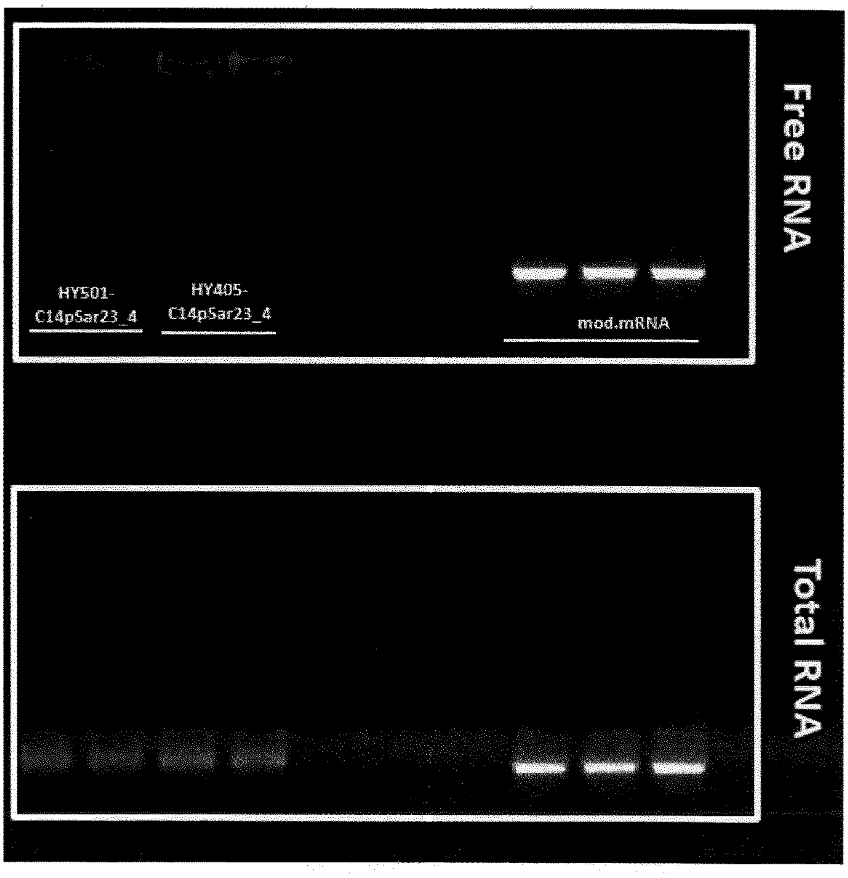
Free RNA
HY501-
C14pSar23_4
HY405-
C14pSar23_4
mod.mRNA
Total RNA

Fig. 12

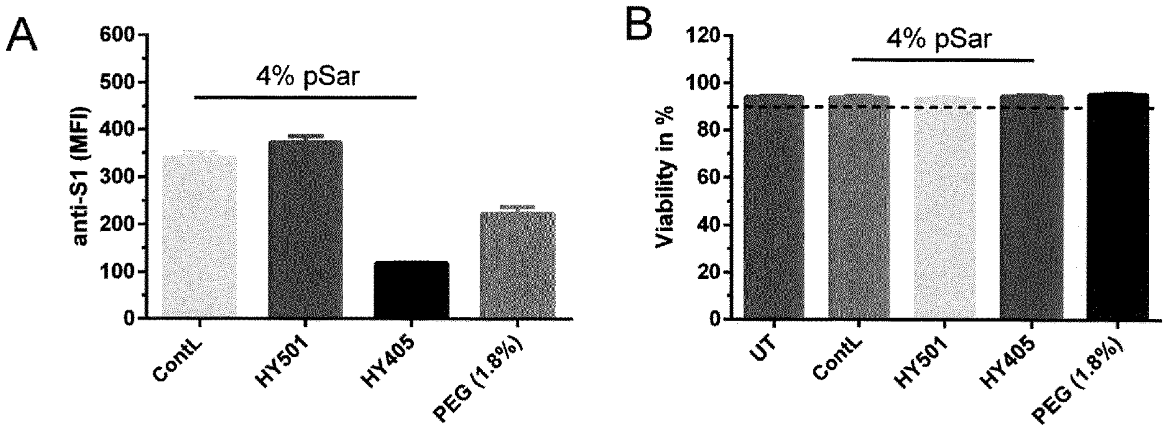


Fig. 13

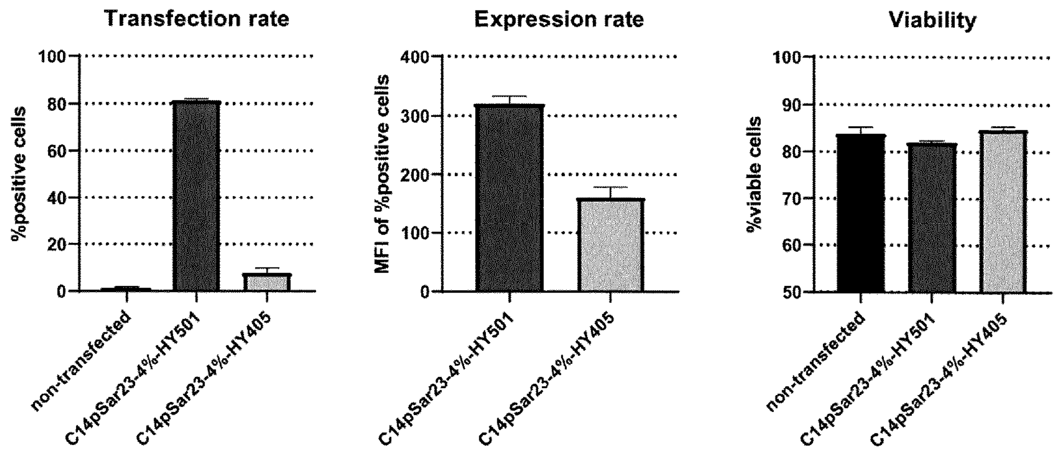


Fig. 14

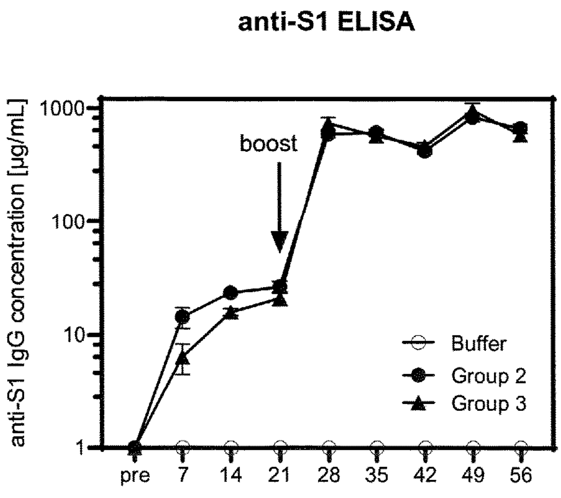

**Fig. 15**

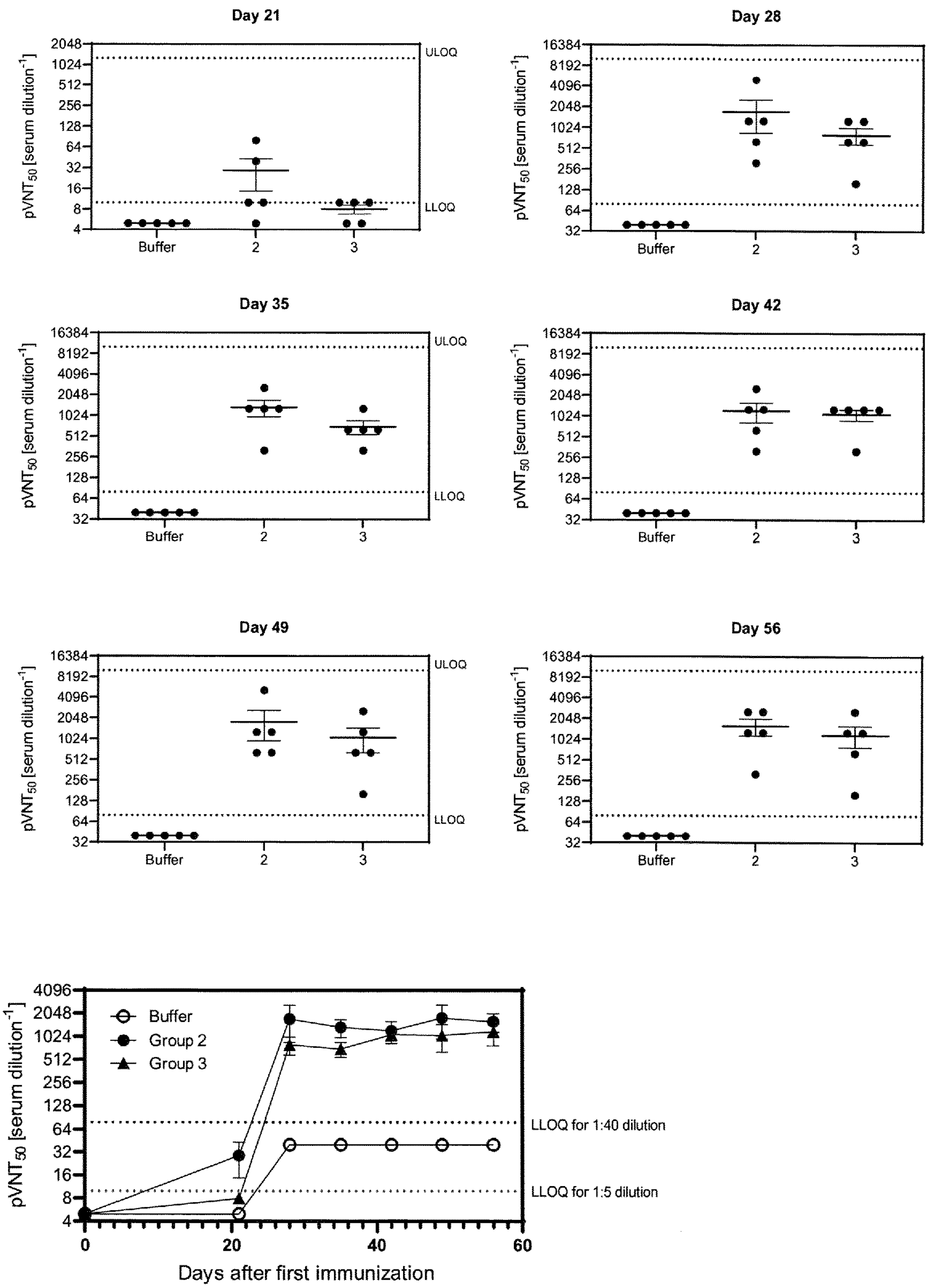
Day 21
Day 28
Day 35
Day 42
Day 49
Day 56
pVNT50 [serum dilution-1]
ULOQ
LLOQ
Buffer
2
3
16384
8192
4096
2048
1024
512
256
128
64
32
16
8
4
Buffer
Group 2
Group 3
LLOQ for 1:40 dilution
LLOQ for 1:5 dilution
0
20
40
60
Days after first immunization

Fig. 16

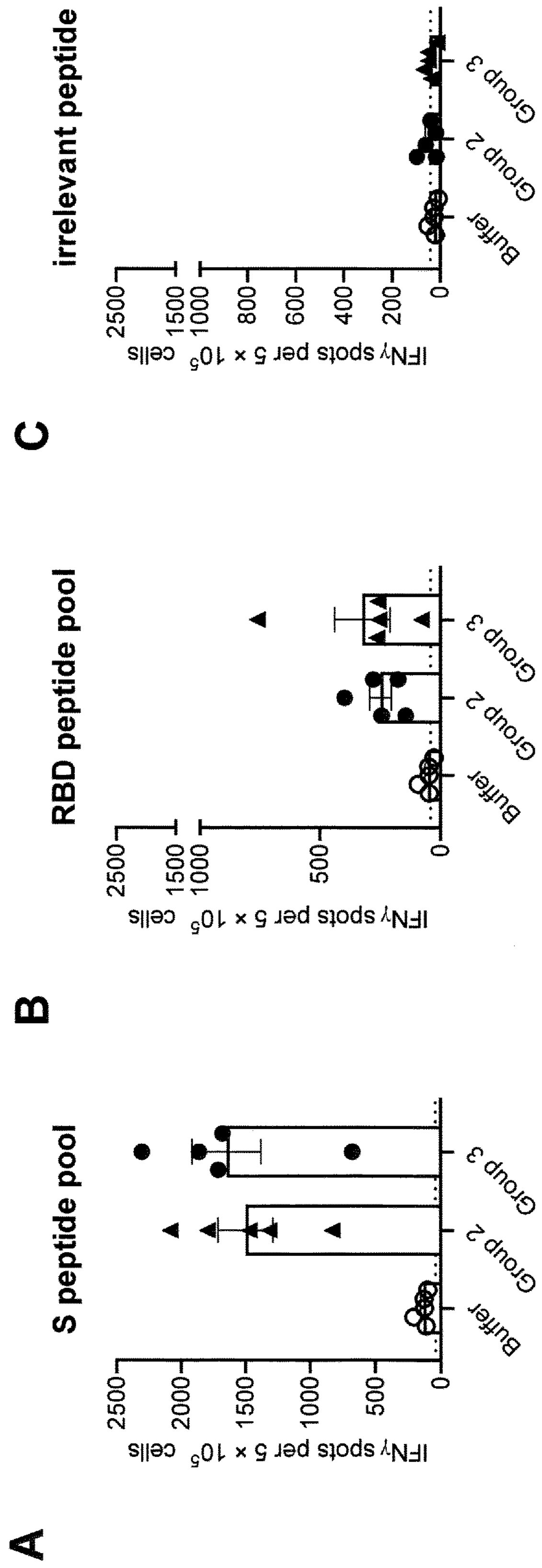
A
S peptide pool
IFNγ spots per 5 x 10^5 cells
2500
2000
1500
1000
500
0
Buffer
Group 2
Group 3
B
RBD peptide pool
IFNγ spots per 5 x 10^5 cells
2500
1500
1000
500
0
Buffer
Group 2
Group 3
C
irrelevant peptide
IFNγ spots per 5 x 10^5 cells
2500
1500
1000
800
600
400
200
0
Buffer
Group 2
Group 3

Fig. 17

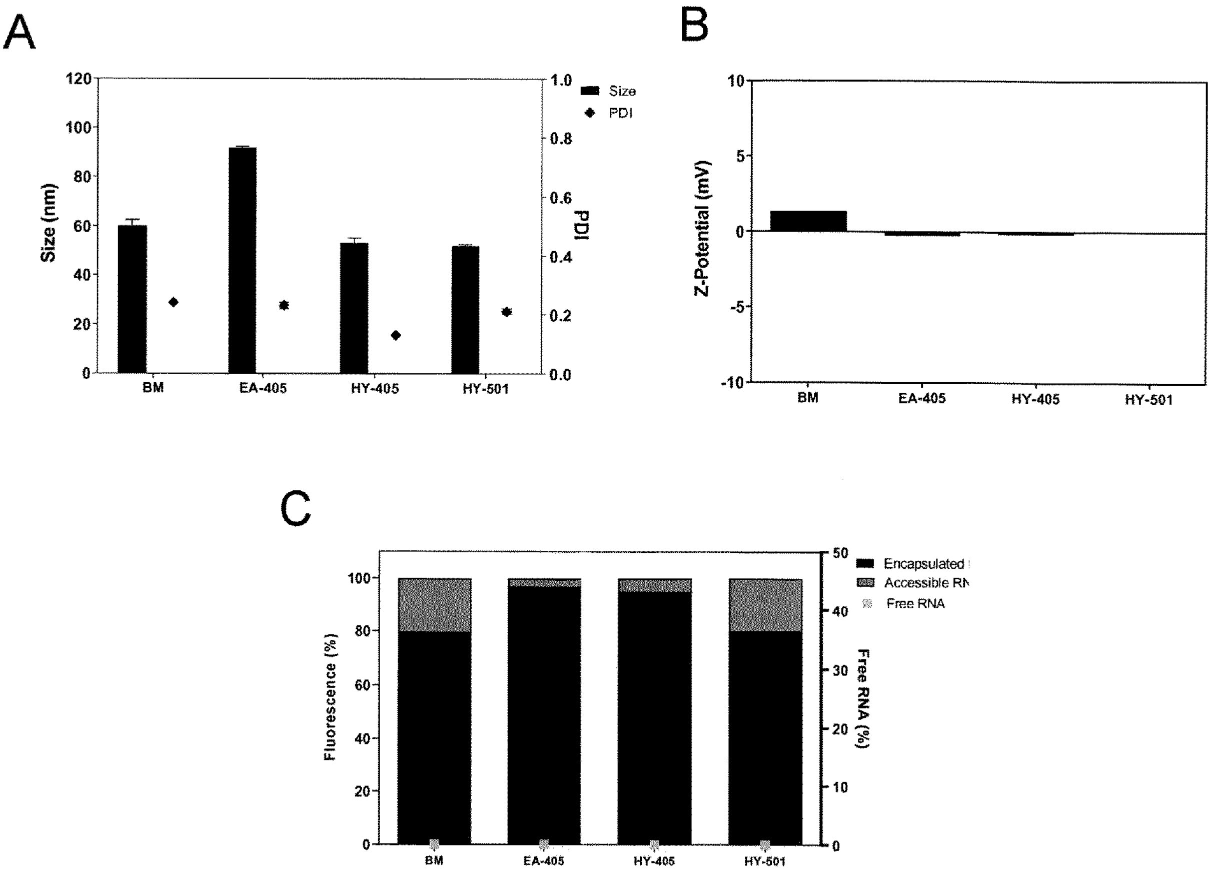
A
Size (nm)
PDI
Size
PDI
BM
EA-405
HY-405
HY-501
B
Z-Potential (mV)
BM
EA-405
HY-405
HY-501
C
Fluorescence (%)
Free RNA (%)
Encapsulated
Accessible RN
Free RNA
BM
EA-405
HY-405
HY-501

Fig. 18

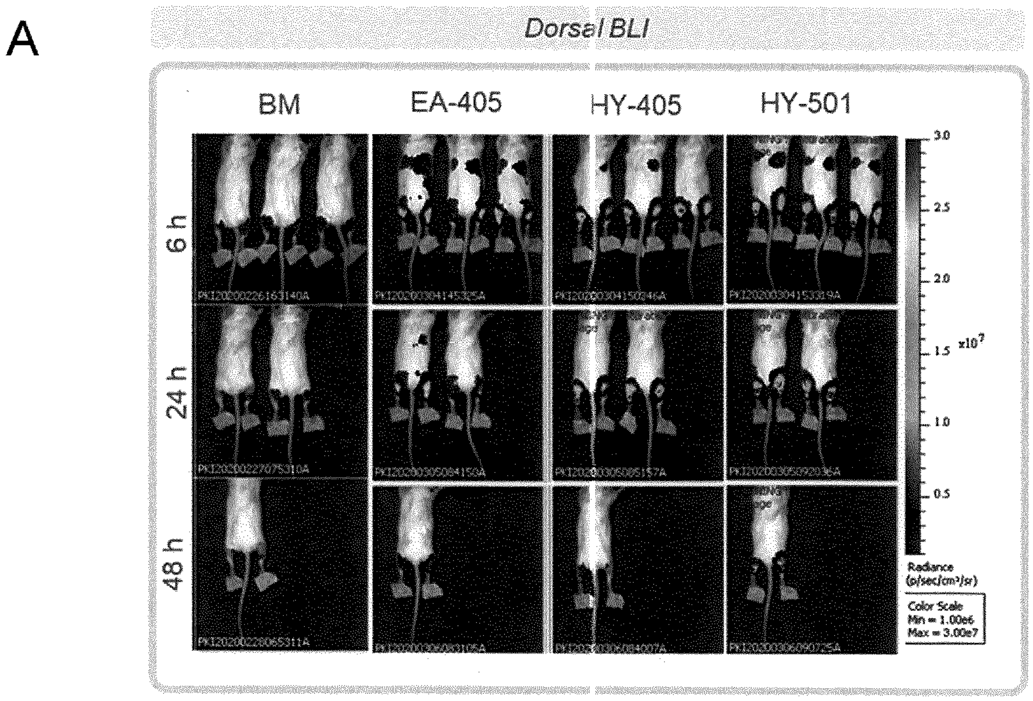
A
Dorsal BLI
BM
EA-405
HY-405
HY-501
6 h
24 h
48 h
x10^7
Radiance (p/sec/cm²/sr)
Color Scale
Min = 1.00e6
Max = 3.00e7

Fig. 18 (contin.)

B

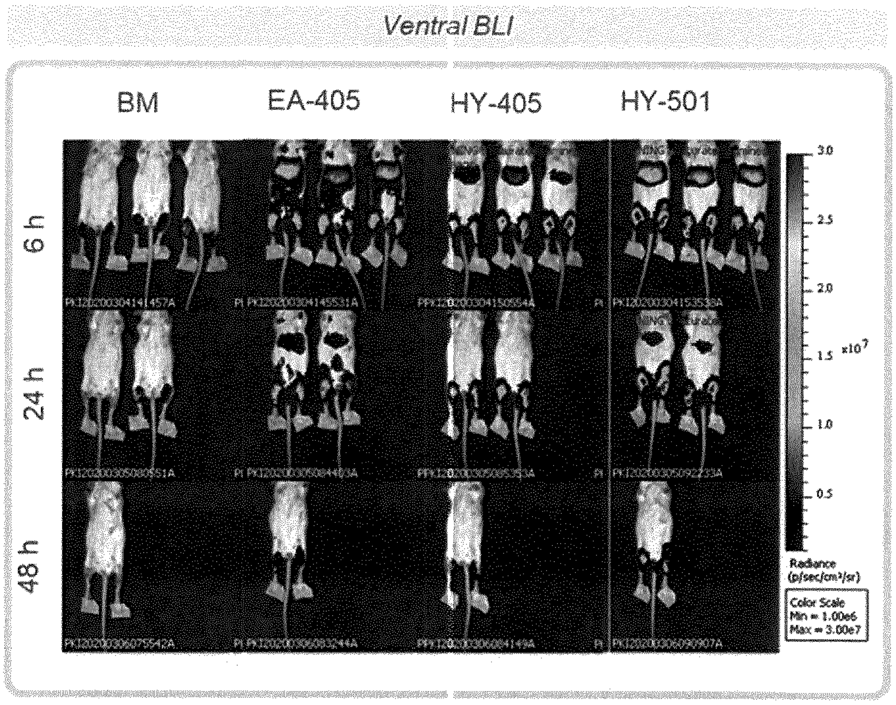


C

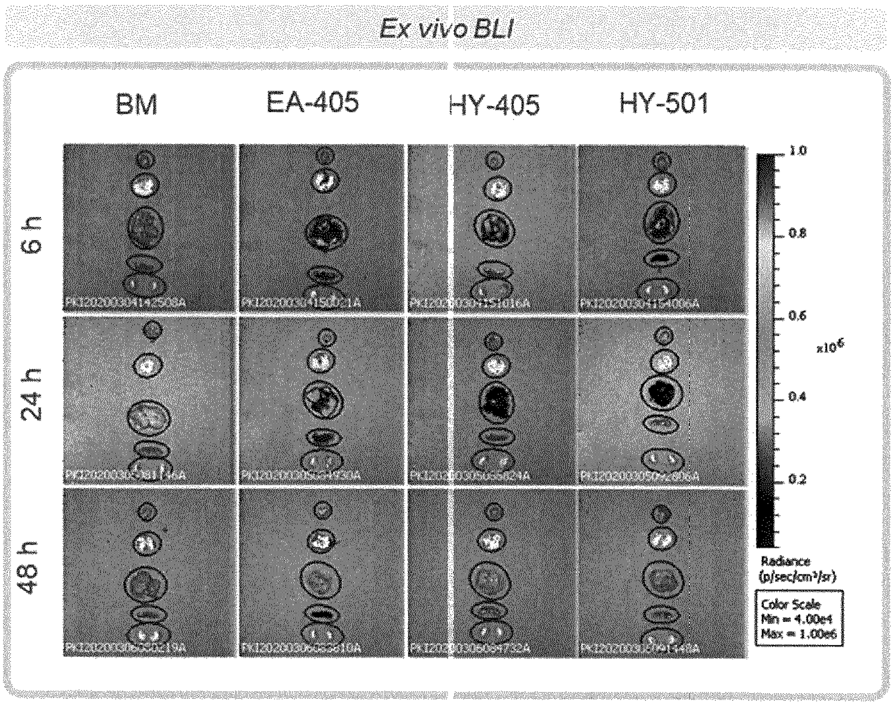

Fig. 19

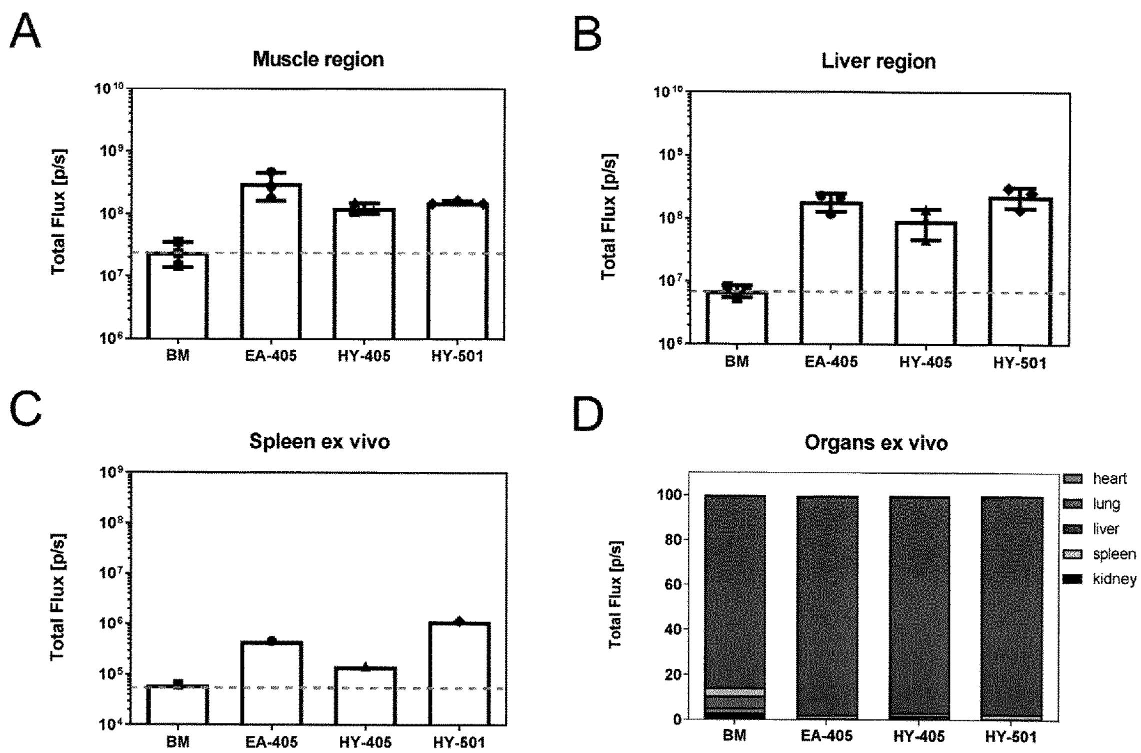
A
Muscle region
Total Flux [p/s]
$10^{10}$
$10^9$
$10^8$
$10^7$
$10^6$
BM
EA-405
HY-405
HY-501
B
Liver region
Total Flux [p/s]
$10^{10}$
$10^9$
$10^8$
$10^7$
$10^6$
BM
EA-405
HY-405
HY-501
C
Spleen ex vivo
Total Flux [p/s]
$10^9$
$10^8$
$10^7$
$10^6$
$10^5$
$10^4$
BM
EA-405
HY-405
HY-501
D
Organs ex vivo
Total Flux [p/s]
100
80
60
40
20
0
BM
EA-405
HY-405
HY-501
heart
lung
liver
spleen
kidney

Fig. 20

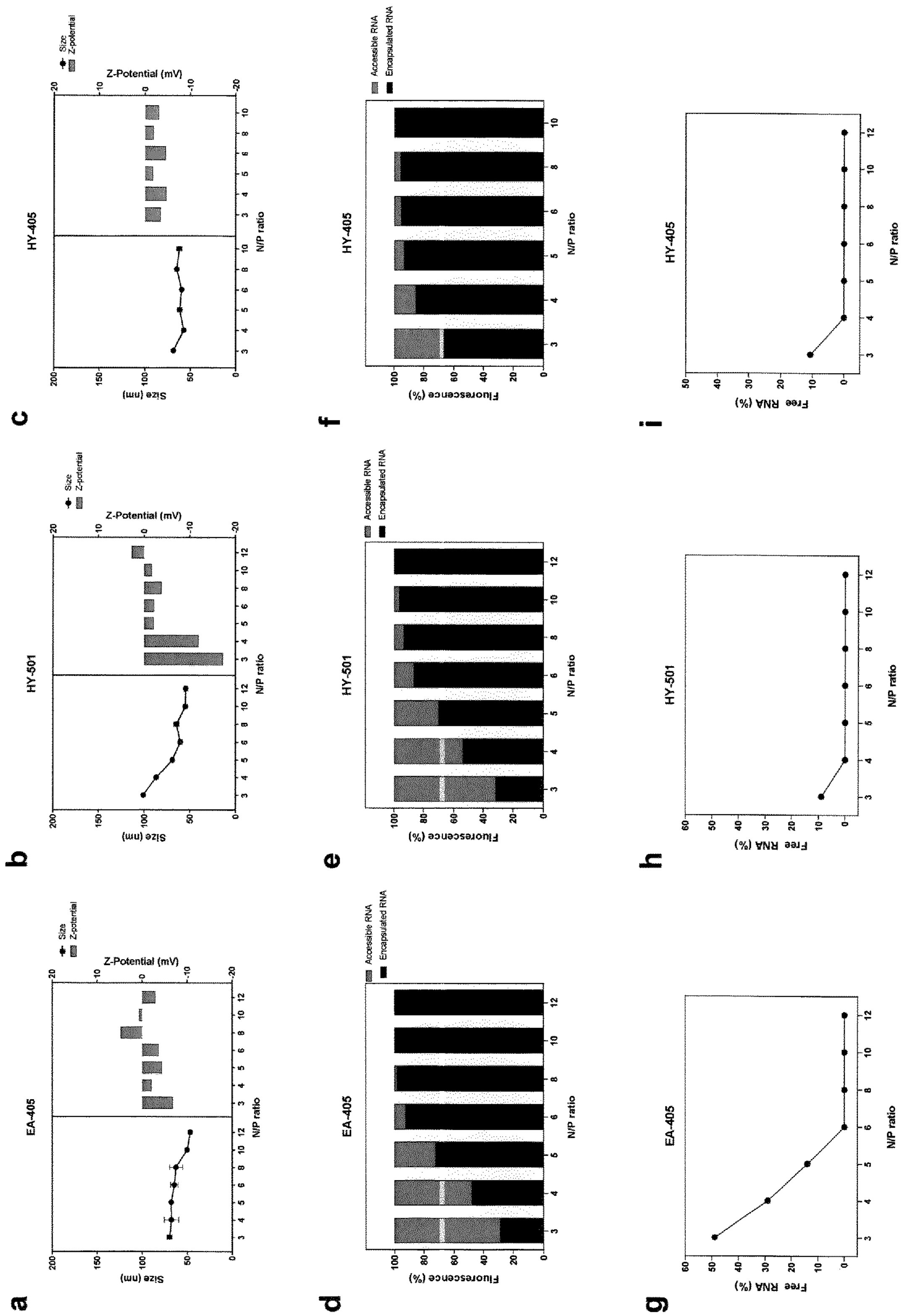
a
b
c
d
e
f
g
h
i
EA-405
HY-501
HY-405
Size (nm)
Z-Potential (mV)
Size
Z-potential
N/P ratio
Fluorescence (%)
Accessible RNA
Encapsulated RNA
Free RNA (%)

Fig. 21

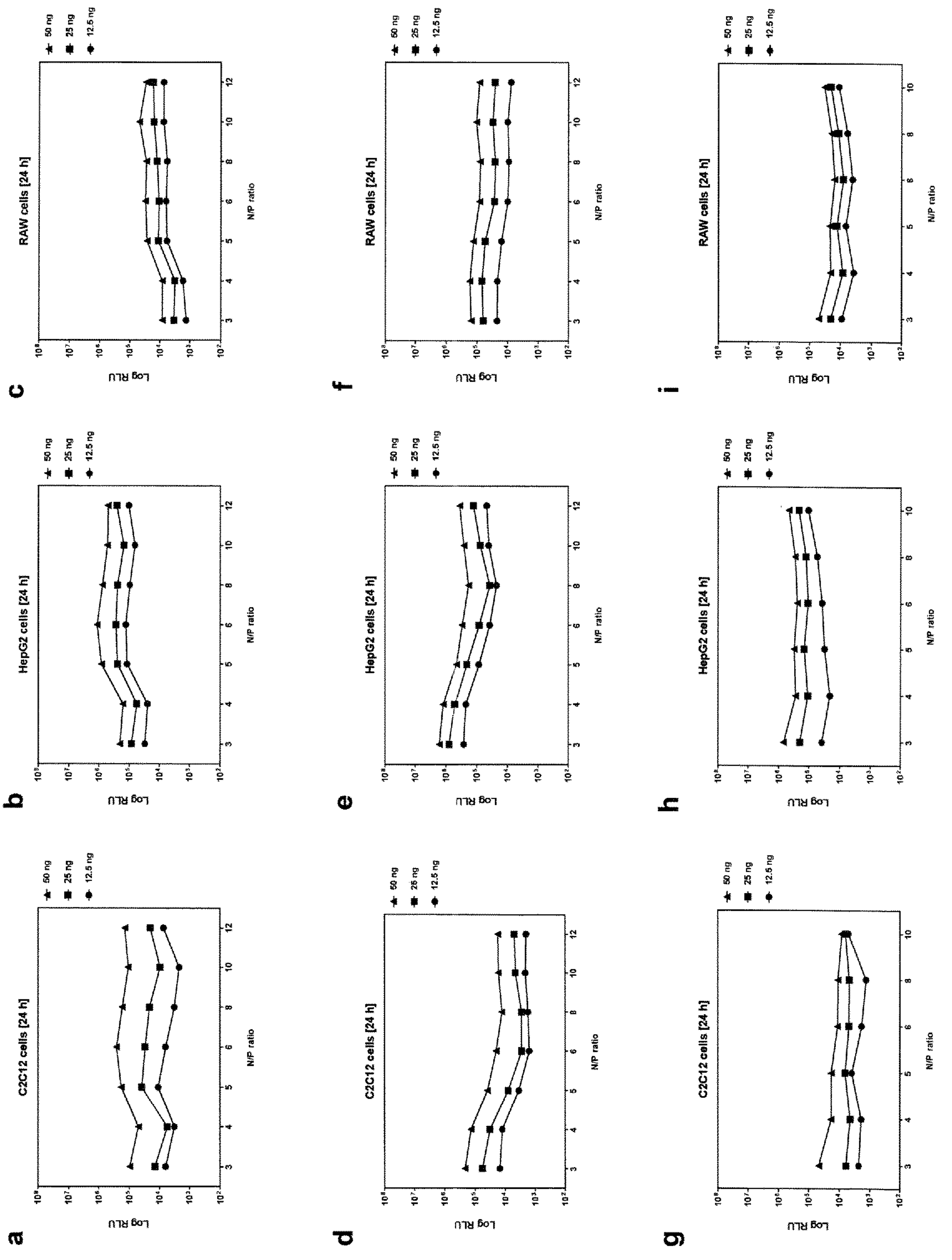
a
C2C12 cells [24 h]
b
HepG2 cells [24 h]
c
RAW cells [24 h]
d
C2C12 cells [24 h]
e
HepG2 cells [24 h]
f
RAW cells [24 h]
g
C2C12 cells [24 h]
h
HepG2 cells [24 h]
i
RAW cells [24 h]
Log RLU
N/P ratio
50 ng
25 ng
12.5 ng

Fig. 22

A

B

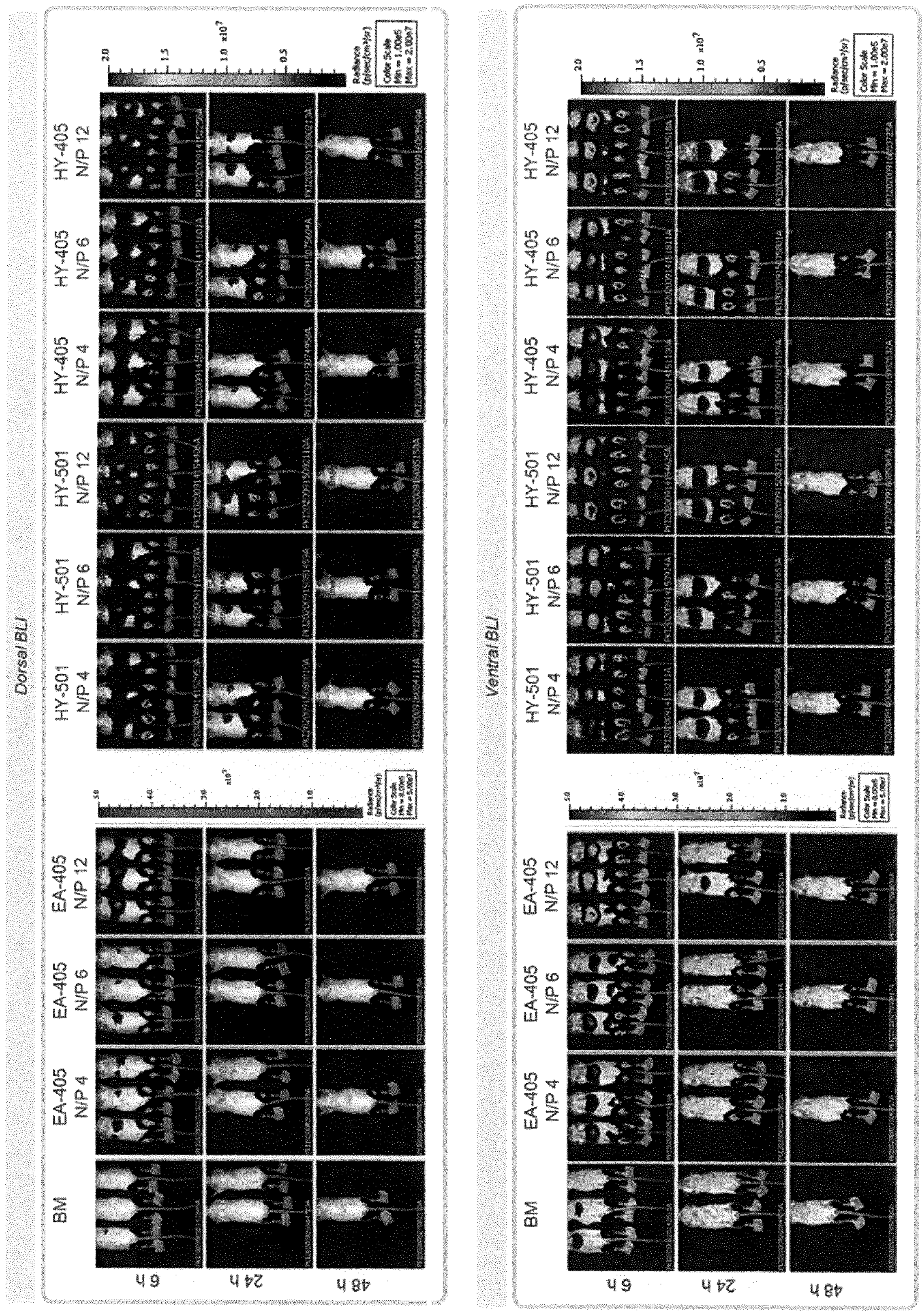
Dorsal BLI
Ventral BLI
BM
EA-405 N/P 4
EA-405 N/P 6
EA-405 N/P 12
HY-501 N/P 4
HY-501 N/P 6
HY-501 N/P 12
HY-405 N/P 4
HY-405 N/P 6
HY-405 N/P 12
6 h
24 h
48 h

Fig. 22 (contin.)

C

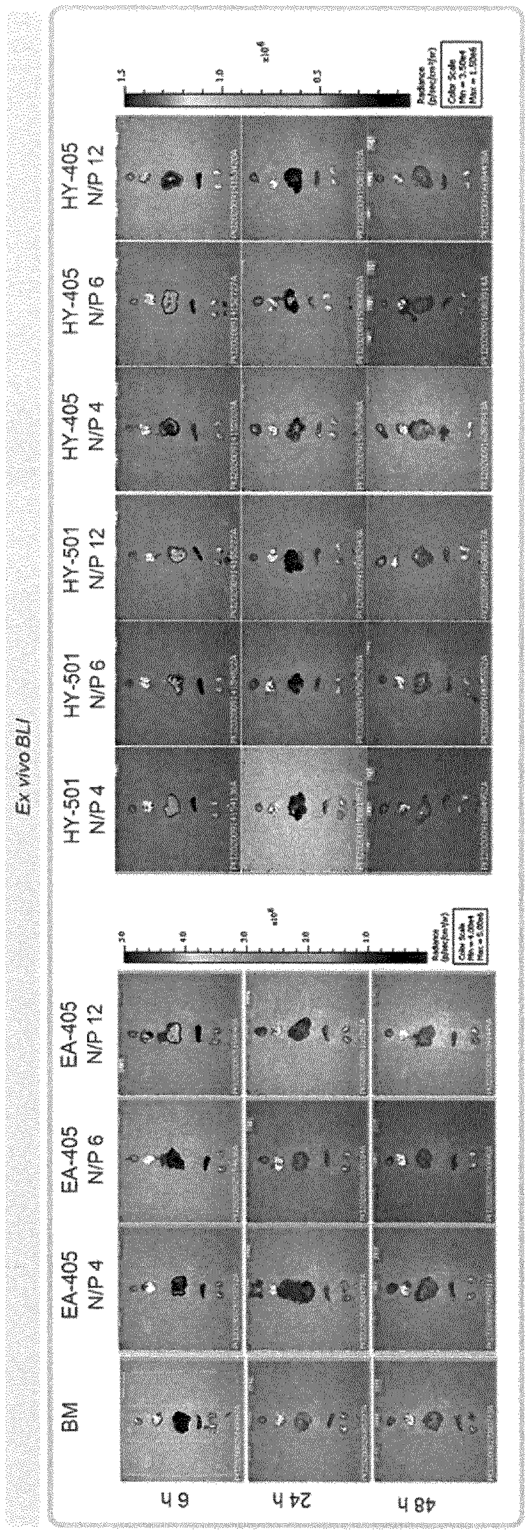

Fig. 23

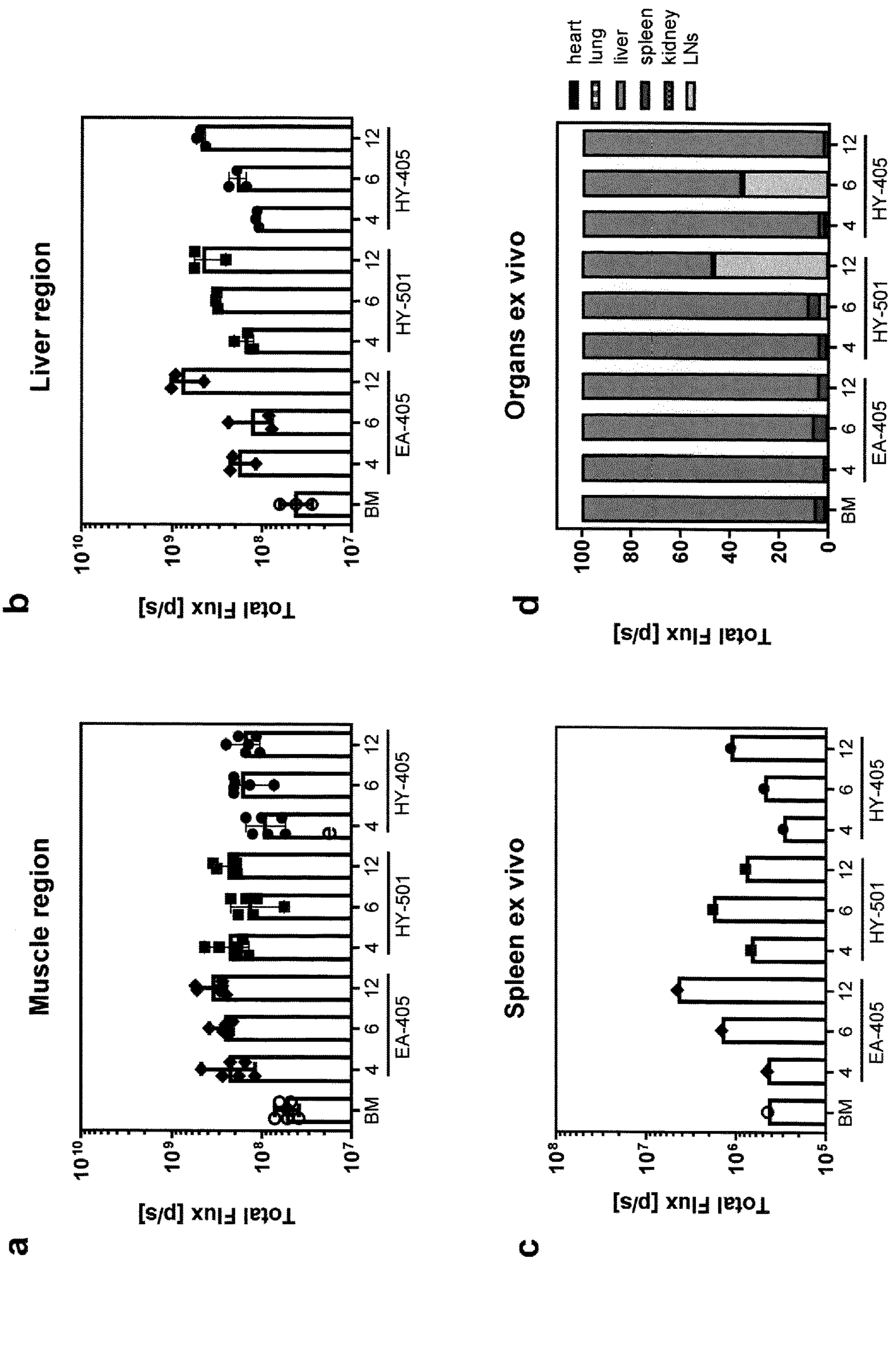
a
Muscle region
Total Flux [p/s]
$10^{10}$
$10^{9}$
$10^{8}$
$10^{7}$
BM
4
6
12
EA-405
HY-501
HY-405
b
Liver region
Total Flux [p/s]
c
Spleen ex vivo
Total Flux [p/s]
$10^{8}$
$10^{7}$
$10^{6}$
$10^{5}$
d
Organs ex vivo
Total Flux [p/s]
100
80
60
40
20
0
heart
lung
liver
spleen
kidney
LNs

Fig. 24

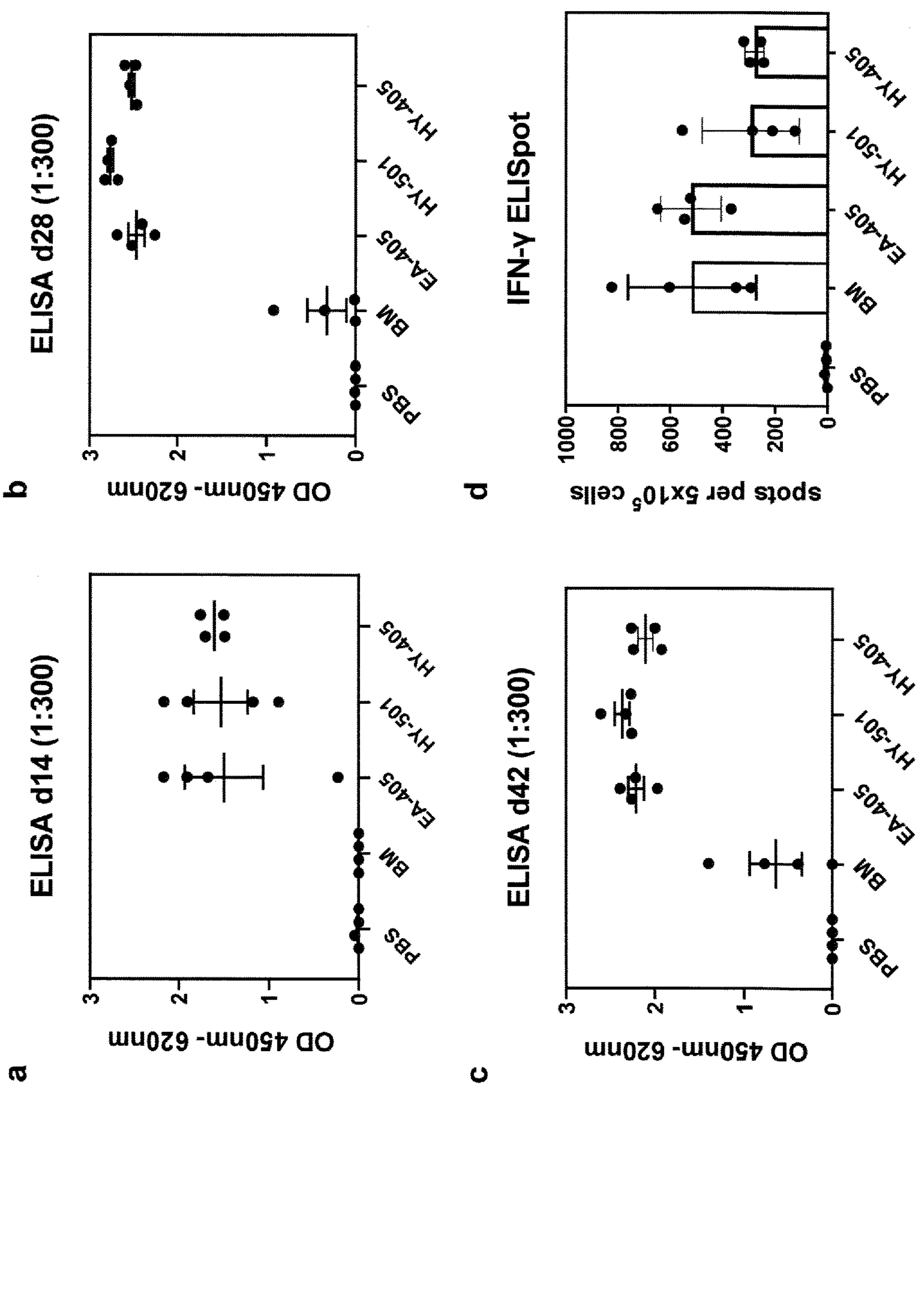
a
ELISA d14 (1:300)
OD 450nm- 620nm
PBS
BM
EA-405
HY-501
HY-405
b
ELISA d28 (1:300)
OD 450nm- 620nm
c
ELISA d42 (1:300)
OD 450nm- 620nm
d
IFN-γ ELISpot
spots per 5x10^5 cells

Fig. 25

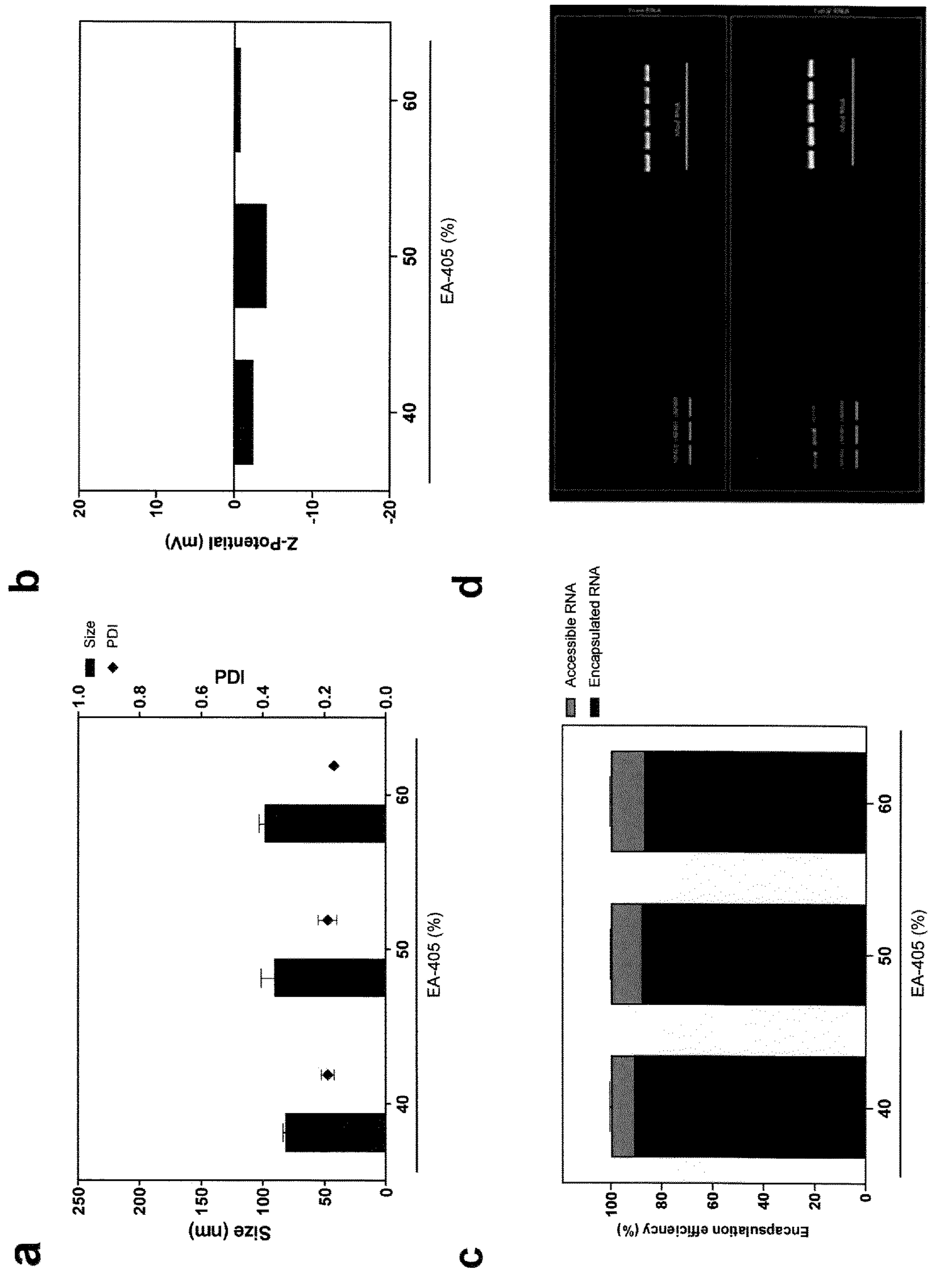
a
Size (nm)
250
200
150
100
50
0
PDI
1.0
0.8
0.6
0.4
0.2
0.0
Size
PDI
40
50
60
EA-405 (%)
b
Z-Potential (mV)
20
10
0
-10
-20
40
50
60
EA-405 (%)
c
Encapsulation efficiency (%)
100
80
60
40
20
0
Accessible RNA
Encapsulated RNA
40
50
60
EA-405 (%)
d

Fig. 26

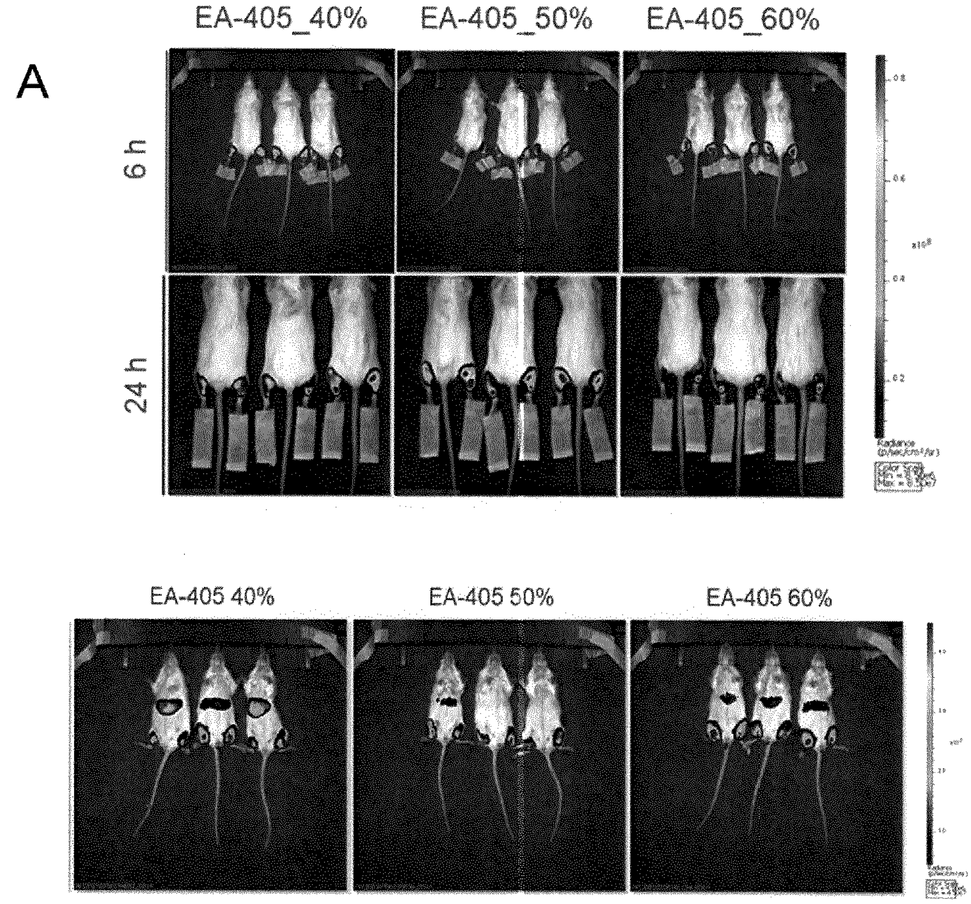

Fig. 27

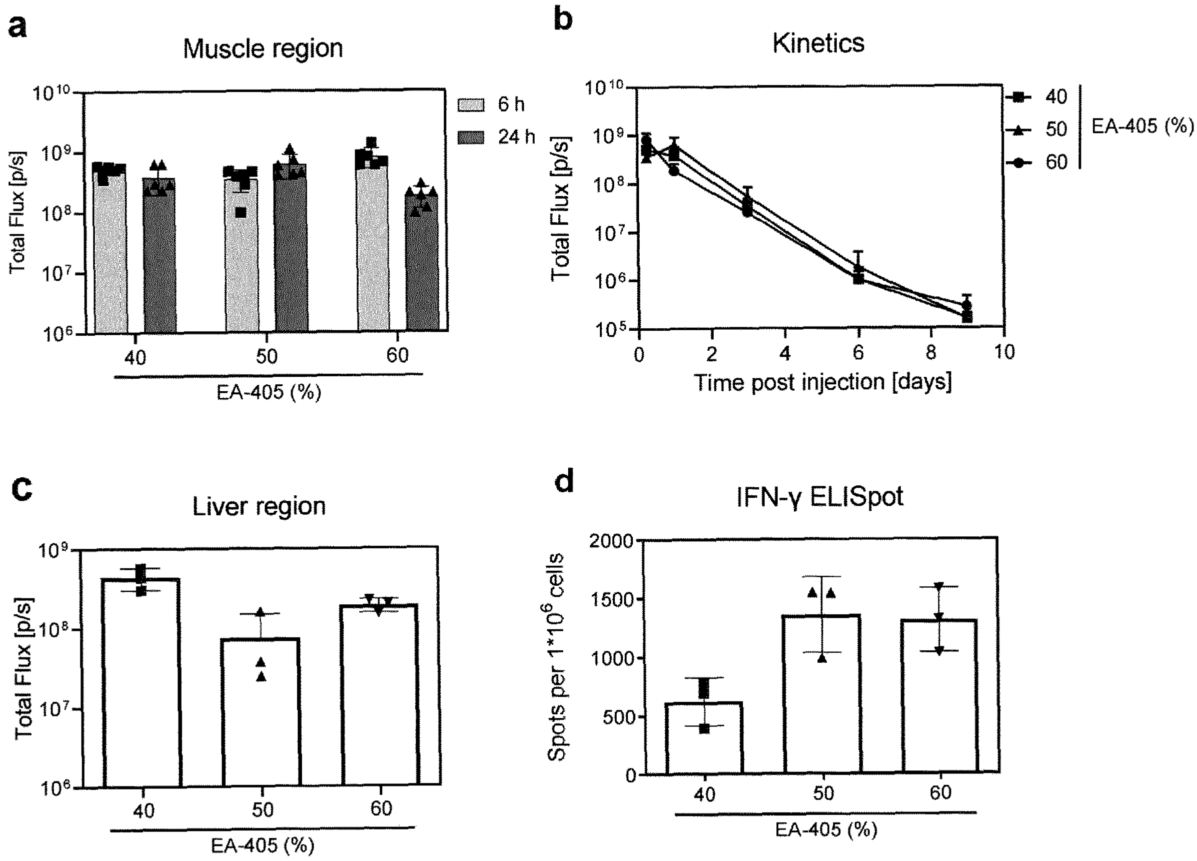
a
Muscle region
Total Flux [p/s]
$10^{10}$
$10^{9}$
$10^{8}$
$10^{7}$
$10^{6}$
6 h
24 h
40
50
60
EA-405 (%)
b
Kinetics
Total Flux [p/s]
$10^{10}$
$10^{9}$
$10^{8}$
$10^{7}$
$10^{6}$
$10^{5}$
0
2
4
6
8
10
Time post injection [days]
40
50
60
EA-405 (%)
c
Liver region
Total Flux [p/s]
$10^{9}$
$10^{8}$
$10^{7}$
$10^{6}$
40
50
60
EA-405 (%)
d
IFN-γ ELISpot
Spots per 1*$10^6$ cells
2000
1500
1000
500
0
40
50
60
EA-405 (%)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017060314 A
- WO 2007036366 A
- EP 2019056502 W
- WO 2016005324 A
- WO 2016005324 A1
- US 20050287153 A
- US 20070003549 A
- US 20070048282 A
- US 5876969 A
- WO 2011124718 A
- WO 2013075066 A
- WO 20110514789 A
- US 20070178082 A
- US 20070269422 A
- US 20100113339 A
- WO 2009083804 A
- WO 2009133208 A
- US 7176278 B
- US 8158579 B
- US 20140220017 A
- US 20170145062 A
- US 20120094909 A
- WO 2017182524 A
- WO 2017075531 A
- WO 2018081480 A
- WO 2013143683 A


### Non-patent literature cited in the description


- **NOGUEIRA et al.** *ACS Appl. Nano Mater.*, 2020, vol. 3, 10634-10645
- **SMITH** ; **WATERMAN**. *Ads App. Math.*, 1981, vol. 2, 482
- **NEDDLEMAN** ; **WUNSCH**. *J. Mol. Biol.*, 1970, vol. 48, 443
- **PEARSON** ; **LIPMAN**. *Proc. Natl Acad. Sci. USA*, 1988, vol. 88, 2444
- Molecular Cloning: A Laboratory Manual. Cold Spring Harbor Laboratory Press, 2012
- **KOPPEL, D.** *J. Chem. Phys.*, 1972, vol. 57, 4814-4820
- **BUCHHOLZ et al.** *Electrophoresis*, 2001, vol. 22, 4118-4128
- **B.H. ZIMM**. *J. Chem. Phys.*, 1945, vol. 13, 141
- **P. DEBYE**. *J. Appl. Phys.*, 1944, vol. 15, 338
- **W. BURCHARD**. *Anal. Chem.*, 2003, vol. 75, 4279-4291
- **JOSÉ et al.** *Future Microbiol.*, 2009, vol. 4, 837-856
- **GOULD et al.** *Antiviral Res.*, 2010, vol. 87, 111-124
- **HOLTKAMP et al.** *Blood*, 2006, vol. 108, 4009-4017
- **KONTERMANN**. *Expert Opin Biol Ther*, July 2016, vol. 16 (7), 903-15
- **KENNETH, A. et al.** Chemical Stability of Pharmaceuticals: A Handbook for Pharmacists
- **PETERS et al.** Pharmacokinetic Analysis: A Practical Approach. 1996
- **GIBALDI, M. et al.** Pharmacokinetics. Marcel Dekker, 1982
- **KACZMAREK, J. C. et al.** *Genome Medicine*, 2017, vol. 9, 60
- Remington's Pharmaceutical Sciences. Mack Publishing Co., 1985